# EUROPEAN PATENT SPECIFICATION

(11) **EP 4 117 655 B1**
(45) Date of publication and mention of the grant of the patent: **09.12.2026**
(21) Application number: 21771600.0
(22) Date of filing: 12.03.2021
(51) Int. Cl.: A61K 31/27, A61K 31/4184, A61K 31/663, A61P 19/02, A61P 35/00, A61P 9/10, A61P 27/02, A61P 11/00, A61P 19/08, C07D 267/20

(54) **DIBENZ(B,F)-1,4-OXAZEPINE COMPOUNDS AND THEIR USE AS MEDICAMENTS**
DIBENZ(B,F)-1,4-OXAZEPIN-VERBINDUNGEN UND IHRE VERWENDUNG ALS ARZNEIMITTEL
COMPOSÉS DE DIBENZ(B,F)-1,4-OXAZÉPINE ET LEUR UTILISATION COMME MÉDICAMENTS

(30) Priority: 14.03.2020 AU 2020900782
(43) Date of publication of application: 18.01.2023
(73) Proprietor: Vascular Therapeutics Pty Ltd, Rhodes, NSW 2138 (AU)
(72) Inventor: MARCUCCIO, Sebastian, M., Scoresby VIC 3179 (AU); KHACHIGIAN, Levon, Michael, Randwick NSW 2052 (AU)
(74) Representative: Sagittarius IP
(86) International application number: PCT/AU2021/050219
(87) International publication number: WO 2021/184059

(56) References cited:
- WO-A1-2006/024092
- GB-A- 1 348 460
- US-A1- 2010 222 401
- LI, Y ET AL.: "The anthelmintic flubendazole blocks human melanoma growth and metastasis and suppresses programmed cell death protein-1 and myeloid-derived suppressor cell accumulatio n", CANCER LETTERS, vol. 459, 2019, pages 268 - 276, XP085739658, DOI: 10.1016/j.canlet.2019.05.026
- OH EUNHYE, KIM YOON‐JAE, AN HYUNSOOK, SUNG DAEIL, CHO TAE‐MIN, FARRAND LEE, JANG SEOJIN, SEO JAE HONG, KIM JI YOUNG: "Flubendazole elicits anti‐metastatic effects in triple‐negative breast cancer via STAT3 inhibition", INTERNATIONAL JOURNAL OF CANCER, JOHN WILEY & SONS, INC., US, vol. 143, no. 8, 15 October 2018 (2018-10-15), US , pages 1978 - 1993, XP055857593, ISSN: 0020-7136, DOI: 10.1002/ijc.31585
- LIN SHICHONG, YANG LEHE, YAO YULEI, XU LINGYUAN, XIANG YOUQUN, ZHAO HAIYANG, WANG LIANGXING, ZUO ZHIGUI, HUANG XIAOYING, ZHAO CHEN: "Flubendazole demonstrates valid antitumor effects by inhibiting STAT3 and activating autophagy", JOURNAL OF EXPERIMENTAL & CLINICAL CANCER RESEARCH, vol. 38, no. 1, 1 December 2019 (2019-12-01), pages 293 - 305, XP055857595, DOI: 10.1186/s13046-019-1303-z
- SINSHEIMER, J. E; GIRI, A. K.; OSORIO, S.; WISE, D. S.; TOWNSEND, L. B.: "Comparative mutagenicity and genotoxicity of the antiparasitic drugs, mebendazole, flubendazole and flubendazole oxime", PROGRESS IN CLINICAL AND BIOLOGICAL RESEARCH, vol. 340E, 1990, US , pages 225 - 234, XP009540521, ISSN: 0361-7742
- DAWSON M, P W, LONG ' M, WATSON T R: "SYNTHESIS AND PURIFICATION OF [4'-'H]-MEBENDAZOLE", JOURNAL OF LABELLED COMPOUNDS AND RADIOPHARMACEUTICALS, 1 January 1985 (1985-01-01), pages 701 - 704, XP055857605, Retrieved from the Internet <URL:https://pillbuys.com/research/Mebendazole/3.pdf> [retrieved on 20211103]
- DATABASE Registry CAS; 5 August 2009 (2009-08-05), "Trideuteriomethyl N-[5-(4-fluorobenzoyl)-lH-benzimidazol-2-yl]carbamate", XP055857615, retrieved from STN Database accession no. RN 1173021-08-3
- DATABASE Registry CAS; 10 August 2001 (2001-08-10), "Carbamic acid, [[[4-(4-chlorobenzoyl)phenyl]amino]carbonyl]-, 2-methoxyethyl ester", XP055857618, retrieved from STN Database accession no. RN 351068-74-1
- DATABASE CAS 15 August 2001 (2001-08-15), "Carbamic acid, [[[4-(4-bromobenzoyl)phenyl]amino]carbonyl]-, 2-methoxyethyl ester", XP055857621, retrieved from STN Database accession no. RN 351460-41-8
- DATABASE Registry CAS; 20 February 2007 (2007-02-20), "Carbamic acid, N-(10,1 1-dihydro-10-methyl-1 1-oxodibenz[b,f][1,4]oxazepin-2-yl)-, ethyl ester", XP055857622, retrieved from STN Database accession no. RN 922055-50-3
- DATABASE Registry CAS; 20 February 2007 (2007-02-20), "Carbamic acid, N-(10,1 1-dihydro-10-methyl-1 1-oxodibenz[b,f][1,4]oxazepin-2-yl)-, methyl ester", XP055857623, retrieved from STN Database accession no. RN 922028-90-8
- DATABASE Registry CAS; 20 February 2007 (2007-02-20), "Carbamic acid, N-(10-ethyl-10,1 1-dihydro-11-oxodibenz[b,f][1,4]oxazepin-2-yl)-, methyl ester", XP055857626, retrieved from STN Database accession no. RN 922029-66-1
- DATABASE Registry CAS; 20 February 2007 (2007-02-20), "Carbamic acid, N-(10-ethyl-10,1 1-dihydro-l 1-oxodibenz[b,f][1,4]oxazepin-2-yl)-, ethyl ester", XP055857628, retrieved from STN Database accession no. RN 922029-50-3
- LI YUE, ALHENDI AHMAD M N, YEH MEI-CHUN, ELAHY MINA, SANTIAGO FERNANDO S, DESHPANDE NANDAN P, WU BEN, CHAN ENOCH, INAM SHAFQAT, : "Thermostable small-molecule inhibitor of angiogenesis and vascular permeability that suppresses a pERK-FosB/FosB–VCAM-1 axis", SCI. ADV, 1 July 2020 (2020-07-01), XP055857607, [retrieved on 20211103], DOI: 10.1126/sciadv.aaz7815

## Description

### FIELD OF THE INVENTION

The present invention also relates to compounds and pharmaceutical compositions for use in reducing vascular permeability, neovascularisation, angiogenesis, inflammation, cell migration and/or cell proliferation.

### BACKGROUND

Vascular permeability and neovascularization are key features underpinning inflammation, wound healing, tumor growth, macular edema in both diabetic retinopathy (DR) and neovascular (wet/exudative) age-related macular degeneration (nAMD). DR is the world's leading cause of vision loss in patients aged 20 to 74 years. AMD has a global prevalence of 170 million with around 11 million people affected with AMD in the United States. Retinal vascular leakage is caused by breakdown of the blood-retinal barrier (BRB) which normally maintains homeostasis. This is precipitated by endothelial dysfunction, angiogenic and inflammatory processes causing retinal capillary leakage into the interstitial space and edema through increased osmotic pressure. Vascular permeabilizing factors include vascular endothelial growth factor (VEGF), tumour necrosis factor-α (TNF-α), histamine, platelet-activating factor, serotonin and interleukin-1β (IL-1β).

Anti-VEGF therapies are widely used clinically for the treatment of DR. Repeated intravitreal injections, however, are needed and many patients do not respond optimally or an improved response is not sustained. Agents that target not only VEGF but other key mediators involved in the pathogenesis of nAMD/DR would have particular pharmaceutical appeal in this area of unmet clinical need.

Vascular permeability is also key to the pathogenesis of rheumatoid arthritis (RA), a process mediated by pro-inflammatory cytokines. RA impacts around 1.3 million people in the US alone.

There has been significant improvement in the management of RA over the last decade using biological agents, such as anti-TNF agents and soluble TNF receptor. However, a significant proportion of patients do not achieve clinical remission with current therapeutic options and are at risk of progressive joint destruction and functional disability.

Given the world's ageing population the significant unmet clinical need for both RA and nAMD/DR, and the global economic burden that the impact of these chronic diseases represent, alternative therapeutic approaches are needed.

US 2010/222401 describes compounds, compositions and methods for inhibiting vascular permeability and pathologic angiogenesis by modulating a signaling pathway.

### SUMMARY

Activator protein-1 (AP-1 or AP1) is a heterodimeric transcription factor involved in the regulation of gene expression in response to a range of pathological stimuli. The inventor has reasoned that compounds which are capable of inhibiting AP-1 dependent gene expression may be useful in treating or preventing diseases or conditions associated with vascular permeability, neovascularization, angiogenesis, inflammation, cell migration and/or cell proliferation.

The inventor has identified compounds that inhibit AP-1 dependent gene expression. The inventor has studied the activity of these compounds and found that these compounds inhibit FosB/ΔFosB expression. The inventor has found that such compounds are able to reduce vascular permeability, neovascularisation, angiogenesis, inflammation, cell migration and cell proliferation.

Accordingly, a first aspect provides a compound of formula (II), or a pharmaceutically acceptable salt thereof: 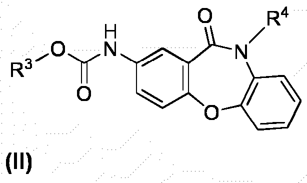 wherein:
R³ is straight or branched C₁-C₆ alkyl; and
R⁴ is straight or branched C₁-C₆ alkyl,
or R⁴ is 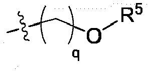 wherein q is 1, 2, 3 or 4; and R⁵ is straight or branched C₁-C₆ alkyl,
for use in treating a disease or condition associated with vascular permeability, neovascularisation, angiogenesis, inflammation, cell migration and/or cell proliferation in the subject, wherein the disease or condition is selected from the group consisting of
- arthritis;
- rheumatoid arthritis;
- bone destruction;
- age-related macular degeneration;
- diabetic retinopathy;
- macular edema;
- vascular leakage;
- vascular permeability;
- retinal vascular permeability;
- angiogenesis;
- endothelial cell dysfunction;
- atherosclerosis;
- stroke;
- myocardial infarction;
- peripheral vascular disease;
- stenosis;
- restenosis;
- inflammation;
- cytokine storm;
- pulmonary inflammation; and
- pulmonary fibrosis.

A second aspect provides a pharmaceutical composition comprising a compound of formula II, or a pharmaceutically acceptable salt thereof, as defined herein, and a pharmaceutically acceptable carrier..

A third aspect provides a compound of formula II, or a pharmaceutically acceptable salt thereof, as defined herein, for use as a medicament.

### BRIEF DESCRIPTION OF THE FIGURES

Preferred embodiments of the present invention will now be described, by way of example only, with reference to the accompanying drawings:

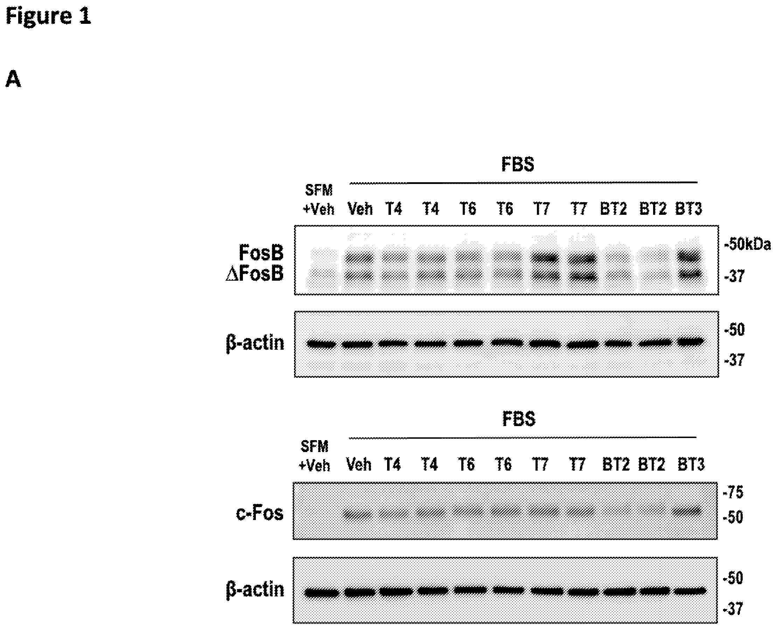

Figure 1A are images of Western blots showing the effect of compounds BT2, T4 and T6 on FosB/ΔFosB and c-Fos expression. HMEC-1 were grown in 6-well plates (in 10% FBS with EGF and hydrocortisone) and serum-arrested for 20h, then treated with 30µM compound (T4, T6, T7, BT2 and BT3) in serum free medium (without EGF or hydrocortisone) at 37°C for 4h. The medium was changed to 10% FBS (with EGF and hydrocortisone) with compound at the same concentration for 1h. Lysates were resolved by SDS-PAGE and Western blotting was performed for FosB or c-Fos. Experiments were performed with independent biological duplicates where indicated. Approximate positions of molecular weight markers are shown. Data represent 3 biologically-independent experiments.

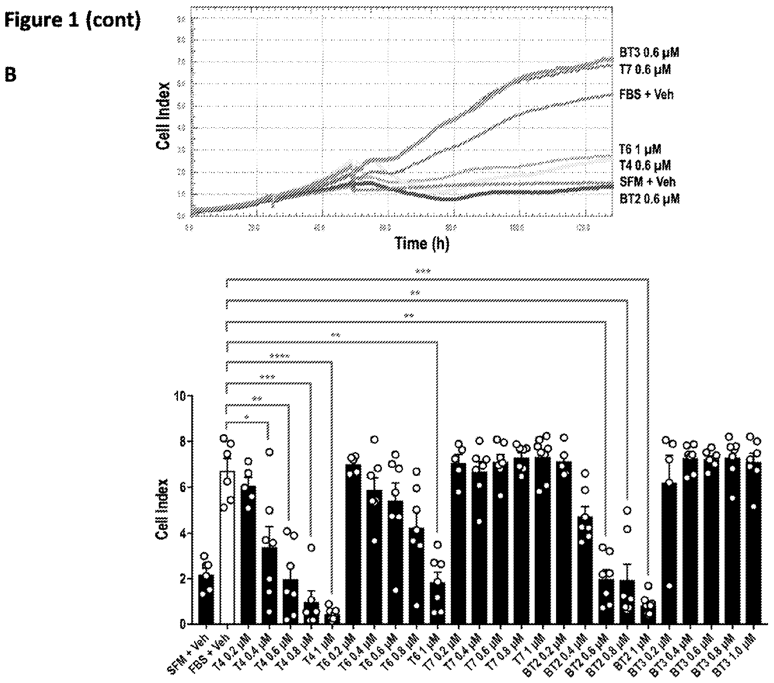

Figure 1B shows the effect of BT2, T4 and T6 on serum-inducible endothelial cell proliferation over time. Serum-deprived HMEC-1 were treated with compound in medium containing 5% FBS (with EGF and hydrocortisone) and cell proliferation monitored using the xCELLigence system. *Upper,* Representative real time profiles from one experiment using the xCELLigence system with concentrations indicated. Cell index is a quantitative measure of cell growth. *Lower,* xCELLigence data represents the mean±SEM of the means of 5-8 independent experiments after 79h. Statistical significance was assessed by one-way ANOVA.

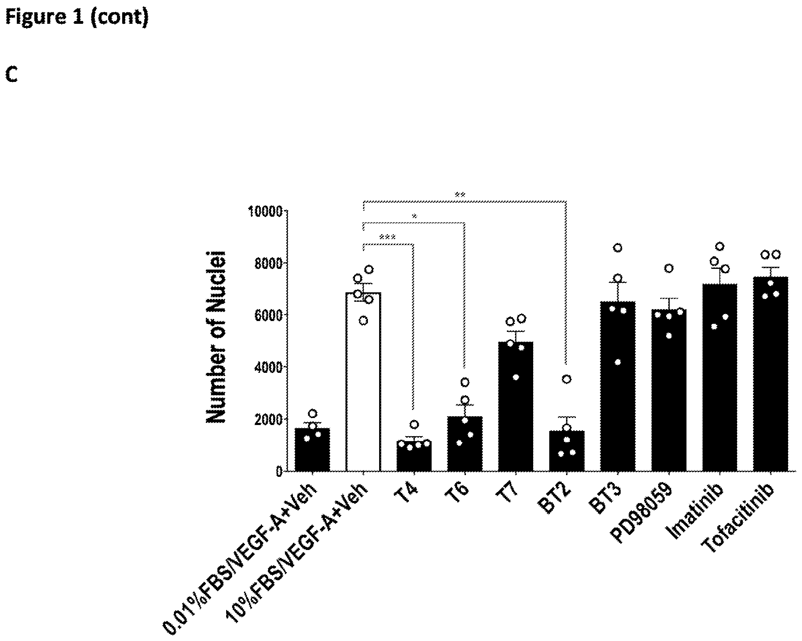

Figure 1C shows the effect of BT2, T4 and T6 on endothelial migration. BAEC in DMEM containing 10% FBS were seeded into 24-well plates fitted with 0.8µm Transwell inserts. After 48h, the medium was changed to DMEM containing 0.01% FBS for 48h. Compounds were added to the upper chamber at 1µM in DMEM containing 0.01% FBS and the medium in the lower chamber was changed to DMEM containing 10% FBS and 50ng/ml VEGF-A₁₆₅. The cells were left for 24h. Nuclei were quantified using NIH ImageJ software. Data represents the mean±SEM of the means of 4-5 independent experiments. Statistical significance was assessed by Kruskal-Wallis multiple comparisons test.

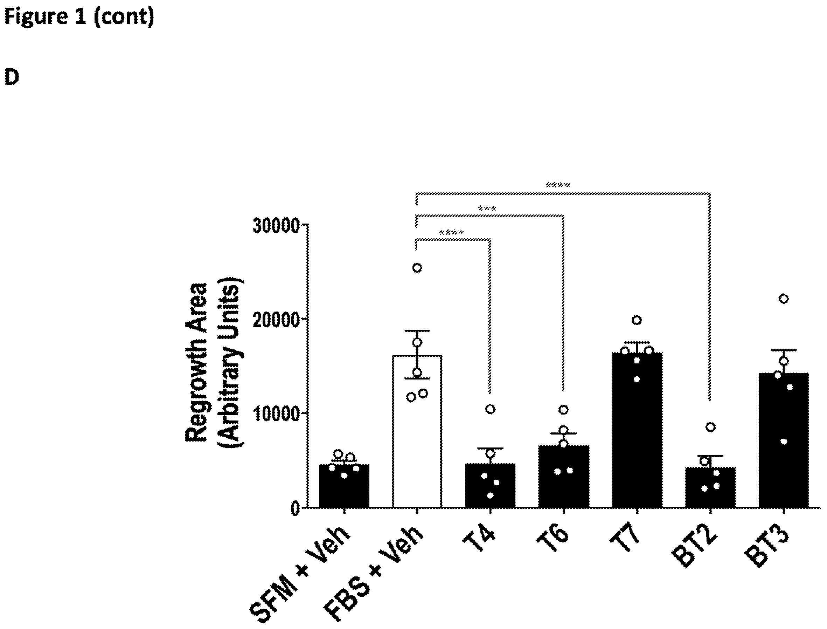

Figure 1D shows the effect of BT2, T4 and T6 on endothelial cell regrowth after mechanical injury *in vitro* using a scratch assay. HMEC-1 monolayers scraped with a sterile toothpick were treated with compound at 0.6µM in medium containing 5% FBS. Regrowth in the denuded area was monitored 48h after scraping. Regrown area was determined using Image-Pro Plus software (Cybernetics). Data represents the mean±SEM of the means of 5 independent experiments. Statistical significance was assessed by one-way ANOVA.

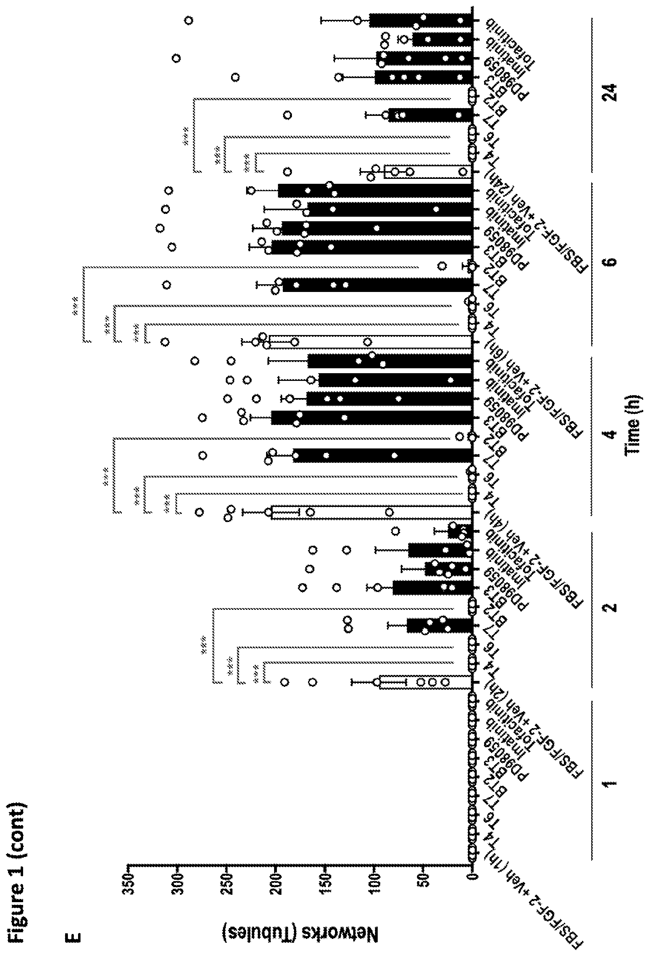

Figure 1E shows the effect of BT2, T4 and T6 on endothelial network (tubule) formation on Matrigel. HMEC-1 in medium containing 1% FBS and 50ng/ml FGF-2 were mixed with compound (3µM final) and seeded in wells coated with Matrigel. Network formation was assessed over the course of 24h. Networks were quantified using Image-Pro Plus software. Data represents the mean±SEM of the means of 5-6 independent experiments. Statistical significance was assessed by Kruskal-Wallis multiple comparisons test.

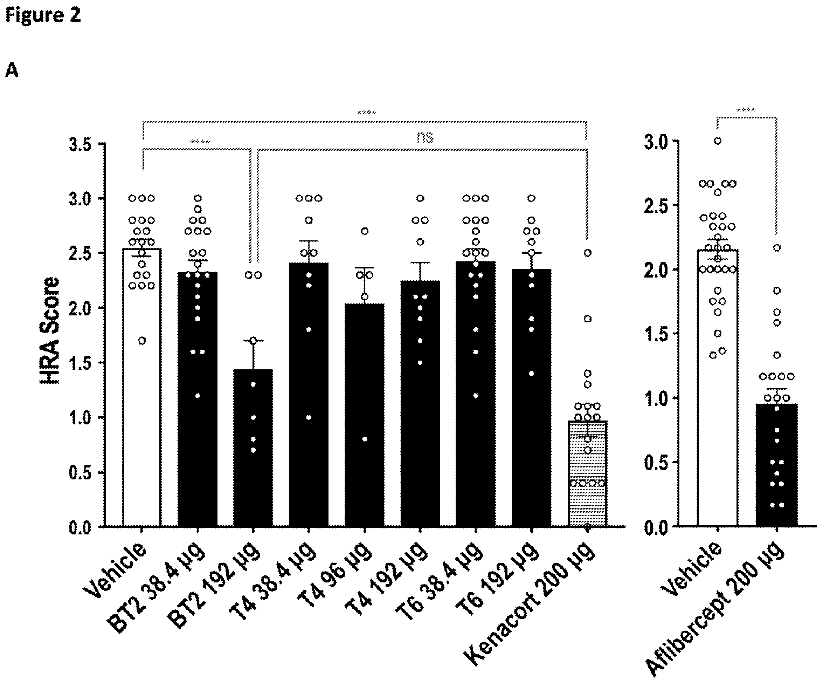

Figure 2A shows that BT2 inhibits retinal permeability in rats following choroidal laser injury. BT2, T4, T6 (doses indicated) or vehicle (control) was injected IVT into both eyes on the day (Day 0) and 7 days following 6 laser burns of the retina. Kenacort was administered IVT on Day 0. Alternatively, aflibercept/Eylea in vehicle (saline) was injected IVT 6 times (Days 0, 3, 7, 10, 14, 17). On Days 14 and 21, sodium fluorescein was injected subcutaneously and after 10min, ocular fluorescence was recorded using Heidelberg retinal angiography (HRA) and scored. HRA score combines Day 14 and 21 data. Data represents mean ± SEM. Statistical significance was assessed using one-way ANOVA (plot at *left*) or t-test (plot at *right,* and BT2 versus Kenacort comparison in plot at *left*). n=5-29 per group.

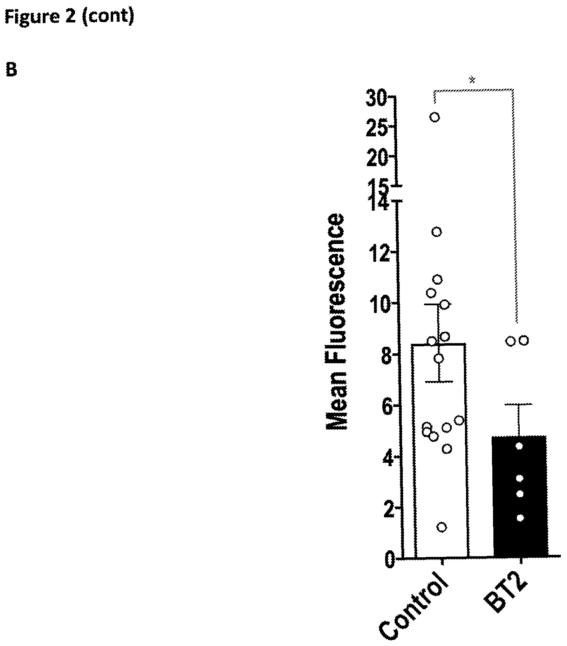

Figure 2B shows that BT2 inhibits retinal vascular permeability in rabbits induced by rhVEGF-A₁₆₅. BT2 or BT3 (600µg) or vehicle was injected IVT into the right eyes of rabbits 5d prior to induction of vascular leakage by IVT injection of 500ng rhVEGF-A₁₆₅ in 50µl in the same eyes. Two days after induction, sodium fluorescein was injected intravenously and after 1h, ocular fluorescence was measured in right (R) and left (L) eyes with an ocular fluorophotometer and expressed as a ratio (R/L) for each rabbit. Ratio data from the vehicle and BT3 groups were pooled (control) as both conditions were inactive and did not differ statistically from the other, for comparison against active compound BT2. Data represents mean±SEM. Statistical significance was assessed by t-test. n=6-8 per group.

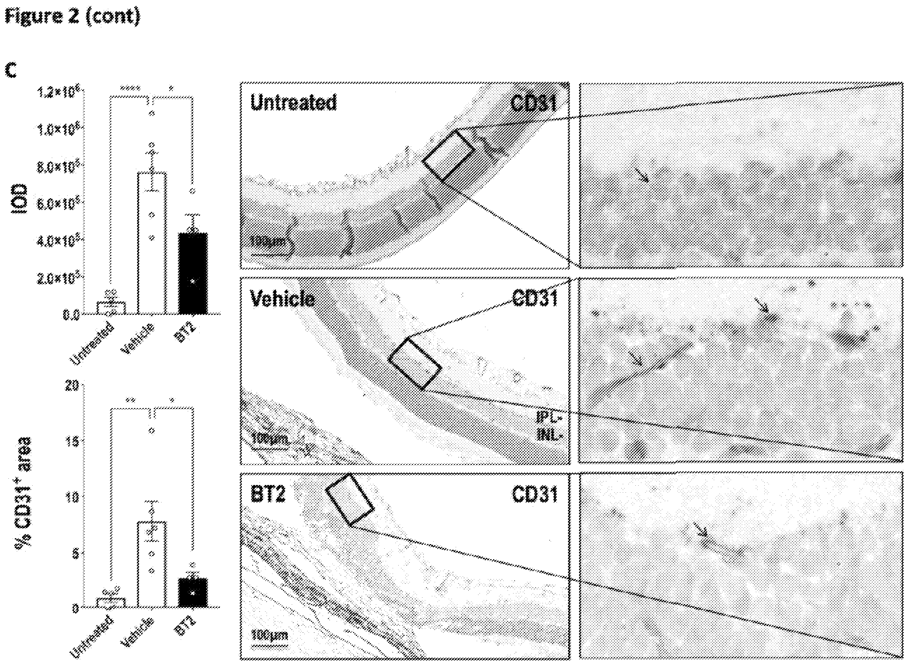

Figures 2C-E show immunohistochemical staining in rat retinal lesions for (C) CD31, (D) VEGF-A₁₆₅, (E) VEGF-A₁₆₅ in 100µm boxed increments relative to the wound. Untreated refers to eyes that were not lasered or injected with vehicle or drug. IOD of positive staining (red chromogen) was assessed using Image-Pro Plus software. Slides were photographed under 10x or 20x objective and magnified views are shown. n=4-6 per group for CD31, n=3-6 per group for VEGF-A₁₆₅ or n=3-5 per group for VEGF-A₁₆₅ gradient analysis. Data represents the mean±SEM of the mean per animal. Statistical significance was assessed by one-way ANOVA, Mann-Whitney or t-test, as appropriate. Arrows provide examples of positive staining.

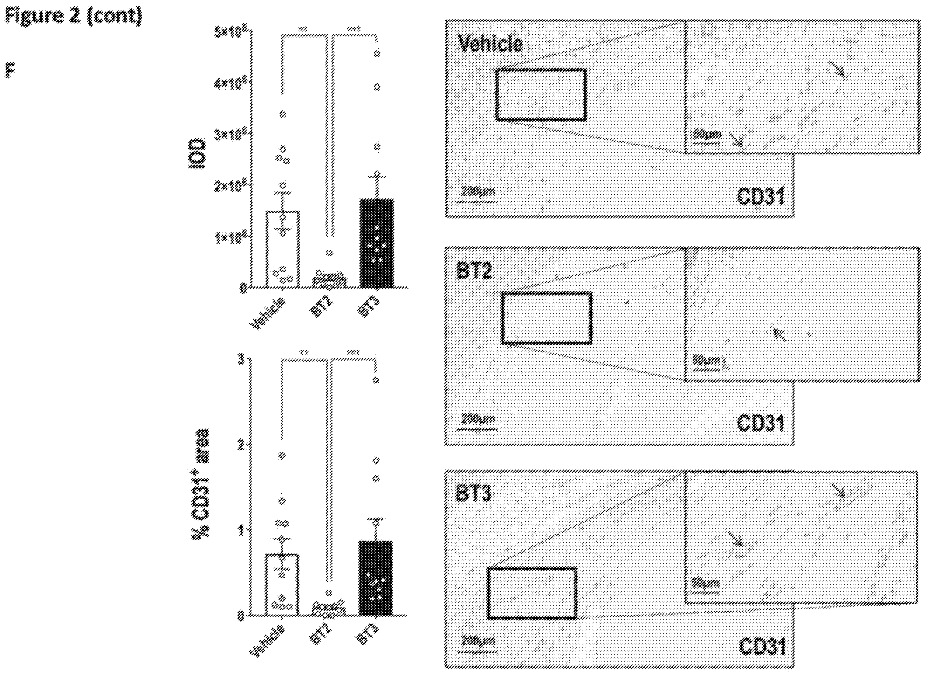

Figure 2F shows that BT2 inhibits angiogenesis in Matrigel plugs in mice. Matrigel (500pl) containing VEGF-A₁₆₅ (100ng/ml), heparin (10U) and BT2 or BT3 (2.5 mg/mouse) or vehicle was injected subcutaneously into the left flanks of male 8 week-old C57BL/6 mice. After 7 days, mice were sacrificed and the plugs stained with CD31 antibodies. Representative immunohistochemical images stained for CD31 photographed under 10x objective with the inset providing a magnified view (photographed under 40x objective). CD31 staining was quantified using Image-Pro Plus software. Data represents mean±SEM of the mean per animal. Statistical significance was assessed by Kruskal-Wallis multiple comparisons test. n=10-11 per group.

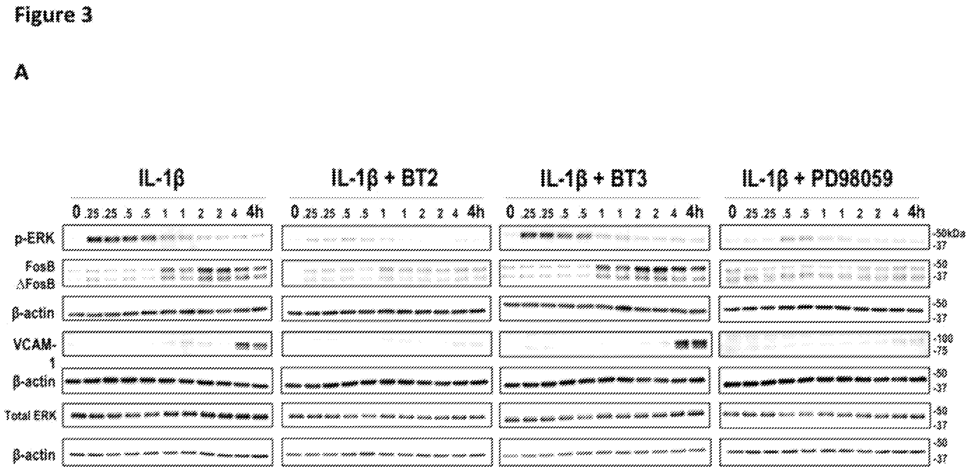

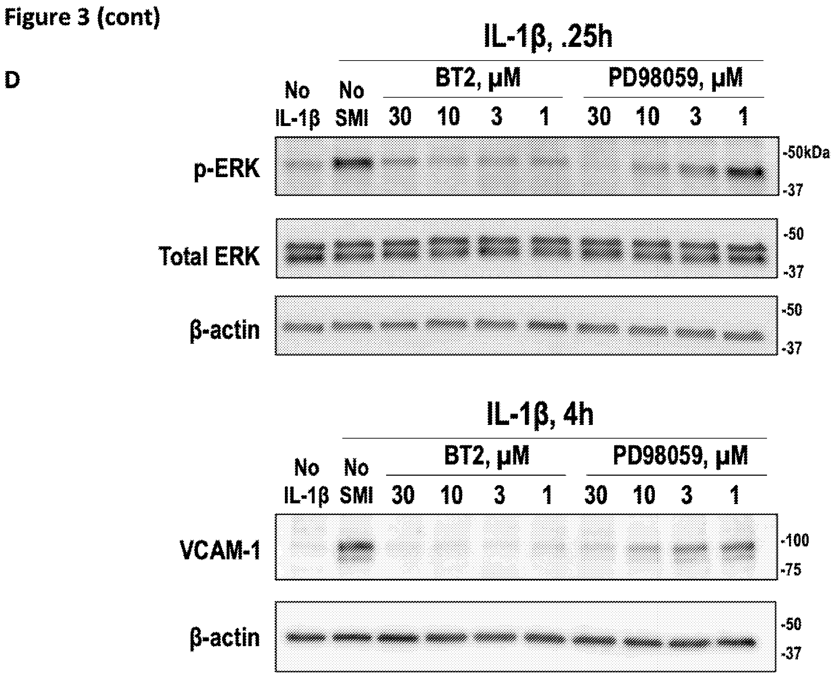

Figure 3A are images of Western blots showing that BT2 inhibits ERK phosphorylation, FosB/ΔFosB and VCAM-1 expression. HMEC treated with 30µM BT2 or 30µM PD98059 were stimulated with 20ng/ml IL-1β for various times up to 4h. Westerns are representative of 2-3 biologically independent experiments each performed with 2 biologically independent replicates run in separate lanes (where shown) with times shown in hours. BT2 inhibition of IL-β1-inducible VCAM-1 and ERK phosphorylation on the same blot is indicated in Figure 3D.

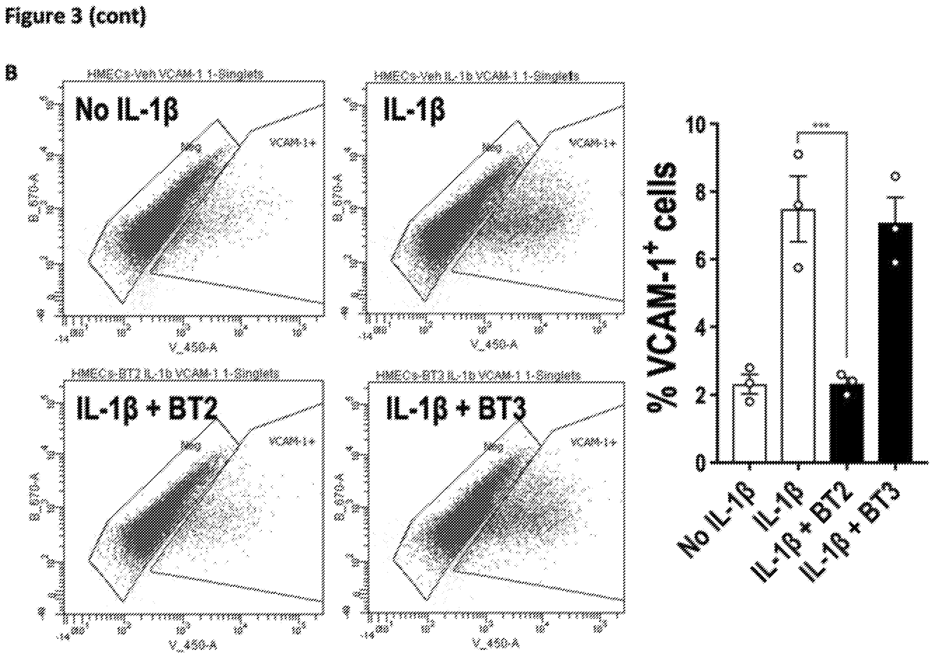

Figure 3B shows that BT2 inhibits VCAM-1 expression by flow cytometry. Flow cytometry was performed with HMEC-1 treated with 30µM BT2 or BT3 and 20ng/ml IL-1β using a BD FACSCanto II. Data represents mean±SEM of the means of 3 independent experiments. Statistical significance was assessed by one-way ANOVA. n=3 per group.

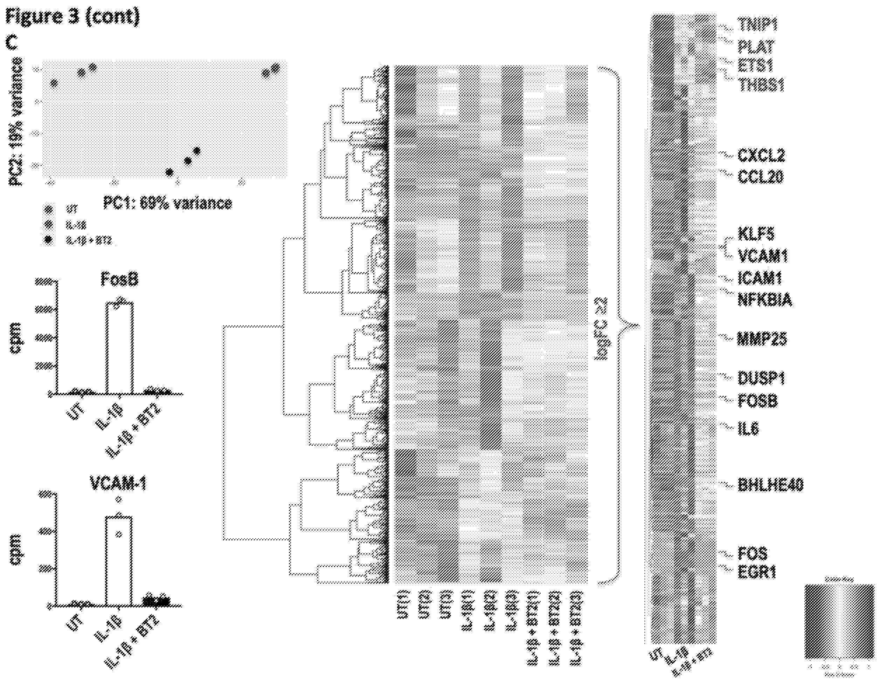

Figure 3C shows that BT2 inhibits FosB, c-Fos, VCAM-1, ICAM-1 and a range of other genes involved in cell proliferation, migration, angiogenesis and/or inflammation, RNA-seq was performed with total RNA prepared from HMEC-1 pre-treated with 30µM BT2 and 4h incubation with 20ng/ml IL-1β. A PCA plot (*upper left*) shows close association between biological replicates within conditions UT, IL-1β and IL-1β+BT2 and clear separation across conditions. The heatmap (*centre,* 1579 genes) was generated for all up-regulated genes for the comparison IL-1β versus UT. Counts per million (cpm) values were used and the genes (rows) were grouped using hierarchical clustering with cpm for FosB and VCAM-1 and plotted. The heatmap (*right*) shows 325 genes with log fold change (FC) ≥2. FosB, c-Fos and VCAM-1 (the subject of this work) are indicated in the figure together with several other genes inhibited by BT2. The figure also shows a small subset of genes (indicated in red) that are further induced by BT2. BHLHE40, basic helix-loop-helix family member e40; CCL20, C-C motif chemokine ligand 20; CXCL2, C-X-C motif chemokine ligand 2; DUSP1, dual specificity phosphatase 1; EGR1, early growth response 1; ETS1, ETS proto-oncogene 1; FOS, FOS proto-oncogene; FOSB, FosB proto-oncogene; ICAM1, intercellular adhesion molecule 1; IL6, interleukin 6; KLF5, Kruppel like factor 5; MMP25, matrix metallopeptidase 25; NFKBIA, NFKB inhibitor α; THBS1, thrombospondin 1; TNIP, TNFAIP3 interacting protein 1; PLAT, plasminogen activator, tissue type; VCAM1, vascular cell adhesion molecule 1.
Figure 3D shows that BT2 inhibits IL-1β-inducible VCAM-1 expression and ERK phosphorylation more potently than PD98059. Concentrations of BT2 and PD98059 (1-30µM) are indicated. Data represents 3 biologically-independent experiments.

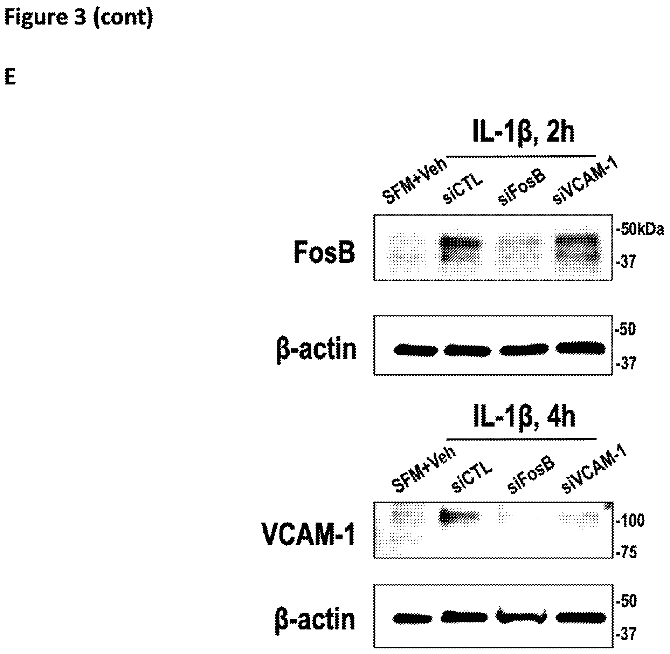

Figure 3E are images of Western blots using siRNA showing that VCAM-1 expression is dependent upon FosB. HMEC-1 treated with 0.6µM siRNA or control siRNA were stimulated with 20ng/ml IL-1β for 2 or 4h. Western blotting was performed with the antibodies indicated. Data is representative of 2 biologically-independent experiments. Approximate positions of molecular weight markers are shown.

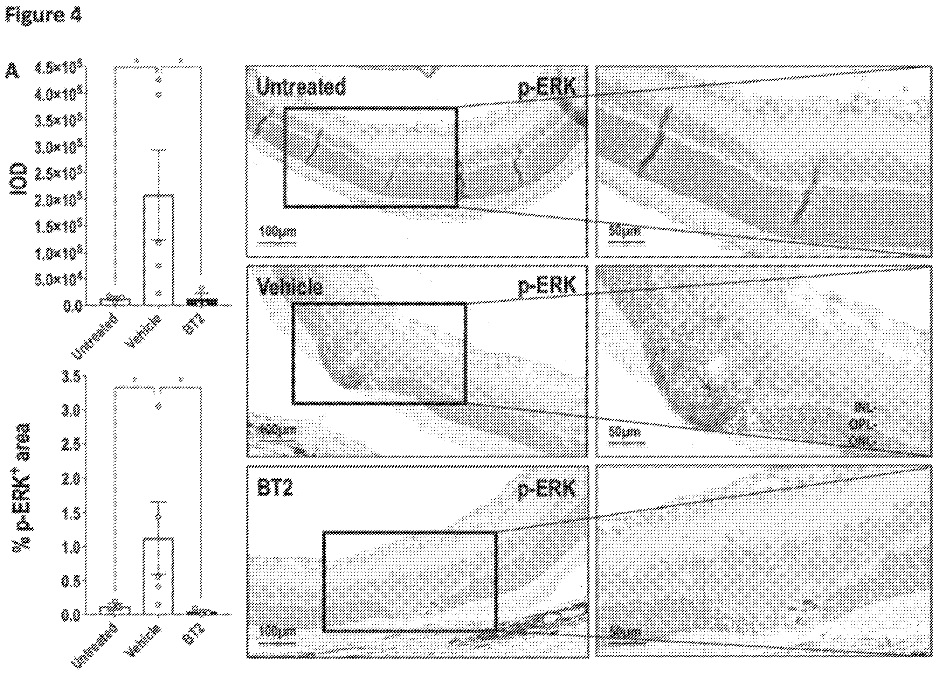

Figures 4A-E show that BT2 inhibits ERK phosphorylation, FosB/ΔFosB and VCAM-1 expression in retinas and Matrigel plugs. Immunohistochemical staining in retinal lesions was performed for (A) pERK, (B) FosB and (C) VCAM-1. IOD of positive staining (red chromogen) was assessed using Image-Pro Plus software. Slides were photographed under 20x or 40x objective and magnified views are shown. n=3-5 per group for pERK and FosB, and n=4-6 per group for VCAM-1. Data represents the mean±SEM of the mean per animal. Statistical significance was assessed by one-way ANOVA, Kruskal-Wallis, Mann-Whitney or t-test, as appropriate. Arrows provide examples of positive staining. INL, inner nuclear layer; OPL, outer plexiform layer; ONL, outer nuclear layer; OLM, outer limiting membrane. Alternatively, Matrigel plugs were stained for (D) FosB or (E) VCAM-1. Representative FosB or VCAM-1 staining was photographed under 40x or 20x objective, respectively, with the inset providing a magnified view. Staining was quantified using Image-Pro Plus software. Data represents mean±SEM. Statistical significance was assessed by Kruskal-Wallis multiple comparisons test (FosB, n=9-11 per group) or one-way ANOVA (VCAM-1, n=10-11 per group). Arrows provide examples of positive staining. IOD denotes integrated optical density.

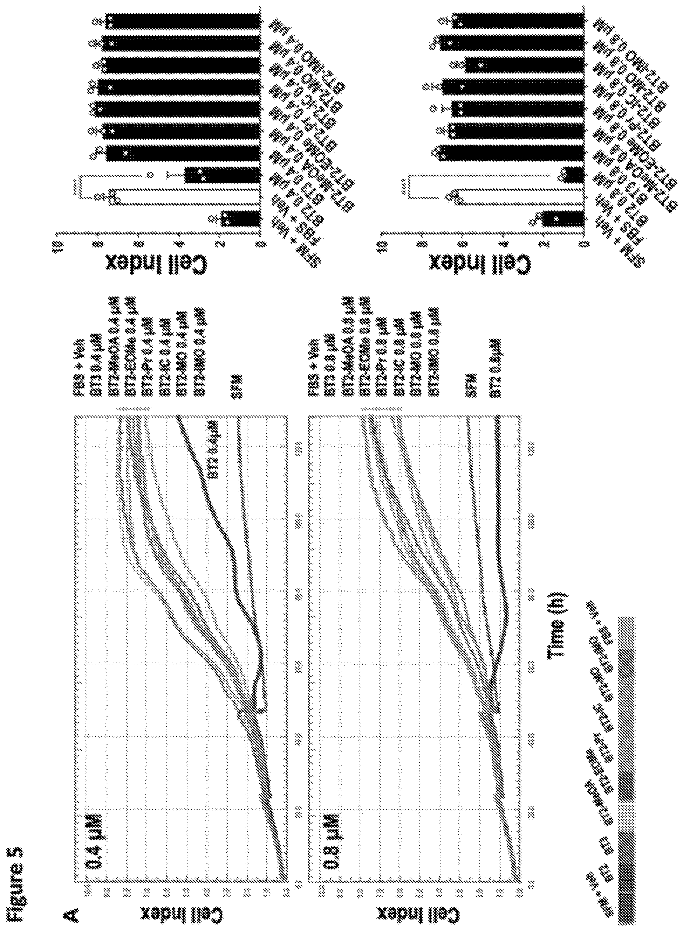

Figures 5A-D show that the carbamate moiety in BT2 is critical to its interaction with MEK1 and functional effects. Figure 5A shows proliferation experiments in which serum-deprived HMEC-1 were treated with compound (0.4 or 0.8µM) in medium containing 5% FBS and cell proliferation monitored using the xCELLigence system (Roche). *Left,* Representative growth profiles from one experiment. *Right,* xCELLigence data representing the mean±SEM of the means of 3 independent experiments after 79h. Statistical significance was assessed by one-way ANOVA or Mann-Whitney test.

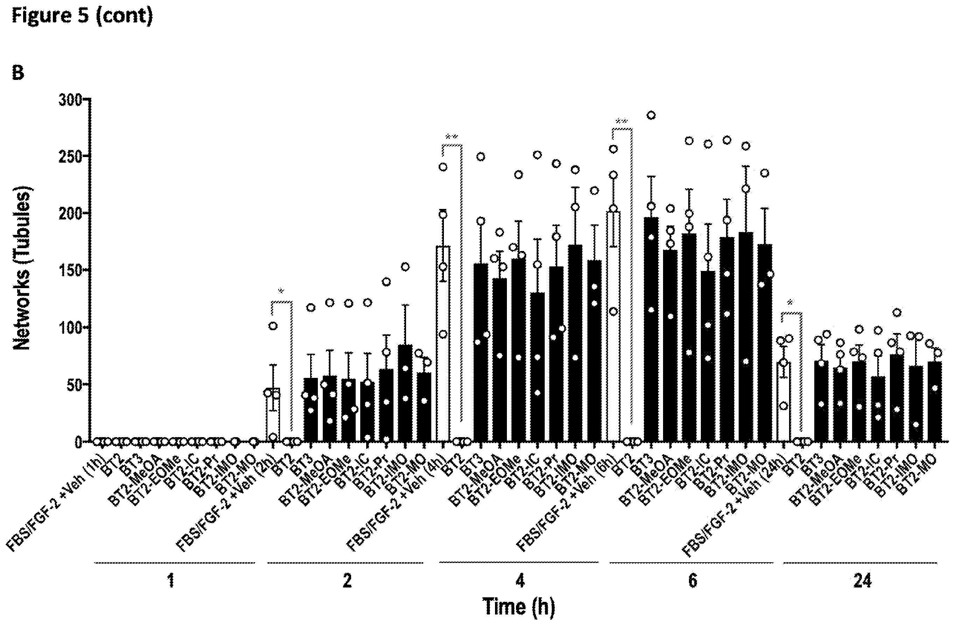

Figure 5B shows HMEC-1 network formation in medium containing 1% FBS and 50ng/ml FGF-2 combined with compound (1µM final) and seeded in wells coated with Matrigel. Networks were quantified using NIH ImageJ software. Data represents the mean±SEM of the means of 3-4 independent experiments. Statistical significance was assessed by Kruskal-Wallis multiple comparisons test.

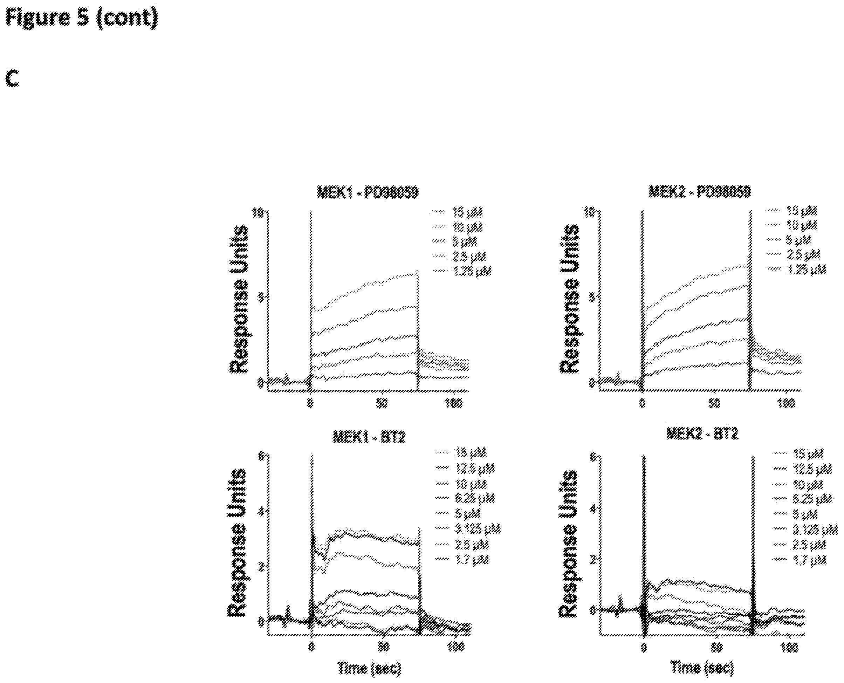

Figure 5C shows SPR analysis testing the interaction of PD98059, BT2 and BT2 analogues with His-MEK1 (*left panels*) and His-MEK2 (*right panels*). Measurements were made on a Biacore T200 at 15°C in a buffer comprising 20mM HEPES, 150mM NaCl, 5% DMSO pH 7.4. Data are representative of 2 independent experiments.

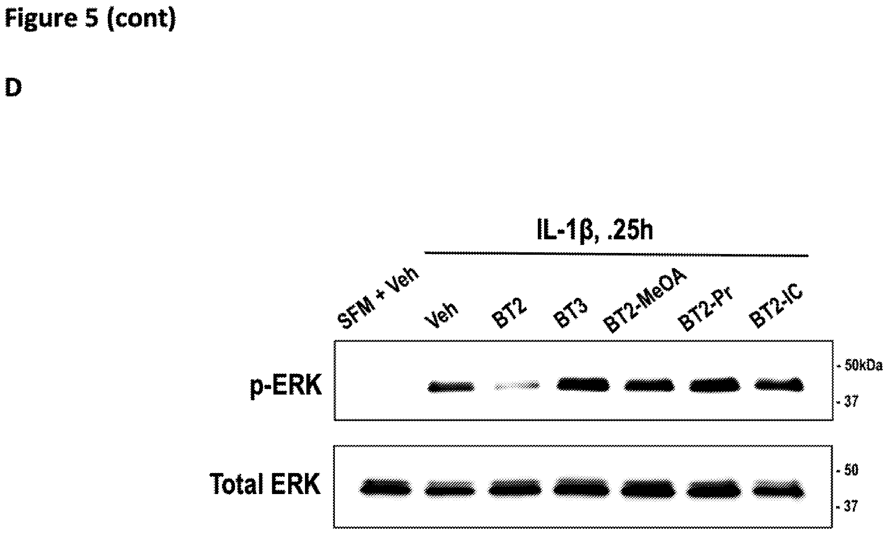

In Figure 5D, HMEC-1 were treated with 1µM compound (BT2 and analogues) in serum free medium at 37°C for 4h. The medium was changed to 20ng/ml IL-1β with compound for 15min. Lysates were resolved by SDS-PAGE and Western blotting was performed for pERK or total ERK. Data is representative of 2 biologically-independent experiments. Approximate positions of molecular weight markers are shown.

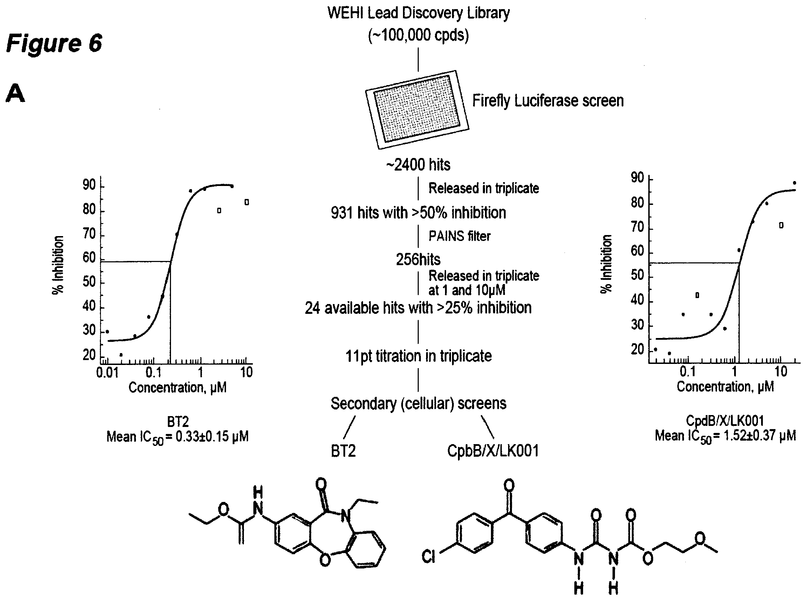

Figure 6A shows a schematic representation of the high throughput compound screen. A luciferase-based high throughput screen was used to identify hits including use of a PAINS frequent hitter filter. Mean IC₅₀ data and typical 11-point titration curves for BT2 and Cpd B/X/LK001 are shown.

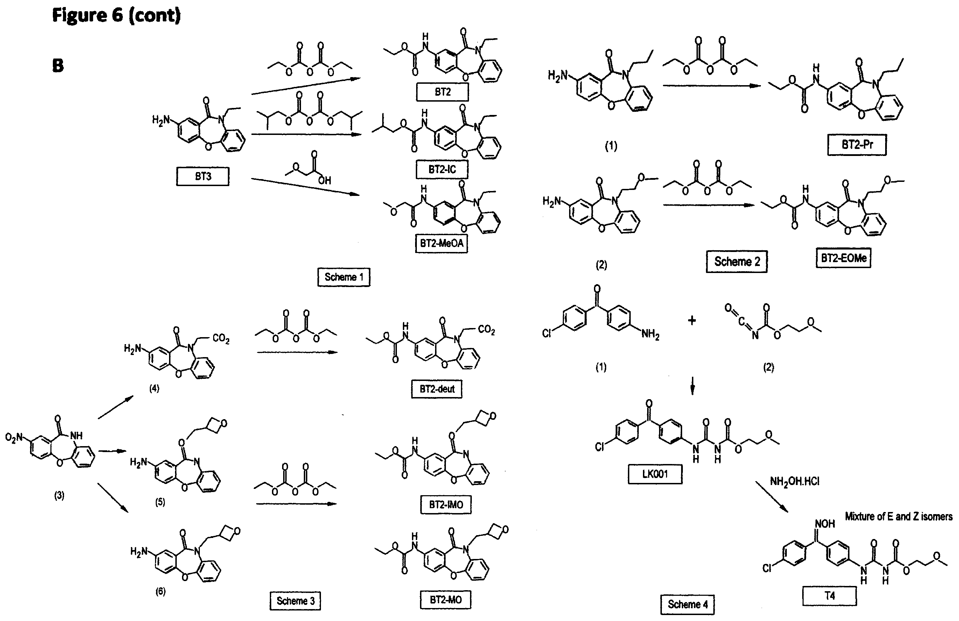

Figure 6B shows reactants in chemical synthesis of Cpd B/X/LK001 or BT2 analogues.

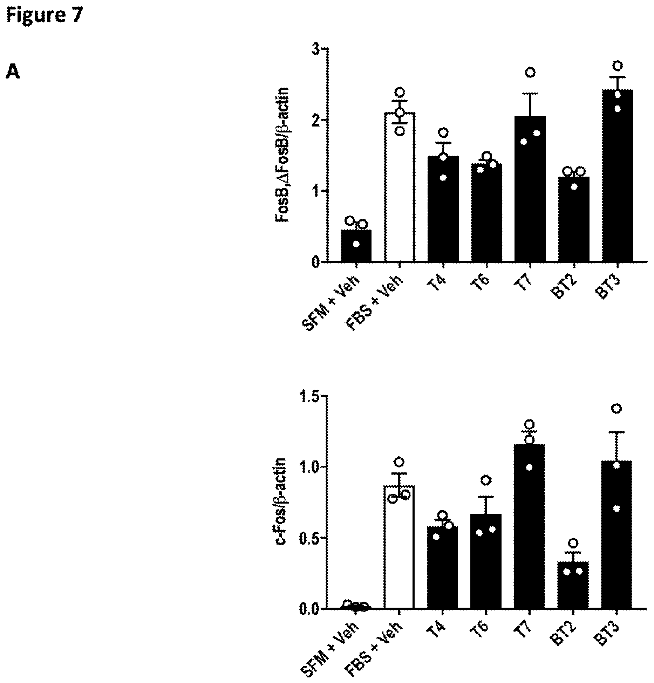

Figures 7A-B show that BT2, T4 and T6 inhibit endothelial FosB/ΔFosB and c-Fos expression and block cell proliferation. Figure 7A shows band intensity (pixel intensity relative to the corresponding control) from Western blot analysis measured using NIH ImageJ software. FosB/ΔFosB band intensity was combined. Plotted data represents the values or means (where independent biological duplicates were used in the one blot) ±SEM of 3 biologically-independent experiments.

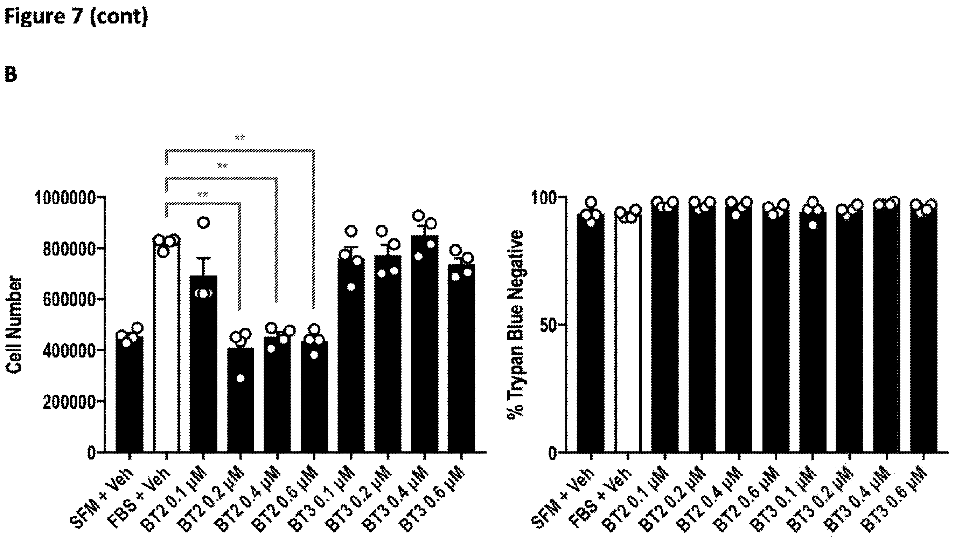

Figure 7B shows total cell numbers and % living cells as a proportion of total cells determined by Trypan Blue exclusion using a Countess II Automated Cell Counter. Countess data represents the mean±SEM of the means of 4 independent experiments. Statistical significance was assessed by Kruskal-Wallis multiple comparisons test.

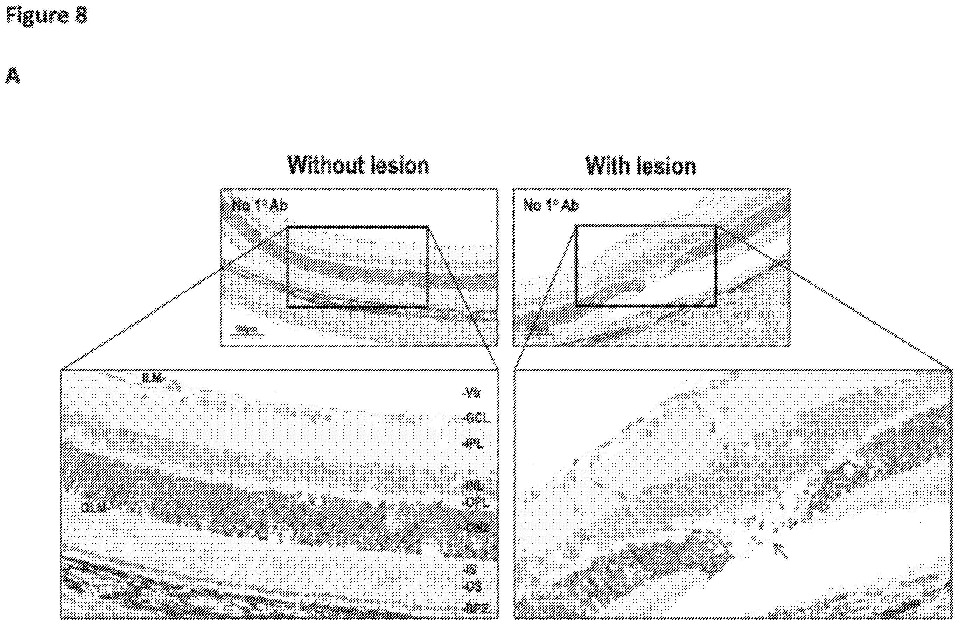

Figures 8A-C shows immunohistochemical staining with primary antibody omitted. Figure 8A shows immunohistochemical staining (vehicle group) using the MACH3 AP-Polymer detection system with primary antibody omitted in a region without or with lesion (arrow). Vitr, vitreous. ILM, inner limiting membrane; GCL, ganglion cell layer; IPL, inner plexiform layer; INL, inner nuclear layer; OPL, outer plexiform layer; ONL, outer nuclear layer; OLM, outer limiting membrane; IS, inner segment; OS, outer segment; RPE, retinal pigment epithelium; Chor, choroid.

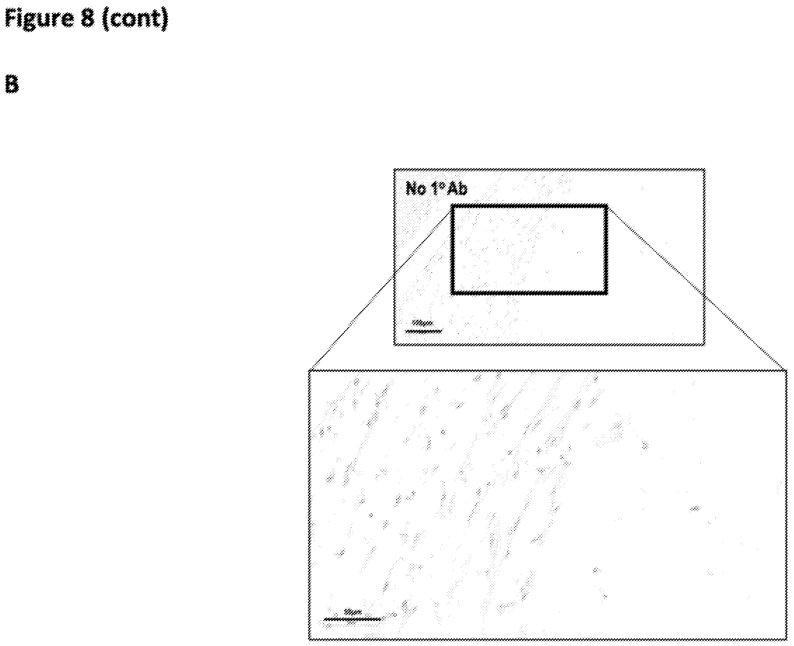

Figure 8B shows immunohistochemical staining (vehicle group) using the DAB chromogen detection system with primary antibody omitted in Matrigel plug.

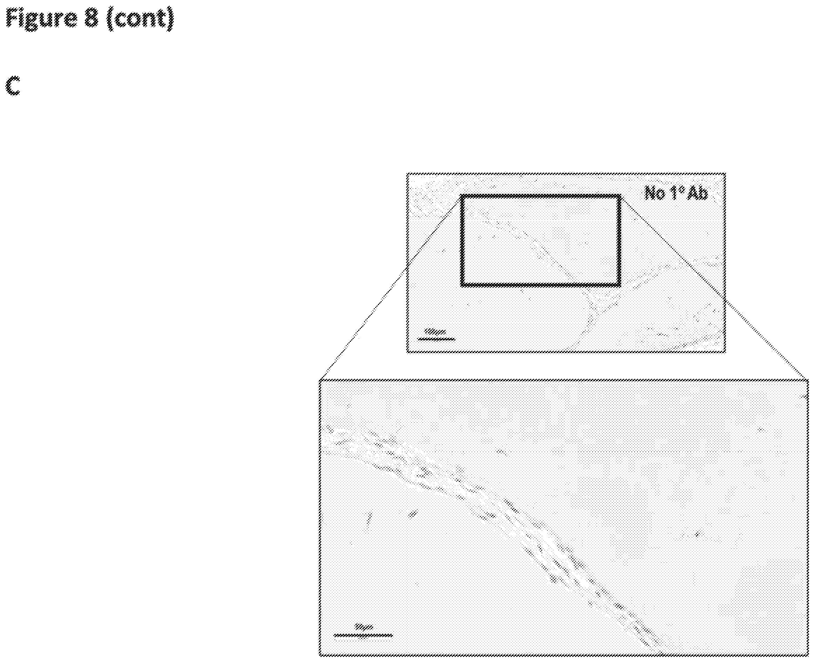

Figure 8C shows immunohistochemical staining (vehicle group) using the MACH3 AP-Polymer detection system with primary antibody omitted in Matrigel plug. No 1° Ab denotes primary antibody omitted.

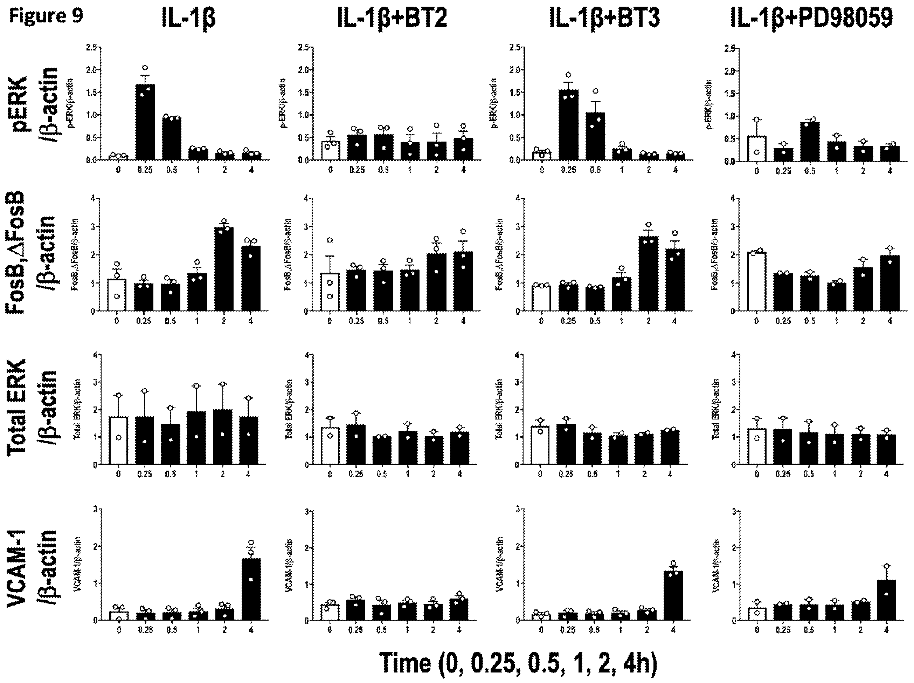

Figure 9 shows BT2 inhibits ERK phosphorylation, FosB/ΔFosB and VCAM-1 expression. Band intensity (pixel intensity relative to the corresponding control) from Western blot analysis was measured using NIH ImageJ software. FosB/ΔFosB band intensity was combined. Plotted data represents the values or means (where independent biological duplicates were used in the one blot) ±SEM of 2-3 biologically-independent experiments.

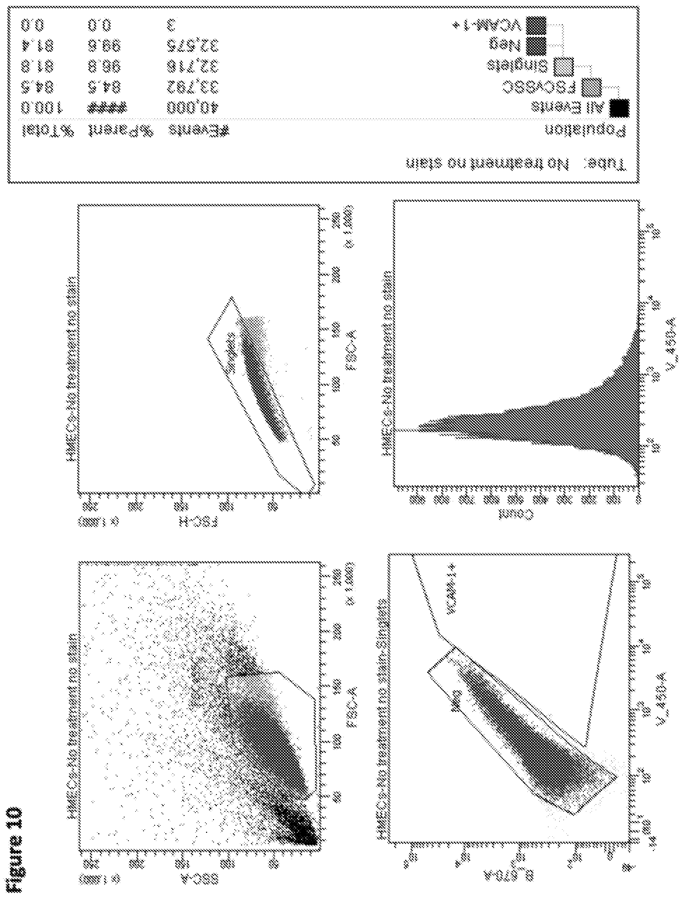

Figure 10 shows gating of VCAM-1⁺ and VCAM-1⁻ cells by flow cytometry. VCAM-1⁺ and VCAM-1⁻ cells were gated by performing flow cytometry (FACSDiva v6.1.3) with or without primary VCAM-1 antibody (non-specific staining), respectively. Representative gating from the latter (i.e. negative control) is shown in the figure.

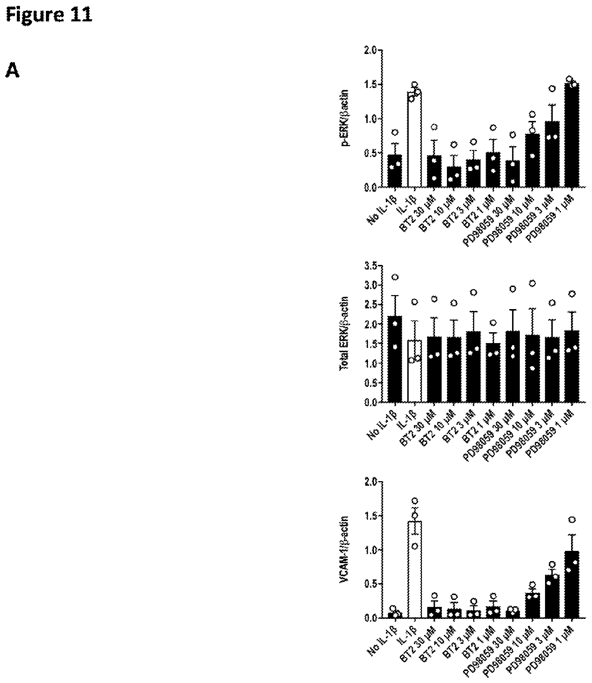

Figures 11A-C show Western blotting experiments with extracts of HMEC-1 exposed to BT2 or plasmid transfected HMEC-1. Figure 11A shows the comparative effect of BT2 and PD98059 on IL-1β-inducible VCAM-1 expression and ERK phosphorylation. Band intensity (pixel intensity relative to the corresponding control) from Western blot analysis was measured using NIH ImageJ software. Plotted data represents the mean±SEM of 3 biologically-independent experiments.

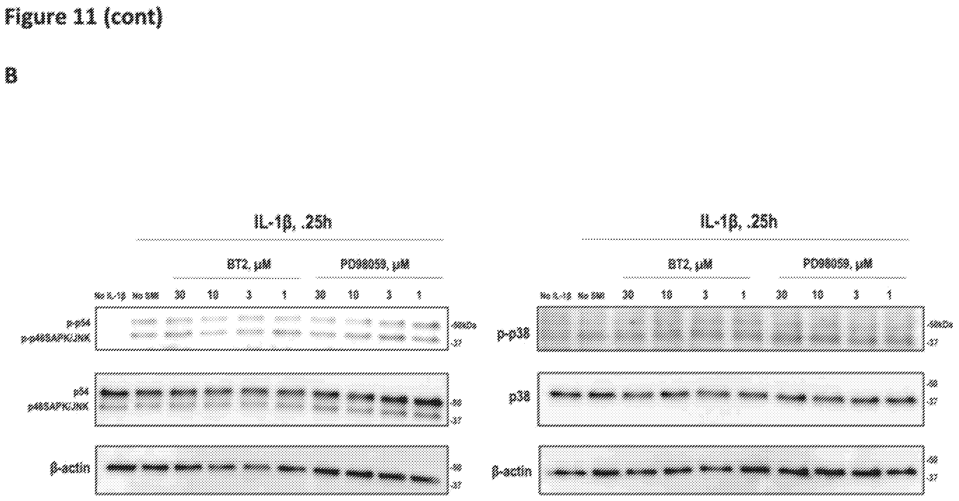

Figure 11B shows the comparative effect of BT2 and PD98059 (1-30µM) on IL-1β-inducible p-SAPK/JNK or p-p38. Data represents the mean±SEM of 3 biologically-independent experiments. Approximate positions of molecular weight markers are shown.

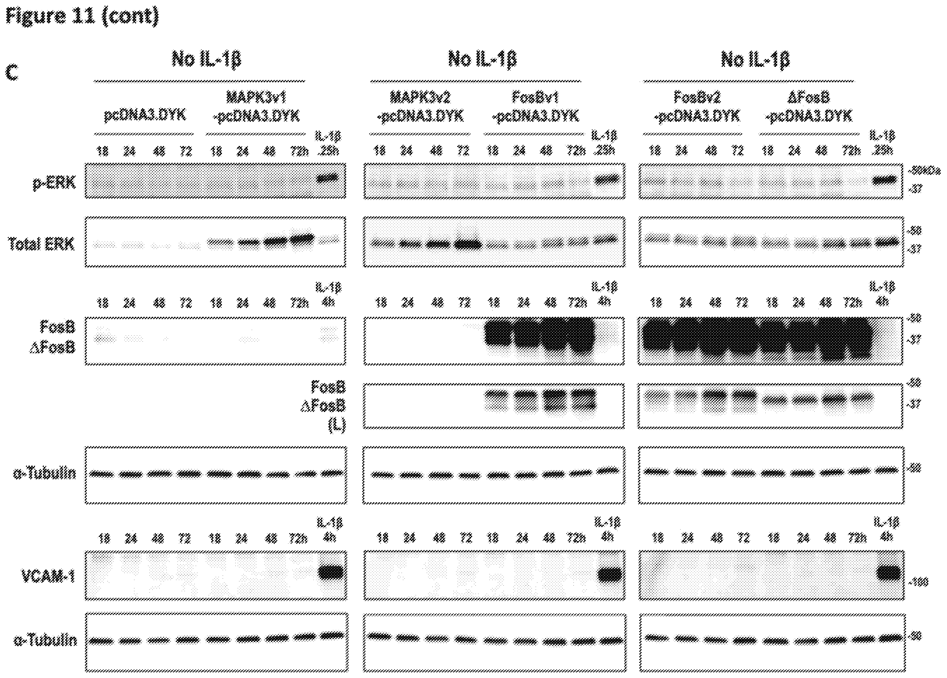

Figure 11C shows the requirement of ERK phosphorylation in the indication of FosB and VCAM-1 expression by Western blotting. HMEC-1 rendered growth quiescent by serum deprivation (and without EGF or hydrocortisone) in 6-well plates were transfected with 6µg of the indicated pcDNA3.1+/C-(K)DYK-based plasmid with insert ERK1 variant 1 (NM_002746.2), ERK1 variant 2 (NM_001040056.3), FosB variant 1 (NM_006732.2), FosB variant 2 (NM_001114171.2) or ΔFosB (XM_005258691.1). Western blotting was performed with total protein lysates (collected 18, 24, 48 and 72h after plasmid transfection) using antibodies indicated. L denotes lighter exposure. Approximate positions of molecular weight markers are shown. Data is representative of 2 independent experiments.

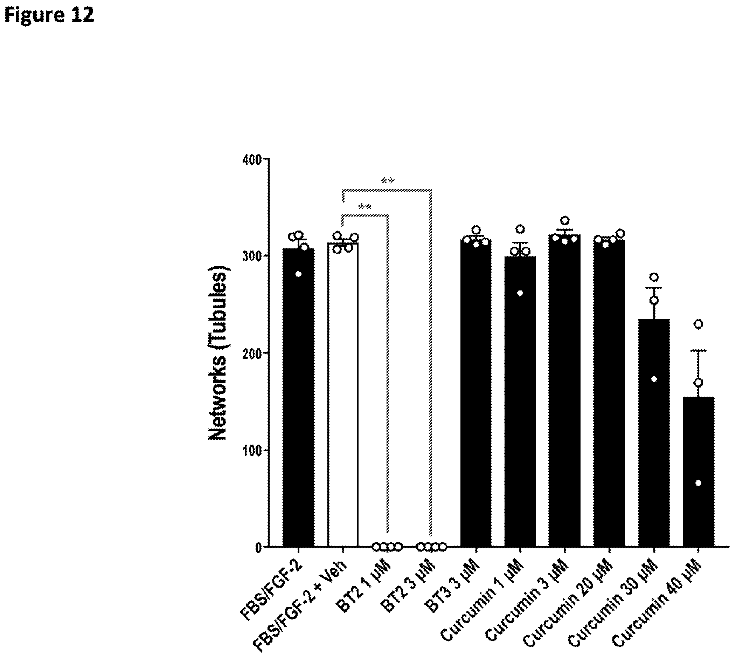

Figure 12 shows that BT2 is more potent than curcumin at inhibiting endothelial network formation on Matrigel. HMEC-1 in medium containing 1% FBS and 50ng/ml FGF-2 were combined with various concentrations of BT2 or curcumin compound and seeded in wells coated with Matrigel. Networks after 4h were quantified using NIH ImageJ software. Data represents the mean±SEM of the means of 3-4 independent experiments. Statistical significance was assessed by Kruskal-Wallis multiple comparisons test.

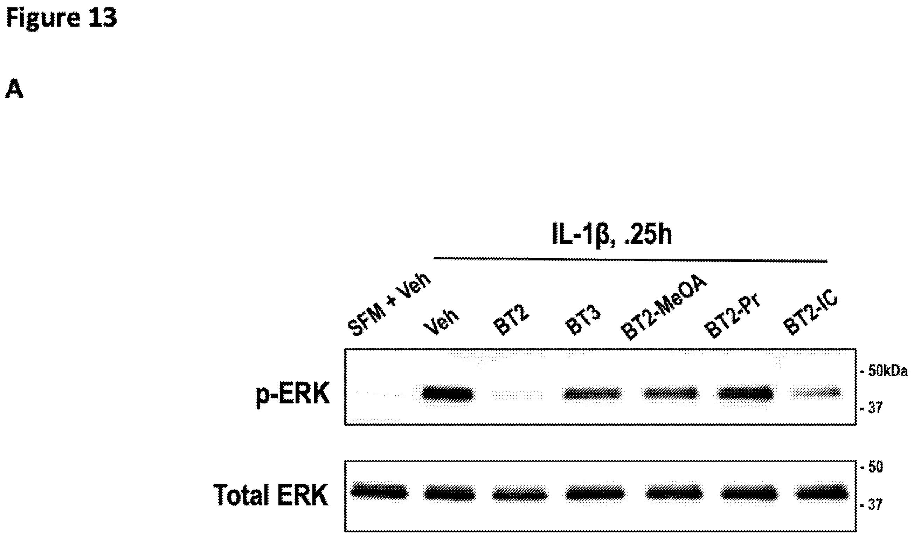

Figures 13A-B show bioactivity of structural analogues of BT2. In Figure 13A, HMEC-1 were treated with 3µM compound (BT2 and analogues) in serum free medium at 37°C for 4h. The medium was changed to 20ng/ml IL-1β with compound for 15min. Lysates were resolved by SDS-PAGE and Western blotting was performed for phosphorylated ERK or total ERK. Approximate positions of molecular weight markers are shown.

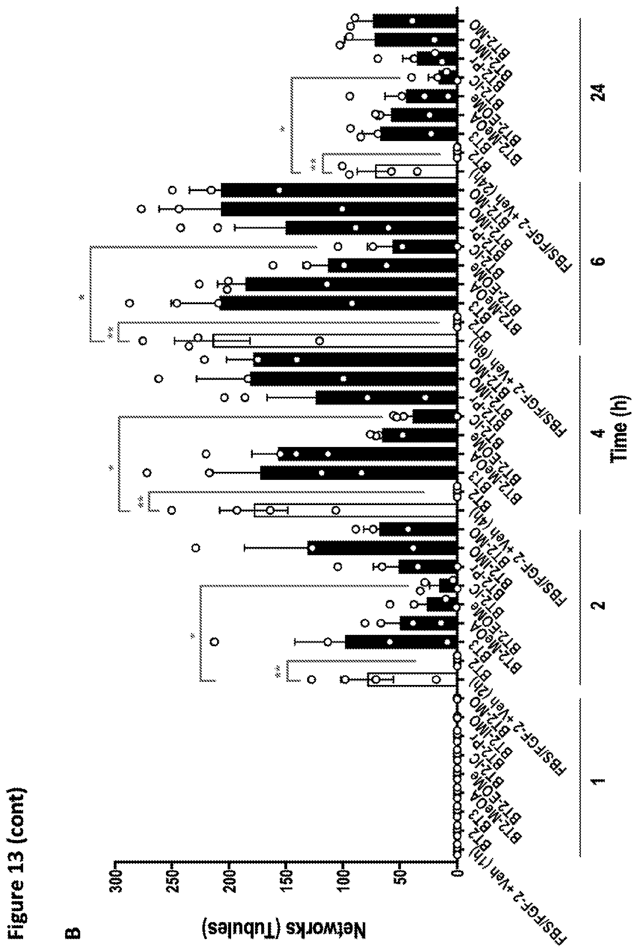

Figure 13B shows HMEC-1 network formation in medium containing 1% FBS and 50ng/ml FGF-2 combined with compound (3µM final) and seeded in wells coated with Matrigel. Networks were quantified using NIH Image J software. Data represents the mean±SEM of the means of 3-4 independent experiments. Statistical significance was assessed by Kruskal-Wallis multiple comparisons test.

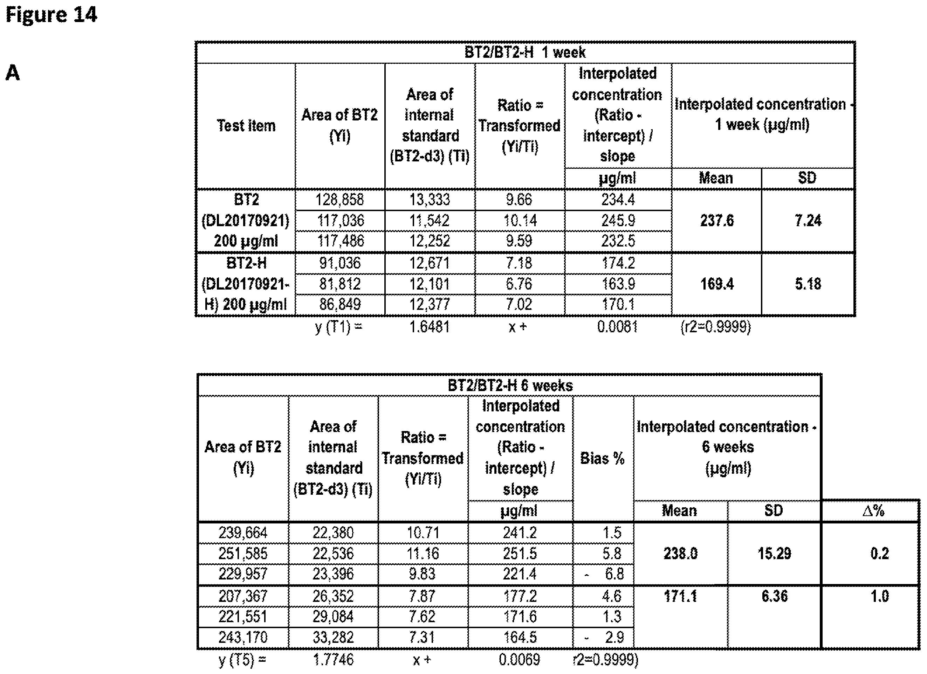

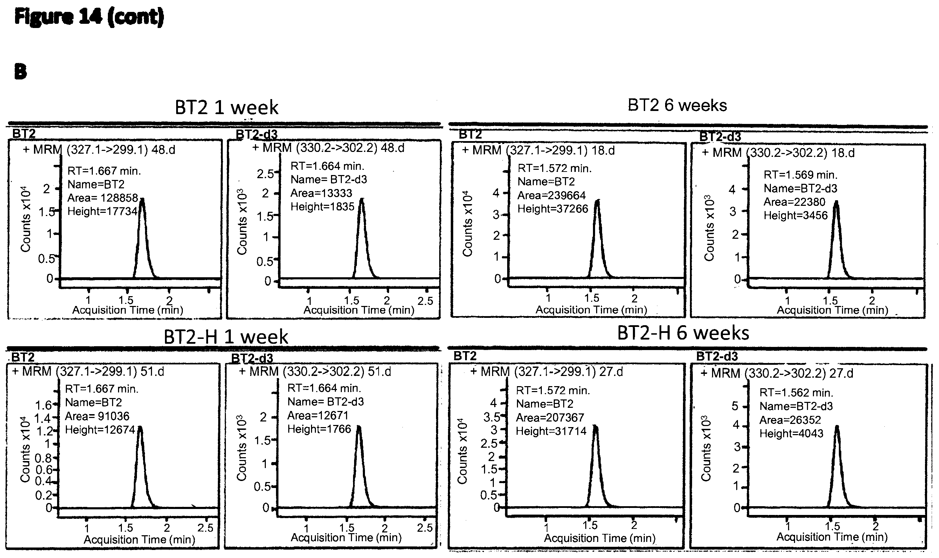

Figures 14A-F show that BT2 retains stability and biological activity after boiling or autoclaving. Figures 14A and 14B show RRLC-MS/MS analysis of heat-treated (100°C water bath for 10min, DL20170921-H) or non-heat treated (DL20170921) sonicated formulations of BT2 (in saline containing 0.5% Tween 80 and 0.01% DMSO) was performed in triplicate 1 or 6 weeks after preparation of the formulation. Representative chromatograms (deuterated (d3)-BT2 controls shown at *right* in each set) are shown.

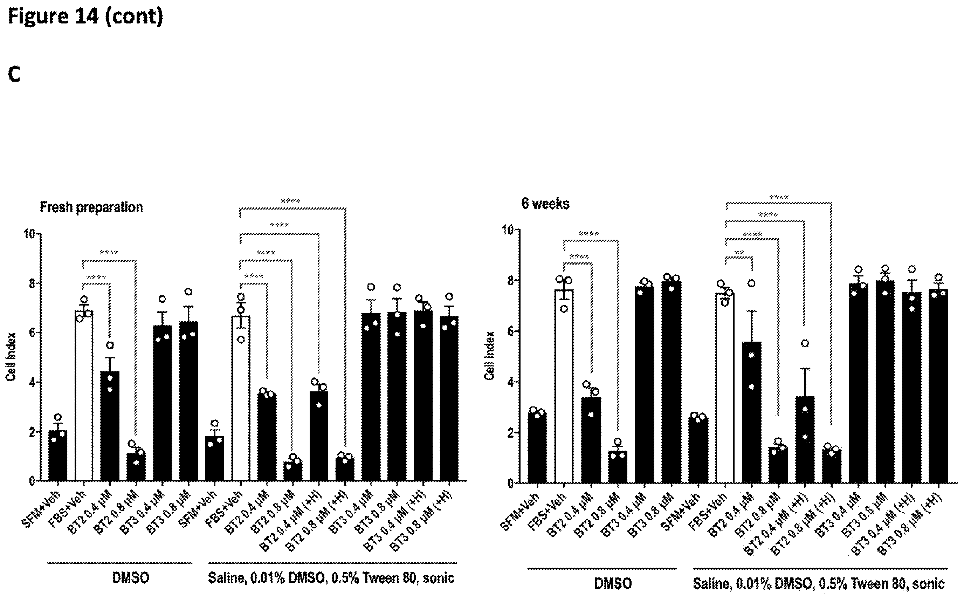

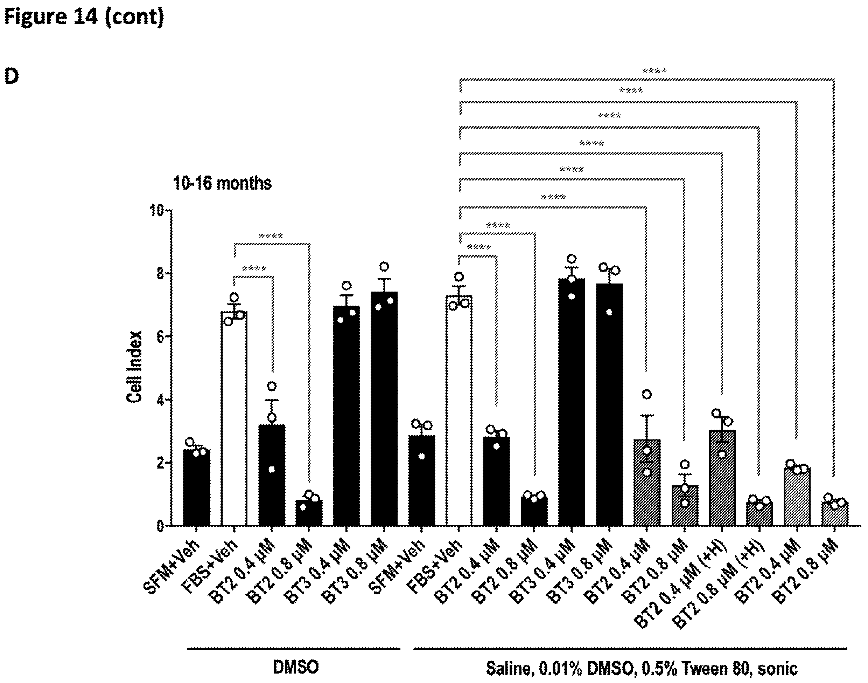

Figures 14C and 14D show tubes containing BT2 or BT3 in vehicle (saline containing 0.01% DMSO and 0.5% Tween 80, sonicated) were kept at 22°C (non heat-treated) or placed in a 100°C water bath for 10min then allowed to cool to 22°C (heat-treated, +H) and freshly used or stored in the dark for 6 weeks or at least 10 months (D, black bars represent 11 months; blue bars represent 10 months; red bars represent 16 months). Serum-deprived HMEC-1 were treated with heat-treated or non heat-treated BT2 or BT3 (0.4, 0.8µM) in medium containing 5% FBS and proliferation monitored using the xCELLigence system (Roche). Data represents the mean±SEM of the means of 3 independent experiments after 79h. Statistical significance was assessed by one-way ANOVA.

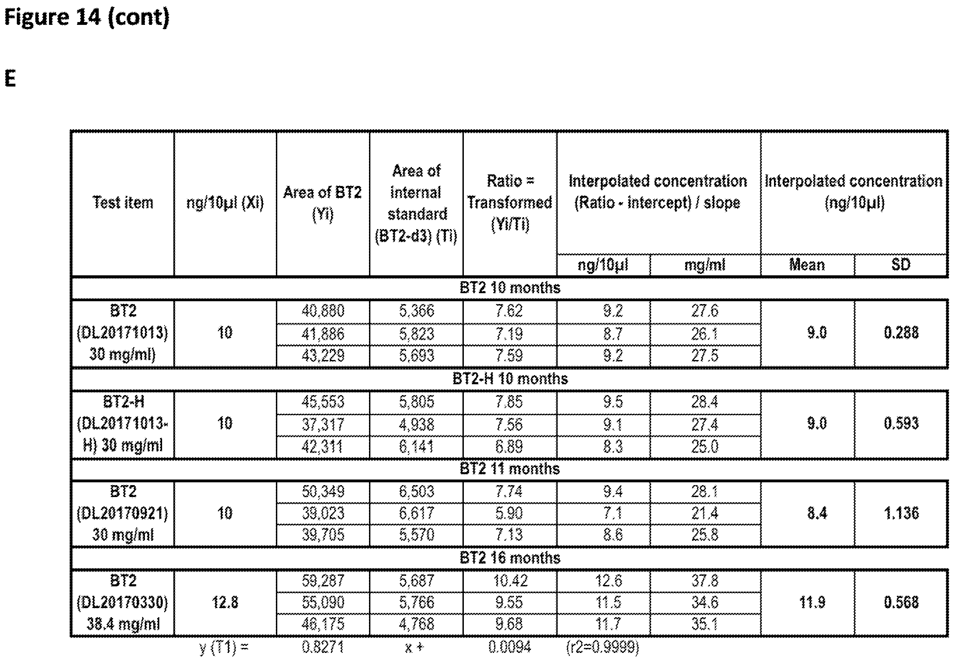

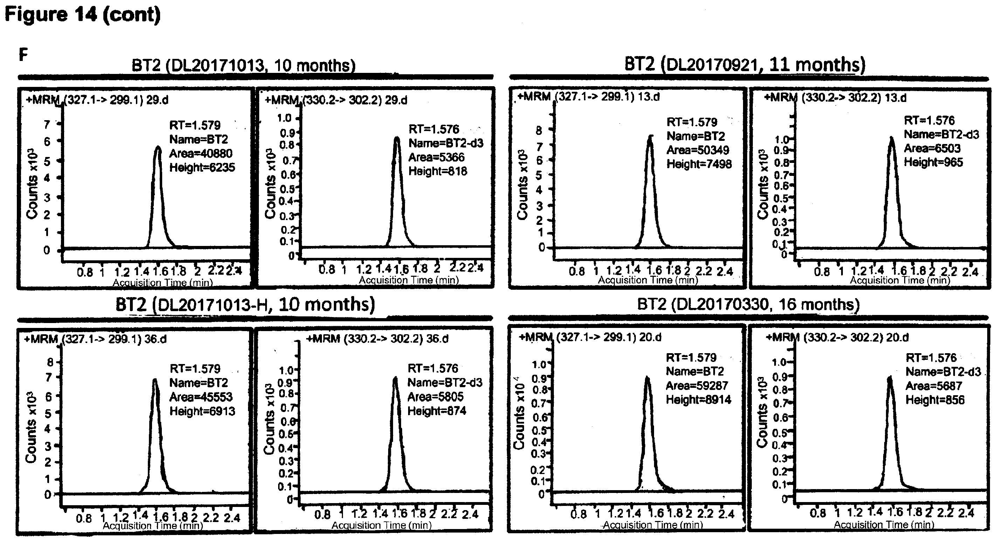

Figures 14E and 14F show RRLC-MS/MS analysis of heat-treated (100°C for 10min) or non-heat treated sonicated formulations of BT2 (in saline containing 0.5% Tween 80 and 0.01% DMSO) was performed in triplicate 10, 11 or 16 months after preparation of the formulation. Representative chromatograms are shown.

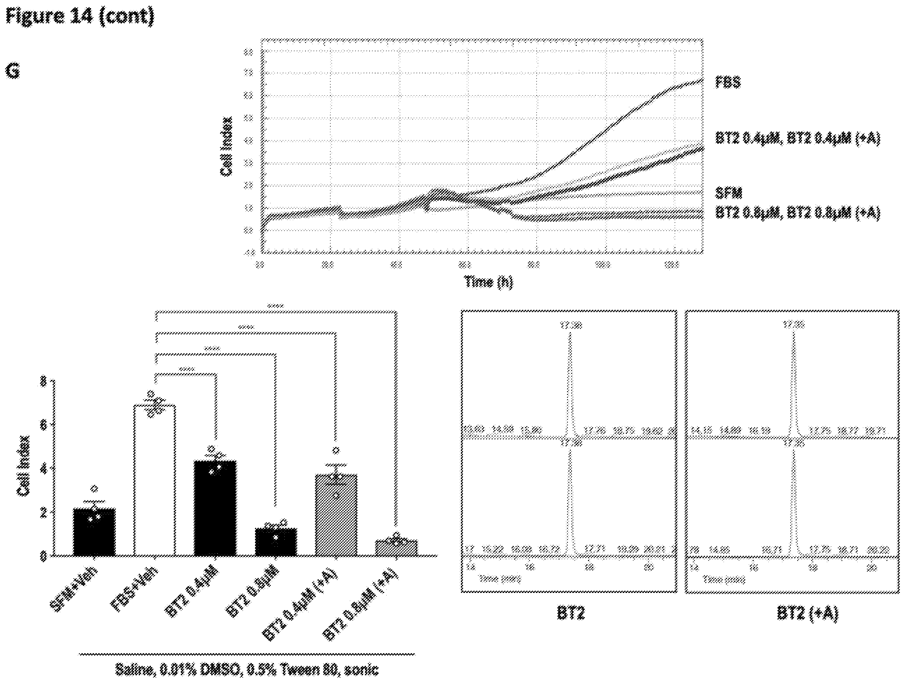

Figure 14G shows tubes containing BT2 in vehicle (saline containing 0.01% DMSO and 0.5% Tween 80, sonicated) that were freshly used or autoclaved (121°C, 15 psi, 20min; +A) and stored in the dark for 4 months (orange bars). Serum-deprived HMEC-1 were treated with autoclaved or freshly used BT2 (0.4, 0.8µM) in medium containing 5% FBS and proliferation monitored using the xCELLigence system (Roche). Proliferation data represents the mean±SEM of the means of 4 independent experiments after 79h. Statistical significance was assessed by one-way ANOVA. Also shown is LC/MS analysis of BT2 freshly prepared or BT2 autoclaved and stored in the dark for 4 months. Figure shows total ion chromatogram integrating peak intensities of each spectrum (*upper,* in black) and extracted ion chromatogram integrating peak intensities of protonated precursor (m/z 327.1319-327.1361) (*lower,* in brown).
Table 3 provides genes induced by IL-1β (logFC ≥2) relative to control (UT) (Table 3C) and inhibited by BT2 (logFC ≥2) relative to IL-1β (Table 3A). Table 3B shows genes induced by IL-1β and inhibited by BT2. RNA-seq was performed with total RNA prepared from HMEC-1 treated with 30µM BT2 and 4h incubation with 20ng/ml IL-1β. These data are sourced from the same experiment represented elsewhere by heatmaps.

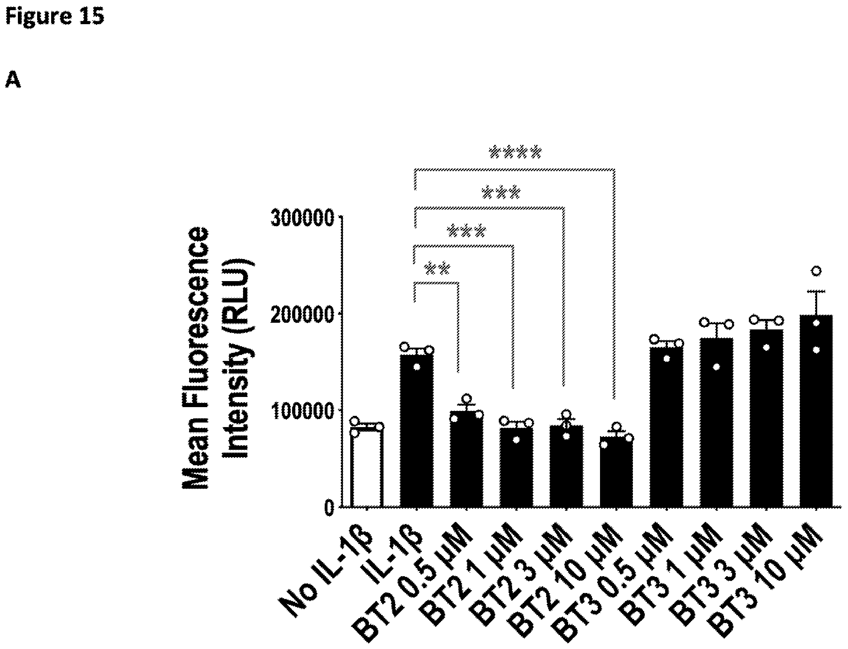

Figure 15A is a graph showing the effect of various concentrations of BT2 and BT3 on monocytic cell adhesion to IL-1β-treated endothelium *in vitro.* THP-1 adhesion to HMEC *in vitro* was assessed by first treating HMEC with various concentrations of BT2 or BT3 for 1h in 96-well plates. HMEC were stimulated with 20ng/ml IL-1β for 1h. Fluorescence intensity of calcein labeled THP-1 that adhered to HMEC monolayers 30 min after adding the cells was then measured via fluorescent plate reader. Data is representative of 3 experiments and expressed as mean ± SEM. Statistical significance was assessed by one-way ANOVA.

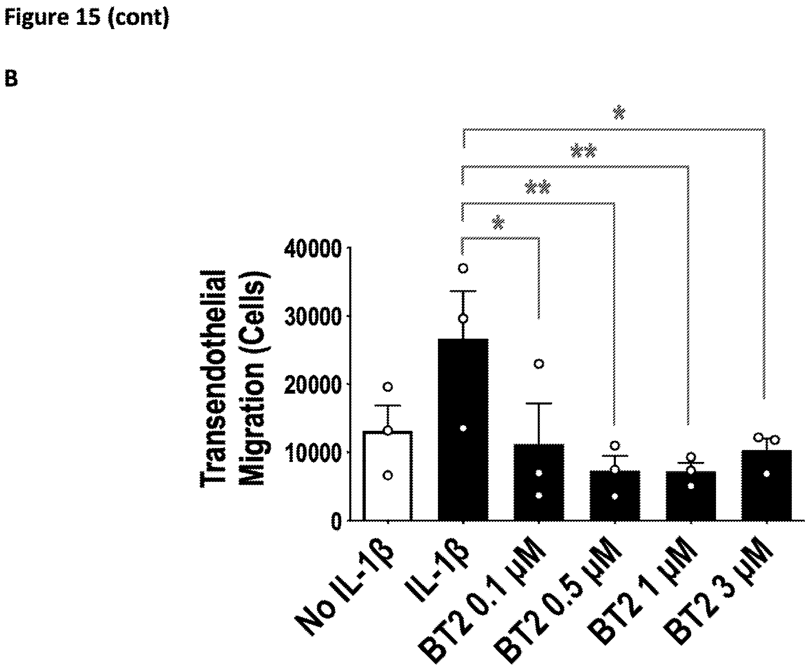

Figure 15B is a graph showing the effect of various concentrations of BT2 on monocytic transendothelial cell migration toward MCP-1 *in vitro.* THP-1 transendothelial cell migration *in vitro* was assessed by treating HMEC with various concentrations of BT2 for 1h in gelatin-coated culture inserts for 1h. HMEC were treated with 20 ng/ml IL-1β for 1h. THP-1 cells that had undergone transendothelial migration toward MCP-1 after 24h was measured using a Coulter counter. Data is representative of 3 experiments and expressed as mean ± SEM. Statistical significance was assessed by one-way ANOVA.

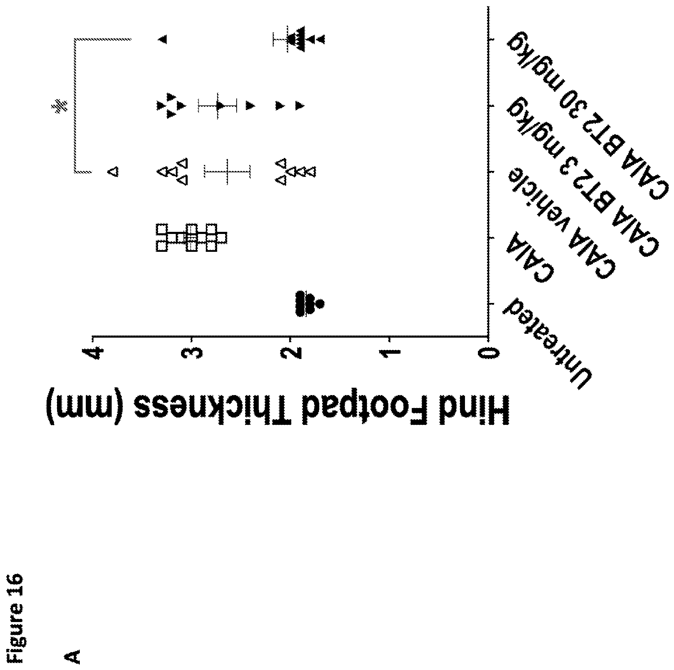

Figure 16A provides a graph showing the effect of vehicle or BT2 at 3mg/kg or 30 mg/kg on hindfoot thickness in a collagen antibody induced arthritic mouse model. Animals were injected i.p. with antibody cocktail on Day 0 with LPS plus BT2 (3 or 30 mg/kg in vehicle) i.p. on Day 3. Hind footpad thickness was measured using digital calipers on Day 9. Data expressed as the hind footpad thickness (mm) of each limb (left and right). n=8-10 per group. Data expressed as mean ± SEM. Statistical significance was assessed by Kruskal-Wallis multiple comparisons test.

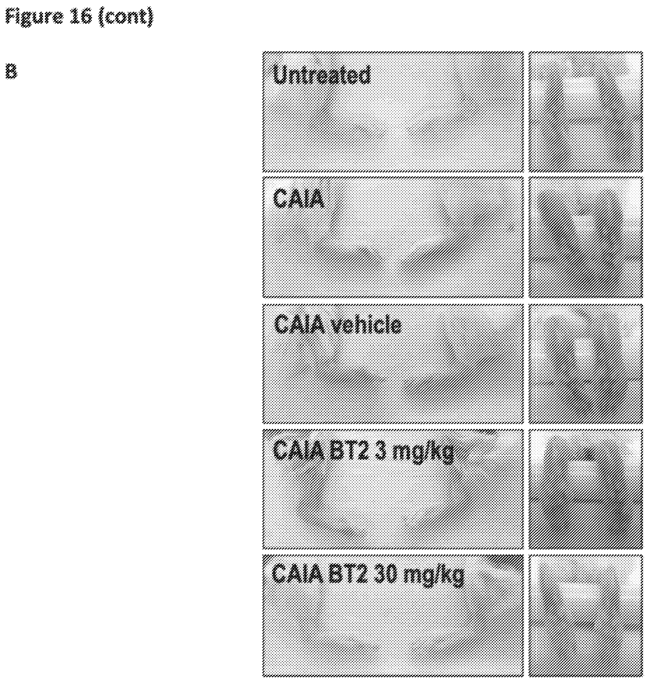

Figure 16B provides images showing the effect of vehicle or BT2 on hindfoot thickness in a collagen antibody induced arthritic mouse model at Day 14 (gross specimens).

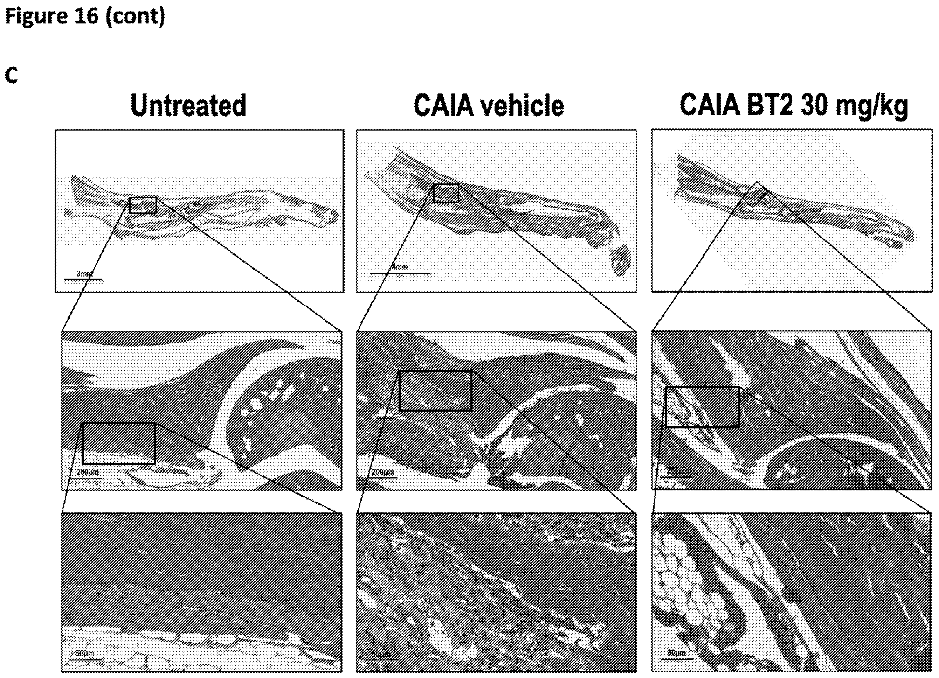

Figure 16C provides images showing H&E staining of mouse footpads following no treatment, or treatment of mice with vehicle or BT2 in a collagen antibody induced arthritic mouse model at Day 14.

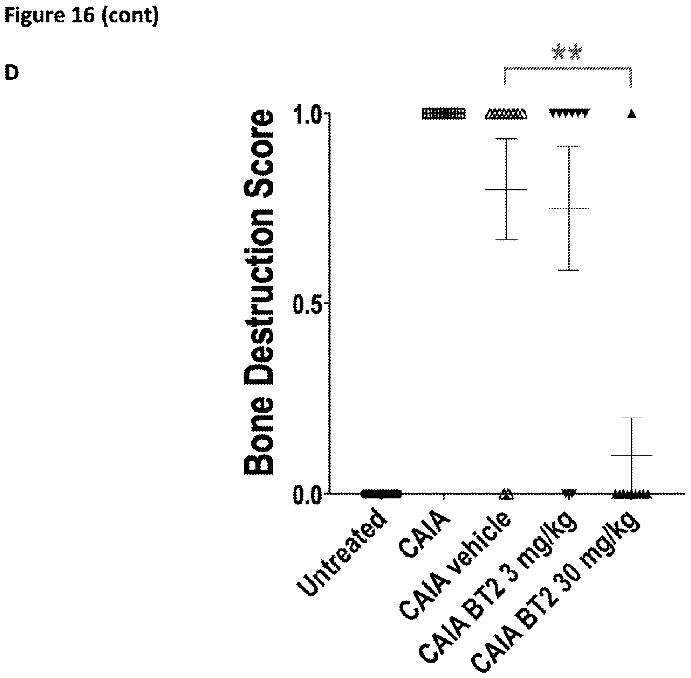

Figure 16D provides a graph showing the effect of no treatment, or treatment with vehicle or BT2 on bone destruction in a collagen antibody induced arthritic mouse model. 3D Micro-CT analysis of Day 14 hind limbs was quantified where a score of 0=no bone destruction and 1=destruction was given to each individual limb. Data is expressed as mean bone destruction score per hind limb (left and right) ± SEM. n=8-10 per group. Statistical significance was assessed using Firth's penalized likelihood method test.

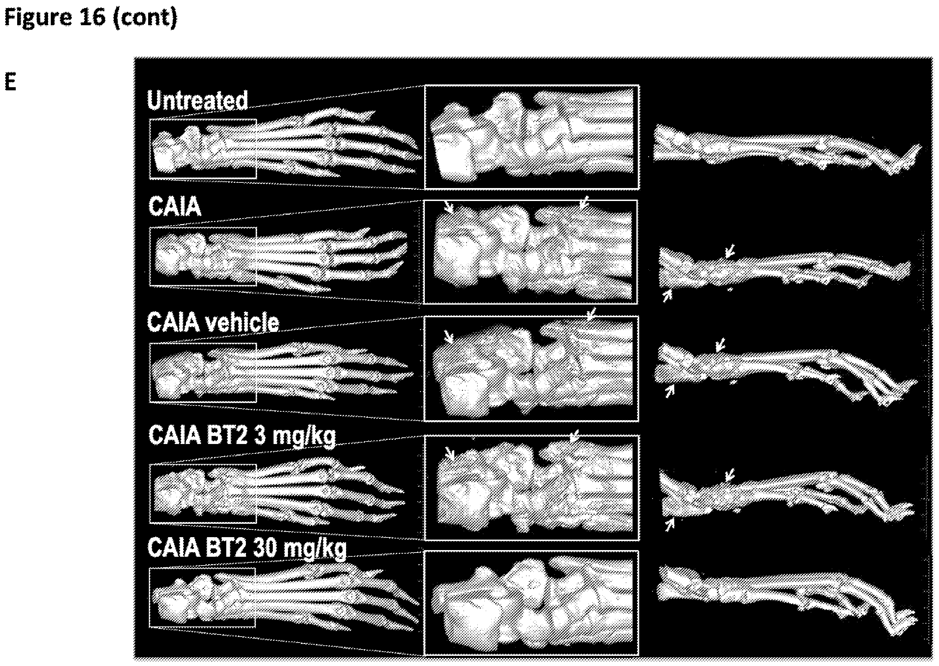

Figure 16E shows Micro-CT images of Day 14 hind limbs in a collagen antibody induced arthritic mouse model following no treatment, or treatment with vehicle or BT2. Arrows denote bone erosion and/or remodeling.

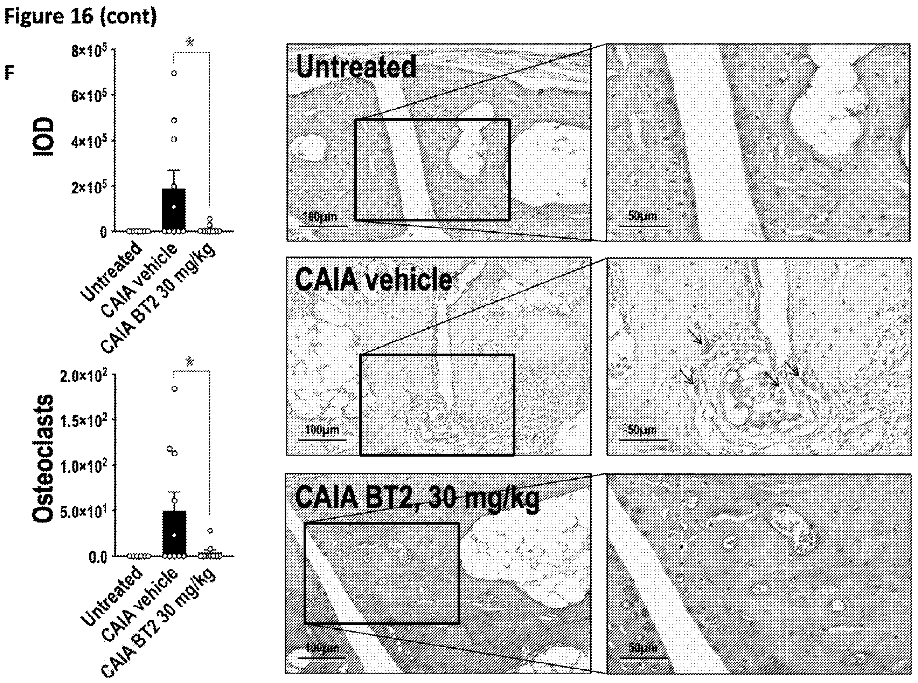

Figure 16F shows graphs and images showing tartrate-resistant acid phosphatase (TRAP) staining in Day 14 hind limb osteoclasts from joints of a collagen antibody induced arthritic mouse model untreated, or treated with vehicle or BT2. Arrows provide examples of positive staining. Slides were photographed under 20x or 40x objectives. IOD of positive staining (red chromogen) was assessed using Image-Pro Plus software. Alternatively numbers of osteoclasts were counted using NIH Image J. Data represents the mean ± SEM of the means. Statistical significance was assessed by Wilcoxon signed-rank test. n=6-10 per group.

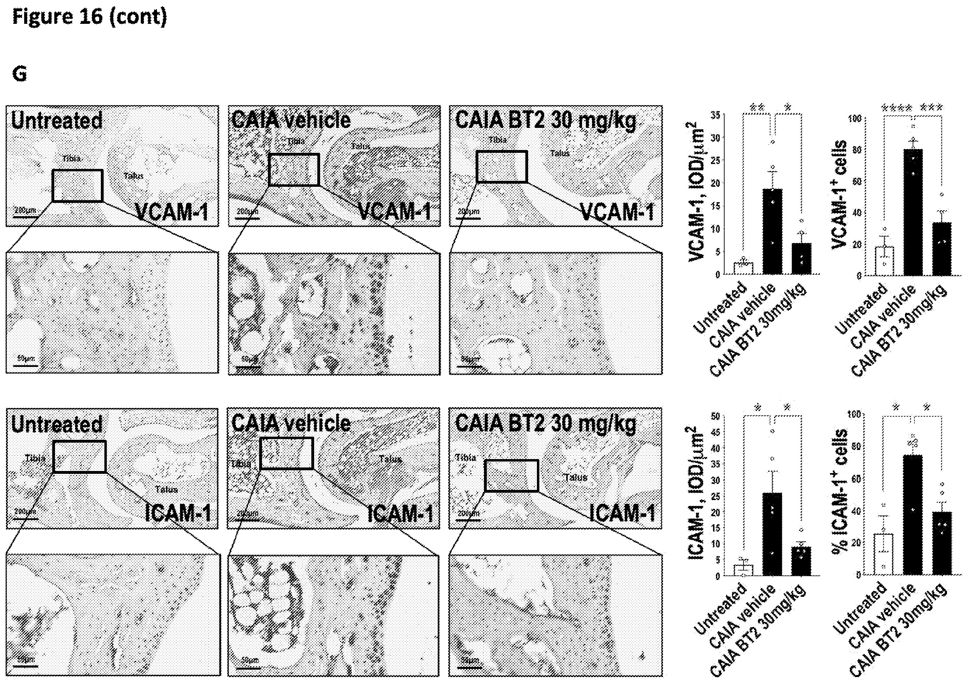

Figure 16G shows immunohistochemical staining for VCAM-1 or ICAM-1 in Day 14 hind limbs. IOD/µm² under 20x objective was assessed using Image-Pro Plus software. Data represents the mean ± SEM of the means. n=3-5 per group. Statistical significance was assessed by one-way ANOVA.

### DETAILED DESCRIPTION

AP-1 is a transcription factor that regulates gene expression in response to a range of pathologic stimuli including cytokines, growth factors, stress, and viral and bacterial infection. AP-1 is a heterodimer formed through the dimerization of proteins belonging to the c-Fos, c-Jun, ATF (activating transcription factor) and/or JDP (Jun dimerization protein 2) protein families. AP-1 family member c-*fos* and c-*jun* expression and DNA binding activity has been observed in human rheumatoid synovium and is associated with disease activity, and have been shown to regulate gene products implicated in angiogenesis, while IL-1β is a mediator of bone and cartilage damage in rheumatoid arthritis. Further, AP-1 factors are expressed in retinal cells after retinal detachment and are elevated in diabetic human retina. AP-1 therefore represents an important therapeutic target for a range of diseases.

As described in the Examples, the inventor has identified and synthesised compounds of formula II having the ability to inhibit AP-1 dependent gene expression. The inventor has further found that these compounds inhibit phosphorylation of ERK1/2, and therefore inhibit ERK1/2-dependent gene expression.

As further described in the Examples, the inventor has shown that compounds of formula II inhibit: serum-inducible endothelial cell proliferation and migration; endothelial wound repair after *in vitro* injury; and microtubule formation on reconstituted basement membrane matrix. The inventor has further found that these compounds inhibit FosB/ΔFosB and c-Fos expression.

As described in the Examples, the inventor has further found that compound BT2 (a compound of formula II), in addition to inhibiting FosB/ΔFosB expression, inhibits phosphorylation of ERK1 and ERK2 (ERK1/2), and inhibits VCAM-1 expression, and VEGF-A expression.

VCAM-1 (also known as CD106) is a cell adhesion molecule expressed on blood vessels following stimulation with cytokines. In particular, VCAM-1 is upregulated in endothelial cells in response to stimulation with, for example, TNF-alpha or IL-1β.

As used herein, an inhibitor of FosB/ΔFosB expression is a compound or agent which reduces the amount of FosB/ΔFosB protein produced by a cell or tissue following contact with the compound or agent relative to the amount of FosB/ΔFosB protein produced by a cell or tissue which has not been contacted with the compound or agent. An inhibitor of ERK1/2 phosphorylation is a compound or agent which reduces the extent of ERK1/2 phosphorylation in a cell or tissue following contact with the compound or agent relative to the extent of ERK1/2 phosphorylation in a cell or tissue that has not been contacted with the compound or agent. An inhibitor of VCAM-1 expression is a compound or agent which reduces the amount of VCAM-1 protein produced by a cell or tissue following contact with the compound or agent relative to the amount of VCAM-1 protein produced by a cell or tissue which has not been contacted with the compound or agent. An inhibitor of VEGF-A expression is a compound or agent which reduces the amount of VEGF-A, typically VEGF-A₁₆₅, protein produced by a cell or tissue following contact with the compound or agent relative to the amount of VEGF-A protein produced by a cell or tissue which has not been contacted with the compound or agent.

A first aspect provides a compound of formula (II), or a pharmaceutically acceptable salt thereof: 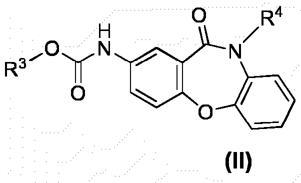 wherein:
R³ is straight or branched C₁-C₆ alkyl; and
R⁴ is straight or branched C₁-C₆ alkyl,
or R⁴ is 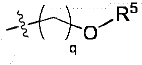 wherein q is 1, 2, 3 or 4; and R⁵ is straight or branched C₁-C₆ alkyl, for use in treating a disease or condition associated with vascular permeability, neovascularisation, angiogenesis, inflammation, cell migration and/or cell proliferation in the subject, wherein the disease or condition is selected from the group consisting of
   - arthritis;
   - rheumatoid arthritis;
   - bone destruction;
   - age-related macular degeneration;
   - diabetic retinopathy;
   - macular edema;
   - vascular leakage;
   - vascular permeability;
   - retinal vascular permeability;
   - angiogenesis;
   - endothelial cell dysfunction;
   - atherosclerosis;
   - stroke;
   - myocardial infarction;
   - peripheral vascular disease;
   - stenosis;
   - restenosis;
   - inflammation;
   - cytokine storm;
   - pulmonary inflammation; and
   - pulmonary fibrosis.

A second aspect provides a pharmaceutical composition comprising a compound of formula II, or a pharmaceutically acceptable salt thereof, as defined herein, and a pharmaceutically acceptable carrier.

A third aspect provides a compound of formula II, or a pharmaceutically acceptable salt thereof, as defined herein, for use as a medicament.

In some embodiments of formula (II), R³ is straight C₁-C₆ alkyl or branched C₁-C₆ alkyl. In some embodiments of formula (II), R³ is -CH₂CH₃ or -CH₂CH(CH₃)₂.

In some embodiments of formula (II), R⁴ is straight C₁-C₆ alkyl or branched C₁-C₆ alkyl. In some embodiments of formula (II), R⁴ is -CH₂CH₃ or -CH₂CH(CH₃)₂.

In some embodiments of formula (II), R⁴ is 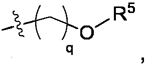 wherein q is 1, 2, 3 or 4; and R⁵ is straight C₁-C₆ alkyl or branched C₁-C₆ alkyl. In some embodiments of formula (II), q is 2. In some embodiments of formula (II), R⁵ is -CH₃. In some embodiments of formula (II), q is 2 and R⁵ is -CH₃.

In some embodiments, the compound of formula (II) may be a compound of formula (II-1): 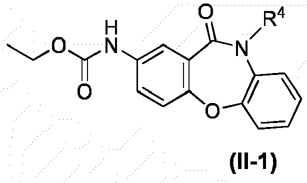 wherein:
R⁴ is straight or branched C₁-C₆ alkyl;
or R⁴ is: 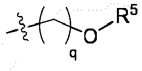 wherein q is 1, 2, 3 or 4; and R⁵ is straight or branched C₁-C₆ alkyl.

For example, the compound of formula (II-1) may be selected from: 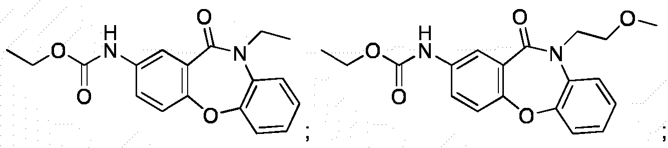 and

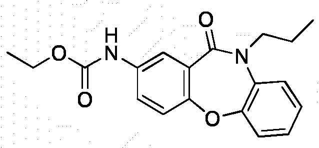

In some unclaimed yet disclosed embodiments, the compound of formula (II) may be a compound of formula (II-2): 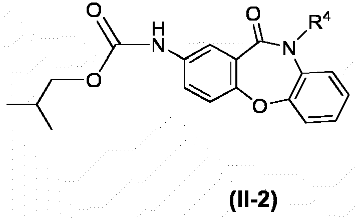 wherein:
R⁴ is straight or branched C₁-C₆ alkyl;
or R⁴ is: 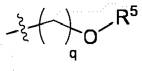 wherein q is 1, 2, 3 or 4; and R⁵ is straight or branched C₁-C₆ alkyl.

For example, the compound of formula (II-2) may be:

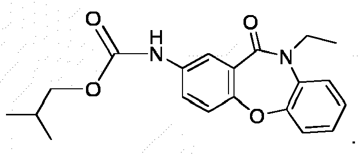

In one embodiment, the compound of formula (II) is:

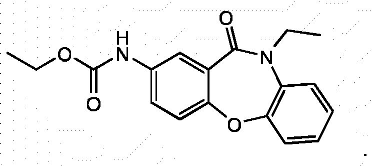

### (also referred to herein as BT2)

Examples of pharmaceutically acceptable salts include salts of pharmaceutically acceptable cations such as sodium, potassium, lithium, calcium, magnesium, ammonium and alkylammonium; acid addition salts of pharmaceutically acceptable inorganic acids such as hydrochloric, orthophosphoric, sulphuric, phosphoric, nitric, carbonic, boric, sulfamic and hydrobromic acids; or salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, trihaloacetic (e.g. trifluoroacetic), methanesulphonic, trihalomethanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

In one unclaimed yet disclosed embodiment, the compound of formula II, or a pharmaceutically acceptable salt thereof, is deuterated.

In one unclaimed yet disclosed embodiment, the compound of formula II, or a pharmaceutically acceptable salt thereof, is an E isomer.

In one unclaimed yet disclosed embodiment, the compound of formula II, or a pharmaceutically acceptable salt thereof, is a Z isomer.

In one unclaimed yet disclosed embodiment, the compound of formula II, or a pharmaceutically acceptable salt thereof, is a mixture of an E isomer and a Z isomer.

Described herein is a pharmaceutical composition comprising a compound of formula II, or a pharmaceutically acceptable salt thereof.

I

The pharmaceutical composition of the present invention may be used in the uses of the invention described herein.

The pharmaceutically composition typically comprises a pharmaceutically acceptable carrier.

The compounds of formula II may be used to treat any diseases or conditions mediated by AP-1 and/or ERK1/2 and/or FosB/ΔFosB, and/or VCAM-1, and/or VEGF-A, and/or IL-1β. A disease or condition is mediated by a protein or protein complex if activity of that protein or protein complex is required for development of, and/or maintaining, the disease or condition.

The compounds of formula II may be used to treat or prevent diseases or conditions associated with vascular permeability, neovascularisation, angiogenesis, inflammation, cell migration and/or cell proliferation.

As described in the Examples, the inventor has shown that administration of compound BT2 inhibits or reduces vascular permeability induced by VEGFA₁₆₅, and inhibits or reduces laser induced vascular leakiness in the eye. Further, the inventor has shown that administration of BT2 reduces inflammation and bone destruction in a collagen antibody-induced arthritis model.

Described herein is a pharmaceutical composition comprising a compound of formula II, or a pharmaceutically acceptable salt thereof, and a pharmaceutically acceptable carrier.

In some unclaimed yet disclosed embodiments, the carrier is a non-naturally occurring carrier.

In some unclaimed yet disclosed embodiments, the compounds described herein or a pharmaceutically acceptable salt thereof may be used in combination with one or more other agents.

It will be understood that the combined administration of a compound described herein or a pharmaceutically acceptable salt thereof with the one or more other agents may be concurrent, sequential or separate administration.

The term "composition" encompasses formulations comprising the active ingredient with conventional carriers and excipients, and also formulations with encapsulating materials as a carrier to provide a capsule in which the active ingredient (with or without other carriers) is surrounded by the encapsulation carrier. In pharmaceutical compositions, the carrier is "pharmaceutically acceptable" meaning that it is compatible with the other ingredients of the composition and is not deleterious to a subject. The pharmaceutical compositions of the present invention may contain other agents or further active agents as described above, and may be formulated, for example, by employing conventional solid or liquid vehicles or diluents, as well as pharmaceutical additives of a type appropriate to the mode of desired administration (for example, excipients, binders, preservatives, stabilizers, flavours, etc.) according to techniques such as those known in the art of pharmaceutical formulation (See, for example, Remington: The Science and Practice of Pharmacy, 21st Ed., 2005, Lippincott Williams & Wilkins).

The pharmaceutical composition may be suitable for intravitreal, oral, rectal, nasal, topical (including dermal, buccal and sub-lingual), vaginal or parenteral (including intramuscular, sub-cutaneous and intravenous) administration or in a form suitable for administration by inhalation or insufflation.

The compounds described herein or a pharmaceutically acceptable salt thereof, together with a pharmaceutically acceptable carrier, may thus be placed into the form of pharmaceutical compositions and unit dosages thereof. The pharmaceutical composition may be a solid, such as a tablet or filled capsule, or a liquid such as solution, suspension, emulsion, elixir, or capsule filled with the same, for oral administration. The pharmaceutical composition may be a liquid such as solution, suspension, or emulsion, for intravitreal administration. The pharmaceutical composition may also be in the form of suppositories for rectal administration or in the form of sterile injectable solutions for parenteral (including subcutaneous) use.

Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

For preparing pharmaceutical compositions from the compounds described herein, pharmaceutically acceptable carriers can be either solid or liquid. Solid form preparations include powders, tablets, pills, capsules, cachets, lozenes (solid or chewable), suppositories, and dispensable granules. A solid carrier can be one or more substances which may also act as diluents, flavouring agents, solubilisers, lubricants, suspending agents, binders, preservatives, tablet disintegrating agents, or an encapsulating material.

Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Tablets, powders, capsules, pills, cachets, and lozenges can be used as solid forms suitable for oral administration.

Liquid form preparations include solutions, suspensions, and emulsions, for example, water or water-propylene glycol solutions. For example, parenteral injection liquid preparations can be formulated as solutions in aqueous polyethylene glycol solution.

Sterile liquid form compositions include sterile solutions, suspensions, emulsions, syrups and elixirs. The active ingredient can be dissolved or suspended in a pharmaceutically acceptable carrier, such as sterile water, sterile organic solvent or a mixture of both.

The pharmaceutical compositions according to the present invention may thus be formulated for parenteral administration (e. g. by injection, for example bolus injection or continuous infusion) and may be presented in unit dose form in ampoules, pre-filled syringes, small volume infusion or in multi-dose containers with an added preservative. The pharmaceutical compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilisation from solution, for constitution with a suitable vehicle, e.g. sterile, pyrogen-free water, before use.

Pharmaceutical forms suitable for injectable use include sterile injectable solutions or dispersions, and sterile powders for the extemporaneous preparation of sterile injectable solutions. They should be stable under the conditions of manufacture and storage and may be preserved against oxidation and the contaminating action of microorganisms such as bacteria or fungi.

The solvent or dispersion medium for the injectable solution or dispersion may contain any of the conventional solvent or carrier systems for injectable solutions or dispersions, and may contain, for example, water, ethanol, polyol (for example, glycerol, propylene glycol and liquid polyethylene glycol, and the like), suitable mixtures thereof, and vegetable oils.

Pharmaceutical forms suitable for injectable use may be delivered by any appropriate route including intravenous, intramuscular, intracerebral, intrathecal, epidural injection or infusion.

Sterile injectable solutions are prepared by incorporating the active ingredient in the required amount in the appropriate solvent with various other ingredients such as those enumerated above, as required, followed by filtered sterilization. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, preferred methods of preparation are vacuum drying or freeze-drying of a previously sterile-filtered solution of the active ingredient plus any additional desired ingredients.

The compounds described herein may be formulated into compositions suitable for oral administration, for example, with an assimilable edible carrier, or enclosed in hard or soft shell gelatin capsule, or compressed into tablets, or incorporated directly with the food of the diet. For oral therapeutic administration, the active compound may be incorporated with excipients and used in the form of ingestible tablets, buccal tablets, troches, capsules, elixirs, suspensions, syrups, wafers, and the like.

The amount of active compound in therapeutically useful compositions should be sufficient that a suitable dosage will be obtained.

The tablets, troches, pills, capsules, lozenges, implants and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavouring agent such as peppermint, oil of wintergreen, or cherry flavouring. When the dosage unit form is a capsule, it may contain, in addition to materials of the above type, a liquid carrier.

Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active ingredient(s) may be incorporated into sustained-release preparations and formulations, including those that allow specific delivery of the active ingredient to specific regions of the gut.

Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well-known suspending agents.

Pharmaceutically acceptable carriers include any and all pharmaceutically acceptable solvents, dispersion media, coatings, antibacterial and antifungal agents, isotonic and absorption delaying agents and the like.

Also included are solid form preparations that are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilising agents, and the like.

For topical administration, the compounds described herein may be formulated as an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Formulations suitable for topical administration in the mouth include lozenges comprising active agent in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions for nasal administration may be applied directly to the nasal cavity by conventional means, for example with a dropper, pipette or spray. The formulations may be provided in single or multidose form. In the case of a dropper or pipette, this may be achieved by the patient administering an appropriate, predetermined volume of the solution or suspension. In the case of a spray, this may be achieved for example by means of a metering atomising spray pump. To improve nasal delivery and retention the compounds of the invention may be encapsulated with cyclodextrins, or formulated with other agents expected to enhance delivery and retention in the nasal mucosa.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas.

The aerosol may conveniently also contain a surfactant such as lecithin. The dose of the active ingredient may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g. gelatin, or blister packs from which the powder may be administered by means of an inhaler.

In formulations intended for administration to the respiratory tract, including intranasal formulations, the active ingredient will generally have a small particle size for example of the order of 5 to 10 microns or less. Such a particle size may be obtained by means known in the art, for example by micronisation.

The compounds described herein can be formulated into compositions for ocular, intraocular, intravitreal or subconjunctival injection. The compounds described herein may be formulated for administration by means of eye drops, contact lens or an implant. Implants may be injected intravitreally into the eye. The implant may allow delivering constant therapeutic levels of the compound. Such slow release implants are typically made with a pelleted compound core surrounded by nonreactive substances such as silicon, ethylene vinyl acetate (EVA), or polyvinyl alcohol (PVA); these implants are nonbiodegradable and can deliver continuous amounts of a compound for months to years. Matrix implants may also be used. They are typically used to deliver a loading dose followed by tapering doses of the compound during a 1-day to 6-month time period. They are most commonly made from the copolymers poly-lactic-acid (PLA) and/or poly-lactic-glycolic acid (PLGA), which degrade to water and carbon dioxide.

Formulations for intravitreal administration may be formulated as aqueous base containing one or more emulsifying agents, stabilising agents, dispersing agents, penetrating agents, or suspending agents.

When desired, formulations adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

It is especially advantageous to formulate parenteral compositions in dosage unit form for ease of administration and uniformity of dosage. Parental compositions may be in the form of physically discrete units suited as unitary dosages for the subjects to be treated, each unit containing a predetermined quantity of the active ingredient calculated to produce the desired therapeutic effect in association a pharmaceutical carrier.

The compounds may also be administered in the absence of carrier where the compounds are in unit dosage form.

The term "effective amount" refers to the amount of a compound effective to achieve the desired response.

An effective amount of a compound described herein, or a pharmaceutically acceptable salt thereof, can be determined by a person skilled in the art having regard to the particular compound.

It will be understood that the specific dose level and frequency of dosage for any particular subject may be varied and will depend upon a variety of factors including the activity of the specific compound employed, the metabolic stability and length of action of that compound, the age, body weight, general health, sex and diet of the subject, the mode and time of administration, rate of excretion, drug combinations, and the severity of the particular condition.

Suitable dosages of the compounds described herein or further active agents administered in combination with compounds described herein can be readily determined by a person skilled in the art having regard to the particular compound of the invention or further active agent selected.

It will further be understood that when the compounds described herein are to be administered in combination with one or more agents, or other active agents, the dosage forms and levels may be formulated for either concurrent, sequential or separate administration or a combination thereof.

The methods of the present invention are intended for use with any subject that may experience the benefits of the methods of the invention. Thus, the term "subject" includes humans as well as non-human mammals. The subject may, for example, be a domestic animal, zoo animal or livestock.

The inventor also envisages that the compounds of formula II can be used for inhibition of AP-1-dependent gene expression and/or ERK1/2-dependent gene expression and/or ERK1/2 phosphorylation, and/or FosB/ΔFosB expression and/or VCAM-1 expression and/or VEGF-A expression *in vitro,* in, for example, laboratory applications.

Unless otherwise herein defined, the following terms will be understood to have the general meanings which follow. The terms referred to below have the general meanings which follow when the term is used alone and when the term is used in combination with other terms, unless otherwise indicated. Hence, for example, the definition of "alkyl" applies to "alkyl" as well as the "alkyl" portions of "haloalkyl", "heteroalkyl", "arylalkyl" etc.

The term "alkyl" refers to a straight chain or branched chain saturated hydrocarbyl group. Unless indicated otherwise, preferred are C₁₋₆alkyl and C₁₋₄alkyl groups. The term "C_{x-y}alkyl", where x and y are integers, refers to an alkyl group having x to y carbon atoms. For example, the term "C₁₋₆alkyl" refers to an alkyl group having 1 to 6 carbon atoms. Examples of C₁₋₆alkyl include methyl (Me), ethyl (Et), propyl (Pr), isopropyl (i-Pr), butyl (Bu), isobutyl (i-Bu), sec-butyl (s-Bu), tert-butyl (t-Bu), pentyl, neopentyl, hexyl and the like. Unless the context requires otherwise, the term "alkyl" also encompasses alkyl groups containing one less hydrogen atom such that the group is attached via two positions, i.e. divalent.

As used herein, "treating" means affecting a subject, tissue or cell to obtain a desired pharmacological and/or physiological effect and includes inhibiting the condition, i.e. arresting its development; or relieving or ameliorating the effects of the condition i.e., cause reversal or regression of the effects of the condition. As used herein, "preventing" means preventing a condition from occurring in a cell or subject that may be at risk of having the condition, but does not necessarily mean that condition will not eventually develop, or that a subject will not eventually develop a condition. Preventing includes delaying the onset of a condition in a cell or subject.

The term "effective amount" refers to the amount of the compound that will elicit the biological or medical response of a tissue, system, animal or human that is being sought by the researcher, veterinarian, medical doctor or other clinician.

**Table 1. Compounds referred to herein.**

| **Com poun d name** | **Chemical name** | **Chemical structure** |
|---|---|---|
| BT2 | (10-ethyl-11-oxo-10,11-dihydro-dibenzo[b,f][1,4]oxazepin-2-yl)-carbamic acid ethyl ester (CAS 922029-50-3) | 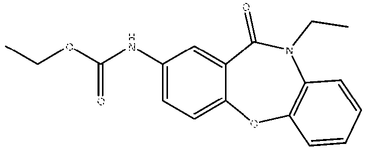 |
| BT3 | 2-amino-10-ethyldibenzo[b,f][1,4] oxazepin-11 (10H)-one (CAS 23474-61-5) | 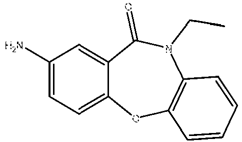 |
| CpdX / CpdB / LK00 1 | 2-methoxyethyl[[[4-(4-chlorobenzoyl)phenyl]amino]ca rbonyl]carbamate (CAS 351068-74-1) | 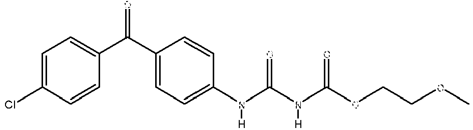 |
| T4 | 2-Methoxyethyl[[[4-(4-chlorophenyl)(hydroxyimino)m ethyl) | 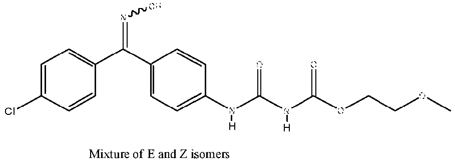 |
| | phenyl]amino]carbonyl]carbam ate | |
| T6 | Flubendazole (CAS 31430-15-6) | 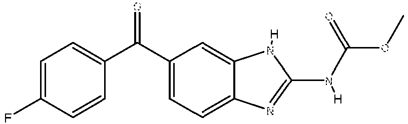 |
| T7 | (4-aminophenyl)(4-fluorophenyl)methanone (CAS 10055-40-0) | 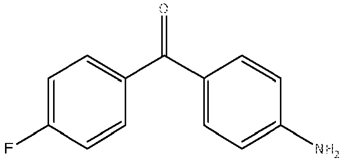 |
| BT2-MeO A | N-(10-ethyl-11-oxo-10,11-dihydrodibenzo[b,f][1,4]oxazep in-2-yl)-2-methoxyacetamide (CAS 922029-59-2) | 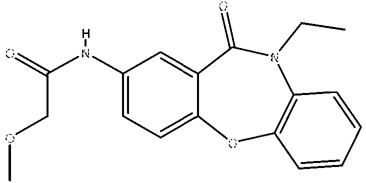 |
| BT2-EOM e | ethyl (10-(2-methoxyethyl)-11-oxo-10,11-dihydrodibenzo[b,f][1,4]oxazep in-2-yl)carbamate | 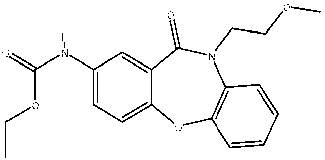 |
| BT2-Pr | ethyl (11-oxo-10-propyl-10,11-dihydrodibenzo[b,f][1,4]oxazep in-2-yl)carbamate (CAS 922029-50-3) | 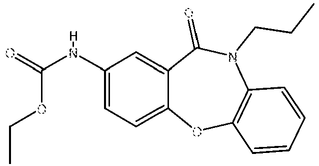 |
| BT2-IC | isobutyl (10-ethyl-11-oxo-10,11-dihydrodibenzo[b,f][1,4]oxazep in-2-yl)carbamate | 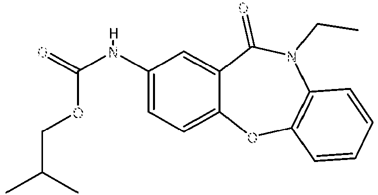 |
| BT2-IMO | ethyl (11-(oxetan-3-ylmethoxy)dibenzo[b,f][1,4]oxa zepin-2-yl)carbamate | 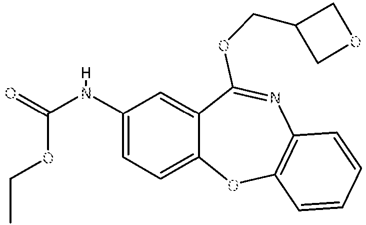 |
| BT2-MO | ethyl (10-(oxetan-3-ylmethyl)-11-oxo-10,11-dihydrodibenzo[b,f][1,4]oxazep in-2-yl)carbamate | 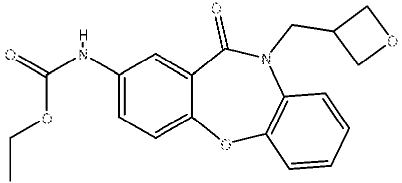 |
| BT2-deut | ethyl (10-ethyl(2',2',2'-d3)-11-oxo-10,11-dihydrodibenzo[b,f][1,4]oxazep in-2-yl)carbamate | 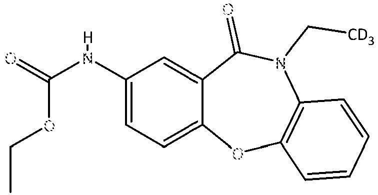 |

The compounds described herein may be synthesised by methods known in the art. The compounds referred to herein as BT2 and T6 are commercially available. For example, BT2 can be purchased from Aurora Building Blocks, USA, or Life Chemicals HTS Compounds, Canada. T6 can be purchased from, for example, Sigma-Aldrich, USA.

The present invention is further described below by reference to the following non-limiting Examples.

### EXAMPLES

Transcription factors, particularly those encoded by immediate-early genes, integrate cues from the extracellular environment with signaling and transcriptional control. While it is clear that transcription factors control disease there are no drugs on the market that directly target such factors (Mapp et al., Nature Chemical Biology 11, 891-894 (2015)). despite encouraging drug development pipelines (Miyoshi, et al., J Invest Dermatol 131, 108-117 (2011); Cho, E.A., et al., The Lancet 381, 1835-1843 (2013)). Basic region-leucine zipper (bZIP) factors comprising AP-1 regulate gene expression in response to a range of pathologic stimuli including cytokines, growth factors, stress and viral and bacterial infection (Hess, et al., Journal of Cell Science 117, 5965-5973 (2004)). AP-1 family members including FosB/ΔFosB (Chen, G., et al., Front Neurosci 11, 112 (2017)) are under the control of mitogen activated protein kinases (MAPK) (Karin, M. J Biol Chem 270, 16483-16486 (1995)) and regulate gene expression in response to a range of pathologic stimuli including cytokines, growth factors, various stresses and viral and bacterial infection (Hess, et al., Journal of Cell Science 117, 5965-5973 (2004)). AP-1 members are elevated in diabetic human retina (Oshitari, T. at el. Current Eye Research 39, 527-531 (2014)) and expressed in retinal cells after retinal detachment (Geller, et al., Invest Ophthalmol Vis Sci 42, 1363-1369 (2001)). AP-1 DNA binding activity has also been observed in human rheumatoid synovium and is associated with disease activity (Asahara, H., et al., Arthritis Rheum 40, 912-918 (1997)) while IL-1β is a known mediator of bone and cartilage damage in RA (Duff, G.W. Cytokines and Rheumatoid Arthritis. in Clinical Applications of Cytokines: Role in Pathogenesis, Diagnosis, and Therapy (eds. Oppenheim, J.J., Rossio, J.L. & Gearing, A.J.H.) (Oxford University Press, Oxfrd, 1993). Attempts have been made to translate AP-1 inhibitors to the clinic, however patient use is hamstrung by the paucity of effective drugs.

We employed a high throughput approach to screen ~100,000 compounds and identified a novel dibenzoxazepinone we termed BT2 which has previously never before been investigated. We found that BT2 inhibits a range of proliferative, migratory angiogenic and inflammatory processes. BT2 directly interacts preferentially with MEK1 and inhibits ERK activation, and suppresses the inducible expression of the AP-1 protein FosB/ΔFosB and that of VCAM-1 and VEGF-A₁₆₅. BT2 abrogates CD31 and tartrate-resistant acid phosphatase (TRAP) staining. BT2 also inhibits retinal vascular leakage in rats and rabbits, and suppresses inflammation and bone destruction in mice. BT2 withstands boiling and remains biologically stable for up to 16 months. Thus, BT2 is a new pharmacologic inhibitor of angiogenesis, vascular permeability and inflammation, and offers a new potential therapeutic tool for nAMD/DR and RA patients.

### Materials and Methods

**High-throughput screen of compound library.** Hits were selected from the ~100,000 compound Lead Discovery Library at the HTS Facility at Walter & Eliza Hall Institute of Medical Research (WEHI, Bundoora, Vic) with a commercially-available human embryonic kidney (HEK)-293 cell-based assay in 384-well microtitre plates in which *Firefly* luciferase was driven by multiple copies of the AP-1 response element (293/AP-1-luc cells, Panomics, Fremont, CA). Briefly, the cell-based assay involved plating 5x10³ cells into 384-well plates in DMEM, pH 7.4 containing 10% FBS. After ~18h, the cells were induced with 10ng/ml 2-O-tetradecanoylphorbol-13-acetate (TPA) (Sigma, St Louis, MO) in the absence or presence of test compound, then after ~18h, luciferase activity was measured using a luminometer. The hit rate of the primary screen was 2.4%. Hits were picked for single point retest in triplicate and 931 test compounds re-confirmed at greater than 50% inhibition. A substructure filter was then applied to remove pan-assay interference compounds (Baell, J.B., et al., J Med Chem 53, 2719-2740 (2010)) and using the most stringent filtering criteria 256 hits were selected for further study. After dose response testing, 24 compounds with molecular weight <400 Da were reordered from suppliers and tested in secondary assays.

**Compound synthesis and purification.** BT2, Cpd B/X/LK001 and structural analogues were synthesized and purified (>95%) at Advanced Molecular Technologies Pty Ltd (Scoresby, Vic) or obtained commercially as indicated below.

*(10-Ethyl-11-oxo-10, 11-dihydro-dibenzo[b,f][1,4]oxazepin-2-yl)-carbamic acid ethyl ester (**BT2**).* Diethyl pyrocarbonate (22.2 ml, 24.43 g, 151 mmol) was added to 2-Amino-10-methyl-10H-dibenzo[b,f][1,4]oxazepin-11-one (BT3) (35.0g, 137mmol) in 100ml of dimethylformamide (DMF), and the mixture stirred for 1h under an atmosphere of nitrogen at 22°C. The solid was filtered and rinsed with ethyl acetate (EtOAc) (100 ml) to give a pure first crop. The combined solvent (DMF and EtOAc) was removed and the mixture was dissolved in dichloromethane (DCM) (200ml) then washed twice with water (100ml). The organic layer was separated, dried with MgSO₄, filtered and the solvent was removed to give a yellow solid. This solid was slurried in EtOAc and filtered to give a pure colorless solid. The crops were combined to give 35.0g (79% yield) of a pure colorless solid. ¹H-NMR (400 MHz, D₆-DMSO): δ=1.15-1.4 (m, 6H); 4.05-4.25 (m, 4H); 7.2 (m, 3H); 7.3 (d, 1H); 7.48 (d, 1H); 7.55 (d, 1H); 7.5 (bs, 1H); 9.7 (s, 1H) ppm.

*Isobutyl(10-ethyl-11-oxo-10,11-dihydrodibenzo[b,f][1,4]oxazepin-2-yl) carbamate (**BT2-IC**).* To 2-Amino-10-methyl-10H-dibenzo[b,f][1,4]oxazepin-11-one (1.5g, 5.89mmol, 1.0eq) in 50ml of DMF under an atmosphere of nitrogen was added diisobutyl dicarbonate (1.55g, 7.08mmol, 1.2eq). The mixture was stirred overnight at 40°C (external). The solvent was removed and the mixture was dissolved in DCM (200ml) and washed twice with water (150ml). Then the organic layer was dried with MgSO₄, filtered on a sintered funnel and the solvent was removed to give 3.0g of a brown solid as a crude product. This solid was purified by column chromatography on silica gel and a mixture of hexane: ethyl acetate (starting from 10% ethyl acetate in hexane, then polarity increased to 20% to give the product 1.55g (74%) as a faint yellow solid. ¹H-NMR (400 MHz, CDCl₃) δ=0.93 (s, 3H); 0.95 (s, 3H); 1.35 (t, 3H); 1.90-2.10 (m, 1H); 3.93 (d, 2H); 4.15 (q, 2H); 6.87 (s, 1H); 7.11-7.21 (m, 3H); 7.23-7.26 (m, 1H); 7.28-7.32 (m, 1H); 7.61 (d, 1H); 7.75 (brs, 1H).

*N-(10-Ethyl-11-ox0-10,11-dihydro-dibenzo[b,f][1,4]oxazepin-2-yl)-2-methoxyacetamide (**BT2-MeOA**).* To methoxy acetic acid (1.169g, 0.996ml, 12.9mmol, 1.1eq) in 60 ml of DMF under an atmosphere of nitrogen was added carbonyldiimidazole (2.487g, 15.0mmol, 1.3eq). The mixture was stirred for 30min. Then the 2-amino-10-methyl-10H-dibenzo[b,f][1,4] oxazepin-11-one (3.0g, 11.8mmol, 1.0eq) was added and the reaction stirred at 30°C (external) overnight. The solvent was removed, water (200ml) and DCM (200ml) were added to the mixture and acidified to pH 6 with 2M HCl. The organic phase was washed twice with 50ml water. The organic layer was dried with MgSO₄, filtered on a sintered funnel and the solvent was removed to give 3.7g of a sticky yellow solid. The crude product was purified by column chromatography using 50% ethyl acetate and in hexane to give the product 3.26g (85%) as a faint brown solid. ¹H-NMR (400 MHz, CDCl₃): δ=1.22 (t, 3H); 3.35 (s, 3H); 3.95 (s, 2H), 4.1 (m, 2H); 7.2-7.3 (m, 3H); 7.35 (d, 1H); 7.52 (d, 1H); 7.8 (d, 1H); 8.03 (s, 1H); 9.9 (s, 1H) ppm.

*(11-oxo-10-propyl-10,11-dihydro-dibenzo[b,f][1,4]oxazepin-2-yl)-carbamic acid ethyl ester (**BT2-Pr**).* To 2-Amino-10-propyl-10H-dibenzo[b,f][1,4]oxazepin-11-one (2.4g, 9.43mmol, 1.0eq) in 70ml of DMF under an atmosphere of nitrogen was added diethyl pyrocarbonate (2.30g, 14.16mmol, 1.5eq). The mixture was stirred overnight at 40°C (external). The solvent was removed and the mixture was dissolved in DCM (200ml) and washed twice with water (150ml). The organic layer was separated and dried with MgSO₄, filtered on a sintered funnel and the solvent removed to give 3.0g of a brown solid as a crude product. This solid was purified by column chromatography in 20% ethyl acetate in hexane to give a pure compound 2.2g (72%) as a faint yellow solid. ¹H-NMR (400 MHz, CDCl₃): δ=1.30 (t, 3H); 3.40 (s, 3H); 3.80(t, 2H); 4.20-4.25 (m, 4H); 6.60 (s, 1H); 7.13-7.26 (m, 5H); 7.55-7.60 (m, 2H); 7.68 (s, 1H) ppm.

*[10-(2-Methoxy-ethyl)-11-oxo-10,11-dihydro-dibenzo[b,f][1,4]oxazepin-2-yl]-carbamic acid ethyl ester (**BT2-EOMe**).* To 2-amino-10-(2-methoxy-ethyl)-10H-dibenzo[b,f][1,4]oxazepin-11-one (2.9g, 10.2mmol, 1.0eq) in 90ml of DMF under an atmosphere of nitrogen was added diethyl pyrocarbonate (1.82g, 11.22mmol, 1.1eq). The mixture was stirred overnight at 40°C (external). The solvent was removed and the mixture was dissolved in DCM (200ml) and the organic phase washed twice with water (150ml). The organic layer was separated and dried with MgSO₄, filtered on a sintered funnel, and the solvent was removed to give 3.6g of a brown solid as a crude product. This solid was purified by column chromatography in 30% EtOAc in hexane to give a pure compound 3.5g (96%) as a colorless solid. ¹H-NMR (400 MHz, CDCl₃) δ=1.30 (t, 3H); 3.40(s, 3H); 3.80(t, 2H); 4.20-4.25 (m, 4H); 6.60 (s, 1H); 7.13-7.26 (m, 5H); 7.55-7.60 (m, 2H); 7.68 (s, 1H) ppm.

*Ethyl (11-(oxetan-3-ylmethyl)dibenzo[b,f][1,4]oxazepin-2-yl)-carbamate and (**BT2-IMO**) and Ethyl (10-(oxetan-3-ylmethyl)-11-oxo-10,11-dihydro-dibenzo[b,f][1,4]oxazepin-2-yl)-carbamate (**BT2-MO**).* Under an atmosphere of nitrogen 2-nitro-10H-dibenzo[b,f][1,4]oxazepin-11-one (3) (**Figure 6B****, Scheme 3**) (7.5g, 0.029mol, 1.0eq) was added to 100 ml of DMF and stirred for 5min. Then, NaH (1.4g, 2.34g (60%) in oil, 0.058mol, 2.0eq) was added in small portion (an exotherm was observed). The mixture was stirred at 40°C (external) for 35min. Then, oxetan-3-ylmethyl methanesulfonate (9.73g, 0.058mol, 2.0eq) was added and the reaction stirred for 3h at 40°C (external). The reaction was followed by TLC in 20% ethyl acetate in hexane. When the reaction was complete the solvent was removed on a kugel (100°C and full vacuum) (or on rotary evaporator, using a strong pump ensuring bath temperature below 70°C) then water (300ml) was added. The solid was stirred with a spatula to make it precipitate in water. After filtration the solid was dried in a vacuum oven overnight at 80°C. The crude product mixture was purified by column chromatography on silica gel and a mixture of hexane: ethyl acetate (starting from 20% ethyl acetate in hexane, with polarity increased to 40%). The first band was the O-alkylated compound (RF=0.65).

A 1.0g (13% yield) of O-alkylated compound yield was obtained as a faint yellow solid. The melting point is 135-137°C (corrected). ¹H-NMR (400 MHz, D₆-DMSO): δ=3.42-3.57 (m, 1H); 4.53 (t, 2H); 4.65 (d, 2H); 4.74-4.79 (app. dd, 2H); 7.19-7.27 (m, 3H); 7.32-7.36 (m, 1H); 7.62 (d, 1H); 8.38 (d, 1H); 8.47 (dd, 1H) ppm.

Second, O-alkyl. To 2-nitro-11-(oxetan-3-ylmethyl)dibenzo[b,f][1,4]oxazepin-11(10H)-one (1.5g, 4.6mmol, 1.0eq) was added 50ml MeOH. The mixture was stirred at 40°C (external) for 15min to dissolve all the solids. The reaction mixture was cooled to 22°C and the flask flushed with nitrogen. 10% Pd/C (200 mg) was added and the compound was hydrogenated under an atmosphere of H₂ at 40°C (external) for 1h. The solvent was removed to give 1.2g (98% yield) of a yellow solid which was used for the next step without further purification (purity ≥97%). Melting point: 150-152°C (corrected). ¹H-NMR (400 MHz, D₆-DMSO): 4.48-4.60 (m, 4H); 4.72-4.78 (app. dd, 2H); 5.2 (s, 2H), 6.71-6.75 (m, 2H); 6.95-6.99 (m, 1H); 7.07-7.20 (m, 4H) ppm.

Last, O-Alkyl. To 2-amino-10-(oxetan-3-ylmethyl)dibenzo[b,f][1,4]oxazepin-11(10H)-one (1.2g, 4.05mmol, 1.0eq) in 40 ml of DMF was added diethyl pyrocarbonate (0.98g, 6.07mmol, 1.5eq). The mixture was stirred overnight at 40°C (external). The solvent was removed, the mixture was dissolved in DCM (150ml) and washed twice with water (150ml). Then, the organic layer was dried with MgSO₄, filtered on a sintered funnel and the solvent was removed to give 1.31g of a faint yellow solid as a crude product. Crude product (1.3g) was purified by column chromatography on silica gel using a mixture of hexane: ethyl acetate (starting from 20% ethyl acetate in hexane, then polarity increases to 35%).

***BT2-IMO**.* First, a 0.5g (34% yield) was obtained as a colorless solid. The melting point is 159-162°C (corrected). ¹H-NMR (400 MHz, CDCl₃): δ=1.30 (t, 3 H); 3.47-3.59 (m, 1 H), 4.22 (q, 2H); 4.63-4.68 (m, 4H); 4.88-4.93 (m, 2 H); 6.60 (s, 1 H); 7.07-7.260 (m, 5 H); 7.50-7.59 (m, 2 H) ppm.

A 3.5g of N-alkylated compound (48% yield) was obtained as a faint yellow solid. (RF= 0.45). The melting point is 106-109°C (corrected). ¹H-NMR (400 MHz, D₆-DMSO) δ=3.17-3.28 (m, 1 H); 4.29 (t, 2 H); 4.47 (br d, 2H); 4.53-4.58 (app. dd, 2H); 7.26-7.37 (m, 2H); 7.46 (dd, 1H); 7.57-7.64 (m, 2H); 8.40 (dd, 1 H); 8.46 (d, 1 H) ppm.

Second, N-alkyl. To a 250ml RBF set up for hydrogenation was added 2-nitro-10-(oxetan-3-ylmethyl)dibenzo[b,f][1,4] oxazepin-11 (10H)-one (2.5g, 6.12 mmol, 1.0 eq) and 50ml MeOH. The mixture was stirred at 40°C (external) for 15min to dissolve all the solids. The flask was cooled to 22°C and flushed with nitrogen again. 10% Pd/C (200mg) was added and the mixture stirrer under an atmosphere of hydrogen at 40°C (external) for 1h at atmospheric pressure. The mixture was filtered through celite and the solvent was removed to yield a pure colorless solid (1.8g, 99% yield) used for the next step without further purification. Melting point: 62-72°C (corrected). ¹H-NMR (400 MHz, D₆-DMSO) δ=3.11-3.22 (m, 1H); 4.26 (t, 2H); 4.53 (app. dd, 2H); 5.17 (br s, 2H), 7.67 (dd, 1H); 6.85 (d, 1H); 6.95 (d, 1H); 7.17-7.29 (m, 3H); 7.49 (dd, 1H) ppm.

Last, N-alkyl. To 2-amino-10-(oxetan-3-ylmethyl)dibenzo[b,f][1,4]oxazepin-11(100H)-one (1.6g, 5.49mmol, 1.0eq) and diethyl pyrocarbonate (1.44g, 8.91mmol, 1.5eq) was added in 50ml of DMF. The mixture was stirred for 1h at 40°C (external). The solvent was removed, the mixture was dissolved in DCM (150ml) and washed twice with water (150ml). The organic layer was dried with MgSO₄, filtered on a sintered funnel and the solvent was removed to give the crude product which was purified by column chromatography using 50% EtOAc in hexane. BT2-MO (1.91g, 87% yield) was obtained as a colorless solid. Melting point: 161-162°C (corrected). ¹H-NMR (400 MHz, CDCl₃) δ=1.30 (t, 3H); 3.36-3.48 (m, 1H), 4.20 (q, 2H); 3.80 (t, 2H); 4.31-4.55 (m, 4 H); 4.70-4.76 (m, 2H); 6.65 (s, 1H); 7.13-7.26 (m, 5H); 7.57 (d, 1H); 7.70 (s, 1H) ppm.

*2-Methoxyethyl[[[4-(4-chlorobenzoyl)phenyl]amino]carbonyl]carbamate (Cpd* ***B*/*X*/*****LK001**)*. A solution of (4-amino-phenyl)-(4-chloro-phenyl)-methanone (49.1g, 210mmol) in DCM (150ml) was cooled in an ice/NaCl bath to ~0°C (internal temperature). 2-methoxyethyl carbonisocyanatidate (40g, 276mmol) in DCM (150ml) was added via a dropping funnel with the internal temperature being kept below 5°C. The ice bath was removed and the solution stirred for 1h at 22°C under nitrogen. The solution was filtered and the solid rinsed with methanol to give a pure faint yellow crop of desired product. A further crop was obtained by concentrating the filtrate (mixture of DCM and MeOH), filtration and methanol wash. Fractions were combined to give 49 g (62%) of the desired product. 1H-NMR (400 MHz, D₆-DMSO) δ=10.52 (s, 1H, NH), 10.10 (s, 1H, NH), 7.75-7.68 (m, 6H), 7.62 (d, 2H), 4.80 (t, 2H), 3.58 (t, 2H), 3.28 (s, 3H) ppm.

*2-Methoxyethyl[[[4-(4-chlorophenyl)(hydroxyimino)methyl) phenyl]amino]carbonyl]carbamate (**T4**).* 2-Methoxyethyl[[[4-(4-chlorobenzoyl)phenyl]amino]carbonyl]carbamate (20.7g, 55mmol), hydroxylamine hydrochloride (11.4g, 165 mmol) and sodium acetate (13.5g, 165mmol) were stirred under a nitrogen atmosphere at reflux for 4h. The reaction mixture was filtered hot to remove any salts. The filtrate was cooled and the product filtered. The filtrate was concentrated by two-thirds, cooled to 22°C and filtered to give a second crop. The solid was vacuum dried at 60°C to give the desired product (15.8g, 73%). ¹H-NMR (400 MHz, D₆-DMSO) as a mixture of E and Z isomers (~1:1) δ=11.43 (s, 0.46H, OH), 11.32 (s, 0.62H, OH), 10.42 (bs, 1H, NH), 9.90 (s, 0.49H, NH), 9.88 (s, 0.63H, NH), 7.59 (d, 0.94H), 7.52 (t, 2.44H), 7.42 (q, 1.92H), 7.35-7.25 (m, 3.44H), 4.28 (m, 2H), 3.58 (m, 2H), 3.27 (s, 1.28H), 3.28 (s, 1.72H) ppm.

***Ethyl (10-ethyl(2',2',2'-d3)-11-oxo-10,11-dihydrodibenzo[b,f][1,4]oxazepin-2-yl)carbamate (BT2-deut).*** First, 2-nitro-10H-dibenzo[b,f][1,4]oxazepin-11-one (1 g, 3.9 mmol, 1 eq) was added to 10 ml of DMF and stirred for 5 min under nitrogen. Then, the NaH (187 mg, 0.32 g in oil, 7.8 mmol, 2 eq) was added in small portions. The mixture was stirred at 40°C external for 35 min. Then, ethyliodide-2,2,2-d₃ (1.24g, 0.62 mL, 7.8 mmol, 2 eq) was added and the reaction was stirred for 3 hours at 40°C external. The solvent was removed via evaporation and trituration three times with water returned a thick paste which was subjected to chromatography eluting using 15% EtOAc in hexane gave 10-(ethyl-2,2,2-d3)-2-nitrodibenzo[b,f][1,4]oxazepin-11(10H)-one as a yellow solid (0.42 g, 38%). The melting point is 142.3°C to 145.6°C (corrected). ¹H-NMR (400 MHz, D₆-DMSO): δ = 4.12 (app s, 2H), 7.25 to 7.38 (m, 2H), 7.45 (dd, 1H), 7.60 (d and dd, 2H), 8.41 (dd, 1H), and 8.45 (d, 1H) ppm.

Second, 10-(ethyl-2,2,2-d3)-2-nitrodibenzo[b,f][1,4]oxazepin-11(10H)-one (0.4 g, 1.41 mmol, 1 eq) and SnCl₂ (0.8 g, 4.2 mmol, 3 eq) were dissolved in 10 ml of EtOH. The mixture was stirred at reflux for 2 hours. The solvent was removed and the mixture was dissolved in EtOAc (100 ml) and 1N NaOH aq. (50 ml). The organic phase was separated and washed with water (2 x 50 ml), dried with MgSO₄, filtered and the solvent evaporated. Chromatography eluting with 50% EtOAc in hexane gave the product 2-amino-10-(ethyl-2,2,2-d3)dibenzo[b,f][1,4]oxazepin-11(10H)-one as a faint beige solid (287 mg, 80%). The melting point is 165.5°C to 167.0°C (corrected). ¹H-NMR (400 MHz, D₆-DMSO): δ = 4.0 (bq, 2H), 5.15 (s, 2H), 6.65 (dd, 1H), 6.84 (d, 1H), 6.95 (d, 1H), 7.15 to 7.30 (m, 3H), and 7.45 (dd, 1H) ppm.

Last, to 2-amino-10-(ethyl-2,2,2-d3)dibenzo[b,f][1,4]oxazepin-11(10H)-one (0.287 g, 1.2 mmol, 1 eq) in DMF (3 ml) was added diethyl pyrocarbonate (0.183 ml, 0.201 g, 1.24 mmol, 1.1 eq). The mixture was stirred for 1 hour under nitrogen at 25°C external. DMF was removed from the reaction mixture and the residual solid was triturated 3 times with EtOAc to give BT2-deut as a colorless solid (260 mg, 66 %). The melting point is 184.3°C to 185.7°C (corrected). ¹H-NMR (400 MHz, D₆-DMSO): δ =1.22 (t, 3H), 4.00 to 4.15 (q and br q, 4H), 7.2-7.3 (m, 3H), 7.35 (dd, 1H), 7.50 (dd, 1H), 7.58 (dd, 1H), 7.80 (d, 1H), and 9.75 (s, 1H) ppm.

*Flubendazole (**T6**), 2-Amino-10-ethyldibenzo[b,f][1,4] oxazepin-11 (10H)-one (**BT3**) and (4-Aminophenyl)(4-fluorophenyl)methanone (**T7**)* are available commercially from AK Scientific Inc.

**Cell culture.** HMEC-1 were obtained from ATCC (Rockville, MD) and grown in MCDB131 medium (Invitrogen, MD), pH 7.4 supplemented with 10% FBS, hydrocortisone (1µg/ml), epidermal growth factor (10ng/ml), L-glutamine (2mM) and penicillin/streptomycin. Bovine aortic endothelial cells (BAEC) were obtained as primary cells from Cell Applications (San Diego, CA) and grown in DMEM, pH 7.4 supplemented with 10% FBS and antibiotics. BAEC were used in experiments between passages 4-6. Cells were routinely passaged after detachment with 0.05% trypsin/5mM EDTA and maintain in a humidified atmosphere of 5% CO₂ at 37°C.

**Western blot analysis with extracts of cells treated with serum.** HMEC-1 (80-90% confluency) were arrested in serum-free MCDB131 medium without EGF or hydrocortisone for 20h. Cells were treated with 30µM compound in serum-free MCDB131 medium for 4h, and the medium was changed to complete medium (with 10% FBS with EGF and hydrocortisone) with 30µM compound for 1h. Total protein was harvested as previously described in radioimmunoprecipitation (RIPA) lysis buffer with protease inhibitors (Li, Y., et al., Int J Cardiol 220, 185-191 (2016)). Proteins were resolved on 4-20% (w/v) sodium dodecyl sulfate (SDS)-polyacrylamide gradient gels (Bio-Rad Mini-PROTEAN TGX) and transferred to Immobilon-P PVDF membranes (Millipore, USA). Membranes were blocked with 5% skim milk and incubated with rabbit monoclonal FosB (cat. 2251, 1:1000, Cell Signaling, USA), rabbit monoclonal c-Fos antibodies (cat. 2250, 1:1000, Cell Signaling, USA) at 4°C overnight or mouse monoclonal β-actin antibodies (cat. A5316, 1:30000, Sigma-Aldrich) at 22°C for 15min then incubated with a horseradish peroxidase conjugated secondary goat anti-rabbit (cat. P0448, 1:1000, DAKO Cytomation, Denmark) or goat anti-mouse (cat. P0447, 1:1000, DAKO Cytomation, Denmark) antibodies for 1h. Chemiluminescence was detected using the Western Lightning Chemiluminescence system (PerkinElmer, USA) and ImageQuant^{™} LAS 4000 biomolecular imager (GE Healthcare Life Sciences, USA). Band intensity in images generated with the LAS 4000 on automatic exposure with sensitivity/resolution setting high was quantified using NIH ImageJ.

**Western blot analysis with extracts of cells treated with IL-1β.** HMEC-1 (80-90% confluency) were arrested in serum-free MCDB131 medium (Invitrogen, MD) without any growth factor for 48h. Cells were treated with 30µM compound in serum-free medium for 4h, and incubated with 20ng/ml IL-1β (Sigma, cat. SRE3083) in serum-free medium with the same concentration of compound for up to 4h, unless otherwise indicated. Total protein was harvested as previously described using RIPA buffer with protease inhibitors. Proteins were resolved on 4-20% (w/v) SDS-polyacrylamide gradient gels and transferred to Immobilon-P PVDF membranes. Membranes were blocked with 5% skim milk and incubated with rabbit monoclonal FosB (cat. 2251S, 1:1000, Cell Signaling, USA), rabbit monoclonal VCAM-1 (cat. 13662S, 1:1000, Cell Signaling, USA), rabbit monoclonal p44/42 MAPK (cat. 4695S, 1:1000, Cell Signaling, USA), rabbit polyclonal p38 MAPK (cat. 9212S, 1:1000, Cell Signaling, USA), rabbit polyclonal SAPK/JNK (cat. 9252S, 1:1000, Cell Signaling, USA), rabbit monoclonal phospho-SAPK/JNK (cat. 4671S, 1:1000, Cell Signaling, USA), rabbit monoclonal phospho-p38 MAPK (cat. 4511S, 1:1000, Cell Signaling, USA), or, mouse monoclonal phospho-p44/42 MAPK antibodies (cat. 9106S, 1:2000, Cell Signaling, USA) at 4°C overnight or mouse monoclonal β-actin antibodies (cat. A5316, 1:10000, Sigma-Aldrich) antibodies at 22°C for 1h. Membranes were then incubated with horseradish peroxidase conjugated secondary goat anti-rabbit (cat. P0448, 1:1000, DAKO Cytomation, Denmark) or goat anti-mouse (cat. P0447, 1:1000, DAKO Cytomation, Denmark) antibodies for 1h. Chemiluminescence was detected using the Western Lightning Chemiluminescence system and ImageQuant^{™} LAS 4000 biomolecular imager. Band intensity in images generated with the LAS 4000 using the same settings were quantified by NIH ImageJ.

**siRNA experiments.** HMEC-1 (70-80% confluency) were arrested in serum-free MCDB131 medium with no hydrocortisone or EGF for 24h and transfected with non-targeting siRNA (cat. D-001810-10-50, Dharmacon, USA) or FosB siRNA (cat. L-010086-01-0020, Dharmacon, USA) or VCAM-1 siRNA (cat. L-013351-00-0020, Dharmacon, USA) and Dharma FECT1 transfection reagent (cat. T-2001-03, Dharmacon, USA) mixed for 24h. siRNA experiments (with 0.6µM FosB, 0.6µM VCAM-1) were performed, shoulder-to-shoulder with non-targeting loading control siRNA at the same concentration. The cells were stimulated with 20ng/ml IL-1β in serum-free complete MCDB131 medium for a further 2 or 4h. Total protein was harvested using RIPA buffer with protease inhibitors and resolved on 4-20% (w/v) SDS-polyacrylamide gradient gels and transferred to Immobilon-P PVDF membranes. Membranes were blocked with 5% skim milk and incubated with rabbit monoclonal FosB (cat. 2251S, 1:1000, Cell Signaling, USA), rabbit monoclonal VCAM-1 (cat. 13662S, 1:1000, Cell Signaling, USA) at 4°C overnight or mouse monoclonal β-actin (cat. A5316, 1:10000, Sigma-Aldrich) antibodies at 22°C for 1h. Membranes were incubated with horseradish peroxidase conjugated secondary goat anti-rabbit (cat. P0448, 1:1000, DAKO Cytomation, Denmark) or goat anti-mouse (cat. P0447, 1:1000, DAKO Cytomation, Denmark) Ig for 1h. Chemiluminescence was detected using the Western Lightning Chemiluminescence system and ImageQuant^{™} LAS 4000 biomolecular imager.

**Plasmid overexpression.** HMEC-1 were seeded into 6-well plates and at 70-80% confluency, the cells were deprived of serum (or EGF and hydrocortisone) overnight. Cells were transfected with 6µg of the indicated plasmid (in pcDNA3.1+/C-(K)DYK) (GenScript, USA) with Fugene 6 (Promega) according to manufacturer's protocol. Total protein lysates were collected 18, 24, 48 and 72h after plasmid transfection in RIPA buffer with protease inhibitors. Proteins were resolved on 4-20% (w/v) SDS-polyacrylamide gradient gels and transferred to Immobilon-P PVDF membranes. Membranes were blocked with 5% skim milk and incubated with rabbit monoclonal p44/42 MAPK (cat. 4695S, 1:1000, Cell Signaling), mouse monoclonal phospho-p44/42 MAPK antibodies (cat. 9106S, 1:2000, Cell Signaling), rabbit monoclonal FosB (cat. 2251S, 1:1000, Cell Signaling, USA), rabbit monoclonal VCAM-1 (cat. 13662S, 1:1000, Cell Signaling) or mouse monoclonal α-tubulin (cat. T5168, 1:40000, Sigma) at 4°C overnight. Membranes were then incubated with horseradish peroxidase conjugated secondary goat anti-rabbit (cat. P0448, 1:1000, DAKO Cytomation, Denmark) or goat anti-mouse (cat. P0447, 1:1000, DAKO Cytomation, Denmark) antibodies for 1h. Chemiluminescence was detected using the Western Lightning Chemiluminescence system and ImageQuant^{™} LAS 4000 biomolecular imager.

**RNA-seq.** HMEC-1 were seeded into nine 100mm petri dishes with complete MCDB131 medium containing 10% FBS. At 70-80% confluency, cells were growth-arrested with serum-free MCDB131 medium with no hydrocortisone or EGF for 44h. Cells were pre-treated with 30µM BT2 in the same medium for 4h then stimulated with 20ng/mL IL-1β for a further 4h. Total RNA was extracted using RNeasy Mini Kit (Qiagen, Amtsgericht Düsseldorf) with modification. Briefly, cells were washed twice with pre-cooled 1x PBS and TRIzol (Thermo Fisher Sci, Waltham, MA) was added to lyze the cells. Chloroform was added to the mixture prior to centrifugation at 13000rpm for 15min at 4°C. Upper aqueous layer containing total RNA was transferred to fresh microtubes and isopropanol was added and loaded into RNeasy column. Columns were washed with Buffers RW1 and RPE. Total RNA was eluted from the column using RNAse-free water. Samples were submitted to The Ramaciotti Centre for Genomics (UNSW, Australia) for TruSeq Stranded mRNA-seq preparation and sequencing by One NextSeq 500 1X75bp High Output flowcell with data output up to 400M reads. Quality control of samples was set at >80% higher than Q30 at 1x75bp.

RNA-seq reads were first assessed for quality using the tool FastQC (v0.11.8) (http://www.bioinformatics.babraham.ac.uk/projects/fastqc/). The tool Salmon was used for quantifying transcript abundance from RNA-seq reads (Patro, R., et al., Nat Methods 14, 417-419 (2017)). The R package DESeq2 (Love, M.I., et al., Genome Biol 15, 550 (2014)) that incorporates a method for differential analysis of count data was then used to identify differentially expressed genes across specific comparisons. The heatmap.2 function from the R package gplots v3.0.1.1 was used to generate heatmaps using counts per million (cpm) values for sets of genes of interest. The database for annotation, visualization and integrated discovery (DAVID) (Jiao, X., et al., Bioinformatics 28, 1805-1806 (2012)), a web-based online bioinformatics resource was used to identify the gene ontologies such as biological processes (BP) found to be enriched for lists of differentially expressed genes for specific comparisons.

**Flow cytometry.** HMEC-1 (at 80-90% confluency) were arrested in serum-free MCDB131 medium without EGF or hydrocortisone for 40h, treated with 30µM BT2 or BT3 for 4h. The cells were incubated in serum-free medium and exposed to 20ng/ml IL-1β with the same concentration of BT2 or BT3 for a further 4h. The cells were washed with PBS then detached with Accutase (Stem Cell Technologies, cat. 07920). The cells were centrifuged at 300g for 5min and resuspended at 5x10⁶ cells/ml containing BT2 or BT3. The cells were incubated with BV421-conjugated mouse anti-human CD106 (VCAM-1) (BD, cat. 744309) or BV421-conjugated mouse IgG₁ (BD, cat. 562438) for 45min at 22°C. The cells were washed with Stain Buffer and the pellet was resuspended in 0.5 ml of 1% paraformaldehyde prior to flow cytometry BD FACSCanto II.

VCAM-1⁺ and VCAM-1⁻ cells were gated by performing flow cytometry with or without primary VCAM-1 antibody (non-specific staining), respectively. Representative gating from the latter (i.e. negative control) is shown as Figure 10 indicate minimal non-specific staining. The gating strategy is based on fluorescence excitation off both the 488nm laser and 405nm laser with emission filters 670LP off 488nm and 450/50 off 405nm. Cells with autofluorescence or negative (blue population) had equal proportion of fluorescence in both channels and VCAM-1 positive cells (red) emit light in the 450/50 filter.

SPR. SPR was performed on a Biacore T200. The active and reference flow cells of a Xantec NIHMC Ni sensor chip were conditioned with 0.5M NaEDTA followed by 5mM NiCl₂ in immobilisation buffer (20mM HEPES, 150mM NaCl, pH 7.4). Recombinant human His-MEK1 and His-MEK2 (500nM, ThermoFisher Scientific, cat. PV3303 and PV3615, respectively) were injected for 15min at 10µl min⁻¹ over separate active flow cells. All immobilisation was carried out at 25°C. Following immobilisation, the temperature was lowered to 15°C, and the buffer changed to 20mM HEPES, 150mM NaCl, 5% DMSO pH 7.4. Samples of PD98059 (2.5-30µM in running buffer) and BT2 (1.25-15µM) were injected at a flow rate of 30ul min⁻¹ over immobilised MEK1 and MEK2. Solvent correction was applied to the data using a DMSO standard curve. Data were analysed using the Biacore T200 Evaluation software. Prior to SPR, limits of compound solubility were determined using ¹H 1D NMR.

Endothelial proliferation assay using the xCELLigence system. HMEC-1 proliferation was evaluated using the xCELLigence System (Roche, Castle Hill). Briefly, HMEC-1 (5x10³ cells/well) were seeded in a 96-well E-plate and inserted into the xCELLigence RTCA station (Roche). Cells were serum-deprived for 24h in MCDB131 medium which contained 10ng/ml EGF (Sigma-Aldrich) and 1µg/ml hydrocortisone (Sigma-Aldrich) then treated with compound (0.2-1µM) in medium containing 5% FBS, 10ng/ml EGF (Sigma-Aldrich) and 1µg/ml hydrocortisone (Sigma-Aldrich). Cell growth was monitored automatically every 15min by xCELLigence system. Cell index (CI) represents a quantitative measure of each well cell growth. In this system, Cl a unitless parameter that reports impedance of electron flow caused by adherent cells.

Endothelial proliferation assay using the Countess system. HMEC-1 proliferation was assessed using a Countess II Automated Cell Counter (ThermoFisher Scientific). Briefly, HMEC-1 (3x10⁵ cells/well) were seeded in a 12-well plate. Cells were serum-deprived for 24h in MCDB131 medium which contained 10ng/ml EGF and 1µg/ml hydrocortisone then treated with compound (0.1-0.6µM) in medium containing 5% FBS, 10ng/ml EGF and 1µg/ml hydrocortisone. The cells were trypsinized after 24h, resuspended in complete medium, a 10µl aliquot was combined with an equal volume of 4% Trypan Blue, and total cell numbers and Trypan Blue-excluding cells as a proportion of total was determined using the Countess.

**Endothelial dual chamber migration assay.** BAEC (6x10³ cells/well) suspended in DMEM supplemented with 10% FBS were seeded into the upper chamber of 24-well plates fitted with Millicell cell culture inserts (cat. PI8P01250, Millipore). After 48h, the medium was changed to DMEM supplemented with 0.01% FBS and the cells were incubated for 48h. Compounds prepared in DMEM containing 0.01% FBS were added to the upper chamber. VEGF-A₁₆₅ (50ng/ml, Sigma, cat. V7259) in medium containing 10% FBS was added to the lower chamber. After 24h, medium from the upper chamber was removed and a cotton swab was used to remove non-migrated cells and excess liquid. The insert was placed in 70% ethanol for 10min to allow cell fixation and membranes were dried for 10-15min. Filters were excised and placed on slides. Mounting medium (Fluoroshield^{™} with DAPI, Sigma, cat. 6057) was added and specimens were visualized using an EVOS FL microscope.

**Endothelial repair following *in vitro* injury.** HMEC-1 (90-100% confluency) in 6-well plates were washed with PBS, and treated with 0.6µM compound in MCDB131 containing 5% FBS. A sterile pointed toothpick was used to scrape the cell monolayer and the wells photographed under 4x objective at 0h and 48h. Cell regrowth in the denuded zone was determined using Image-Pro Plus (Cybernetics, USA).

**BT2 formulation analysis using RRLC-MS/MS.** A rapid resolution liquid chromatography/tandem mass spectrometry (RRLC-MS/MS) method was developed under GLP by Iris Pharma using an Agilent 1200 Triple Quad G6410B to determine BT2 content in heat-treated or non-heat treated BT2 formulations at 1 week (T1 week) or 6 weeks (T6 weeks) after preparation at room temperature. The formulations were heat (H)-treated (tubes placed in a 100°C water bath for 10min) or non-heat treated sonicated formulations of BT2 in saline containing 0.5% Tween 80 and 0.01% DMSO). Standard curves were constructed with 8 concentrations between the lower limit of quantification (LLOQ) and the upper limit of quantification (ULOQ). Evaluations were performed on 3 preparations at the same dilution. Chromatograms were integrated using MassHunter software. For BT2 content analysis (T1 week and T6 weeks), calculation of mean, SD, CV (%) and bias (%) were performed as follows: For T1, the theoretical concentration (*i.e.* the weighed/formulated material supplied) was used as reference to calculate the bias (%) of each preparation containing the test sample: $% bias = \frac{Calculated Value - Theoretical Concentration}{Theoretical Concentration} \times 100 .$

Standard curves were fitted using Excel^{®} version 2011. For each run, bias on back-calculated concentration of the standard curve and QC was determined, with back-calculated concentrations of the calibration standards being set within ± 15% of the theoretical value, except for the LLOQ for which it was set within ± 20%. At least 75% of the calibration standards, with a minimum of six, must have had to fulfil this criterion and the coefficient of determination (r²) was set at ≥0.98.

### BT2 formulation analysis using liquid chromatography mass spectrometry (LC/MS).

DMSO (100µl) and samples (~50µl) were combined along with formic acid (1µl). These solutions (10µl) were further diluted with H₂O:CH₃CN (1:1) 0.1% formic acid (90µl) for LC/MS analysis. Samples were separated by UPLC using an HPG-3400RS UPLC pump, autosampler and column compartment system (Thermo Scientific, CA). Samples (0.1µl) were loaded onto a Hypersil Gold aQ column (2.1 x 50 mm) containing 1.9µ media (Thermo Scientific). Compounds were eluted using a linear gradient of H₂O:CH₃CN with A containing H₂O (0.1% formic acid) and B containing H₂O:CH₃CN (1:4, 0.1% formic acid). The gradient was: T=0min 2% B, T=20min 75% B, T=23min 95% B, T=25min, 95% B, T=25.2min 2% B, T=30min, 2% at 200µl/min over 30min. The column oven was heated to 45°C. Positive ions were generated by electrospray and the QExactive Plus mass spectrometer (Thermo Fisher, Bremen, Germany) operated in data dependent acquisition mode (DDA). The heated electrospray source (HESI) was used with a high voltage 3.8 kV applied; a vaporizer temp of 250°C; sheath gas 20; aux gas 5 and the heated capillary set to T=290°C. A survey scan m/z 140-800 was acquired (resolution=70,000 at m/z 200, with an AGC target value of 3x10⁶ ions, max IT 250 msec) with lockmass was enabled (m/z 391.28429). Up to the 10 most abundant ions combining 2 microscans (with a minimum AGC target of 5x10⁴, maximum IT 110 msec) were sequentially isolated (width m/z 1.8) and fragmented by HCD (NCE = 20, 30, 50) with an AGC target of 2x10⁵ ions (resolution=17,500 at m/z 200). M/Z ratios selected for MS/MS were dynamically excluded for 12sec and charge state exclusion was not enabled. LC/MS chromatograms were processed using Xcalibur Qual Browser.

**Endothelial network formation assay.** HMEC-1 (4x10⁴ cells/well) in MCDB131 containing 1% FBS and compound (1 or 3µM) or curcumin (1-40µM) and 50ng/ml FGF-2 were added to 96-well plates coated overnight at 4°C with 100µl of growth factor-reduced reconstituted basement membrane matrix (Matrigel, cat. 354230, Corning, NY). Network formation was observed over subsequent hours and photographed under 4x or 10x objective using an Olympus CKX41 microscope.

**Matrigel plug assay.** Matrigel (500µl) containing VEGF-A₁₆₅ (100ng/ml), heparin (10U) and BT2 or BT3 (2.5 mg/mouse) or its vehicle (saline containing 0.01% DMSO and 0.5% Tween 80) was injected subcutaneously into the left flanks of male 8 week-old C57BL/6 mice. After 7d the mice were sacrificed by CO₂ asphyxiation and the plugs carefully removed. Formalin-fixed paraffin embedded sections were prepared from Matrigel plugs for immunohistological assessment. Heat-induced epitope retrieval was applied to all deparaffinized sections (4µm Superfrost slides) in citrate buffer, pH 6 for 5min at 110°C. Immunostaining for all groups with a given antibody was performed simultaneously and development time was identical. Animal experiments were approved by the Animal Care and Ethics Committee at the University of New South Wales.

For CD31 staining, sections were blocked with endogenous enzyme blocking agent (cat. S2003, DAKO) for 10min and then with 2% skim milk for 20min. Slides were incubated with primary antibody rabbit polyclonal CD31 antibody, 1:25 dilution (cat. ab28364, Abcam) for 1h at room temperature. Slides were rinsed with buffer and incubated with secondary antibody (goat anti-rabbit (cat. P0448, DAKO)) for 30min, rinsed with buffer and incubated with diaminobenzidine (DAB) chromagen (cat. K3468, DAKO) for 5min and counterstained in hematoxylin and Scott blue. Slides were dehydrated in 100% ethanol and xylene then coverslipped.

For FosB or VCAM-1 staining, sections were blocked with dual endogenous enzyme blocking agent (cat. S2003, DAKO) for 10min and then with 2% skim milk for 20min. The slides were incubated with primary rabbit monoclonal FosB (cat. 2251, Cell Signaling, USA) or rabbit polyclonal VCAM-1 (cat. sc-8304, Santa Cruz) for 1h at room temperature and then incubated for 10min with the probe component of MACH3 Rabbit AP-Polymer Detection (Biocare Medical, M3R533 G, H, L). After rinsing with buffer the slides were incubated with polymer component of MACH3 Rabbit AP-Polymer Detection (Biocare Medical, M3R533 G, H, L) for a further 10min. The slides were incubated with red chromogen (Warp Red^{™} Chromogen Kit) for 7min and counterstained in hematoxylin and Scott blue. The slides were dried using filter paper and dehydrated in xylene then coverslipped.

Slides were scanned using an Aperio ScanScope XT slide scanner (Leica Biosystems, Mt Waverley, Vic Australia) and images captured using ImageScope software (Leica Biosystems). Positive intraplug staining was assessed using Image-Pro Plus software (Cybernetics, Bethesda, MD) in 5-12 randomly selected fields of view for each plug photographed under 10x (CD31), 20x (VCAM-1) and 40x (FosB) objectives and expressed as integrated optical density (IOD, the product of calibrated intensity (optical density) and area, *i.e.* IOD = intensity (mean) × area) (Media Cybernetics) (Liu, H., et al., Sci Rep 6, 21319 (2016)). We also expressed positive immunostaining as the area of positive staining as a proportion (%) of plug area (Kim, J.Y., et al., Biomolecules 10, pii: E11 (2019)).

**Rabbit retinal vascular hyperpermeability model.** Male HY79b pigmented rabbits (8-12 week-old) were anesthetized by an intramuscular injection of Rompun^{®} (xylazine)/Imalgene^{®} (ketamine). Compound (600µg BT2, BT3 or saline vehicle containing 0.5% Tween 80 and 10% DMSO vehicle in 100µl) was injected into the right eye 5d prior to rhVEGF-A₁₆₅ induction. Injections were performed on anesthetized animals under an operating microscope using a 250µl Hamilton syringe (fitted with 30G needle). Retinal vascular permeability was induced by a single 50µl IVT injection of 500ng rhVEGF-A₁₆₅ (diluted in PBS with carrier protein) into the right eye. Forty-seven hours (+/-3h) after induction, sodium fluorescein (10% in saline, 50mg/kg) was injected into the marginal ear vein. One hour after fluorescein injection, animals were anaesthetized and pupils were dilated by instillation of one drop of 0.5% tropicamide. Ocular fluorescence in both eyes was measured with a FM-2 Fluorotron Master ocular fluorophotometer. Animals were euthanized by injection of pentobarbital. The study was performed by Iris Pharma (La Gaude, France) with approval from the Animal Ethics Committee of Iris Pharma and the Animal Care and Ethics Committee at the University of New South Wales.

**Rat choroidal laser injury model.** Male Brown Norway pigmented rats (8-14 week-old) were anesthetized by an intramuscular injection of Rompun^{®} (xylazine)/Imalgene^{®} (ketamine). Pupils were dilated by instillation of one drop of 0.5% tropicamide before laser burn. Six burns were created in both eyes on Day 0 by applying 170mW of 532nm laser light (Viridis laser, Quantel, France) on 75µm spots around the optic nerve, between the main retinal vessel branches, for 0.1s, through the slit lamp and contact lens. Production of a bubble at the time of laser application confirmed the rupture of Bruch's membrane. Compounds in vehicle (saline containing 0.01% DMSO and 0.5% Tween 80, sonicated) in 2-5µl were injected IVT on Days 0 and 7 under an operating microscope using a 30G needle mounted on a 100µl Hamilton syringe. Kenacort was administered IVT into each eye on Day 0. Alternatively, aflibercept/Eylea in vehicle (saline) was injected IVT 6 times (Days 0, 3, 7, 10, 14, 17). Fluorescein angiography was performed using Heidelberg retinal angiography. After anaesthesia, 10% sodium fluorescein (250µl/100g body weight) was injected subcutaneously, and ocular fluorescence was recorded 10min after dye injection. Fluorescein leakage was evaluated on Days 14 and 21 in the angiograms by two examiners masked to the study groups and graded for fluorescein intensity as follows: score 0: no leakage; 1: slightly stained; 2: moderately stained; 3: strongly stained. The studies were performed by Iris Pharma (La Gaude, France) with approval from the Animal Ethics Committee of Iris Pharma and the Animal Care and Ethics Committee at the University of New South Wales.

**Immunohistochemical staining of rat retina.** Rabbit monoclonal anti-CD31 (cat. ab182981), rabbit monoclonal anti-VCAM-1 (cat. ab134047) and rabbit polyclonal anti-VEGF-A (cat. ab46154) were obtained from Abcam. Rabbit monoclonal phospho-p44/42 MAPK (pERK1/2, Thr²⁰²/Tyr²⁰⁴) (cat. 4370) and rabbit monoclonal FosB (cat. 2251) were obtained from Cell Signaling. Formalin-fixed, paraffin embedded sections were prepared from resected rat eyes. Heat-induced epitope retrieval was applied to all deparaffinized sections (4µm Superfrost slides) with either citrate buffer, pH 6 (VEGF-A, pERK, VCAM-1) or EDTA buffer, pH 9 (CD31) for 5min at 110°C. Sections were blocked with dual endogenous enzyme blocking agent (cat. S2003, DAKO) for 10min and then with 2% skim milk for 20min. Slides were incubated with primary antibody for 60min at room temperature and then for 10min with the probe component of MACH3 Rabbit AP-Polymer Detection (Biocare Medical, cat. M3R533 G, H, L). After rinsing with buffer, the slides were incubated with the polymer component of MACH3 Rabbit AP-Polymer Detection (Biocare Medical, M3R533 G, H, L) for a further 10min. Slides were incubated with red chromogen (Warp Red^{™} Chromogen Kit) for 7min and counterstained in hematoxylin and Scott blue. Slides were dried with filter paper and dehydrated in xylene then coverslipped. Immunostaining with a given antibody was performed for all groups at the same time. Immunostained slides were scanned using an Aperio ScanScope XT slide scanner (Leica Biosystems, Mt Waverley, Vic, Australia) and images were captured using ImageScope software (Leica Biosystems). IOD of positive staining (red chromogen) was assessed for CD31, VEGF-A₁₆₅, pERK, FosB and VCAM-1 using Image-Pro Plus software (Cybernetics, Bethesda, MD). IOD in IPL and INL was quantified for CD31; OPL to OS for VEGF-A₁₆₅; INL to ONL for pERK; GCL to OS for FosB; OLM for VCAM-1, using Image-Pro Plus. In addition, we expressed positive immunostaining as area of positive staining relative to retinal tissue area (%) (Kim, J.Y., et al., Biomolecules 10, pii E11 (2019)). On image selection for quantification, for the vehicle and BT2 groups, all wounds in 2-4 sections/eye were identified and photographed under 20x objective. In the untreated group, which had no injury, photographs of 1-3 sections/eye were taken under 20x objective. Staining was quantified with n=3-6 per group. Where VEGF-A₁₆₅ gradient staining was assessed relative to the wound, immunostaining was assessed in 10 consecutive 100µm boxes starting 150µm (double headed arrow) from wound center with IOD in each box quantified with Image-Pro Plus.

**Endothelial-monocytic cell adhesion assay.** HMEC (80-90% confluency) in 96-well plates were deprived of serum for 24h and treated with compound at indicated concentrations for 1h then incubated with IL-1β (20 ng/ml) for 4h. Meanwhile THP-1 were labeled with 5µM calcein (5x10⁶ cells/ml, BD Bioscience) for 30 min at 37°C followed by washing 3 times with PBS. THP-1 (2.5x10⁵ cells/well) were then added for 30 min, unbound cells were washed off 3 times in PBS. Adhesion of calcein-labeled THP-1 to the endothelium layer was determined in a fluorescent plate reader at excitation 485 nm and emission 530 nm.

**Monocyte-transendothelial migration assay.** Millicell 8 µm polycarbonated culture plate inserts (Millipore) were coated with 0.1% porcine gelatin type A (Sigma) and then placed into 24-well plates. HMEC (5x10⁴ cells/well) were seeded onto the insert and allowed to adhere overnight. Cells were then serum deprived for 24h and treated with various compound treatments for 1h. IL-1β (20ng/ml) was added to stimulate the cells for 4h and 500µl of serum-free medium was added to the bottom of the 24-well plate along with the compound. THP-1 (5x10⁵ cells in 100µl) were added into the insert and 100 ng/ml MCP-1 (Sigma) was added to the lower well. After 24 h, the number of cells that had migrated though the endothelial layer was assessed by counting 100µl of the suspension in the lower chamber using a Coulter cell counter (Beckman Coulter).

**Collagen antibody-induced arthritis.** Arthritis was induced in female Balb/c mice (6-8 week-old) as previously described with a commercially obtained cocktail of 5 monoclonal antibodies to type II collagen at 2 mg/mouse (Chondrex, Inc. Redmond, WA) followed by LPS (50µg/mouse) with or without BT2 (3 or 30 mg/kg mouse) in DMSO vehicle was administered i.p. on Day 3. Hind footpad thickness was measured on Day 9 using digital calipers. Mice were sacrificed on Day 14 and microCT scanning of hind limbs was performed. Animal experiments were approved by the Animal Care and Ethics Committee at the University of New South Wales.

**Micro-CT scanning and analysis.** Formalin-ethanol fixed hind limbs were micro-CT scanned prior to histology processing using a Siemens Inveon micro-CT scanner (Victoria, Australia). Data was acquired with the Inveon Acquisition Workplace at 16.84µm pixel size, 360 projections, 4100ms integration time, 80keV photon energy and 140µA current. 3D models were visualized and snap shots of the limbs acquired with the Inveon Research Workplace software. Data was quantified by binary score where 0=no bone destruction and 1=destruction was given to each individual limb.

**Tartrate-resistant acid phosphatase (TRAP) staining.** Osteoclasts were stained using TRAP kit (Cosmo Bio, Japan, cat. PMC-AK04F-COS). Sections were heated at 65°C for 1h prior dewaxing. Tissue sections were deparaffinised with 100% xylene and rehydrated with 100, 70 and 30% ethanol and rinsed with distilled water for 5 min. Sections were covered with TRAP staining solution containing 3 mg tartaric acid per 50 ml tartaric acid buffer. The sections were incubated at 37°C for 1h, then rinsed in distilled water 3 times to halt the reaction. Sections were counterstained with hematoxylin for 5s then washed in running water until clear then dried. Sections were dehydrated with xylene and air-dried then mounted with aqueous permanent mounting medium. Within the synovium on the medial aspect of each animal joint, 6 random areas photographed under 20x objective were selected in the blinded fashion. Numbers of osteoclasts were counted using NIH Image J. Alternatively TRAP staining was quantitated using IOD (Image-Pro Plus).

**Immmunohistochemical staining of hind limbs for VCAM-1 and ICAM-1 and analysis.** Formalin-fixed, paraffin embedded of hind limbs were sectioned (5 µm). Dako EnVision Rabbit Kit (cat. K4011, Dako) was used for immmunohistochemical staining for VCAM-1 and ICAM-1. Briefly, sections were blocked with peroxidase for 30 min and then immunostained with rabbit monoclonal VCAM-1 (cat. ab134047, 1:100, Abcam), or rabbit polyclonal ICAM-1 (cat. ab124759, 1:100, Abcam) at 4°C for overnight. Staining was visualized using labeled polymer-Horse Radish Peroxidase (HRP) (anti-rabbit) and Diaminobenzidine (DAB) system followed by counter staining with haematoxylin and Scott blue. Immunostained slides were scanned using an Aperio ScanScope XT slide scanner (Leica Biosystems, Mt Waverley, Vic, Australia) and images were captured using ImageScope software (Leica Biosystems). Integrated optical density (IOD) of positive staining in ankle joint (tibia and talus) articular cartilage was assessed for VCAM-1 and ICAM-1 using Image-Pro Plus software (Cybernetics, Bethesda, MD, USA). Area (µm²) of ankle joint articular cartilage was measured using Image-Pro Plus software. Total cell number and positive staining cell number in ankle joint articular cartilage were counted manually using Image-Pro Plus software. Data was represented as IOD/µm² and percentage of positive staining cell per 20x objective view.

**Toxicology.** Female Balb/c mice (8-9 week-old) were given 3 or 30 mg/kg BT2 (DMSO vehicle) via intraperitoneal injection (Days 0 and 5 in DMSO), oral gavage (Days 0-4 in DMSO/methylcellulose) or intraarticular injection (Day 0 in DMSO). Mice were euthanized after 8-11 d. Tissues was fixed in 10% formalin, processed routinely, sectioned at 4µm and stained with hematoxylin and eosin. Sections were examined histologically for signs of toxicity by a board-certified diplomate of the American College of Veterinary Pathologists. Animal experiments were approved by the Animal Care and Ethics Committee at the University of New South Wales.

**Statistics.** Statistical analysis was performed as described in the legends using PRISM v7.0d and differences were considered significant when P<0.05. Where indicated, *P<0.05, **P<0.01, ***P<0.001, ****P< 0.0001.

### Results

**Identification of BT2, T4, T6.** To identify novel small molecule inhibitors of AP-1, the ~100,000 compound WEHI Lead Discovery Library was screened using a 293 cell-based assay in which *Firefly* luciferase was driven by multiple copies of the AP-1 response element. A substructure filter was applied during the course of screening to remove pan assay interference compounds (PAINS) (Baell, J.B., et al., J Med Chem 53, 2719-2740 (2010)) that typically captures the AP-1 inhibitor curcumin (Nelson, K.M., et al., J Med Chem 60, 1620-1637 (2017)). This yielded 24 available hits with IC₅₀ in the micromolar or submicromolar range determined using 11-point titration curves, including the dibenzoxazepinone BT2. This followed an earlier screen of a 960 compound DIVERSet library (ChemBridge) that yielded the benzophenone Cpd B/X/LK001 **(****Figure 6A****).** T4, T6 and T7 are structural analogues of Cpd B/X/LK001, whereas BT3 is an analogue of BT2 **(Table 1).** BT2 was synthesized subsequent to the screen by reacting commercially available 2-amino-10-ethyldibenzo[b,f][1,4] oxazepin-11 (10H)-one (BT3) with diethyl pyrocarbonate **(****Figure 6B****, Scheme 1).** Cpd B/X/LK001 was produced by reacting 2-methoxyethyl carbonisocyanatidate (2) (Krebs, A, et al., European Patent Office EP0230224B1 (1991)) with the commercially available (4-aminophenyl)(4-chlorophenyl)methanone (1) **(****Figure 6B****, Scheme 4).** Treatment of Cpd B/X/LK001 with hydroxylamine hydrochloride gave T4 as a ~1:1 mixture of E and Z isomers **(****Figure 6B****, Scheme 4).** Flubendazole (T6) and (4-aminophenyl)(4-fluorophenyl)methanone (T7) were sourced commercially.

**BT2, T4 and T6 inhibit serum-inducible endothelial FosB/ΔFosB and c-Fos expression, and block proliferation, migration and network formation *in vitro.*** We determined the effect of BT2, T4 and T6 on the serum-inducible expression of two different AP-1 subunits in cultured human microvascular endothelial cells (HMEC-1). Endothelial cells provide a vital barrier between the flowing blood and tissue that become hyperpermeable when activated or stressed (van Hinsbergh, V.W., et al., Arterioscler Thromb Vasc Biol 17, 1018-1023 (1997)). BT2 blocked the inducible expression of FosB and ΔFosB **(****Figures 1A** & **7A****).** T4 and T6 inhibited less potently, while BT3 and T7 demonstrated no inhibition **(****Figure 1A****).** BT2 also blocked the inducible expression of c-Fos, a known mediator of angiogenesis (Marconcini, L., et al., Proc Natl Acad Sci USA 96, 9671-9676 (1999)) **(****Figures 1A** & **7A****).**

We next investigated the effects of these compounds on endothelial cell growth using the xCELLigence system that monitors cell proliferation in real time. We found that BT2, T4 and T6 each inhibited serum-inducible proliferation at concentrations in a dose-dependent manner **(****Figure 1B****).** In contrast, BT3 or T7 had no inhibitory effect. To confirm that growth inhibition was not merely due to cell death, we tested one of these compounds (BT2) in a whole cell proliferation assay in which the Countess system is coupled with Trypan Blue exclusion. BT2 inhibited serum-inducible proliferation without affecting Trypan Blue incorporation **(****Figure 7B****).** In the dual chamber Transwell system, BT2, T4 and T6 inhibited migration of bovine aortic endothelial cells (BAEC) toward VEGF-A₁₆₅ in serum-containing medium **(****Figure 1C****).** BAEC were used for this purpose since HMEC-1 cells lack VEGFR-2 (Flk/KDR) and only weakly migrate toward VEGF (Shao, R., et al., Biochem Biophys Res Commun 321, 788-794 (2004)). BAEC, on the other hand, express VEGFR-2 (Lamy, S., et al., Cancer Res 62, 381-385 (2002)) and migrate to VEGF-A (Hussain, S., et al., BMC Cell Biol 9, 7 (2008)). In contrast, neither BT3, nor PD98059 (allosteric MEK inhibitor), imatinib (tyrosine kinase inhibitor) or tofacitinib (Janus kinase inhibitor) had an inhibitory effect at the same concentration **(****Figure 1C****).**

Endothelial cell repair after mechanical injury *in vitro* evokes a proliferative and migratory response. BT2, T4 and T6 blocked this reparative response within 48h, whereas BT3 or T7 had no such effect **(****Figure 1D****).** We also evaluated the effect of these compounds in an endothelial network formation assay (also known as tubule formation) atop reconstituted basement membrane matrix typically used to characterize angiogenic factors and processes (Arnaoutova, I., et al., Angiogenesis 12, 267-274 (2009)). Endothelial cells in this assay form capillary-like networks maximally within a few hours and regress thereafter. BT2, T4 and T6 inhibited network formation at 2, 4, 6 and 24 h **(****Figure 1E****).**

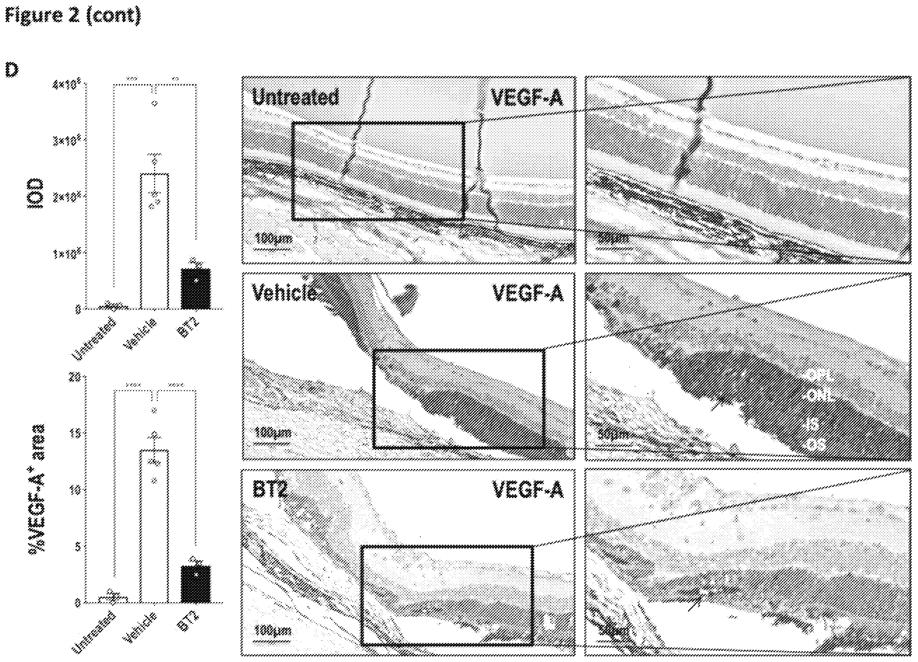

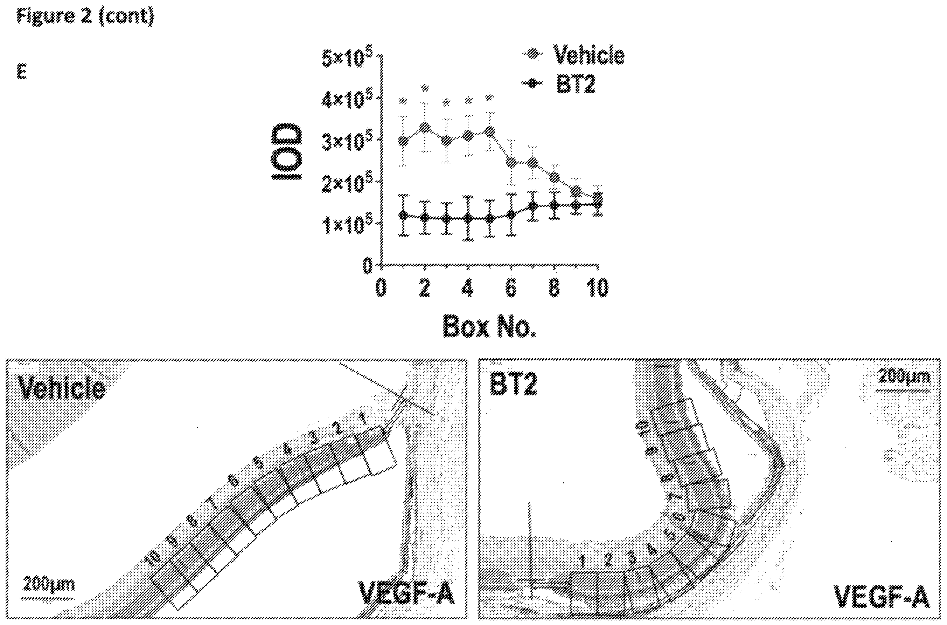

**BT2 prevents retinal vascular permeability and angiogenesis.** Since retinal vascular permeability is a key pathologic feature in nAMD and DME/DR (Campochiaro, P.A., et al., J Mol Med (Berl) 91, 311-321 (2013)), we sought to determine the effect of BT2, T4 and T6, on fluorescein leakage induced in eyes of Brown Norway pigmented rats after multiple laser burns of Bruch's membrane around the optic nerve (Grossniklaus, H.E., et al., Prog Retin Eye Res 29, 500-519 (2010)). BT2 (192µg) reduced retinal permeability by ~50%, an effect similar to aflibercept/Eylea (200µg administered 6 times (Days 0, 3, 7, 10, 14, 17) by intravitreal (IVT) injection over 21 days as compared with 2 injections (Days 0, 7) of BT2) or triamcinolone acetonide (Kenacort^{®} 200µg IVT, Day 0) **(****Figure 2A****).** In contrast, T4 and T6, delivered as per BT2, had no inhibitory effect **(****Figure 2A****).** Aflibercept is first-line therapy for nAMD and DME in the US, Europe and Asia-Pacific (Parikh, R., et al., Ophthalmol Retina 3, 16-26 (2019), while Kenacort is a corticosteroid commonly used to treat DME (Karacorlu, M., Eye (Lond) 19, 382-386 (2005)). BT2 also reduced vascular permeability induced by rhVEGF-A₁₆₅ in pigmented rabbits causing fluorescein leakage. Single IVT delivery of BT2 (600µg) inhibited retinal leakage after 2 days by ~50% **(****Figure 2B**). Immunohistochemical staining of lasered rat eyes 21 days after injury revealed that BT2 inhibited inducible CD31 staining in the IPL and INL **(****Figures 2C** **&** **8A****),** where CD31 is expressed after laser injury (Ju, X., et al., Clin Exp Pharmacol Physiol 46, 75-85 (2019)). BT2 also inhibited the inducible expression of VEGF-A₁₆₅ **(****Figure 2D****),** consistent with findings of VEGF expression mainly in the outer retina (Wang, X., et al., Int J Mol Sci 8, 61-69 (2007); Foureaux, G., et al., Braz J Med Biol Res 48, 1109-1114 (2015)). VEGF-A₁₆₅ stained in a gradient relative to the wound which was inhibited by BT2 **(****Figure 2E****).** The murine Matrigel plug assay confirmed the anti-angiogenic properties of BT2. Matrigel containing VEGF-A₁₆₅, heparin and compound was implanted subcutaneously into C57BL/6 mice and CD31 staining in plugs after 7 days was quantified. BT2 suppressed new blood vessel formation, whereas BT3 had no effect **(****Figures 2F** **&** **8B****).**

### BT2 inhibits ERK phosphorylation, FosB/ΔFosB and VCAM-1 expression.

Endothelial cells exposed to IL-1β undergo rapid ERK phosphorylation. IL-1β causes endothelial cell permeability (Puhlmann, M., et al., J Transl Med 3, 37 (2005)) and retinal leukostasis (Vinores, S.A., et al., J Neuroimmunol 182, 73-79 (2007)). Diabetics with macular edema have significantly higher concentrations of IL-1β among other cytokines and VEGF in the aqueous humor (Dong, N., et al., PLoS ONE 10, e0125329 (2015)). We used IL-1β as a model agonist with HMEC-1 in Western blotting experiments. BT2 inhibited IL-1β-inducible ERK phosphorylation, FosB/ΔFosB and VCAM-1 expression (**Figures 3A** **&** **9**). BT2 inhibition of VCAM-1 was further demonstrated by flow cytometry (**Figures 3B** **&** **10**).

RNA-sequencing affirmed BT2's ability to suppress IL-1β-inducible FosB and VCAM-1 expression **(****Figure 3C****).** From a pool of 33379 gene IDs, there were 325 genes induced by IL-1β 2-fold or more (logFC≥2) (Table 3C), 89 (27.5%) of which were inhibited by BT2 (logFC≥2) (**Table 3B**). Principal component analysis (PCA) (**Figure 3C****, *upper left***) showed close association between biological replicates. BT2 also inhibited a range of other regulatory genes involved in cell proliferation, migration, angiogenesis and inflammation including ICAM-1, CXCL2, KLF5, Egr-1 and Fos (**Figure 3C**).

Dose escalation and Western blotting experiments revealed that BT2 inhibited VCAM-1 and ERK phosphorylation more potently than PD98059 (**Figures 3D** **&** **11A**). In contrast, BT2, like PD98059, had no effect on IL-1β-inducible p-SAPK/JNK or p-p38 (**Figure 11B**). To explore the hitherto unrecognized dependence of VCAM-1 expression upon FosB/ΔFosB we performed siRNA knockdown experiments. FosB siRNA inhibited both FosB/ΔFosB and VCAM-1 whereas VCAM-1 siRNA inhibited VCAM-1 but not FosB/ΔFosB (**Figure 3E**). Overexpression of ERK1 did not increase levels of phosphorylated ERK, compared with IL-1β stimulation, nor did it increase levels of FosB, ΔFosB or VCAM-1 (**Figure 11C**). Similarly, overexpression of FosB or ΔFosB did not increase VCAM-1 expression compared with IL-1β stimulation (**Figure 11C**). These findings indicate that, in this agonist-free system where ERK is not phosphorylated, FosB and VCAM-1 are not directly activated by ERK1 overexpression, nor is VCAM-1 directly activated by FosB or ΔFosB overexpression. These data complement our demonstration (under conditions of agonist stimulation) that BT2, which prevents ERK phosphorylation, abrogates the induction of FosB/ΔFosB and VCAM-1 *in vitro* (**Figures 3A-D**) and *in vivo* (**Figures 4A-E**). Indeed, BT2 physically interacts with MEK1 (**Figure 5C**), which phosphorylates ERK (Qi, M., et al., Journal of Cell Science 118, 3569-3572 (2005)).

These findings, taken together with our demonstration that IL-1β induction of VCAM-1 is blocked with FosB siRNA (Figure 3E) suggest that while FosB is necessary for cytokine-inducible VCAM-1 expression, FosB overexpression alone is not sufficient to induce VCAM-1 without cytokine stimulation. FosB likely relies upon co-factors (or post-translational modifications) under conditions of cytokine stimulation.

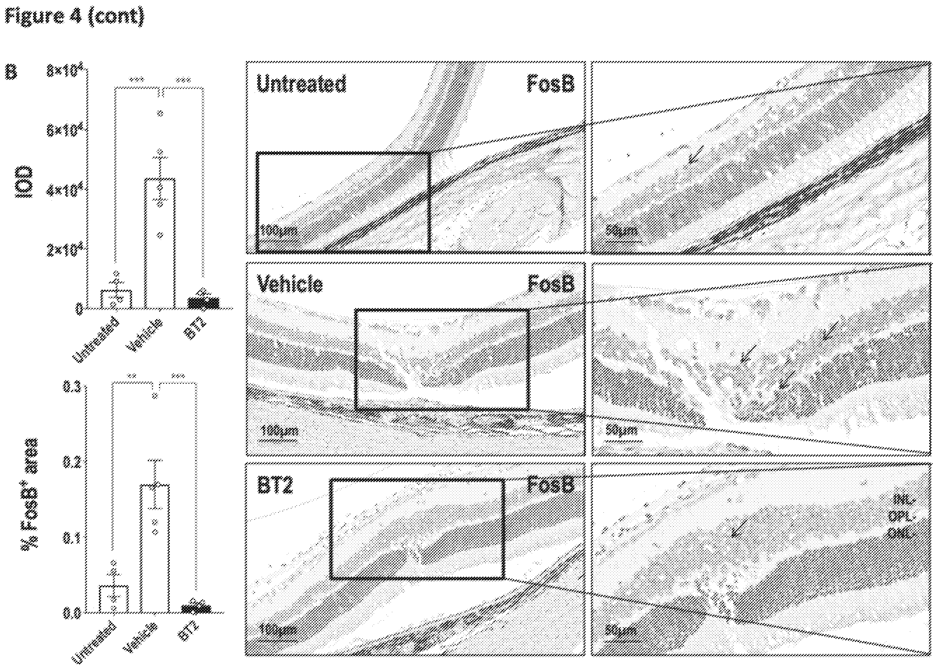

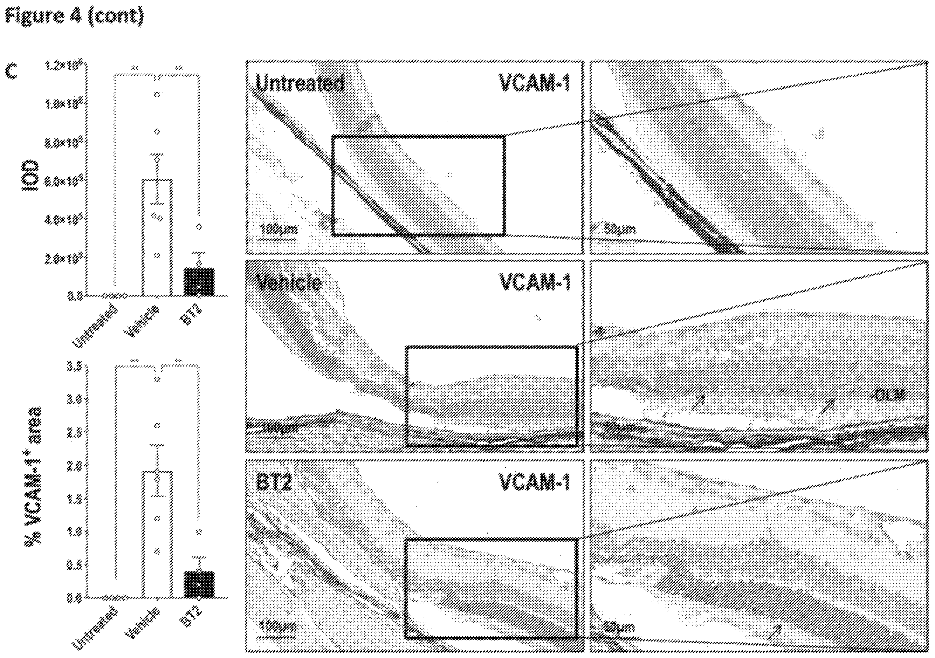

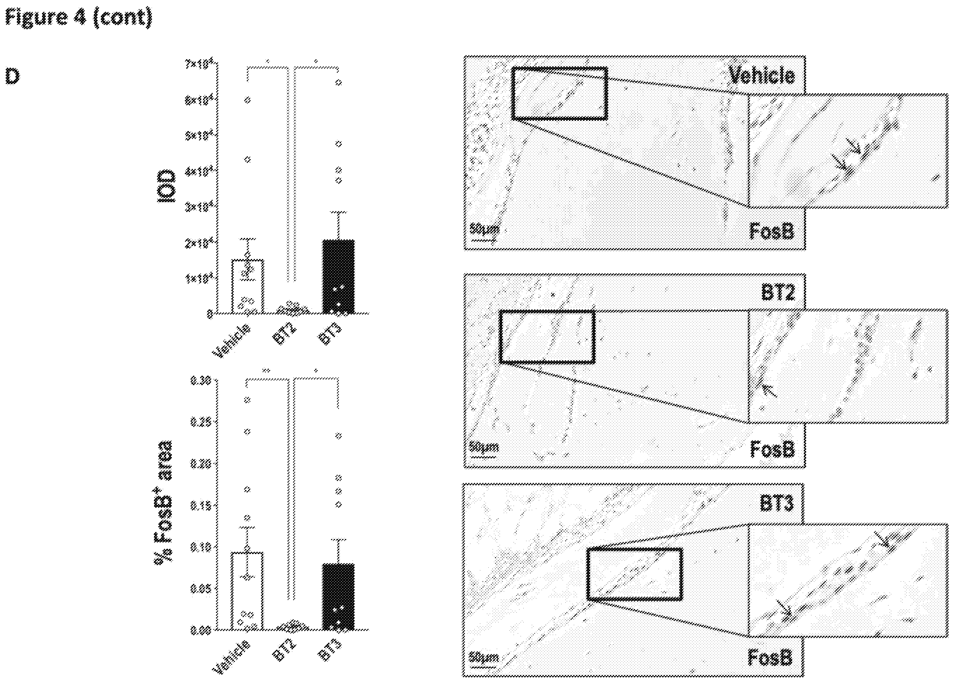

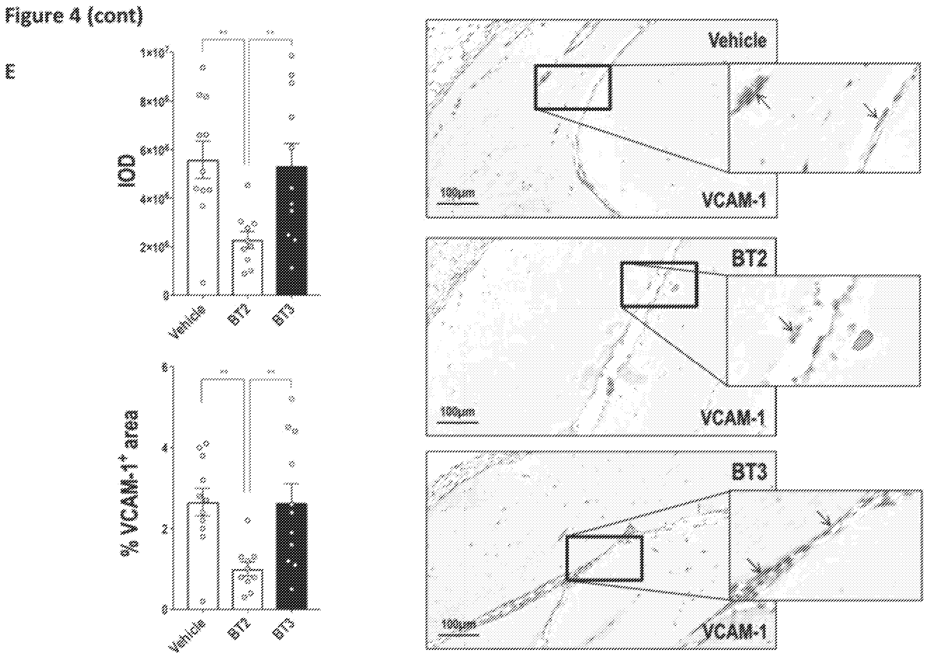

Immunohistochemical staining of rat retinas revealed that BT2 suppressed inducible pERK staining in the INL, OPL and ONL (Figure 4A) consistent with pERK expression in this proximity (Takeda, M., et al., Invest Ophthalmol Vis Sci 43, 907-911 (2002); Caicedo, A., et al., Exp Eye Res 81, 38-47 (2005)). BT2 also inhibited retinal FosB immunostaining (Figure 4B). Moreover, BT2 reduced inducible VCAM-1 expression in the OLM (Figure 4C) where others have found that VCAM-1 is expressed (Makhoul, M., et al., Exp Eye Res 101, 27-35 (2012)). BT2 also inhibited FosB (Figures 4D & 8C) and VCAM-1 (Figure 4E) immunoreactivity in Matrigel plugs.

In further experiments, we compared the biological potency of BT2 with curcumin (Ye, N., et al., J Med Chem 57, 6930-6948 (2014)) in the endothelial network formation assay. While BT2 abolished network formation at 1µM after 4h, no inhibition was observed with curcumin at this concentration (Figure 12). Curcumin appeared to inhibit network formation at 30µM by ~25% and 40µM by ~50% (Figure 12), indicating BT2 is >40-fold more potent than curcumin in this assay.

**BT2 structural analogues lack the biological potency of BT2.** We next explored whether the biological potency or solubility of BT2 could be improved by structural modification. Dibenzoxazepinones are typically poorly soluble in water. Six BT2 analogues (aside from BT3) were generated (BT2-MeOA, BT2-EOMe, BT2-Pr, BT2-IC, BT2-MO, BT2-IMO) (Table 1). BT2-MeOA was synthesized by coupling methoxyacetic acid with 2-amino-10-ethyldibenzo[b,f][1,4] oxazepin-11 (10H)-one (BT3) while BT2-IC was synthesized using the diisobutyl dicarbonate **(****Figure 6B****, Scheme 1).** BT2-Pr and BT2-EOMe were synthesized from commercially available (1) and (2) (Figure 6B, Scheme 2) with the same protocol used to prepare BT2. A tri-deuterated derivation of BT2 was synthesized for stability analysis by alkylating 2-nitro-10H-dibenzo[b,f][1,4]oxazepin-11-one (3) with d₃-iodoethane followed by reduction of the nitro group to give compound (4) (Figure 6B, Scheme 3). This intermediate was reacted with diethyl pyrocarbonate to give the desired product. Alkylation of 2-nitro-10H-dibenzo[b,f][1,4]oxazepin-11-one (3) with oxetan-3-ylmethyl methanesulfonate gave both O- and N- alkylated products in 13% and 48% yield respectively after column chromatography (Figure 6B, Scheme 3). The O- and N-alkylated products were reduced to give the corresponding anilines (5) and (6) (Figure 6B, Scheme 3) and then converted in the usual manner using diethyl pyrocarbonate to the target products BT2-IMO and BT2-MO.

Dilution of these compounds in medium containing serum revealed that only one these analogues (BT2-MeOA) had greater solubility than BT2 and that BT3 was the most soluble of all these dibenzoxazepinones. Adding serum to the diluent increased BT2 solubility, consistent with reports that serum albumin can increase the dissolution of unionizable drugs (Khoder, M., et al., Pharm Dev Technol 23, 732-738 (2018)). Neither BT2-MeOA nor any other BT2 analogue had the ability to inhibit serum-inducible proliferation (Figure 5A) or network formation on Matrigel (Figure 5B) as, or more potently than BT2. BT2-IC showed some inhibition of network formation at higher concentrations (Figure 13B).

Since BT2 suppressed ERK phosphorylation, we hypothesized that BT2 may interact with MEK1 or MEK2. Binding of BT2 and PD98059 to recombinant His-MEK-1 or His-MEK2 was tested by surface plasmon resonance (SPR). Over the concentration range able to be assayed, BT2 bound to His-MEK1 significantly better than to His-MEK2 (Figure 5C). In contrast, and as expected, PD98059 bound to both His-MEK1 and His-MEK2 (Dudley, D.T., et al., Proc Natl Acad Sci U S A 92, 7686-7689 (1995)) (Figure 5C). Over equivalent concentration ranges, there was no appreciable binding of BT3, BT2-MeOA or BT2-Pr to either MEK1 or MEK2. BT2-IC showed some interaction with MEK1 (but not MEK2). The drop-off in interaction at higher concentrations is possibly due to BT2-IC insolubility (limit of solubility 8±2µM determined by ¹H 1D NMR spectroscopy). Western blotting revealed that BT2-IC inhibited both ERK phosphorylation (Figure 13A) and network formation (Figure 13B) at 3µM (albeit less potently than BT2) but not at 1µM (Figures 5B & D).

BT2 retains stability and biological potency after sonication and 100°C treatment or autoclaving. Finally, in considering the potential pharmaceutical appeal of BT2, we explored whether this compound (as a sonicated preparation in saline containing 0.01% DMSO and 0.5% Tween 80) retained biological potency and stability after extreme heat treatment. Rapid resolution liquid chromatography/tandem mass spectrometry (RRLC-MS/MS) revealed that BT2 remains stable with or without heat treatment (100°C for 10min) and 6 weeks storage at 22°C, with only 0.2% and 1% discrepancy in BT2 content in non-heat treated and heat-treated formulations, respectively (Figures 14A-B). BT2 retained its ability to inhibit serum-inducible endothelial proliferation under these conditions **(****Figure 14C****).** Even more surprisingly, there was no loss in biological efficacy or degradation even up to 16 months **(****Figures 14D-F).** Remarkably, the BT2 formulation remained stable and biologically active 4 months after standard autoclaving and storage at 22°C **(****Figure 14G****).** Antibodies and other proteins, which comprise all current nAMD/DME drugs, are typically inactivated by extreme heat (Jones, F.S., J Exp Med 46, 291-301 (1927).

**BT2 inhibits monocytic cell adhesion to IL-1β-treated endothelium** *in vitro* **and monocytic transendothelial migration toward MCP-1** *in vitro.* VCAM-1 mediates monocyte adhesion in human umbilical vein endothelial cells (Gerszten, R.E., et al., Circ Res 82, 871-878 (1998)). In an *in vitro* model involving calcein-labeled THP-1 monocytic cells and endothelial cells pretreated with IL-1β, THP-1 adhesion to endothelial cells is inhibited by BT2 **(****Figure 15A****).** BT2 also inhibits the transendothelial migration of THP-1 monocytes toward MCP-1 from the upper chamber to the lower chamber **(****Figure 15B****).**

**Intraperitoneal administration of BT2 prevents footpad swelling, bone destruction and VCAM-1 and ICAM-1 expression in arthritic mice.** Having established the *in vitro* anti-angiogenic and anti-inflammatory properties of BT2, we hypothesized that BT2 may be useful in a complex pro-inflammatory setting such as collagen antibody induced arthritis (Khachigian, L.M. Nature Protocols 1, 2512-2516 (2006)). Hind footpad thickness induced in this model is inhibited by a single administration of 30mg/kg BT2 **(****Figures 16A** **&** **B).** H&E staining revealed significant inflammation in CAIA mice injected is reduced by BT2 **(****Figure 16C****).** 3D micro-CT analysis on hind limbs revealed that BT2 inhibits bone destruction **(****Figures 16D** **&** **E).** In support of these findings, we made use of the fact that tartrate-resistant acid phosphatase (TRAP) activity is a key histocytochemical marker of bone-degrading osteoclasts (Ballanti, P., et al., Osteoporosis International 7, 39-43 (1997)). BT2 reduced TRAP staining in the joints **(****Figure 16F****).** BT2 was found to inhibit VCAM-1 and ICAM-1 expression in bone **(****Figure 16G****).** Additionally, BT2 (30 mg/kg) reduced plasma levels of IL-1β, IL-2 and IL-6 to normal levels but did not change IL-4 or IL-10.

**No evidence of BT2 toxicity following intraperitoneal, intraarticular or gavage administration.** BT2 (3 or 30mg/kg) was administered to Balb/c mice by one of 3 routes (intraperitoneal injection, intraarticular injection or oral gavage) and tissues were assessed for signs of toxicity. There was no histopathological evidence of toxic damage due to BT2 **(Table** 2). Livers from most mice in all groups contained minimal to mild, infrequent inflammatory foci, occasionally associated with necrosis of individual hepatocytes or small groups of hepatocytes. This is likely a common, spontaneous background lesion in laboratory mice (Taylor, I. Mouse. in Background lesions in laboratory animals (ed. McInnes, E.F.) 45-75 (Saunders Elsevier, Edinburgh, 2012)) and not test item related. Livers from most mice in groups administered i.p. exhibited minimal to mild inflammation over the capsule, consistent with a non-specific peritoneal reaction to the injection and the effect being unrelated to the test item. Kidneys from one of 5 control mice and 4 of 30 BT2-treated mice contained infrequent inflammatory foci. Again this is a common spontaneous background lesion in laboratory mice and not test item related. Inflammation involving the pelvis of the kidney may have been due to ascending bacterial infection of the urinary tract. In liver and lung, there were other very infrequent, minimal changes not related to the treatment group. In summary, there was no histopathological evidence of toxicity following intraperitoneal, intraarticular injection or gavage administration of BT2.

Finally, GLP-compliant pharmacokinetics and ocular tolerance studies conducted by Iris Pharma (France), single intravitreal injection in rabbits (10µg/50µl BT2) revealed that BT2 is well tolerated macroscopically and histologically after 28 days with an ocular half-life (t_{1/2}) of 3.3 days.

**Table 2. Severity grading of histopathology findings following intraarticular, intraperitoneal or gavage delivery of BT2. Severity of lesions was graded histologically as follows: 0=no abnormalities detected; 1=minimal change; 2=mild change; 3=moderate change; 4=severe change; NA=not assessed. n=5 mice per group. IA denotes intraarticular, IP intraperitoneal. Vehicle was DMSO.**

| Group | Control (Untreated) | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 1 | 1 | 1 | 0 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 1 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | IA Vehicle | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 0 | 1 | 1 | 1 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 0 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | IA BT2 3 mg/kg | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 1 | 1 | 1 | 0 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 1 | 0 | 0 | 0 |
| . inflammation, renal pelvis | 2 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | IA BT2 30 mg/kg | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 1 | 1 | 1 | 1 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 0 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | IP Vehicle | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 0 | 1 | 1 | 1 | 1 |
| . inflammation, peritoneal | 1 | 2 | 1 | 1 | 1 |
| . mineralisation, subcapsular | 0 | 0 | 0 | 0 | 0 |
| Kidney (left and right) | | | | | |
| . inflammation, interstitial | 0 | 0 | 0 | 0 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | IP BT2 3 mg/kg | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 1 | 0 | 1 | 1 |
| . inflammation, peritoneal | 0 | 0 | 2 | 1 | 1 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 0 | 0 |
| . inflammation, renal pelvis | 0 | 0 | 0 | 0 | 0 |

| Lung | | | | | |
|---|---|---|---|---|---|
| . osseous metaplasia | 0 | 1 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | IP BT2 30 mg/kg | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 1 | 1 | 1 | 2 |
| . inflammation, peritoneal | 1 | 1 | 2 | 1 | 2 |
| . mineralisation, subcapsular | 0 | 1 | 0 | 0 | 0 |

| Kidney (left and right) | | | | | |
|---|---|---|---|---|---|
| . inflammation, interstitial | 0 | 0 | 0 | 0 | 0 |
| . inflammation, renal pelvis | 0 | 0 | 0 | 4 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | Gavage Vehicle | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 0 | 1 | 1 | 1 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 0 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | NA |

| Group | Gavage BT2 3 mg/k | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 0 | 0 | 0 | 1 | 1 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 1 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

| Group | Gavage BT2 30 mg/kg | | | | |
|---|---|---|---|---|---|
| Animal | Mouse 1 | Mouse 2 | Mouse 3 | Mouse 4 | Mouse 5 |
| Liver | | | | | |
| . inflammation, parenchymal | 1 | 0 | 0 | 1 | 0 |
| . inflammation, peritoneal | 0 | 0 | 0 | 0 | 0 |
| . mineralisation, subcapsular | 0 | 0 | 0 | 0 | 0 |

| Kidney (left and right) | | | | | |
|---|---|---|---|---|---|
| . inflammation, interstitial | 0 | 0 | 0 | 0 | 0 |
| . inflammation, renal pelvis | 0 | 0 | 0 | 0 | 0 |

| Lung | | | | | |
|---|---|---|---|---|---|
| . osseous metaplasia | 0 | 0 | 0 | 0 | 0 |
| Spleen | 0 | 0 | 0 | 0 | 0 |
| Heart | 0 | 0 | 0 | 0 | 0 |
| Eye (left and right) | 0 | 0 | 0 | 0 | 0 |

### Discussion

New therapeutic approaches complementing existing VEGF-based strategies for nAMD/DR are needed (Apte, R.S., et al., Cell 176, 1248-1264 (2019)). While IVT anti-VEGF remains first-line therapy for retinal leakage, alternative therapies are required as many patients do not respond optimally, or the response is not sustained. The Comparison of AMD Treatments Trials (CATT) study with 647 nAMD patients treated with ranibizumab or bevacizumab showed that vision gains during the first 2 years were not maintained at 5 years (Maguire, M.G., et al., Ophthalmology 123, 1751-1761 (2016); Pedrosa, A.C., et al., Clin Ophthalmol 10, 541-546 (2016)). The AURA^{iv} study of 2227 nAMD patients in 8 European countries also revealed that while anti-VEGF therapy resulted in initial improvement in visual acuity, gains were not maintained over time and declined, mainly due to undertreatment (Holz, F.G., et al., Br J Ophthalmol 99, 220-226 (2015)).

Here we report our discovery and biological characterization of a novel dibenzoxazepinone from a high throughput screen of ~100,000 compounds. BT2 blocks cell proliferation, migration, wound repair and network formation *in vitro.* This compound demonstrates efficacy in animal models of vascular leakage and angiogenesis (Carneiro, A., et al., Acta Ophthalmol 87, 517-523 (2009); Ameri, H., et al., Invest Ophthalmol Vis Sci 48, 5708-5715 (2007); Pan, C.K., et al., J Ocul Pharmacol Ther 27, 219-224 (2011)) that have served as key platforms in the development of nAMD/DR therapies used by millions today. BT2 prevented retinal vascular permeability in rats following choroidal laser injury as effectively as first-line therapy for nAMD and DME following 6 aflibercept injections compared with 2 of BT2 at the same dose. BT2 reduced CD31 staining in the IPL and INL, consistent with VEGF-A gain-of-function studies in amacrine and horizontal cells after studies that crossed *Ptf1a-Cre* mice with floxed *Vhl (Vhlf*/*f)* mice to induce pseudohypoxia revealed massive neovascularization in the IPL and INL (Usui, Y., et al., J Clin Invest 125, 2335-2346 (2015)). In rabbits, we found that BT2 inhibited retinal vascular leakiness induced by VEGF-A₁₆₅.

While BT2 suppressed the inducible expression of VEGF-A₁₆₅, its effects in the retina were not confined to VEGF. BT2 inhibited ERK activation and VCAM-1 expression, both implicated in the pathogenesis of nAMD and DR (Kyosseva, S.V., et al., Ophthalmol Eye Dis 8, 23-30 (2016); Ye, X., et al., Invest Ophthalmol Vis Sci 53, 3481-3489 (2012); Jonas, J.B., et al., Arch Ophthalmol 128, 1281-6 (2010); Barile, G.R., et al., Curr Eye Res 19, 219-227 (1999)). Our findings suggest the existence of a pERK-FosB/ΔFosB-VCAM-1 cascade under conditions of cytokine stimulation. BT2 also inhibited a range of other genes involved in cell growth, migration, angiogenesis and inflammation. BT2 is more potent than PD98059 and >40-fold more potent than curcumin, the main active ingredient in the golden spice turmeric that inhibits AP-1 (Ye, N., et al., J Med Chem 57, 6930-6948 (2014) and is widely used for medicinal purposes despite double-blind placebo controlled clinical trials of curcumin not having been successful (Nelson, K.M., et al., J Med Chem 60, 1620-1637 (2017)).

We synthesised BT2 analogues bearing a variety of substitutions at the 2- and 10-positions of the **2-amino-dibenzo[b,f][1,4]** oxazepin-11(10H)-one ring system. Minor variations of the carbamate moiety (BT2-MeOA and BT2-IC) markedly affected activity as did modifications at the 10- position (BT2-Pr, BT2-EOMe, BT2-MO and BT2-IMO). We expected that BT2-EOMe, BT2-MO and BT2-IMO, all of which have lower calculated log Ps, would have increased water solubility. Although BT2-MeOA (and BT3) were more soluble than BT2, two separate assays revealed BT2 remained the most biologically potent of all these compounds indicating that larger substituents at the 2- and 10-positions are not advantageous. Comparison of BT2 with BT2-MeOA, which has the same molecular formula/weight and is an isomer of BT2 (linked through an amide) indicates that the carbamate moiety at the 2- position in BT2 is critical to BT2 function. BT2 may be amenable to lipid-based drug delivery systems, such as self-emulsifying delivery methodologies, that have improved oral absorption of poorly water-soluble drugs and facilitated high-dose toxicological studies (Chen, X.Q., et al., J Pharm Sci 107, 1352-1360 (2018)).

Rodent and rabbit models are useful in recreating certain features of retinal disease in humans, but may not totally recapitulate the human condition since nAMD and DR are complex, multifactorial chronic diseases that cannot be precisely recreated in acute experiments with single stimuli (Robinson, R., et al., Dis Model Mech 5, 444-456 (2012)). While rats offer advantages of rapid disease progression and comparative low cost, rats (like mice) do not possess a macula (Pennesi, M.E., et al., Mol Aspects Med 33, 487-509 (2012)). The size of the rabbit eye is more akin to the human eye but its posterior segment circulation differs from primates and rodents and rabbits also lack a macula (Chen, S., et al., Expert Rev Opthalmol 9, 285-295 (2014)). BT2 may overcome limitations in translatability that have hampered the broader use of humanized and species-specific reagents in animal models (Lu, F., et al., Graefes Arch Clin Exp Ophthalmol 247, 171-177 (2009)).

*BT2 effects outside the retina.* There is also a need for new and effective anti-inflammatory and anti-arthritic agents. Around one third of patients treated with a TNF inhibitor do not achieve 20% improvement based on American College of Rheumatology criteria (Klak, A., et al., Rheumatologia 54, 177-186 (2016)); Rubbert-Roth, A. & Finckh, A. Arthritis Research & Therapy 11 Suppl 1, S1 (2009)) which may relate to serum IFN-β/α ratio (Wampler Muskardin, T., et al., Annals of the Rheumatic Diseases 75, 1757-1762 (2016)). p-ERK levels are elevated in synovial tissue from RA patients compared with normal individuals (Thiel, M.J., et al., Arthritis Rheum 56, 3347-3357 (2007)). Moreover, serum sVCAM1 levels reflect the clinical status in RA (Navarro-Hernandez, R.E. et al., Disease Markers 26, 119-126 (2009)) and decrease in RA patients as the condition is relieved (Wang, L., et al., Experimental and Therapeutic Medicine 10, 1229-1233 (2015)). We found that BT2 delivered systemically in CAIA mice inhibited joint inflammation and bone erosion. BT2 also suppressed monocytic cell adhesion to endothelial cells and monocytic transendothelial migration to MCP-1 *in vitro.* Moreover systemic administration of BT2 in mice prevents footpad swelling, TRAP staining and bone destruction. Inflammation is also thought to drive all phases of atherosclerosis, from initiation, progression, and ultimately plaque rupture and infarction, causing further inflammation. The recent CANTOS (Hansson, G.K. Circulation 136, 1875-7 (2017); Ridker, P.M., et al. N Engl J Med 377, 1119-31 (2017)), COLCOT (Tardif, J.C., et al. N Engl J Med 381:2497-2505 (2019)) and tocilizumab (Kleveland, O., et al. Eur Heart J 37, 2406-13 (2016)) clinical trials revealed that inflammation is a treatable mechanism in cardiovascular disease. However, patients treated with existing anti-inflammatory approaches (such as canakinumab and colchicine) remain at considerable risk for major adverse cardiac events even with the widespread use of statins and antiplatelet therapies (Ridker, P.M., et al. N Engl J Med 377, 1119-31 (2017); Tardif, J.C., et al. N Engl J Med 381:2497-2505 (2019); Thompson, P.L. Clin Ther. 41, 41:8-10 (2019). There is also a paucity of clinically effective anti-inflammatory small molecule drugs for cardiovascular disease beyond statins (Collins, R. et al. Lancet 388, 2532-61 (2016)). This indicates the therapeutic potential of BT2 in RA and other inflammatory disease.

In conclusion, BT2 offers a new tool in the armamentarium targeting vascular permeability, angiogenic and inflammatory indications. BT2 served as a molecular tool to establish an ERK-FosB-VCAM1 axis mediating vascular permeability. Together with a favorable toxicological profile, our findings suggest clinical utility of this compound for retinal disease and RA. Unlike current clinically used antibody- or protein-based therapies that principally target the VEGF system, BT2 inhibits the inducible expression of multiple genes that underpin angiogenic and inflammatory processes not limited to VEGF. That BT2 retains biological potency even after boiling or autoclaving and several months' storage at room temperature adding further to its pharmaceutical appeal. Like triamcinolone acetonide, BT2 is poorly soluble in water and as such, could potentially offer a further advantage that a bolus injection can form a depot at the site of injection facilitating gradual release (Yang, Y., et al., Retina 35, 2440-2449 (2015)). Moreover, BT2 may be used in intravitreal reservoirs or implant strategies and ocular delivery systems facilitating sustained release (Kang-Mieler, J.J., et al. Eye (Lond) 34, 1371-1379 (2021)).

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

**Table 3A: Difference in Gene expression induced by IL-1β and induced by IL-1β in the presence of BT2**

| | | FC = Fold Change | | | |
|---|---|---|---|---|---|
| **Gene** | **Description** | **logFC** | **lfcSE** | **PValue** | **FDR** |
| ENSG00000130518 | KIAA1683(KIAA1683) | -6.046181314 | 0.203731903 | 1.51E-193 | 4.45E-190 |
| ENSG00000170345 | Fos proto-oncogene, AP-1 transcription factor subunit(FOS) | -5.81280372 | 0.165839111 | 3.78E-269 | 4.45E-265 |
| ENSG00000105371 | intercellular adhesion molecule 4 (Landsteiner-Wiener blood group)(ICAM4) | -5.291180651 | 0.217067201 | 3.09E-131 | 3.64E-128 |
| ENSG00000128016 | ZFP36 ring finger protein(ZFP36) | -5.233766514 | 0.751072833 | 3.21E-12 | 7.96E-11 |
| ENSG00000104833 | tubulin beta 4A class IVa(TUBB4A) | -5.230664532 | 0.764574959 | 7.85E-12 | 1.83E-10 |
| ENSG00000081041 | C-X-C motif chemokine ligand 2(CXCL2) | -4.84494193 | 0.148984477 | 5.60E-232 | 3.30E-228 |
| ENSG00000198576 | activity regulated cytoskeleton associated protein(ARC) | -4.770568608 | 0.232366353 | 1.15E-93 | 8.48E-91 |
| ENSG00000203811 | histone cluster 2 H3 family member c(HIST2H3C) | -4.763925928 | 0.250691678 | 1.61E-80 | 1.05E-77 |
| ENSG00000179388 | early growth response 3(EGR3) | -4.748606448 | 0.274536514 | 4.97E-67 | 2.34E-64 |
| ENSG00000274276 | cystathionine-beta-synthase like(CBSL) | -4.578933527 | 1.764517748 | 0.009458936 | 0.027015035 |
| ENSG00000163734 | C-X-C motif chemokine ligand 3(CXCL3) | -4.487286536 | 0.161163632 | 1.31E-170 | 3.08E-167 |
| ENSG00000171223 | JunB proto-oncogene, AP-1 transcription factor subunit(JUNB) | -4.439704537 | 0.276779365 | 6.65E-58 | 2.52E-55 |
| ENSG00000125740 | FosB proto-oncogene, AP-1 transcription factor subunit(FOSB) | -4.39246161 | 0.176769766 | 2.68E-136 | 4.51E-133 |
| ENSG00000188483 | immediate early response 5 like(IER5L) | -4.233292009 | 0.241812304 | 1.28E-68 | 6.54E-66 |
| ENSG00000116741 | regulator of G-protein signaling 2(RGS2) | -4.113629168 | 0.277042966 | 7.13E-50 | 2.21E-47 |
| ENSG00000120738 | early growth response 1(EGR1) | -4.070710146 | 0.157557175 | 3.46E-147 | 6.78E-144 |
| ENSG00000123358 | nuclear receptor subfamily 4 group A member 1(NR4A1) | -4.035692161 | 0.171853391 | 6.04E-122 | 5.92E-119 |
| ENSG00000164400 | colony stimulating factor 2(CSF2) | -3.818708412 | 0.167915715 | 1.73E-114 | 1.56E-111 |
| ENSG00000136244 | interleukin 6(IL6) | -3.773082183 | 0.151957758 | 4.27E-136 | 6.28E-133 |
| ENSG00000214391 | NA | -3.757571821 | 0.6688129 | 1.93E-08 | 2.56E-07 |
| ENSG00000177606 | Jun proto-oncogene, AP-1 transcription factor subunit(JUN) | -3.713380171 | 0.172873661 | 2.38E-102 | 1.87E-99 |
| ENSG00000105289 | tight junction protein 3(TJP3) | -3.625909887 | 0.623153392 | 5.93E-09 | 8.69E-08 |
| ENSG00000181218 | histone cluster 3 H2A(HIST3H2A) | -3.620778057 | 0.401690942 | 1.99E-19 | 1.21E-17 |
| ENSG00000267059 | ubiquinol-cytochrome c reductase, complex III subunit XI(UQCR11) | -3.610917086 | 1.248901826 | 0.003836837 | 0.012646835 |
| ENSG00000169429 | C-X-C motif chemokine ligand 8(CXCL8) | -3.607945957 | 0.230957755 | 5.18E-55 | 1.85E-52 |
| ENSG00000158373 | histone cluster 1 H2B family member d(HIST1H2BD) | -3.501034382 | 0.231843553 | 1.60E-51 | 5.37E-49 |
| ENSG00000137285 | tubulin beta 2B class IIb(TUBB2B) | -3.467774689 | 0.343920164 | 6.56E-24 | 6.08E-22 |
| ENSG00000163739 | C-X-C motif chemokine ligand 1(CXCL1) | -3.421722765 | 0.143479818 | 1.06E-125 | 1.13E-122 |
| ENSG00000089692 | lymphocyte activating 3(LAG3) | -3.415913177 | 0.271278765 | 2.34E-36 | 4.37E-34 |
| ENSG00000162692 | vascular cell adhesion molecule 1(VCAM1) | -3.357244281 | 0.270405547 | 2.15E-35 | 3.72E-33 |
| ENSG00000130513 | growth differentiation factor 15(GDF15) | -3.349977694 | 0.242284901 | 1.76E-43 | 4.23E-41 |
| ENSG00000156427 | fibroblast growth factor 18(FGF18) | -3.320172319 | 0.285046241 | 2.35E-31 | 3.38E-29 |
| ENSG00000168477 | tenascin XB(TNXB) | -3.263931496 | 0.272800853 | 5.45E-33 | 8.55E-31 |
| ENSG00000218426 | NA | -3.206669489 | 1.290276202 | 0.012945751 | 0.035002025 |
| ENSG00000115844 | distal-less homeobox 2(DLX2) | -3.200287357 | 0.265673919 | 2.04E-33 | 3.38E-31 |
| ENSG00000272196 | histone cluster 2 H2A family member a4(HIST2H2AA4) | -3.149273568 | 0.706938519 | 8.40E-06 | 6.10E-05 |
| ENSG00000221869 | CCAAT/enhancer binding protein delta(CEBPD) | -3.11155979 | 0.191503048 | 2.30E-59 | 9.68E-57 |
| ENSG00000160888 | immediate early response 2(IER2) | -3.10770709 | 0.177166769 | 6.95E-69 | 3.89E-66 |
| ENSG00000115009 | C-C motif chemokine ligand 20(CCL20) | -3.088261993 | 0.206803164 | 2.00E-50 | 6.36E-48 |
| ENSG00000278828 | histone cluster 1 H3 family member h(HIST1H3H) | -3.070062967 | 0.431188994 | 1.08E-12 | 2.87E-11 |
| ENSG00000188293 | IGF like family member 1(IGFL1) | -3.038575344 | 0.189136331 | 4.45E-58 | 1.75E-55 |
| ENSG00000125968 | inhibitor of DNA binding 1, HLH protein(ID1) | -3.021043337 | 0.226721958 | 1.66E-40 | 3.55E-38 |
| ENSG00000118503 | TNF alpha induced protein 3(TNFAIP3) | -2.937976363 | 0.147054779 | 8.42E-89 | 5.83E-86 |
| ENSG00000163435 | E74 like ETS transcription factor 3(ELF3) | -2.915501624 | 0.155672702 | 2.90E-78 | 1.80E-75 |
| ENSG00000127129 | endothelin 2(EDN2) | -2.848547622 | 0.206057819 | 1.83E-43 | 4.30E-41 |
| ENSG00000123416 | tubulin alpha 1b(TUBA1B) | -2.84448183 | 0.153278627 | 7.08E-77 | 4.17E-74 |
| ENSG00000167549 | coronin 6(CORO6) | -2.842329488 | 0.232015853 | 1.67E-34 | 2.84E-32 |
| ENSG00000164949 | GTP binding protein overexpressed in skeletal muscle(GEM) | -2.820631629 | 0.342870063 | 1.93E-16 | 8.40E-15 |
| ENSG00000167992 | von Willebrand factor C and EGF domains(VWCE) | -2.750162871 | 0.381950327 | 6.01E-13 | 1.64E-11 |
| ENSG00000196787 | histone cluster 1 H2A family member g(HIST1H2AG) | -2.743526046 | 0.374289447 | 2.30E-13 | 6.89E-12 |
| ENSG00000182759 | MAF bZIP transcription factor A(MAFA) | -2.741653406 | 0.2948384 | 1.42E-20 | 9.83E-19 |
| ENSG00000139329 | lumican(LUM) | -2.7112042 | 0.317961886 | 1.50E-17 | 7.37E-16 |
| ENSG00000107731 | unc-5 netrin receptor B(UNC5B) | -2.688686545 | 0.624988231 | 1.69E-05 | 0.00011412 |
| ENSG00000136826 | Kruppel like factor 4(KLF4) | -2.684611128 | 0.193028264 | 5.67E-44 | 1.42E-41 |
| ENSG00000250067 | YjeF N-terminal domain containing 3(YJEFN3) | -2.680562267 | 0.320868637 | 6.59E-17 | 3.05E-15 |
| ENSG00000120129 | dual specificity phosphatase 1(DUSP1) | -2.659219592 | 0.153210752 | 1.76E-67 | 8.62E-65 |
| ENSG00000197903 | histone cluster 1 H2B family member k(HIST1H2BK) | -2.657370655 | 0.686153716 | 0.00010757 | 0.000581817 |
| ENSG00000102878 | heat shock transcription factor 4(HSF4) | -2.650455425 | 0.227371798 | 2.12E-31 | 3.07E-29 |
| ENSG00000152518 | ZFP36 ring finger protein like 2(ZFP36L2) | -2.598134923 | 0.160517911 | 6.34E-59 | 2.57E-56 |
| ENSG00000184588 | phosphodiesterase 4B(PDE4B) | -2.596960186 | 0.43036967 | 1.60E-09 | 2.58E-08 |
| ENSG00000105376 | intercellular adhesion molecule 5(ICAM5) | -2.593632739 | 0.25816455 | 9.53E-24 | 8.75E-22 |
| ENSG00000115738 | inhibitor of DNA binding 2, HLH protein(ID2) | -2.582567927 | 0.233411178 | 1.87E-28 | 2.29E-26 |
| ENSG00000139289 | pleckstrin homology like domain family A member 1(PHLDA1) | -2.567199114 | 0.154753572 | 8.38E-62 | 3.65E-59 |
| ENSG00000168209 | DNA damage inducible transcript 4(DDIT4) | -2.556020952 | 0.180975322 | 2.72E-45 | 7.27E-43 |
| ENSG00000167552 | tubulin alpha 1a(TUBA1A) | -2.555390948 | 0.16329339 | 3.37E-55 | 1.24E-52 |
| ENSG00000162733 | discoidin domain receptor tyrosine kinase 2(DDR2) | -2.538720005 | 0.364525905 | 3.30E-12 | 8.11E-11 |
| ENSG00000222047 | chromosome 10 open reading frame 55(C10orf55) | -2.534916862 | 0.26306961 | 5.64E-22 | 4.28E-20 |
| ENSG00000108691 | C-C motif chemokine ligand 2(CCL2) | -2.525591194 | 0.149994352 | 1.29E-63 | 5.82E-61 |
| ENSG00000137267 | tubulin beta 2A class IIa(TUBB2A) | -2.520283371 | 0.173040878 | 4.71E-48 | 1.39E-45 |
| ENSG00000162924 | REL proto-oncogene, NF-kB subunit(REL) | -2.517805862 | 0.25798788 | 1.68E-22 | 1.33E-20 |
| ENSG00000108551 | ras related dexamethasone induced 1(RASD1) | -2.512046377 | 0.233988416 | 6.91E-27 | 7.53E-25 |
| ENSG00000184260 | histone cluster 2 H2A family member c(HIST2H2AC) | -2.496959246 | 0.461616062 | 6.33E-08 | 7.47E-07 |
| ENSG00000089902 | REST corepressor 1(RCOR1) | -2.496519035 | 0.88478045 | 0.004778095 | 0.015167163 |
| ENSG00000185432 | methyltransferase like 7A(METTL7A) | -2.496513051 | 0.282686227 | 1.03E-18 | 5.74E-17 |
| ENSG00000109321 | amphiregulin(AREG) | -2.491152934 | 0.238468994 | 1.52E-25 | 1.54E-23 |
| ENSG00000143850 | pleckstrin homology domain containing A6(PLEKHA6) | -2.487338375 | 0.478941684 | 2.06E-07 | 2.22E-06 |
| ENSG00000108342 | colony stimulating factor 3(CSF3) | -2.486011924 | 0.198745497 | 6.71E-36 | 1.18E-33 |
| ENSG00000104899 | microRNA 4321(MIR4321) | -2.479501612 | 0.275204221 | 2.07E-19 | 1.25E-17 |
| ENSG00000180447 | growth arrest specific 1(GAS1) | -2.477155982 | 0.252786737 | 1.13E-22 | 9.06E-21 |
| ENSG00000128917 | delta like canonical Notch ligand 4(DLL4) | -2.473176483 | 0.223230558 | 1.59E-28 | 1.96E-26 |
| ENSG00000234851 | NA | -2.460269598 | 0.714378172 | 0.000573321 | 0.002475062 |
| ENSG00000168140 | vasorin(VASN) | -2.43068175 | 0.244300724 | 2.53E-23 | 2.21E-21 |
| ENSG00000173391 | oxidized low density lipoprotein receptor 1(OLR1) | -2.426590495 | 0.238780502 | 2.92E-24 | 2.77E-22 |
| ENSG00000137449 | cytoplasmic polyadenylation element binding protein 2(CPEB2) | -2.416506755 | 0.271147699 | 5.01E-19 | 2.89E-17 |
| ENSG00000169242 | ephrin A1(EFNA1) | -2.409787071 | 0.160210369 | 3.93E-51 | 1.28E-48 |
| ENSG00000273802 | histone cluster 1 H2B family member g(HIST1H2BG) | -2.395692476 | 0.513461033 | 3.07E-06 | 2.52E-05 |
| ENSG00000146278 | proline rich nuclear receptor coactivator 1(PNRC1) | -2.392607114 | 0.16836345 | 7.83E-46 | 2.19E-43 |
| ENSG00000004777 | Rho GTPase activating protein 33(ARHGAP33) | -2.365826313 | 0.217750297 | 1.69E-27 | 1.99E-25 |
| ENSG00000188229 | tubulin beta 4B class IVb(TUBB4B) | -2.352516876 | 0.173150108 | 4.81E-42 | 1.09E-39 |
| ENSG00000019549 | snail family transcriptional repressor 2(SNAI2) | -2.336223423 | 0.249346216 | 7.29E-21 | 5.20E-19 |
| ENSG00000066230 | solute carrier family 9 member A3(SLC9A3) Cbp/p300 interacting transactivator with Glu/Asp rich carboxy-terminal domain | -2.300290022 | 0.371494574 | 5.94E-10 | 1.04E-08 |
| ENSG00000164442 | 2(CITED2) | -2.290371541 | 0.156079988 | 9.41E-49 | 2.84E-46 |
| ENSG00000182580 | EPH receptor B3(EPHB3) | -2.282145354 | 0.254291496 | 2.85E-19 | 1.68E-17 |
| ENSG00000274272 | NA | -2.27836831 | 0.373573077 | 1.07E-09 | 1.77E-08 |
| ENSG00000183496 | mex-3 RNA binding family member B(MEX3B) | -2.273063239 | 0.203805175 | 6.91E-29 | 8.65E-27 |
| ENSG00000112773 | family with sequence similarity 46 member A(FAM46A) | -2.261388559 | 0.390102219 | 6.75E-09 | 9.77E-08 |
| ENSG00000163874 | microRNA 6732(MIR6732) | -2.240141482 | 0.171912868 | 8.19E-39 | 1.69E-36 |
| ENSG00000114315 | hes family bHLH transcription factor 1(HES1) | -2.238445441 | 0.187406522 | 6.95E-33 | 1.08E-30 |
| ENSG00000162772 | activating transcription factor 3(ATF3) | -2.20839475 | 0.15342621 | 5.66E-47 | 1.62E-44 |
| ENSG00000143878 | ras homolog family member B(RHOB) | -2.194356004 | 0.15812153 | 8.65E-44 | 2.12E-41 |
| ENSG00000258947 | tubulin beta 3 class III(TUBB3) | -2.193469798 | 0.191698835 | 2.57E-30 | 3.44E-28 |
| ENSG00000113742 | cytoplasmic polyadenylation element binding protein 4(CPEB4) | -2.183085711 | 0.284884093 | 1.82E-14 | 6.23E-13 |
| ENSG00000105672 | ETS variant 2(ETV2) | -2.170070518 | 0.29995652 | 4.67E-13 | 1.30E-11 |
| ENSG00000124875 | C-X-C motif chemokine ligand 6(CXCL6) | -2.165343451 | 0.212940464 | 2.73E-24 | 2.61E-22 |
| ENSG00000169583 | chloride intracellular channel 3(CLIC3) | -2.161182799 | 0.275213414 | 4.07E-15 | 1.53E-13 |
| ENSG00000233822 | histone cluster 1 H2B family member n(HIST1H2BN) | -2.156175293 | 0.33386154 | 1.06E-10 | 2.13E-09 |
| ENSG00000099860 | growth arrest and DNA damage inducible beta(GADD45B) | -2.155058852 | 0.164425457 | 3.02E-39 | 6.34E-37 |
| ENSG00000178814 | 5-oxoprolinase (ATP-hydrolysing)(OPLAH) | -2.152637783 | 0.415549297 | 2.22E-07 | 2.37E-06 |
| ENSG00000105327 | microRNA 3191(MIR3191) | -2.152382139 | 0.272276734 | 2.68E-15 | 1.04E-13 |
| ENSG00000186834 | hexamethylene bisacetamide inducible 1(HEXIM1) | -2.145742165 | 0.184762754 | 3.52E-31 | 4.99E-29 |
| ENSG00000106538 | retinoic acid receptor responder 2(RARRES2) | -2.141112848 | 0.224350882 | 1.38E-21 | 1.02E-19 |
| ENSG00000090238 | yippee like 3(YPEL3) | -2.133728477 | 0.196053001 | 1.38E-27 | 1.64E-25 |
| ENSG00000188833 | ectonucleoside triphosphate diphosphohydrolase 8(ENTPD8) | -2.132374824 | 0.350831604 | 1.22E-09 | 2.00E-08 |
| ENSG00000185338 | suppressor of cytokine signaling 1(SOCS1) | -2.130055169 | 0.277581729 | 1.67E-14 | 5.77E-13 |
| ENSG00000188738 | fibrous sheath interacting protein 2(FSIP2) | -2.121266929 | 0.372264703 | 1.21E-08 | 1.68E-07 |
| ENSG00000243449 | chromosome 4 open reading frame 48(C4orf48) | -2.112571645 | 0.815336222 | 0.009568539 | 0.027259846 |
| ENSG00000266714 | myosin XVB(MYO15B) | -2.110698344 | 0.342584505 | 7.22E-10 | 1.24E-08 |
| ENSG00000204839 | maestro heat like repeat family member 6(MROH6) | -2.110544407 | 0.268802053 | 4.11E-15 | 1.54E-13 |
| ENSG00000142235 | lemur tyrosine kinase 3(LMTK3) | -2.103511072 | 0.448991478 | 2.80E-06 | 2.32E-05 |
| ENSG00000087074 | protein phosphatase 1 regulatory subunit 15A(PPP1R15A) | -2.101900503 | 0.15100514 | 4.83E-44 | 1.24E-41 |
| ENSG00000185650 | ZFP36 ring finger protein like 1(ZFP36L1) | -2.089088105 | 0.162092746 | 5.24E-38 | 1.03E-35 |
| ENSG00000184371 | colony stimulating factor 1(CSF1) | -2.085693014 | 0.160775141 | 1.75E-38 | 3.48E-36 |
| ENSG00000100625 | SIX homeobox 4(SIX4) | -2.081865037 | 0.305318044 | 9.19E-12 | 2.12E-10 |
| ENSG00000130522 | JunD proto-oncogene, AP-1 transcription factor subunit(JUND) | -2.075823946 | 0.164211509 | 1.25E-36 | 2.38E-34 |
| ENSG00000172602 | Rho family GTPase 1(RND1) | -2.057976964 | 0.216824768 | 2.28E-21 | 1.67E-19 |
| ENSG00000144802 | NFKB inhibitor zeta(NFKBIZ) | -2.054645605 | 0.163586916 | 3.50E-36 | 6.44E-34 |
| ENSG00000102554 | Kruppel like factor 5(KLF5) | -2.05370008 | 0.193409976 | 2.45E-26 | 2.55E-24 |
| ENSG00000188163 | family with sequence similarity 166 member A(FAM166A) | -2.048710774 | 0.173437577 | 3.37E-32 | 5.08E-30 |
| ENSG00000102934 | plasmolipin(PLLP) | -2.043564987 | 0.229533139 | 5.43E-19 | 3.10E-17 |
| ENSG00000213420 | glypican 2(GPC2) | -2.041827162 | 0.476268724 | 1.81E-05 | 0.000120704 |
| ENSG00000165312 | OTU deubiquitinase 1(OTUD1) | -2.029552583 | 0.279709159 | 3.99E-13 | 1.13E-11 |
| ENSG00000145020 | aminomethyltransferase(AMT) | -2.028945764 | 0.39429682 | 2.66E-07 | 2.81E-06 |
| ENSG00000162572 | sodium channel epithelial 1 delta subunit(SCNN1D) | -2.026636946 | 0.300500721 | 1.54E-11 | 3.43E-10 |
| ENSG00000147883 | cyclin dependent kinase inhibitor 2B(CDKN2B) | -2.024327858 | 0.212292377 | 1.49E-21 | 1.10E-19 |
| ENSG00000197483 | zinc finger protein 628(ZNF628) | -2.020027279 | 0.54101041 | 0.000188609 | 0.00095268 |
| ENSG00000115257 | proprotein convertase subtilisin/kexin type 4(PCSK4) | -2.001447207 | 0.2888169 | 4.21E-12 | 1.02E-10 |
| ENSG00000184678 | histone cluster 2 H2B family member e(HIST2H2BE) | -2.000619696 | 0.20311196 | 6.87E-23 | 5.69E-21 |
| ENSG00000188199 | NUT family member 2B(NUTM2B) | -1.993266208 | 0.454077254 | 1.14E-05 | 7.98E-05 |
| ENSG00000122861 | plasminogen activator, urokinase(PLAU) | -1.975687413 | 0.174402248 | 9.50E-30 | 1.26E-27 |
| ENSG00000141933 | tubulin polyglutamylase complex subunit 1(TPGS1) | -1.970830571 | 0.79550903 | 0.013232647 | 0.03566297 |
| ENSG00000124635 | histone cluster 1 H2B family member j(HIST1H2BJ) | -1.959671412 | 0.238247144 | 1.95E-16 | 8.45E-15 |
| ENSG00000150907 | forkhead box 01(FOXO1) | -1.959194272 | 0.213686828 | 4.80E-20 | 3.17E-18 |
| ENSG00000125538 | interleukin 1 beta(IL1B) | -1.956685622 | 0.182363261 | 7.39E-27 | 7.98E-25 |
| ENSG00000249936 | NA | -1.953635992 | 0.562512195 | 0.000514569 | 0.002255175 |
| ENSG00000163431 | leiomodin 1(LMOD1) | -1.943370751 | 0.350107377 | 2.84E-08 | 3.64E-07 |
| ENSG00000143507 | dual specificity phosphatase 10(DUSP10) | -1.943324581 | 0.223464803 | 3.43E-18 | 1.81E-16 |
| ENSG00000141682 | phorbol-12-myristate-13-acetate-induced protein 1(PMAIP1) | -1.941475377 | 0.230607985 | 3.80E-17 | 1.81E-15 |
| ENSG00000180096 | septin 1(SEPT1) | -1.940856 | 0.3767614 | 2.59E-07 | 2.73E-06 |
| ENSG00000203812 | histone cluster 2 H2A family member a3(HIST2H2AA3) | -1.935875727 | 0.26385978 | 2.19E-13 | 6.57E-12 |
| ENSG00000242265 | paternally expressed 10(PEG10) | -1.932841063 | 0.231307871 | 6.48E-17 | 3.01E-15 |
| ENSG00000141232 | transducer of ERBB2, 1(TOB1) | -1.929879211 | 0.225488827 | 1.14E-17 | 5.69E-16 |
| ENSG00000167711 | serpin family F member 2(SERPINF2) | -1.92949002 | 0.373803039 | 2.45E-07 | 2.60E-06 |
| ENSG00000177943 | MAM domain containing 4(MAMDC4) | -1.926832726 | 0.267541448 | 5.93E-13 | 1.62E-11 |
| ENSG00000177045 | SIX homeobox 5(SIX5) | -1.925858102 | 0.199130124 | 3.99E-22 | 3.07E-20 |
| ENSG00000055208 | TGF-beta activated kinase 1/MAP3K7 binding protein 2(TAB2) | -1.919584233 | 0.418095771 | 4.41E-06 | 3.47E-05 |
| ENSG00000180573 | histone cluster 1 H2A family member c(HIST1H2AC) | -1.918382268 | 0.284756907 | 1.62E-11 | 3.60E-10 |
| ENSG00000187961 | kelch like family member 17(KLHL17) | -1.908358374 | 0.264554699 | 5.45E-13 | 1.50E-11 |
| ENSG00000142303 | ADAM metallopeptidase with thrombospondin type 1 motif 10(ADAMTS10) | -1.905392946 | 0.383572574 | 6.78E-07 | 6.50E-06 |
| ENSG00000141854 | microRNA 1199(MIR1199) | -1.899486742 | 0.43054423 | 1.03E-05 | 7.29E-05 |
| ENSG00000173918 | C1q and tumor necrosis factor related protein 1(C1QTNF1) | -1.891862571 | 0.199985249 | 3.08E-21 | 2.24E-19 |
| ENSG00000196275 | GTF2I repeat domain containing 2(GTF2IRD2) | -1.881904107 | 0.255273813 | 1.68E-13 | 5.12E-12 |
| ENSG00000173825 | tigger transposable element derived 3(TIGD3) | -1.87611588 | 0.225124325 | 7.84E-17 | 3.60E-15 |
| ENSG00000173597 | sulfotransferase family 1B member 1(SULT1B1) | -1.874923707 | 0.35957178 | 1.84E-07 | 2.00E-06 |
| ENSG00000143867 | odd-skipped related transciption factor 1(OSR1) | -1.87490095 | 0.240100341 | 5.77E-15 | 2.12E-13 |
| ENSG00000095066 | hook microtubule tethering protein 2(HOOK2) | -1.874007571 | 0.227309202 | 1.66E-16 | 7.29E-15 |
| ENSG00000167553 | tubulin alpha 1c(TUBA1C) | -1.873570246 | 0.156945281 | 7.52E-33 | 1.15E-30 |
| ENSG00000171150 | suppressor of cytokine signaling 5(SOCS5) | -1.871595365 | 0.240620553 | 7.36E-15 | 2.67E-13 |
| ENSG00000122035 | RAS like family 11 member A(RASL11A) | -1.867673808 | 0.271833436 | 6.39E-12 | 1.52E-10 |
| ENSG00000213199 | acid sensing ion channel subunit 3(ASIC3) | -1.86056168 | 0.245763512 | 3.72E-14 | 1.24E-12 |
| ENSG00000180592 | SKI/DACH domain containing 1(SKIDA1) | -1.859559532 | 0.448387341 | 3.37E-05 | 0.000209143 |
| ENSG00000282827 | three prime repair exonuclease 1(TREX1) | -1.858575428 | 0.401064106 | 3.58E-06 | 2.89E-05 |
| ENSG00000198435 | NOTCH-regulated ankyrin repeat protein(NRARP) | -1.858085575 | 0.357479679 | 2.02E-07 | 2.17E-06 |
| ENSG00000100055 | cytohesin 4(CYTH4) | -1.844920784 | 0.38918094 | 2.13E-06 | 1.82E-05 |
| ENSG00000167676 | perilipin 4(PLIN4) | -1.844553952 | 0.426911862 | 1.56E-05 | 0.000105707 |
| ENSG00000175602 | coiled-coil domain containing 85B(CCDC85B) | -1.844124119 | 0.50756675 | 0.000279858 | 0.001336141 |
| ENSG00000125657 | tumor necrosis factor superfamily member 9(TNFSF9) | -1.843073388 | 0.182183702 | 4.66E-24 | 4.39E-22 |
| ENSG00000124216 | snail family transcriptional repressor 1(SNAI1) | -1.832231771 | 0.186118571 | 7.24E-23 | 5.92E-21 |
| ENSG00000152082 | mitotic spindle organizing protein 2B(MZT2B) | -1.820619294 | 0.641370293 | 0.004530626 | 0.014548831 |
| ENSG00000189221 | monoamine oxidase A(MAOA) | -1.816031146 | 0.418089759 | 1.40E-05 | 9.64E-05 |
| ENSG00000141582 | chromobox 4(CBX4) | -1.814006138 | 0.185883385 | 1.69E-22 | 1.33E-20 |
| ENSG00000172216 | CCAAT/enhancer binding protein beta(CEBPB) | -1.812713232 | 0.181031677 | 1.33E-23 | 1.21E-21 |
| ENSG00000176945 | mucin 20, cell surface associated(MUC20) | -1.812352081 | 0.35404549 | 3.07E-07 | 3.19E-06 |
| ENSG00000244694 | patched domain containing 4(PTCHD4) | -1.809163209 | 0.669159738 | 0.006858573 | 0.020593224 |
| ENSG00000177595 | p53-induced death domain protein 1(PIDD1) | -1.804391851 | 0.275047768 | 5.37E-11 | 1.13E-09 |
| ENSG00000137193 | Pim-1 proto-oncogene, serine/threonine kinase(PIM1) | -1.796178248 | 0.149534486 | 3.08E-33 | 4.96E-31 |
| ENSG00000158106 | rhophilin Rho GTPase binding protein 1(RHPN1) | -1.79178538 | 0.255135351 | 2.17E-12 | 5.50E-11 |
| ENSG00000176244 | acyl-CoA binding domain containing 7(ACBD7) | -1.791314557 | 0.412673802 | 1.42E-05 | 9.75E-05 |
| ENSG00000144655 | cysteine and serine rich nuclear protein 1(CSRNP1) | -1.788246735 | 0.14809058 | 1.43E-33 | 2.40E-31 |
| ENSG00000119922 | interferon induced protein with tetratricopeptide repeats 2(IFIT2) | -1.787977604 | 0.360246158 | 6.93E-07 | 6.63E-06 |
| ENSG00000155090 | Kruppel like factor 10(KLF10) | -1.785667386 | 0.192646574 | 1.88E-20 | 1.28E-18 |
| ENSG00000048392 | ribonucleotide reductase regulatory TP53 inducible subunit M2B(RRM2B) | -1.784896254 | 0.313478741 | 1.24E-08 | 1.72E-07 |
| ENSG00000010310 | gastric inhibitory polypeptide receptor(GIPR) | -1.780904948 | 0.527031803 | 0.000727176 | 0.003036742 |
| ENSG00000184557 | suppressor of cytokine signaling 3(SOCS3) | -1.78040118 | 0.164670038 | 3.02E-27 | 3.35E-25 |
| ENSG00000167617 | CDC42 effector protein 5(CDC42EP5) | -1.776064264 | 0.226648469 | 4.64E-15 | 1.72E-13 |
| ENSG00000169715 | metallothionein 1E(MT1E) | -1.775054056 | 0.169060366 | 8.68E-26 | 8.88E-24 |
| ENSG00000169174 | proprotein convertase subtilisin/kexin type 9(PCSK9) | -1.774868404 | 0.377810577 | 2.63E-06 | 2.20E-05 |
| ENSG00000168916 | zinc finger protein 608(ZNF608) | -1.774823944 | 0.297090862 | 2.31E-09 | 3.61E-08 |
| ENSG00000205560 | carnitine palmitoyltransferase 1B(CPT1B) | -1.77322761 | 0.240370749 | 1.62E-13 | 4.96E-12 |
| ENSG00000170271 | fatty acid hydroxylase domain containing 2(FAXDC2) | -1.772919799 | 0.230778202 | 1.56E-14 | 5.42E-13 |
| ENSG00000134339 | serum amyloid A2(SAA2) | -1.77223139 | 0.304209239 | 5.69E-09 | 8.37E-08 |
| ENSG00000212864 | ring finger protein 208(RNF208) | -1.76696907 | 0.393877133 | 7.25E-06 | 5.38E-05 |
| ENSG00000006283 | calcium voltage-gated channel subunit alpha1 G(CACNA1G) | -1.765979533 | 0.31667939 | 2.45E-08 | 3.18E-07 |
| ENSG00000231160 | KLF3 antisense RNA 1(KLF3-AS1) | -1.758310845 | 0.272040861 | 1.02E-10 | 2.06E-09 |
| ENSG00000172508 | carnosine synthase 1(CARNS1) | -1.750686946 | 0.355385383 | 8.39E-07 | 7.87E-06 |
| ENSG00000166016 | ankyrin repeat and BTB domain containing 2(ABTB2) | -1.746337918 | 0.536282412 | 0.001128437 | 0.004448702 |
| ENSG00000171236 | leucine rich alpha-2-glycoprotein 1(LRG1) | -1.744654278 | 0.304151942 | 9.69E-09 | 1.37E-07 |
| ENSG00000215788 | TNF receptor superfamily member 25(TNFRSF25) | -1.741958403 | 0.315437984 | 3.34E-08 | 4.19E-07 |
| ENSG00000104856 | RELB proto-oncogene, NF-kB subunit(RELB) | -1.740127001 | 0.229980137 | 3.84E-14 | 1.27E-12 |
| ENSG00000064270 | ATPase secretory pathway Ca2+ transporting 2(ATP2C2) | -1.73420168 | 0.31049373 | 2.33E-08 | 3.05E-07 |
| ENSG00000141574 | secreted and transmembrane 1(SECTM1) | -1.731435382 | 0.213706787 | 5.41E-16 | 2.25E-14 |
| ENSG00000250510 | G protein-coupled receptor 162(GPR162) | -1.728550696 | 0.209218671 | 1.43E-16 | 6.36E-15 |
| ENSG00000151364 | potassium channel tetramerization domain containing 14(KCTD14) | -1.722382016 | 0.48363294 | 0.000368965 | 0.001692204 |
| ENSG00000142102 | protein-glucosylgalactosylhydroxylysine glucosidase(PGGHG) | -1.718278002 | 0.179830855 | 1.24E-21 | 9.26E-20 |
| ENSG00000101222 | sperm flagellar 1(SPEF1) | -1.718229178 | 0.371315924 | 3.70E-06 | 2.97E-05 |
| ENSG00000273049 | NA | -1.716950428 | 0.520090049 | 0.00096253 | 0.003877809 |
| ENSG00000162591 | multiple EGF like domains 6(MEGF6) | -1.715274581 | 0.251022168 | 8.31E-12 | 1.92E-10 |
| ENSG00000205746 | NA | -1.712171875 | 0.648932784 | 0.008328781 | 0.024264433 |
| ENSG00000163602 | RING1 and YY1 binding protein(RYBP) | -1.709863631 | 0.236201935 | 4.52E-13 | 1.26E-11 |
| ENSG00000099812 | mitotic spindle positioning(MISP) | -1.70732312 | 0.235696288 | 4.36E-13 | 1.22E-11 |
| ENSG00000023445 | baculoviral IAP repeat containing 3(BIRC3) | -1.705999464 | 0.242519425 | 2.00E-12 | 5.09E-11 |
| ENSG00000120696 | kelch repeat and BTB domain containing 7(KBTBD7) | -1.702507876 | 0.372358543 | 4.83E-06 | 3.75E-05 |
| ENSG00000145743 | F-box end leucine rich repeat protein 17(FBXL17) | -1.698137382 | 0.302845052 | 2.06E-08 | 2.72E-07 |
| ENSG00000069399 | microRNA 8085(MIR8085) | -1.697586219 | 0.180101389 | 4.27E-21 | 3.08E-19 |
| ENSG00000114812 | vasoactive intestinal peptide receptor 1(VIPR1) | -1.695072401 | 0.214332626 | 2.60E-15 | 1.02E-13 |
| ENSG00000148344 | prostaglandin E synthase(PTGES) | -1.692010453 | 0.18744605 | 1.77E-19 | 1.08E-17 |
| ENSG00000184381 | phospholipase A2 group VI(PLA2G6) | -1.681970126 | 0.22280423 | 4.38E-14 | 1.45E-12 |
| ENSG00000088002 | sulfotransferase family 2B member 1(SULT2B1) | -1.680173241 | 0.403198774 | 3.08E-05 | 0.000193738 |
| ENSG00000099998 | gamma-glutamyltransferase 5(GGT5) | -1.67885923 | 0.183469334 | 5.66E-20 | 3.72E-18 |
| ENSG00000189184 | protocadherin 18(PCDH18) | -1.678347353 | 0.442075579 | 0.000146743 | 0.000762832 |
| ENSG00000165272 | aquaporin 3 (Gill blood group)(AQP3) | -1.676320743 | 0.212892286 | 3.43E-15 | 1.30E-13 |
| ENSG00000127528 | Kruppel like factor 2(KLF2) | -1.670368488 | 0.213234367 | 4.75E-15 | 1.76E-13 |
| ENSG00000206172 | hemoglobin subunit alpha 1(HBA1) | -1.66968786 | 0.3845072 | 1.41E-05 | 9.68E-05 |
| ENSG00000163659 | TCDD inducible poly(ADP-ribose) polymerase(TIPARP) | -1.66910326 | 0.258472768 | 1.06E-10 | 2.14E-09 |
| ENSG00000185112 | family with sequence similarity 43 member A(FAM43A) | -1.668335364 | 0.18392045 | 1.18E-19 | 7.30E-18 |
| ENSG00000174749 | chromosome 4 open reading frame 32(C4orf32) | -1.66413041 | 0.246717708 | 1.53E-11 | 3.41E-10 |
| ENSG00000204469 | proline rich coiled-coil 2A(PRRC2A) | -1.662820699 | 0.437585253 | 0.0001447 | 0.000753878 |
| ENSG00000070731 | ST6 N-acetylgalactosaminide alpha-2,6-sialyltransferase 2(ST6GALNAC2) | -1.661097172 | 0.286761023 | 6.93E-09 | 1.00E-07 |
| ENSG00000220201 | zinc finger, GATA-like protein 1(ZGLP1) | -1.660886472 | 0.265741282 | 4.10E-10 | 7.44E-09 |
| ENSG00000168874 | atonal bHLH transcription factor 8(ATOH8) | -1.658959924 | 0.185046743 | 3.10E-19 | 1.82E-17 |
| ENSG00000074855 | anoctamin 8(ANO8) | -1.655984891 | 0.237131492 | 2.88E-12 | 7.21E-11 |
| ENSG00000157514 | TSC22 domain family member 3(TSC22D3) | -1.654990553 | 0.146737104 | 1.67E-29 | 2.16E-27 |
| ENSG00000169184 | MN1 proto-oncogene, transcriptional regulator(MN1) | -1.651867448 | 0.267896274 | 7.00E-10 | 1.21E-08 |
| ENSG00000068078 | fibroblast growth factor receptor 3(FGFR3) | -1.649957499 | 0.20084239 | 2.12E-16 | 9.16E-15 |
| ENSG00000168216 | LMBR1 domain containing 1(LMBRD1) | -1.649710139 | 0.366851099 | 6.89E-06 | 5.14E-05 |
| ENSG00000153132 | calmegin(CLGN) | -1.647511965 | 0.313785006 | 1.52E-07 | 1.67E-06 |
| ENSG00000122641 | inhibin beta A subunit(INHBA) | -1.647041745 | 0.235515639 | 2.68E-12 | 6.73E-11 |
| ENSG00000196428 | TSC22 domain family member 2(TSC22D2) | -1.64550207 | 0.212678579 | 1.02E-14 | 3.63E-13 |
| ENSG00000232434 | chromosome 9 open reading frame 172(C9orf172) | -1.645266144 | 0.487124113 | 0.000731455 | 0.003052444 |
| ENSG00000177791 | myozenin 1(MYOZ1) | -1.643802058 | 0.315334203 | 1.86E-07 | 2.02E-06 |
| ENSG00000049769 | protein phosphatase 1 regulatory subunit 3F(PPP1R3F) | -1.641884124 | 0.209427495 | 4.51E-15 | 1.68E-13 |
| ENSG00000173272 | microRNA 4784(MIR4784) | -1.641603977 | 0.614587606 | 0.007561081 | 0.022360119 |
| ENSG00000197019 | SERTA domain containing 1(SERTAD1) | -1.641131111 | 0.190246515 | 6.33E-18 | 3.21E-16 |
| ENSG00000243649 | complement factor B(CFB) | -1.640956129 | 0.279749429 | 4.47E-09 | 6.67E-08 |
| ENSG00000100906 | NFKB inhibitor alpha(NFKBIA) | -1.638877097 | 0.145070285 | 1.36E-29 | 1.77E-27 |
| ENSG00000129194 | SRY-box 15(SOX15) | -1.634258643 | 0.26798384 | 1.07E-09 | 1.77E-08 |
| ENSG00000198355 | Pim-3 proto-oncogene, serine/threonine kinase(PIM3) | -1.632717354 | 0.199331001 | 2.59E-16 | 1.11E-14 |
| ENSG00000197757 | homeobox C6(HOXC6) | -1.63245452 | 0.224248553 | 3.35E-13 | 9.63E-12 |
| ENSG00000198598 | matrix metallopeptidase 17(MMP17) | -1.632159887 | 0.287644598 | 1.39E-08 | 1.91E-07 |
| ENSG00000132470 | integrin subunit beta 4(ITGB4) | -1.627934415 | 0.184212957 | 9.81E-19 | 5.47E-17 |
| ENSG00000099251 | hydroxysteroid 17-beta dehydrogenase 7 pseudogene 2(HSD17B7P2) | -1.626905006 | 0.392783599 | 3.44E-05 | 0.000213293 |
| ENSG00000110876 | selectin P ligand(SELPLG) | -1.626595762 | 0.216224501 | 5.36E-14 | 1.75E-12 |
| ENSG00000005961 | integrin subunit alpha 2b(ITGA2B) | -1.625303381 | 0.333792195 | 1.12E-06 | 1.02E-05 |
| ENSG00000163009 | NA | -1.624896288 | 0.436683762 | 0.000198443 | 0.000991901 |
| ENSG00000167578 | RAB4B, member RAS oncogene family(RAB4B) | -1.622974975 | 0.278044952 | 5.31E-09 | 7.84E-08 |
| ENSG00000169962 | taste 1 receptor member 3(TAS1R3) | -1.619475754 | 0.313803102 | 2.46E-07 | 2.61E-06 |
| ENSG00000119669 | interferon regulatory factor 2 binding protein like(IRF2BPL) | -1.616477269 | 0.208438215 | 8.82E-15 | 3.16E-13 |
| ENSG00000186868 | microtubule associated protein tau(MAPT) | -1.609378195 | 0.374641333 | 1.74E-05 | 0.000116686 |
| ENSG00000181744 | chromosome 3 open reading frame 58(C3orf58) | -1.608253399 | 0.3411211 | 2.42E-06 | 2.04E-05 |
| ENSG00000113240 | CDC like kinase 4(CLK4) | -1.608061545 | 0.254124 | 2.49E-10 | 4.66E-09 |
| ENSG00000187091 | phospholipase C delta 1(PLCD1) | -1.605503891 | 0.184907074 | 3.86E-18 | 2.03E-16 |
| ENSG00000158473 | CD1d molecule(CD1D) | -1.604827019 | 0.285777665 | 1.96E-08 | 2.59E-07 |
| ENSG00000171159 | chromosome 9 open reading frame 16(C9orf16) | -1.59282655 | 0.240840757 | 3.75E-11 | 8.05E-10 |
| ENSG00000240342 | NA | -1.590714769 | 0.259705857 | 9.06E-10 | 1.53E-08 |
| ENSG00000111199 | transient receptor potential cation channel subfamily V member 4(TRPV4) | -1.59003361 | 0.295254655 | 7.23E-08 | 8.48E-07 |
| ENSG00000196136 | serpin family A member 3(SERPINA3) | -1.586760612 | 0.165384303 | 8.44E-22 | 6.37E-20 |
| ENSG00000113555 | protocadherin 12(PCDH12) | -1.585634725 | 0.48338112 | 0.00103697 | 0.004128625 |
| ENSG00000197847 | solute carrier family 22 member 20(SLC22A20) | -1.584322499 | 0.3665176 | 1.54E-05 | 0.000104903 |
| ENSG00000103269 | rhomboid like 1(RHBDL1) | -1.577618839 | 0.388615648 | 4.92E-05 | 0.000291636 |
| ENSG00000179833 | SERTA domain containing 2(SERTAD2) | -1.576203467 | 0.173627113 | 1.10E-19 | 6.88E-18 |
| ENSG00000135722 | F-box and leucine rich repeat protein 8(FBXL8) | -1.568743386 | 0.346849908 | 6.10E-06 | 4.59E-05 |
| ENSG00000171219 | CDC42 binding protein kinase gamma(CDC42BPG) | -1.564281711 | 0.324944073 | 1.48E-06 | 1.31E-05 |
| ENSG00000113916 | B-cell CLL/lymphoma 6(BCL6) | -1.563364842 | 0.169883259 | 3.49E-20 | 2.32E-18 |
| ENSG00000188596 | cilia and flagella associated protein 54(CFAP54) | -1.5629499 | 0.37389857 | 2.91E-05 | 0.000183958 |
| ENSG00000112769 | laminin subunit alpha 4(LAMA4) | -1.562479425 | 0.448406798 | 0.000493033 | 0.002176376 |
| ENSG00000069122 | adhesion G protein-coupled receptor F5(ADGRF5) | -1.560553257 | 0.473363114 | 0.000978154 | 0.003927304 |
| ENSG00000064300 | nerve growth factor receptor(NGFR) | -1.55790422 | 0.24072604 | 9.69E-11 | 1.97E-09 |
| ENSG00000196338 | neuroligin 3(NLGN3) | -1.554770032 | 0.388549681 | 6.30E-05 | 0.000363904 |
| ENSG00000095397 | whirlin(WHRN) | -1.5544847 | 0.186999465 | 9.35E-17 | 4.26E-15 |
| ENSG00000243566 | uroplakin-3b(LOC105375355) | -1.553270106 | 0.516354744 | 0.002628474 | 0.009194579 |
| ENSG00000165030 | nuclear factor, interleukin 3 regulated(NFIL3) | -1.553219913 | 0.213005557 | 3.06E-13 | 8.85E-12 |
| ENSG00000172789 | homeobox C5(HOXC5) | -1.55310046 | 0.340480861 | 5.08E-06 | 3.92E-05 |
| ENSG00000132016 | chromosome 19 open reading frame 57(C19orf57) | -1.552868726 | 0.260532128 | 2.52E-09 | 3.91E-08 |
| ENSG00000138650 | protocadherin 10(PCDH10) | -1.548797823 | 0.261336926 | 3.10E-09 | 4.74E-08 |
| ENSG00000196378 | zinc finger protein 34(ZNF34) | -1.547504331 | 0.237103097 | 6.72E-11 | 1.39E-09 |
| ENSG00000113369 | arrestin domain containing 3(ARRDC3) | -1.544213064 | 0.285008006 | 6.02E-08 | 7.15E-07 |
| ENSG00000101096 | nuclear factor of activated T-cells 2(NFATC2) | -1.543121721 | 0.200028252 | 1.21E-14 | 4.29E-13 |
| ENSG00000222009 | BTB domain containing 19(BTBD19) | -1.541184262 | 0.239833432 | 1.31E-10 | 2.59E-09 |
| ENSG00000185621 | leishmanolysin like peptidase(LMLN) | -1.53990564 | 0.310315217 | 6.96E-07 | 6.65E-06 |
| ENSG00000127824 | tubulin alpha 4a(TUBA4A) | -1.539111584 | 0.253492644 | 1.27E-09 | 2.07E-08 |
| ENSG00000186081 | keratin 5(KRT5) | -1.536323615 | 0.399644385 | 0.000120933 | 0.000645196 |
| ENSG00000141338 | ATP binding cassette subfamily A member 8(ABCA8) | -1.534743237 | 0.26687224 | 8.88E-09 | 1.27E-07 |
| ENSG00000138688 | KIAA1109(KIAA1109) | -1.53367673 | 0.252160592 | 1.19E-09 | 1.95E-08 |
| ENSG00000153208 | MER proto-oncogene, tyrosine kinase(MERTK) | -1.532444914 | 0.274649379 | 2.41E-08 | 3.14E-07 |
| ENSG00000113721 | platelet derived growth factor receptor beta(PDGFRB) | -1.53118311 | 0.15544624 | 6.84E-23 | 5.69E-21 |
| ENSG00000106415 | glucocorticoid induced 1(GLCCI1) | -1.529739031 | 0.374447388 | 4.40E-05 | 0.000265128 |
| ENSG00000114378 | hyaluronoglucosaminidase 1(HYAL1) | -1.527776373 | 0.389807112 | 8.88E-05 | 0.000492764 |
| ENSG00000152527 | pleckstrin homology, MyTH4 and FERM domain containing H2(PLEKHH2) | -1.525806247 | 0.321272715 | 2.04E-06 | 1.75E-05 |
| ENSG00000198753 | plexin B3(PLXNB3) | -1.525781137 | 0.42172642 | 0.000296957 | 0.001405228 |
| ENSG00000105246 | Epstein-Barr virus induced 3(EBI3) | -1.524723265 | 0.238605891 | 1.66E-10 | 3.20E-09 |
| ENSG00000166924 | neuronal tyrosine phosphorylated phosphoinositide-3-kinase adaptor 1(NYAP1) | -1.522610889 | 0.381171801 | 6.48E-05 | 0.000372861 |
| ENSG00000106537 | tetraspanin 13(TSPAN13) | -1.522025575 | 0.323056032 | 2.46E-06 | 2.07E-05 |
| ENSG00000182324 | potassium voltage-gated channel subfamily J member 14(KCNJ14) | -1.52145477 | 0.254174478 | 2.15E-09 | 3.40E-08 |
| ENSG00000115255 | receptor accessory protein 6(REEP6) | -1.520125558 | 0.20507585 | 1.24E-13 | 3.86E-12 |
| ENSG00000185950 | insulin receptor substrate 2(IRS2) | -1.519636523 | 0.179928003 | 3.02E-17 | 1.46E-15 |
| ENSG00000205236 | NA | -1.519302681 | 0.242234979 | 3.56E-10 | 6.52E-09 |
| ENSG00000121691 | catalase(CAT) | -1.517636365 | 0.278474034 | 5.04E-08 | 6.09E-07 |
| ENSG00000127220 | abhydrolase domain containing 8(ABHD8) | -1.513237919 | 0.260769868 | 6.52E-09 | 9.47E-08 |
| ENSG00000184489 | protein tyrosine phosphatase type IVA, member 3(PTP4A3) | -1.503240022 | 0.256453615 | 4.58E-09 | 6.82E-08 |
| ENSG00000173432 | serum amyloid A1(SAA1) | -1.501709865 | 0.231803649 | 9.27E-11 | 1.89E-09 |
| ENSG00000250151 | ARPC4-TTLL3 readthrough(ARPC4-TTLL3) | -1.500931361 | 0.263825454 | 1.28E-08 | 1.76E-07 |
| ENSG00000242252 | bone gamma-carboxyglutamate protein(BGLAP) | -1.500149532 | 0.364748176 | 3.91E-05 | 0.000238841 |
| ENSG00000102057 | potassium voltage-gated channel subfamily D member 1(KCND1) | -1.49962232 | 0.353360644 | 2.20E-05 | 0.000143335 |
| ENSG00000205189 | zinc finger and BTB domain containing 10(ZBTB10) | -1.497917054 | 0.342272422 | 1.21E-05 | 8.43E-05 |
| ENSG00000254413 | CHKB-CPT1B readthrough (NMD candidate)(CHKB-CPT1B) | -1.494891069 | 0.367549828 | 4.76E-05 | 0.000283729 |
| ENSG00000165997 | ADP ribosylation factor like GTPase 5B(ARLSB) | -1.494366313 | 0.270643898 | 3.36E-08 | 4.21E-07 |
| ENSG00000102886 | glycerophosphodiester phosphodiesterase domain containing 3(GDPD3) | -1.493297072 | 0.266911237 | 2.21E-08 | 2.91E-07 |
| ENSG00000130702 | microRNA 4758(MIR4758) | -1.491831476 | 0.256676613 | 6.17E-09 | 9.02E-08 |
| ENSG00000109705 | NK3 homeobox 2(NKX3-2) | -1.490383038 | 0.423080152 | 0.00042718 | 0.00192542 |
| ENSG00000258839 | melanocortin 1 receptor(MC1R) | -1.488710978 | 0.234363307 | 2.12E-10 | 4.02E-09 |
| ENSG00000124171 | par-6 family cell polarity regulator beta(PARD6B) | -1.486306939 | 0.436563697 | 0.000662709 | 0.002811437 |
| ENSG00000148926 | adrenomedullin(ADM) | -1.483988979 | 0.19331041 | 1.63E-14 | 5.65E-13 |
| ENSG00000267281 | NA | -1.478097585 | 0.429648435 | 0.000581179 | 0.002504673 |
| ENSG00000101888 | nuclear transport factor 2 like export factor 2(NXT2) | -1.477032531 | 0.339627416 | 1.37E-05 | 9.43E-05 |
| ENSG00000142156 | collagen type VI alpha 1 chain(COL6A1) | -1.474086542 | 0.19630812 | 5.96E-14 | 1.90E-12 |
| ENSG00000139318 | dual specificity phosphatase 6(DUSP6) | -1.473527853 | 0.16757654 | 1.45E-18 | 7.99E-17 |
| ENSG00000186480 | insulin induced gene 1(INSIG1) | -1.471200488 | 0.151414768 | 2.57E-22 | 1.99E-20 |
| ENSG00000105427 | cornifelin(CNFN) | -1.468355871 | 0.259824655 | 1.59E-08 | 2.15E-07 |
| ENSG00000037042 | tubulin gamma 2(TUBG2) | -1.463899957 | 0.156114394 | 6.78E-21 | 4.86E-19 |
| ENSG00000282951 | NA | -1.46079567 | 0.225766145 | 9.77E-11 | 1.98E-09 |
| ENSG00000054598 | forkhead box C1(FOXC1) | -1.453630912 | 0.163052105 | 4.87E-19 | 2.84E-17 |
| ENSG00000160781 | progestin and adipoQ receptor family member 6(PAQR6) | -1.453377111 | 0.245085139 | 3.03E-09 | 4.65E-08 |
| ENSG00000163661 | pentraxin 3(PTX3) | -1.453084824 | 0.182027397 | 1.43E-15 | 5.80E-14 |
| ENSG00000196220 | SLIT-ROBO Rho GTPase activating protein 3(SRGAP3) | -1.451735059 | 0.425291608 | 0.00064133 | 0.002732561 |
| ENSG00000145779 | TNF alpha induced protein 8(TNFAIP8) | -1.44733524 | 0.225306552 | 1.33E-10 | 2.62E-09 |
| ENSG00000184897 | H1 histone family member X(H1FX) | -1.447205023 | 0.237125158 | 1.04E-09 | 1.73E-08 |
| ENSG00000218537 | NA | -1.445419508 | 0.519728309 | 0.005417405 | 0.016864343 |
| ENSG00000124102 | peptidase inhibitor 3(PI3) | -1.445048691 | 0.301133398 | 1.60E-06 | 1.40E-05 |
| ENSG00000099957 | purinergic receptor P2X 6(P2RX6) | -1.441744233 | 0.292675376 | 8.39E-07 | 7.87E-06 |
| ENSG00000115468 | EF-hand domain family member D1(EFHD1) | -1.440594355 | 0.218248404 | 4.09E-11 | 8.74E-10 |
| ENSG00000196550 | family with sequence similarity 72 member A(FAM72A) | -1.439749711 | 0.283944146 | 3.97E-07 | 4.01E-06 |
| ENSG00000131979 | GTP cyclohydrolase 1(GCH1) | -1.435944361 | 0.258742188 | 2.86E-08 | 3.65E-07 |
| ENSG00000104883 | peroxisomal biogenesis factor 11 gamma(PEX11G) | -1.43245526 | 0.437549464 | 0.001061067 | 0.0042156 |
| ENSG00000099994 | sushi domain containing 2(SUSD2) | -1.431034891 | 0.278591406 | 2.80E-07 | 2.93E-06 |
| ENSG00000073150 | pannexin 2(PANX2) | -1.429235335 | 0.266350363 | 8.05E-08 | 9.37E-07 |
| ENSG00000105088 | olfactomedin 2(OLFM2) | -1.427882513 | 0.354335917 | 5.58E-05 | 0.000327293 |
| ENSG00000131069 | acyl-CoA synthetase short-chain family member 2(ACSS2) | -1.427116427 | 0.189411319 | 4.90E-14 | 1.62E-12 |
| ENSG00000163683 | small integral membrane protein 14(SMIM14) | -1.426886161 | 0.258224433 | 3.28E-08 | 4.13E-07 |
| ENSG00000116678 | leptin receptor(LEPR) | -1.426690743 | 0.381666135 | 0.000185446 | 0.000938317 |
| ENSG00000162076 | FLYWCH family member 2(FLYWCH2) | -1.424586121 | 0.210330048 | 1.26E-11 | 2.86E-10 |
| ENSG00000165655 | zinc finger protein 503(ZNF503) | -1.424146368 | 0.261573071 | 5.19E-08 | 6.24E-07 |
| ENSG00000059804 | solute carrier family 2 member 3(SLC2A3) | -1.422706563 | 0.163523275 | 3.31E-18 | 1.75E-16 |
| ENSG00000184226 | protocadherin 9(PCDH9) | -1.422358419 | 0.418057642 | 0.000668207 | 0.002832717 |
| ENSG00000167470 | midnolin(MIDN) | -1.422036144 | 0.18640615 | 2.37E-14 | 7.99E-13 |
| ENSG00000114107 | centrosomal protein 70(CEP70) | -1.421648817 | 0.333144992 | 1.98E-05 | 0.000130801 |
| ENSG00000104093 | Dmx like 2(DMXL2) | -1.42059139 | 0.292077835 | 1.15E-06 | 1.05E-05 |
| ENSG00000171056 | SRY-box 7(SOX7) | -1.419039141 | 0.333087988 | 2.04E-05 | 0.000134424 |
| ENSG00000164039 | 3-hydroxybutyrate dehydrogenase, type 2(BDH2) | -1.413000751 | 0.26135096 | 6.43E-08 | 7.57E-07 |
| ENSG00000213402 | protein tyrosine phosphatase, receptor type C associated protein(PTPRCAP) | -1.411500539 | 0.435820114 | 0.001200662 | 0.004689438 |
| ENSG00000081803 | calcium dependent secretion activator 2(CADPS2) | -1.41000998 | 0.479158956 | 0.003253878 | 0.011021775 |
| ENSG00000140961 | oxidative stress induced growth inhibitor 1(OSGIN1) | -1.409905505 | 0.202033215 | 2.98E-12 | 7.43E-11 |
| ENSG00000064547 | lysophosphatidic acid receptor 2(LPAR2) | -1.409808905 | 0.234576358 | 1.85E-09 | 2.96E-08 |
| ENSG00000127666 | toll like receptor adaptor molecule 1(TICAM1) | -1.409468841 | 0.192550735 | 2.48E-13 | 7.39E-12 |
| ENSG00000205277 | mucin 12, cell surface associated(MUC12) | -1.407310016 | 0.295811231 | 1.96E-06 | 1.69E-05 |
| ENSG00000205517 | ral guanine nucleotide dissociation stimulator like 3(RGL3) | -1.405634992 | 0.194731673 | 5.26E-13 | 1.45E-11 |
| ENSG00000134574 | damage specific DNA binding protein 2(DDB2) | -1.405486839 | 0.154365039 | 8.63E-20 | 5.49E-18 |
| ENSG00000148357 | hemicentin 2(HMCN2) | -1.40535989 | 0.378142543 | 0.000202016 | 0.001006999 |
| ENSG00000161996 | WD repeat domain 90(WDR90) | -1.404042187 | 0.224563071 | 4.04E-10 | 7.35E-09 |
| ENSG00000175505 | cardiotrophin-like cytokine factor 1(CLCF1) | -1.40360778 | 0.177797909 | 2.92E-15 | 1.12E-13 |
| ENSG00000227766 | NA | -1.402014307 | 0.257472056 | 5.17E-08 | 6.22E-07 |
| ENSG00000166963 | microtubule associated protein 1A(MAP1A) | -1.399789524 | 0.349870641 | 6.31E-05 | 0.000364638 |
| ENSG00000168010 | autophagy related 16 like 2(ATG16L2) | -1.399690787 | 0.191327866 | 2.56E-13 | 7.59E-12 |
| ENSG00000197536 | chromosome 5 open reading frame 56(C5orf56) | -1.399499401 | 0.228175594 | 8.60E-10 | 1.46E-08 |
| ENSG00000151413 | nucleotide binding protein like(NUBPL) | -1.398261844 | 0.438530189 | 0.001430036 | 0.005453795 |
| ENSG00000189129 | placenta specific 9(PLAC9) | -1.397656395 | 0.335459674 | 3.09E-05 | 0.000194055 |
| ENSG00000067082 | Kruppel like factor 6(KLF6) | -1.396802169 | 0.187722992 | 1.00E-13 | 3.13E-12 |
| ENSG00000167371 | proline rich transmembrane protein 2(PRRT2) | -1.392677415 | 0.296336767 | 2.61E-06 | 2.19E-05 |
| ENSG00000131650 | kringle containing transmembrane protein 2(KREMEN2) | -1.391626191 | 0.278343681 | 5.74E-07 | 5.58E-06 |
| ENSG00000117318 | inhibitor of DNA binding 3, HLH protein(ID3) | -1.389003656 | 0.187596688 | 1.32E-13 | 4.10E-12 |
| ENSG00000111780 | NA | -1.387032008 | 0.472650919 | 0.003339993 | 0.011284226 |
| ENSG00000140465 | cytochrome P450 family 1 subfamily A member 1(CYP1A1) | -1.386030357 | 0.43929316 | 0.001604224 | 0.006028366 |
| ENSG00000151470 | chromosome 4 open reading frame 33(C4orf33) | -1.385607871 | 0.444152785 | 0.001810569 | 0.006674876 |
| ENSG00000213658 | linker for activation of T-cells(LAT) | -1.38517042 | 0.308151409 | 6.95E-06 | 5.18E-05 |
| ENSG00000175906 | ADP ribosylation factor like GTPase 4D(ARL4D) | -1.3835423 | 0.166892204 | 1.13E-16 | 5.08E-15 |
| ENSG00000119138 | Kruppel like factor 9(KLF9) | -1.382289587 | 0.247124333 | 2.23E-08 | 2.93E-07 |
| ENSG00000187678 | sprouty RTK signaling antagonist 4(SPRY4) | -1.378151976 | 0.183894168 | 6.67E-14 | 2.11E-12 |
| ENSG00000166823 | mesoderm posterior bHLH transcription factor 1(MESP1) | -1.377498969 | 0.364321774 | 0.000156203 | 0.000805244 |
| ENSG00000185507 | interferon regulatory factor 7(IRF7) | -1.377480893 | 0.236110283 | 5.41E-09 | 7.97E-08 |
| ENSG00000010704 | hemochromatosis(HFE) | -1.375872358 | 0.192133593 | 8.01E-13 | 2.16E-11 |
| ENSG00000185684 | EP400 N-terminal like(EP400NL) | -1.373756617 | 0.220983509 | 5.08E-10 | 9.00E-09 |
| ENSG00000136378 | ADAM metallopeptidase with thrombospondin type 1 motif 7(ADAMTS7) | -1.371919488 | 0.20396772 | 1.74E-11 | 3.86E-10 |
| ENSG00000132005 | regulatory factor X1(RFX1) | -1.370888874 | 0.221869919 | 6.46E-10 | 1.13E-08 |
| ENSG00000089820 | Rho GTPase activating protein 4(ARHGAP4) | -1.362181178 | 0.47718226 | 0.004308632 | 0.013978694 |
| ENSG00000152475 | zinc finger protein 837(ZNF837) | -1.360353813 | 0.405720738 | 0.000799605 | 0.003297075 |
| ENSG00000162775 | RNA binding motif protein 15(RBM15) | -1.35907714 | 0.191469457 | 1.26E-12 | 3.34E-11 |
| ENSG00000138172 | calcium homeostasis modulator 2(CALHM2) | -1.358194602 | 0.415305139 | 0.001074132 | 0.004261749 |
| ENSG00000026103 | Fas cell surface death receptor(FAS) | -1.356675556 | 0.296412779 | 4.72E-06 | 3.68E-05 |
| ENSG00000148700 | adducin 3(ADD3) | -1.355690134 | 0.289160924 | 2.75E-06 | 2.29E-05 |
| ENSG00000019582 | CD74 molecule(CD74) | -1.353714328 | 0.427993553 | 0.00156189 | 0.005885355 |
| ENSG00000176692 | forkhead box C2(FOXC2) | -1.350787189 | 0.210883424 | 1.50E-10 | 2.93E-09 |
| ENSG00000132002 | DnaJ heat shock protein family (Hsp40) member B1(DNAJB1) | -1.350685599 | 0.154273824 | 2.04E-18 | 1.10E-16 |
| ENSG00000135437 | retinol dehydrogenase 5(RDH5) | -1.347181203 | 0.238360489 | 1.59E-08 | 2.15E-07 |
| ENSG00000111727 | host cell factor C2(HCFC2) | -1.347147568 | 0.359014678 | 0.000175187 | 0.000892937 |
| ENSG00000140511 | hyaluronan and proteoglycan link protein 3(HAPLN3) | -1.345041311 | 0.195092626 | 5.41E-12 | 1.29E-10 |
| ENSG00000126246 | IGF like family receptor 1(IGFLR1) | -1.344218553 | 0.24936917 | 7.03E-08 | 8.26E-07 |
| ENSG00000114166 | lysine acetyltransferase 2B(KAT2B) | -1.343305181 | 0.322947804 | 3.19E-05 | 0.000199568 |
| ENSG00000198189 | hydroxysteroid 17-beta dehydrogenase 11(HSD17B11) | -1.342271396 | 0.241758683 | 2.82E-08 | 3.61E-07 |
| ENSG00000268434 | NA | -1.341928396 | 0.313043011 | 1.81E-05 | 0.000120859 |
| ENSG00000179403 | von Willebrand factor A domain containing 1(VWA1) | -1.340602643 | 0.23326985 | 9.08E-09 | 1.29E-07 |
| ENSG00000007944 | myosin regulatory light chain interacting protein(MYLIP) | -1.336611252 | 0.249432322 | 8.39E-08 | 9.73E-07 |
| ENSG00000149809 | transmembrane 7 superfamily member 2(TM7SF2) | -1.335219048 | 0.162610787 | 2.19E-16 | 9.41E-15 |
| ENSG00000164877 | MICAL like 2(MICALL2) | -1.334158053 | 0.271108385 | 8.60E-07 | 8.05E-06 |
| ENSG00000145632 | polo like kinase 2(PLK2) | -1.327071556 | 0.223121982 | 2.72E-09 | 4.21E-08 |
| ENSG00000040608 | reticulon 4 receptor(RTN4R) | -1.325092193 | 0.528690962 | 0.012197984 | 0.033293988 |
| ENSG00000111981 | UL16 binding protein 1(ULBP1) | -1.324457917 | 0.292638759 | 6.01E-06 | 4.54E-05 |
| ENSG00000179111 | hes family bHLH transcription factor 7(HES7) | -1.324109283 | 0.359959575 | 0.000234615 | 0.001147136 |
| ENSGO0000172382 | protease, serine 27(PRSS27) | -1.323392547 | 0.367625893 | 0.000318419 | 0.001491196 |
| ENSG00000257704 | InaF motif containing 1(INAFM1) | -1.323215307 | 0.198653995 | 2.72E-11 | 5.93E-10 |
| ENSG00000111912 | nuclear receptor coactivator 7(NCOA7) | -1.32275892 | 0.235006158 | 1.82E-08 | 2.43E-07 |
| ENSG00000172889 | EGF like domain multiple 7(EGFL7) | -1.322640988 | 0.246428941 | 8.00E-08 | 9.31E-07 |
| ENSG00000152076 | coiled-coil domain containing 74B(CCDC74B) | -1.321288717 | 0.231612284 | 1.17E-08 | 1.63E-07 |
| ENSG00000176532 | proline rich 15(PRR15) | -1.315823861 | 0.366987616 | 0.000336469 | 0.001564514 |
| ENSG00000171298 | glucosidase alpha, acid(GAA) | -1.314130802 | 0.201330675 | 6.70E-11 | 1.39E-09 |
| ENSG00000145861 | C1q and tumor necrosis factor related protein 2(C1QTNF2) | -1.310255954 | 0.256971565 | 3.42E-07 | 3.51E-06 |
| ENSG00000132793 | lipin 3(LPIN3) | -1.308546865 | 0.473795444 | 0.005747677 | 0.017732479 |
| | WAP, follistatin/kazal, immunoglobulin, kunitz and netrin domain containing | | | | |
| ENSG00000127578 | 1(WFIKKN1) | -1.305993492 | 0.434472508 | 0.002647709 | 0.009250859 |
| ENSG00000244270 | NA | -1.305682978 | 0.320270858 | 4.57E-05 | 0.000273902 |
| ENSG00000087253 | lysophosphatidylcholine acyltransferase 2(LPCAT2) | -1.301504404 | 0.212658984 | 9.35E-10 | 1.57E-08 |
| ENSG00000182993 | chromosome 12 open reading frame 60(Cl2orf60) | -1.30139818 | 0.44190418 | 0.003229774 | 0.010946431 |
| ENSG00000161544 | cytoglobin(CYGB) | -1.299876647 | 0.292036924 | 8.54E-06 | 6.20E-05 |
| ENSG00000182871 1 | collagen type XVIII alpha 1 chain(COL18A1) | -1.299107934 | 0.248492356 | 1.71E-07 | 1.87E-06 |
| ENSG00000203724 | chromosome 1 open reading frame 53(Clorf53) | -1.293489454 | 0.257260035 | 4.96E-07 | 4.89E-06 |
| ENSG00000116044 | nuclear factor, erythroid 2 like 2(NFE2L2) | -1.291898077 | 0.25337019 | 3.42E-07 | 3.51E-06 |
| ENSG00000090339 | intercellular adhesion molecule 1(ICAM1) | -1.290532655 | 0.180477431 | 8.64E-13 | 2.31E-11 |
| ENSG00000173334 | tribbles pseudokinase 1(TRIB1) | -1.288888965 | 0.15034907 | 1.01E-17 | 5.06E-16 |
| ENSG00000163545 | NUAK family kinase 2(NUAK2) | -1.287942514 | 0.152928335 | 3.71E-17 | 1.77E-15 |
| ENSG00000076864 | RAP1 GTPase activating protein(RAP1GAP) | -1.287751299 | 0.182447923 | 1.69E-12 | 4.37E-11 |
| ENSG00000154646 | transmembrane protease, serine 15(TMPRSS15) | -1.287535918 | 0.295191741 | 1.29E-05 | 8.94E-05 |
| ENSG00000176472 | zinc finger protein 575(ZNF575) | -1.285880384 | 0.268053936 | 1.61E-06 | 1.41E-05 |
| ENSG00000043355 | Zic family member 2(ZIC2) | -1.285245814 | 0.318579778 | 5.48E-05 | 0.000321971 |
| ENSG00000146350 | TBC1 domain family member 32(TBC1D32) | -1.283980338 | 0.411860803 | 0.001823855 | 0.006711239 |
| ENSG00000130720 | fibrinogen C domain containing 1(FIBCD1) | -1.283002851 | 0.209608348 | 9.30E-10 | 1.56E-08 |
| ENSG00000133639 | BTG anti-proliferation factor 1(BTG1) | -1.282346966 | 0.157953121 | 4.72E-16 | 1.98E-14 |
| ENSG00000188157 | agrin(AGRN) | -1.282299532 | 0.237258974 | 6.49E-08 | 7.65E-07 |
| ENSG00000163660 | cyclin L1(CCNL1) | -1.280574467 | 0.20996724 | 1.07E-09 | 1.77E-08 |
| ENSG00000165113 | G kinase anchoring protein 1(GKAP1) | -1.280526654 | 0.42999792 | 0.002901517 | 0.010012746 |
| ENSG00000168672 | family with sequence similarity 84 member B(FAM84B) | -1.280369803 | 0.214139137 | 2.24E-09 | 3.52E-08 |
| ENSG00000163283 | alkaline phosphatase, placental(ALPP) | -1.279485247 | 0.18399845 | 3.56E-12 | 8.68E-11 |
| ENSG00000121753 | adhesion G protein-coupled receptor B2(ADGRB2) | -1.277603325 | 0.246162054 | 2.10E-07 | 2.26E-06 |
| ENSG00000266086 | NA | -1.277240193 | 0.435765409 | 0.003378434 | 0.011401003 |
| ENSG00000240370 | ribosomal protein L13 pseudogene 5(RPL13P5) | -1.277150082 | 0.320568034 | 6.78E-05 | 0.000387136 |
| ENSG00000177721 | annexin A2 receptor(ANXA2R) | -1.274363264 | 0.341353812 | 0.000189016 | 0.000953917 |
| ENSG00000142920 | antizyme inhibitor 2(AZIN2) | -1.273258573 | 0.17969792 | 1.38E-12 | 3.63E-11 |
| ENSG00000167508 | mevalonate diphosphate decarboxylase(MVD) | -1.272969462 | 0.21865659 | 5.82E-09 | 8.54E-08 |
| ENSG00000186567 | carcinoembryonic antigen related cell adhesion molecule 19(CEACAM19) | -1.272730821 | 0.241979559 | 1.44E-07 | 1.60E-06 |
| ENSG00000266094 | Ras association domain family member 5(RASSF5) | -1.271853405 | 0.239487414 | 1.09E-07 | 1.24E-06 |
| ENSG00000080298 | regulatory factor X3(RFX3) | -1.271852988 | 0.338166348 | 0.000169217 | 0.000867015 |
| ENSG00000168497 | serum deprivation response(SDPR) | -1.269449986 | 0.230121545 | 3.46E-08 | 4.32E-07 |
| ENSG00000114698 | phospholipid scramblase 4(PLSCR4) | -1.26784236 | 0.333412785 | 0.000143173 | 0.000746915 |
| ENSG00000088448 | ankyrin repeat domain 10(ANKRD10) | -1.266655861 | 0.160495099 | 2.97E-15 | 1.14E-13 |
| ENSG00000197766 | complement factor D(CFD) | -1.266366716 | 0.330127047 | 0.000125055 | 0.000664777 |
| ENSG00000196569 | laminin subunit alpha 2(LAMA2) | -1.265277663 | 0.288753747 | 1.18E-05 | 8.25E-05 |
| ENSG00000112149 | CD83 molecule(CD83) | -1.264100872 | 0.146184615 | 5.27E-18 | 2.71E-16 |
| ENSG00000183506 | phosphatidylinositol 4-kinase alpha pseudogene 2(PI4KAP2) | -1.263955666 | 0.248772958 | 3.76E-07 | 3.83E-06 |
| ENSG00000237846 | NA | -1.26358563 | 0.308968135 | 4.32E-05 | 0.000261348 |
| ENSG00000144366 | GULP, engulfment adaptor PTB domain containing 1(GULP1) | -1.257165094 | 0.287771276 | 1.25E-05 | 8.68E-05 |
| ENSG00000002330 | BCL2 associated agonist of cell death(BAD) | -1.256069693 | 0.252099012 | 6.28E-07 | 6.06E-06 |
| ENSG00000173269 | multimerin 2(MMRN2) | -1.254853691 | 0.35940856 | 0.000480423 | 0.002130304 |
| ENSG00000114796 | kelch like family member 24(KLHL24) | -1.253685747 | 0.340744662 | 0.000233918 | 0.001144883 |
| ENSG00000186395 | keratin 10(KRT10) | -1.252066604 | 0.177793237 | 1.89E-12 | 4.83E-11 |
| ENSG00000124766 | SRY-box 4(SOX4) | -1.248492458 | 0.192950354 | 9.77E-11 | 1.98E-09 |
| ENSG00000117000 | rearranged L-myc fusion(RLF) | -1.248310893 | 0.281525227 | 9.25E-06 | 6.64E-05 |
| ENSG00000147251 | dedicator of cytokinesis 11(DOCK11) | -1.24823725 | 0.363233857 | 0.000589378 | 0.002536005 |
| ENSG00000143554 | solute carrier family 27 member 3(SLC27A3) | -1.246506733 | 0.299317239 | 3.12E-05 | 0.000195462 |
| ENSG00000121410 | alpha-1-B glycoprotein(A1BG) | -1.246430559 | 0.210887854 | 3.41E-09 | 5.19E-08 |
| ENSG00000184545 | dual specificity phosphatase 8(DUSP8) | -1.243609246 | 0.18994626 | 5.86E-11 | 1.23E-09 |
| ENSG00000175198 | propionyl-CoA carboxylase alpha subunit(PCCA) | -1.243072176 | 0.324610229 | 0.00012844 | 0.000680312 |
| ENSG00000221883 | ariadne homolog 2 opposite strand(ARIH2OS) | -1.240910532 | 0.488573731 | 0.011089578 | 0.030841085 |
| ENSG00000185522 | lamin tail domain containing 2(LMNTD2) | -1.238601973 | 0.280940768 | 1.04E-05 | 7.39E-05 |
| ENSG00000179085 | dolichyl-phosphate mannosyltransferase subunit 3(DPM3) | -1.238434242 | 0.25361922 | 1.04E-06 | 9.59E-06 |
| ENSG00000167968 | deoxyribonuclease 1 like 2(DNASE1L2) | -1.238079241 | 0.310627458 | 6.73E-05 | 0.000384724 |
| ENSG00000158467 | adenosylhomocysteinase like 2(AHCYL2) | -1.237270757 | 0.352239983 | 0.00044378 | 0.001988811 |
| ENSG00000237515 | shisa family member 9(SHISA9) | -1.236212322 | 0.31061319 | 6.89E-05 | 0.00039294 |
| ENSG00000167996 | ferritin heavy chain 1(FTH1) | -1.235939832 | 0.204273049 | 1.44E-09 | 2.35E-08 |
| ENSG00000109501 | wolframin ER transmembrane glycoprotein(WFS1) | -1.235681418 | 0.185037203 | 2.42E-11 | 5.32E-10 |
| ENSG00000134107 | basic helix-loop-helix family member e40(BHLHE40) | -1.234937929 | 0.148807055 | 1.05E-16 | 4.75E-15 |
| ENSG00000119227 | phosphatidylinositol glycan anchor biosynthesis class Z(PIGZ) | -1.232786998 | 0.245957136 | 5.38E-07 | 5.25E-06 |
| ENSG00000232774 | uncharacterized LOC400221(FU22447) | -1.232638152 | 0.341177464 | 0.000302798 | 0.001428847 |
| ENSG00000205544 | transmembrane protein 256(TMEM256) | -1.229789471 | 0.163452339 | 5.32E-14 | 1.74E-12 |
| ENSG00000105997 | homeobox A3(HOXA3) | -1.229545376 | 0.33563127 | 0.000248907 | 0.001203513 |
| ENSG00000067064 | isopentenyl-diphosphate delta isomerase 1(IDI1) | -1.229157627 | 0.203705887 | 1.60E-09 | 2.58E-08 |
| ENSG00000162148 | protein phosphatase 1 regulatory subunit 32(PPP1R32) | -1.227965592 | 0.371622329 | 0.000952012 | 0.003840694 |
| ENSG00000251209 | long intergenic non-protein coding RNA 923(LINC00923) | -1.227566241 | 0.293302589 | 2.85E-05 | 0.000180394 |
| ENSG00000187240 | dynein cytoplasmic 2 heavy chain 1(DYNC2H1) | -1.227514932 | 0.39812392 | 0.002047542 | 0.007411471 |
| ENSG00000244731 | complement C4A (Rodgers blood group)(C4A) | -1.227494073 | 0.292948821 | 2.79E-05 | 0.000177281 |
| ENSG00000143028 | synaptophysin like 2(SYPL2) | -1.22725427 | 0.451325922 | 0.006543616 | 0.019788971 |
| ENSG00000174938 | seizure related 6 homolog like 2(SEZ6L2) | -1.227075654 | 0.160020133 | 1.74E-14 | 6.00E-13 |
| ENSG00000135111 | T-box 3(TBX3) | -1.22699259 | 0.145256862 | 2.99E-17 | 1.45E-15 |
| ENSG00000183246 | RIMS binding protein 3C(RIMBP3C) | -1.226765947 | 0.246339633 | 6.36E-07 | 6.13E-06 |
| ENSG00000258643 | BCL2L2-PABPN1 readthrough(BCL2L2-PABPN1) | -1.226316559 | 0.292057833 | 2.68E-05 | 0.000171589 |
| ENSG00000142856 | integrin subunit beta 3 binding protein(ITGB3BP) | -1.224019574 | 0.17083165 | 7.78E-13 | 2.10E-11 |
| ENSG00000090006 | latent transforming growth factor beta binding protein 4(LTBP4) | -1.223205364 | 0.217854296 | 1.97E-08 | 2.60E-07 |
| ENSG00000170540 | ADP ribosylation factor like GTPase 6 interacting protein 1(ARL6IP1) | -1.22293556 | 0.199260282 | 8.39E-10 | 1.43E-08 |
| ENSG00000102854 | mesothelin(MSLN) | -1.221569434 | 0.198659063 | 7.79E-10 | 1.33E-08 |
| ENSG00000183891 | tetratricopeptide repeat domain 32(TTC32) | -1.220509006 | 0.429616107 | 0.004498228 | 0.014470099 |
| ENSG00000131094 | complement C1q like 1(C1QL1) | -1.220151489 | 0.197797291 | 6.89E-10 | 1.19E-08 |
| ENSG00000049089 | collagen type IX alpha 2 chain(COL9A2) | -1.219482505 | 0.214115318 | 1.23E-08 | 1.71E-07 |
| ENSG00000189409 | matrix metallopeptidase 23B(MMP23B) | -1.218574717 | 0.285054242 | 1.91E-05 | 0.000127029 |
| ENSG00000133466 | C1q and tumor necrosis factor related protein 6(C1QTNF6) | -1.216444765 | 0.178522264 | 9.49E-12 | 2.18E-10 |
| ENSG00000130758 | mitogen-activated protein kinase kinase kinase 10(MAP3K10) | -1.21556266 | 0.232813087 | 1.78E-07 | 1.94E-06 |
| ENSG00000007080 | coiled-coil domain containing 124(CCDC124) | -1.214901753 | 0.257008132 | 2.28E-06 | 1.93E-05 |
| ENSG00000070404 | follistatin like 3(FSTL3) | -1.214582956 | 0.167587012 | 4.25E-13 | 1.20E-11 |
| ENSG00000168675 | low density lipoprotein receptor class A domain containing 4(LDLRAD4) | -1.21455556 | 0.313713944 | 0.000108151 | 0.000584648 |
| ENSG00000143320 | cellular retinoic acid binding protein 2(CRABP2) | -1.214185331 | 0.276240745 | 1.11E-05 | 7.79E-05 |
| ENSG00000019102 | V-set and immunoglobulin domain containing 2(VSIG2) | -1.214163691 | 0.329481037 | 0.000228632 | 0.001123016 |
| ENSG00000165507 | chromosome 10 open reading frame 10(C10orf10) | -1.213191681 | 0.202759516 | 2.19E-09 | 3.45E-08 |
| ENSG00000178401 | DnaJ heat shock protein family (Hsp40) member C22(DNAJC22) | -1.212480831 | 0.245664079 | 7.99E-07 | 7.53E-06 |
| ENSG00000167702 | kinesin family member C2(KIFC2) | -1.209573163 | 0.250471072 | 1.37E-06 | 1.22E-05 |
| ENSG00000167646 | dynein axonemal assembly factor 3(DNAAF3) | -1.206687392 | 0.272104761 | 9.22E-06 | 6.62E-05 |
| ENSG00000156500 | family with sequence similarity 122C(FAM122C) | -1.20598013 | 0.327184186 | 0.000227863 | 0.001120177 |
| ENSG00000115963 | Rho family GTPase 3(RND3) | -1.205548671 | 0.221716811 | 5.41E-08 | 6.49E-07 |
| ENSG00000105409 | ATPase Na+/K+ transporting subunit alpha 3(ATP1A3) | -1.205210098 | 0.27909871 | 1.57E-05 | 0.000106711 |
| ENSG00000008516 | matrix metallopeptidase 25(MMP25) | -1.204866326 | 0.245609457 | 9.31E-07 | 8.63E-06 |
| ENSG00000172349 | interleukin 16(IL16) | -1.204164901 | 0.303180568 | 7.13E-05 | 0.000404255 |
| ENSG00000196993 | nuclear pore complex interacting protein family member B9(NPIPB9) | -1.203699314 | 0.461216965 | 0.00905864 | 0.0260107 |
| ENSG00000203965 | EF-hand calcium binding domain 7(EFCAB7) | -1.203548672 | 0.349544325 | 0.000574888 | 0.002480009 |
| ENSG00000196337 | chorionic gonadotropin beta subunit 7(CGB7) | -1.201802165 | 0.286503243 | 2.73E-05 | 0.000174482 |
| ENSG00000232133 | NA | -1.20125889 | 0.187371297 | 1.44E-10 | 2.83E-09 |
| ENSG00000137880 | GTP cyclohydrolase I feedback regulator(GCHFR) | -1.200882318 | 0.183480049 | 5.95E-11 | 1.24E-09 |
| ENSG00000072571 | hyaluronan mediated motility receptor(HMMR) | -1.199672273 | 0.230566868 | 1.96E-07 | 2.12E-06 |
| ENSG00000159339 | peptidyl arginine deiminase 4(PADI4) | -1.199099961 | 0.382920386 | 0.001739393 | 0.006448857 |
| ENSG00000151338 | mirror-image polydactyly 1(MIPOL1) | -1.197444652 | 0.264980763 | 6.21E-06 | 4.66E-05 |
| ENSG00000205771 | cation channel sperm associated 2 pseudogene 1(CATSPER2P1) | -1.197220496 | 0.329495157 | 0.000279603 | 0.001336007 |
| ENSG00000184985 | sortilin related VPS10 domain containing receptor 2(SORCS2) | -1.197000615 | 0.20184475 | 3.02E-09 | 4.65E-08 |
| ENSG00000246705 | H2A histone family member J(H2AFJ) | -1.19648205 | 0.215193878 | 2.70E-08 | 3.47E-07 |
| ENSG00000170385 | solute carrier family 30 member 1(SLC30A1) | -1.195735361 | 0.281588646 | 2.17E-05 | 0.000141901 |
| ENSG00000130653 | patatin like phospholipase domain containing 7(PNPLA7) | -1.192230975 | 0.287288665 | 3.33E-05 | 0.000206888 |
| ENSG00000162490 | dorsal inhibitory axon guidance protein(DRAXIN) | -1.192112885 | 0.392281695 | 0.002374275 | 0.008417292 |
| ENSG00000114200 | butyrylcholinesterase(BCHE) | -1.189934634 | 0.2328682 | 3.22E-07 | 3.33E-06 |
| ENSG00000186998 | EMI domain containing 1(EMID1) | -1.187421606 | 0.339825465 | 0.000475466 | 0.002110712 |
| ENSG00000149781 | fermitin family member 3(FERMT3) | -1.187333512 | 0.311639013 | 0.000138987 | 0.000727093 |
| ENSG00000176912 | NA | -1.186476373 | 0.387809732 | 0.002217593 | 0.007951162 |
| ENSG00000175920 | docking protein 7(DOK7) | -1.186280164 | 0.267833891 | 9.46E-06 | 6.78E-05 |
| ENSG00000144152 | fibulin 7(FBLN7) | -1.184876263 | 0.221621554 | 8.97E-08 | 1.03E-06 |
| ENSG00000144485 | hes family bHLH transcription factor 6(HES6) | -1.184535254 | 0.184309119 | 1.30E-10 | 2.59E-09 |
| ENSG00000122257 | RB binding protein 6, ubiquitin ligase(RBBP6) | -1.183253302 | 0.212333487 | 2.51E-08 | 3.25E-07 |
| ENSG00000260916 | microRNA 628(MIR628) | -1.181117938 | 0.261473686 | 6.27E-06 | 4.70E-05 |
| ENSG00000117569 | polypyrimidine tract binding protein 2(PTBP2) | -1.180927691 | 0.322990477 | 0.000255951 | 0.001234526 |
| ENSG00000174059 | CD34 molecule(CD34) | -1.179405741 | 0.220443017 | 8.79E-08 | 1.02E-06 |
| ENSG00000013441 | CDC like kinase 1(CLK1) | -1.179316015 | 0.203120845 | 6.40E-09 | 9.33E-08 |
| ENSG00000159733 | zinc finger FYVE-type containing 28(ZFYVE28) | -1.179033587 | 0.292617759 | 5.60E-05 | 0.0003278 |
| ENSG00000140379 | BCL2 related protein A1(BCL2A1) | -1.178600303 | 0.316025646 | 0.000191902 | 0.00096641 |
| ENSG00000107331 | ATP binding cassette subfamily A member 2(ABCA2) | -1.177120123 | 0.186404178 | 2.70E-10 | 5.04E-09 |
| ENSG00000092096 | solute carrier family 22 member 17(SLC22A17) | -1.175199236 | 0.207950469 | 1.59E-08 | 2.15E-07 |
| ENSG00000108813 | distal-less homeobox 4(DLX4) | -1.175016137 | 0.260337922 | 6.38E-06 | 4.77E-05 |
| ENSG00000131462 | tubulin gamma 1(TUBG1) | -1.174285626 | 0.151282581 | 8.35E-15 | 3.00E-13 |
| ENSG00000145335 | synuclein alpha(SNCA) | -1.173998066 | 0.414662957 | 0.004637221 | 0.014828015 |
| ENSG00000127586 | chromosome transmission fidelity factor 18(CHTF18) | -1.170761306 | 0.469364764 | 0.012618706 | 0.03427533 |
| ENSG00000197579 | TOP1 binding arginine/serine rich protein(TOPORS) | -1.169752915 | 0.27196964 | 1.70E-05 | 0.000114538 |
| ENSG00000170629 | DPY19L2 pseudogene 2(DPY19L2P2) | -1.168586074 | 0.199188538 | 4.44E-09 | 6.65E-08 |
| ENSG00000130066 | spermidine/spermine N1-acetyltransferase 1(SAT1) | -1.167798887 | 0.187046729 | 4.28E-10 | 7.68E-09 |
| ENSG00000122644 | ADP ribosylation factor like GTPase 4A(ARL4A) | -1.167131197 | 0.267064172 | 1.24E-05 | 8.63E-05 |
| ENSG00000184500 | protein S (alpha)(PROS1) | -1.167085866 | 0.25762088 | 5.89E-06 | 4.46E-05 |
| ENSG00000087245 | matrix metallopeptidase 2(MMP2) | -1.166808995 | 0.161121819 | 4.43E-13 | 1.24E-11 |
| ENSG00000164056 | sprouty RTK signaling antagonist 1(SPRY1) | -1.166788374 | 0.196483671 | 2.88E-09 | 4.45E-08 |
| ENSG00000153140 | centrin 3(CETN3) | -1.166625543 | 0.227049833 | 2.77E-07 | 2.91E-06 |
| ENSG00000233608 | twist family bHLH transcription factor 2(TWIST2) | -1.166158283 | 0.274582641 | 2.17E-05 | 0.000141581 |
| ENSG00000003137 | cytochrome P450 family 26 subfamily B member 1(CYP26B1) | -1.165711873 | 0.268686586 | 1.43E-05 | 9.82E-05 |
| ENSG00000176046 | nuclear protein 1, transcriptional regulator(NUPR1) | -1.164206727 | 0.195154956 | 2.44E-09 | 3.79E-08 |
| ENSG00000262209 | protocadherin gamma subfamily B, 3(PCDHGB3) | -1.163721068 | 0.386824229 | 0.002626293 | 0.009189683 |
| ENSG00000064205 | WNT1 inducible signaling pathway protein 2(WISP2) | -1.163463607 | 0.40570023 | 0.00413348 | 0.013481082 |
| ENSG00000100985 | matrix metallopeptidase 9(MMP9) | -1.162013363 | 0.357295934 | 0.001144979 | 0.004509385 |
| ENSG00000064687 | ATP binding cassette subfamily A member 7(ABCA7) | -1.161753049 | 0.19813309 | 4.53E-09 | 6.75E-08 |
| ENSG00000113448 | phosphodiesterase 4D(PDE4D) | -1.15988012 | 0.305638583 | 0.000147679 | 0.000767018 |
| ENSG00000204060 | forkhead box O6(FOXO6) | -1.15957387 | 0.227090966 | 3.29E-07 | 3.39E-06 |
| ENSG00000006015 | chromosome 19 open reading frame 60(C19orf60) | -1.159064515 | 0.230279489 | 4.82E-07 | 4.77E-06 |
| ENSG00000167034 | NK3 homeobox 1(NKX3-1) | -1.157146137 | 0.240939513 | 1.57E-06 | 1.37E-05 |
| ENSG00000255513 | NA | -1.153434569 | 0.250225999 | 4.04E-06 | 3.21E-05 |
| ENSG00000127990 | sarcoglycan epsilon(SGCE) | -1.149580561 | 0.318303043 | 0.000304324 | 0.001435471 |
| ENSG00000066248 | neuronal guanine nucleotide exchange factor(NGEF) | -1.149137393 | 0.238669818 | 1.47E-06 | 1.31E-05 |
| ENSG00000161664 | ankyrin repeat and SOCS box containing 16(ASB16) | -1.149082083 | 0.382074072 | 0.00263419 | 0.009209098 |
| ENSG00000129226 | CD68 molecule(CD68) | -1.148543164 | 0.211273717 | 5.44E-08 | 6.51E-07 |
| ENSG00000178821 | transmembrane protein 52(TMEM52) | -1.147512129 | 0.247945049 | 3.69E-06 | 2.96E-05 |
| ENSG00000133138 | TBC1 domain family member 8B(TBC1D8B) | -1.147283955 | 0.322545844 | 0.000375166 | 0.001717295 |
| ENSG00000135477 | NA | -1.147240898 | 0.288740469 | 7.09E-05 | 0.000402129 |
| ENSG00000103024 | NME/NM23 nucleoside diphosphate kinase 3(NME3) | -1.146966272 | 0.452247333 | 0.011207928 | 0.031094528 |
| ENSG00000137642 | sortilin related receptor 1(SORL1) | -1.145699172 | 0.222127875 | 2.50E-07 | 2.65E-06 |
| ENSG00000130962 | proline rich and Gla domain 1(PRRG1) | -1.145009153 | 0.214586077 | 9.51E-08 | 1.09E-06 |
| ENSG00000182809 | cysteine rich protein 2(CRIP2) | -1.144615442 | 0.246695535 | 3.49E-06 | 2.82E-05 |
| ENSG00000176293 | zinc finger protein 135(ZNF135) | -1.14375124 | 0.3362022 | 0.000669009 | 0.002834075 |
| ENSG00000105227 | periaxin(PRX) | -1.143673145 | 0.202710354 | 1.68E-08 | 2.26E-07 |
| ENSG00000005020 | src kinase associated phosphoprotein 2(SKAP2) | -1.143341079 | 0.290621572 | 8.35E-05 | 0.000467987 |
| ENSG00000123384 | LDL receptor related protein 1(LRP1) | -1.143249805 | 0.186819141 | 9.38E-10 | 1.57E-08 |
| ENSG00000213398 | lecithin-cholesterol acyltransferase(LCAT) | -1.141080803 | 0.178208497 | 1.52E-10 | 2.96E-09 |
| ENSG00000227081 | NA | -1.141062865 | 0.461217105 | 0.013360017 | 0.035965044 |
| ENSG00000054654 | spectrin repeat containing nuclear envelope protein 2(SYNE2) | -1.140086186 | 0.200962214 | 1.40E-08 | 1.92E-07 |
| ENSG00000116661 | F-box protein 2(FBXO2) | -1.139528638 | 0.378255029 | 0.002590256 | 0.009071681 |
| ENSG00000073350 | LLGL2, scribble cell polarity complex component(LLGL2) | -1.13811109 | 0.227036386 | 5.36E-07 | 5.23E-06 |
| ENSG00000132535 | discs large MAGUK scaffold protein 4(DLG4) | -1.137465093 | 0.15530963 | 2.41E-13 | 7.19E-12 |
| ENSG00000180902 | D-2-hydroxyglutarate dehydrogenase(D2HGDH) | -1.136029387 | 0.233369595 | 1.13E-06 | 1.03E-05 |
| ENSG00000198168 | small VCP interacting protein(SVIP) | -1.135618834 | 0.350154985 | 0.001181997 | 0.004628834 |
| ENSG00000159714 | zinc finger DHHC-type containing 1(ZDHHC1) | -1.134877443 | 0.294820834 | 0.000118417 | 0.000633497 |
| ENSG00000121039 | retinol dehydrogenase 10 (all-trans)(RDH10) | -1.134068633 | 0.371734873 | 0.002282737 | 0.008152434 |
| ENSG00000183873 | sodium voltage-gated channel alpha subunit 5(SCN5A) | -1.133891883 | 0.397197308 | 0.004307315 | 0.013978278 |
| ENSG00000167487 | kelch like family member 26(KLHL26) | -1.133562539 | 0.22695914 | 5.90E-07 | 5.72E-06 |
| ENSG00000250251 | NA | -1.133437283 | 0.242330669 | 2.91E-06 | 2.40E-05 |
| ENSG00000187837 | histone cluster 1 H1 family member c(HIST1H1C) | -1.132860268 | 0.156860615 | 5.12E-13 | 1.42E-11 |
| ENSG00000185022 | MAF bZIP transcription factor F(MAFF) | -1.132219693 | 0.152073849 | 9.68E-14 | 3.04E-12 |
| ENSG00000171680 | pleckstrin homology and RhoGEF domain containing G5(PLEKHG5) | -1.131197808 | 0.20840687 | 5.70E-08 | 6.81E-07 |
| ENSG00000227097 | NA | -1.130938565 | 0.238303162 | 2.08E-06 | 1.78E-05 |
| ENSG00000103227 | lipase maturation factor 1(LMF1) | -1.129824405 | 0.312506632 | 0.000299929 | 0.001417583 |
| ENSG00000169710 | fatty acid synthase(FASN) | -1.128614631 | 0.249951543 | 6.32E-06 | 4.73E-05 |
| ENSG00000187193 | metallothionein 1X(MT1X) | -1.128418314 | 0.163766321 | 5.56E-12 | 1.32E-10 |
| ENSG00000164463 | CREB3 regulatory factor(CREBRF) | -1.128100896 | 0.317969321 | 0.00038842 | 0.001769798 |
| ENSG00000125148 | metallothionein 2A(MT2A) | -1.127412656 | 0.245618814 | 4.43E-06 | 3.49E-05 |
| ENSG00000173083 | heparanase(HPSE) | -1.127233471 | 0.174691371 | 1.10E-10 | 2.20E-09 |
| ENSG00000170955 | protein kinase C delta binding protein(PRKCDBP) | -1.126250077 | 0.198471913 | 1.39E-08 | 1.91E-07 |
| ENSG00000171227 | transmembrane protein 37(TMEM37) | -1.12591033 | 0.371944601 | 0.002469192 | 0.00870209 |
| ENSG00000142173 | collagen type VI alpha 2 chain(COL6A2) | -1.125845338 | 0.223599994 | 4.78E-07 | 4.74E-06 |
| ENSG00000186469 | G protein subunit gamma 2(GNG2) | -1.125025408 | 0.233362538 | 1.43E-06 | 1.27E-05 |
| ENSG00000196878 | microRNA 4260(MIR4260) | -1.12271331 | 0.213404052 | 1.43E-07 | 1.59E-06 |
| ENSG00000104936 | dystrophia myotonica protein kinase(DMPK) | -1.122447929 | 0.198852959 | 1.66E-08 | 2.23E-07 |
| ENSG00000179029 | transmembrane protein 107(TMEM107) | -1.121494266 | 0.182924672 | 8.74E-10 | 1.48E-08 |
| ENSG00000198718 | family with sequence similarity 179 member B(FAM179B) | -1.12128867 | 0.36716295 | 0.002258674 | 0.00807876 |
| ENSG00000099977 | D-dopachrome tautomerase(DDT) | -1.12104546 | 0.249479322 | 7.00E-06 | 5.21E-05 |
| ENSG00000104419 | N-myc downstream regulated 1(NDRG1) | -1.120408701 | 0.154425318 | 4.01E-13 | 1.13E-11 |
| ENSG00000126787 | DLG associated protein 5(DLGAP5) | -1.120021321 | 0.237648747 | 2.44E-06 | 2.05E-05 |
| ENSG00000135424 | integrin subunit alpha 7(ITGA7) | -1.119671666 | 0.179432732 | 4.37E-10 | 7.82E-09 |
| ENSG00000122971 | acyl-CoA dehydrogenase, C-2 to C-3 short chain(ACADS) | -1.118463306 | 0.323279087 | 0.000540687 | 0.002354048 |
| ENSG00000136603 | SKI like proto-oncogene(SKIL) | -1.116930953 | 0.276537856 | 5.37E-05 | 0.00031672 |
| ENSG00000163354 | DC-STAMP domain containing 2(DCST2) | -1.116805589 | 0.43606756 | 0.010434617 | 0.029279287 |
| ENSG00000173991 | titin-cap(TCAP) | -1.112846195 | 0.277319904 | 6.00E-05 | 0.000348438 |
| ENSG00000188641 | dihydropyrimidine dehydrogenase(DPYD) | -1.112802529 | 0.275682394 | 5.42E-05 | 0.000319552 |
| ENSG00000128342 | leukemia inhibitory factor(LIF) | -1.112050155 | 0.157880754 | 1.87E-12 | 4.79E-11 |
| ENSG00000172663 | transmembrane protein 134(TMEM134) | -1.110720956 | 0.205169273 | 6.17E-08 | 7.30E-07 |
| ENSG00000187244 | basal cell adhesion molecule (Lutheran blood group)(BCAM) | -1.109807884 | 0.190570043 | 5.76E-09 | 8.47E-08 |
| ENSG00000167244 | insulin like growth factor 2(IGF2) | -1.10951772 | 0.330983876 | 0.000801777 | 0.003303715 |
| ENSG00000136213 | uncharacterized LOC101927181(LOC101927181) | -1.109200103 | 0.21782664 | 3.54E-07 | 3.62E-06 |
| ENSG00000142765 | synaptotagmin like 1(SYTL1) | -1.108833475 | 0.326337469 | 0.00067927 | 0.002868246 |
| ENSG00000084764 | microtubule associated protein RP/EB family member 3(MAPRE3) | -1.108515513 | 0.193465138 | 1.01E-08 | 1.42E-07 |
| ENSG00000256073 | URB1 antisense RNA 1 (head to head)(URB1-AS1) | -1.108456172 | 0.223060572 | 6.72E-07 | 6.44E-06 |
| ENSG00000187608 | ISG15 ubiquitin-like modifier(ISG15) | -1.107935176 | 0.242482102 | 4.90E-06 | 3.80E-05 |
| ENSG00000090539 | chordin(CHRD) | -1.107269146 | 0.215550627 | 2.79E-07 | 2.93E-06 |
| ENSG00000250474 | NA | -1.106963222 | 0.402300964 | 0.005930856 | 0.018202607 |
| ENSG00000167771 | REST corepressor 2(RCOR2) | -1.106624835 | 0.275556521 | 5.92E-05 | 0.000344787 |
| ENSG00000121749 | TBC1 domain family member 15(TBC1D15) | -1.106362835 | 0.265235696 | 3.03E-05 | 0.000190679 |
| ENSG00000148950 | inner mitochondrial membrane peptidase subunit 1(IMMP1L) | -1.104790607 | 0.26072614 | 2.26E-05 | 0.000146993 |
| ENSG00000109929 | sterol-C5-desaturase(SC5D) | -1.104660329 | 0.219016897 | 4.57E-07 | 4.55E-06 |
| ENSG00000007255 | trafficking protein particle complex 6A(TRAPPC6A) | -1.103399734 | 0.215911734 | 3.21E-07 | 3.32E-06 |
| ENSG00000229644 | NA | -1.103281735 | 0.374146695 | 0.00319016 | 0.010824644 |
| ENSG00000142530 | family with sequence similarity 71 member E1(FAM71E1) | -1.103161343 | 0.362907161 | 0.002367436 | 0.00839883 |
| ENSG00000133460 | solute carrier family 2 member 11(SLC2A11) | -1.102308347 | 0.199370401 | 3.22E-08 | 4.08E-07 |
| ENSG00000136870 | zinc finger protein 189(ZNF189) | -1.101021783 | 0.287636344 | 0.00012928 | 0.000682916 |
| ENSG00000130270 | ATPase phospholipid transporting 8B3(ATP8B3) | -1.100581727 | 0.255708211 | 1.68E-05 | 0.000113251 |
| ENSG00000105854 | paraoxonase 2(PON2) | -1.099668703 | 0.24039423 | 4.77E-06 | 3.72E-05 |
| ENSG00000182712 | C-X9-C motif containing 4(CMC4) | -1.098895896 | 0.27301832 | 5.70E-05 | 0.00033331 |
| ENSG00000170801 | HtrA serine peptidase 3(HTRA3) | -1.098776879 | 0.201536868 | 4.98E-08 | 6.03E-07 |
| ENSG00000269374 | NA | -1.098482272 | 0.44234168 | 0.013015887 | 0.035159332 |
| ENSG00000138036 | dynein cytoplasmic 2 light intermediate chain 1(DYNC2LI1) | -1.096483354 | 0.316588714 | 0.000533332 | 0.002327196 |
| ENSG00000260103 | uncharacterized LOC101929333(LOC101929333) | -1.094681756 | 0.191660099 | 1.12E-08 | 1.57E-07 |
| ENSG00000130529 | transient receptor potential cation channel subfamily M member 4(TRPM4) | -1.094359782 | 0.192775691 | 1.37E-08 | 1.89E-07 |
| ENSG00000137575 | syndecan binding protein(SDCBP) | -1.093772189 | 0.22536116 | 1.21E-06 | 1.09E-05 |
| ENSG00000071243 | inhibitor of growth family member 3(ING3) | -1.093356997 | 0.289231578 | 0.000156694 | 0.000807425 |
| ENSG00000185187 | single Ig and TIR domain containing(SIGIRR) | -1.09296249 | 0.226350599 | 1.37E-06 | 1.23E-05 |
| ENSG00000161642 | zinc finger protein 385A(ZNF385A) | -1.092800624 | 0.17047787 | 1.45E-10 | 2.84E-09 |
| ENSG00000127419 | transmembrane protein 175(TMEM175) | -1.091658722 | 0.176593499 | 6.34E-10 | 1.11E-08 |
| ENSG00000144445 | KAT8 regulatory NSL complex subunit 1 like(KANSL1L) | -1.089943005 | 0.297302741 | 0.000246273 | 0.001194211 |
| ENSG00000143578 | cAMP responsive element binding protein 3 like 4(CREB3L4) | -1.088668484 | 0.196934121 | 3.24E-08 | 4.09E-07 |
| ENSG00000137501 | synaptotagmin like 2(SYTL2) | -1.08858528 | 0.41701737 | 0.009043442 | 0.025989114 |
| ENSG00000116883 | NA | -1.08809691 | 0.33653098 | 0.001223801 | 0.004765571 |
| ENSG00000153487 | inhibitor of growth family member 1(ING1) | -1.086282722 | 0.198250624 | 4.27E-08 | 5.26E-07 |
| ENSG00000186174 | B-cell CLL/lymphoma 9-like(BCL9L) | -1.086247345 | 0.204829497 | 1.14E-07 | 1.28E-06 |
| ENSG00000009694 | teneurin transmembrane protein 1(TENM1) | -1.086247035 | 0.244215439 | 8.67E-06 | 6.29E-05 |
| ENSG00000154265 | ATP binding cassette subfamily A member 5(ABCA5) | -1.085919753 | 0.290690029 | 0.000187222 | 0.000946488 |
| ENSG00000149289 | zinc finger CCCH-type containing 12C(ZC3H12C) | -1.085183304 | 0.214598996 | 4.26E-07 | 4.28E-06 |
| ENSG00000167842 | MIS12, kinetochore complex component(MIS12) | -1.08471645 | 0.237595617 | 4.99E-06 | 3.86E-05 |
| ENSG00000141540 | tweety family member 2(TTYH2) | -1.083705624 | 0.374217974 | 0.003780478 | 0.012489059 |
| ENSG00000184384 | mastermind like transcriptional coactivator 2(MAML2) | -1.083000129 | 0.246838164 | 1.15E-05 | 8.06E-05 |
| ENSG00000125534 | pancreatic progenitor cell differentiation and proliferation factor(PPDPF) | -1.082285105 | 0.197636707 | 4.35E-08 | 5.34E-07 |
| ENSG00000081087 | osteopetrosis associated transmembrane protein 1(OSTM1) | -1.081719843 | 0.236385776 | 4.74E-06 | 3.69E-05 |
| ENSG00000129355 | cyclin dependent kinase inhibitor 2D(CDKN2D) | -1.081396053 | 0.173162917 | 4.24E-10 | 7.63E-09 |
| ENSG00000158014 | solute carrier family 30 member 2(SLC30A2) | -1.080994561 | 0.33764985 | 0.00136702 | 0.005252001 |
| ENSG00000182362 | ybeY metallopeptidase (putative)(YBEY) | -1.079356015 | 0.20831121 | 2.20E-07 | 2.36E-06 |
| ENSG00000254221 | protocadherin gamma subfamily B, 1(PCDHGB1) | -1.079090017 | 0.439210842 | 0.0140148 | 0.037496044 |
| ENSG00000161509 | glutamate ionotropic receptor NMDA type subunit 2C(GRIN2C) | -1.078621516 | 0.369414483 | 0.003502405 | 0.011738546 |
| ENSG00000124006 | obscurin like 1(OBSL1) | -1.078550485 | 0.191028209 | 1.64E-08 | 2.22E-07 |
| ENSG00000157873 | TNF receptor superfamily member 14(TNFRSF14) | -1.07838428 | 0.399853021 | 0.006997742 | 0.020915 |
| ENSG00000163093 | Bardet-Biedl syndrome 5(BBS5) | -1.077371212 | 0.353196223 | 0.002285767 | 0.008158303 |
| ENSG00000258472 | NA | -1.076918535 | 0.258772234 | 3.16E-05 | 0.000197816 |
| ENSG00000243477 | N-acetyltransferase 6(NAT6) | -1.07647781 | 0.173806572 | 5.88E-10 | 1.04E-08 |
| ENSG00000100290 | BCL2 interacting killer(BIK) | -1.075786944 | 0.382274962 | 0.004890327 | 0.015469161 |
| ENSG00000061938 | tyrosine kinase non receptor 2(TNK2) | -1.074819105 | 0.259609114 | 3.47E-05 | 0.000214902 |
| ENSG00000134899 | ERCC excision repair 5, endonuclease(ERCC5) | -1.074786997 | 0.289808175 | 0.000208396 | 0.001036166 |
| ENSG00000171992 | synaptopodin(SYNPO) | -1.07297422 | 0.15626133 | 6.58E-12 | 1.56E-10 |
| ENSG00000141448 | GATA binding protein 6(GATA6) | -1.072038819 | 0.267747591 | 6.23E-05 | 0.000360338 |
| ENSG00000105642 | potassium calcium-activated channel subfamily N member 1(KCNN1) | -1.071347578 | 0.377553784 | 0.004545374 | 0.014585866 |
| ENSG00000131480 | amine oxidase, copper containing 2(AOC2) | -1.071151719 | 0.318560756 | 0.000772479 | 0.003197551 |
| ENSG00000104763 | N-acylsphingosine amidohydrolase 1(ASAH1) | -1.069917458 | 0.262504367 | 4.59E-05 | 0.000274802 |
| ENSG00000107872 | F-box and leucine rich repeat protein 15(FBXL15) | -1.069345623 | 0.338115785 | 0.001563342 | 0.00588894 |
| ENSG00000184451 | C-C motif chemokine receptor 10(CCR10) | -1.066834007 | 0.312867815 | 0.000649973 | 0.002764384 |
| ENSG00000182612 | tetraspanin 10(TSPAN10) | -1.065890517 | 0.247680254 | 1.68E-05 | 0.000113473 |
| ENSG00000111319 | sodium channel epithelial 1 alpha subunit(SCNN1A) | -1.065206539 | 0.230891645 | 3.96E-06 | 3.15E-05 |
| ENSG00000138468 | SUMO1/sentrin specific peptidase 7(SENP7) | -1.064071829 | 0.367889183 | 0.003823467 | 0.012606298 |
| ENSG00000158887 | myelin protein zero(MPZ) | -1.063830036 | 0.399458402 | 0.007740576 | 0.022798577 |
| ENSG00000182685 | BRICHOS domain containing 5(BRICD5) | -1.062750042 | 0.277029351 | 0.000124934 | 0.000664433 |
| ENSG00000117984 | cathepsin D(CTSD) | -1.062401025 | 0.177708853 | 2.25E-09 | 3.53E-08 |
| ENSG00000243708 | phospholipase A2 group IVB(PLA2G4B) | -1.061950652 | 0.204767149 | 2.15E-07 | 2.30E-06 |
| ENSG00000204977 | tripartite motif containing 13(TRIM13) | -1.06124557 | 0.281978094 | 0.000167502 | 0.000858976 |
| ENSG00000139496 | nucleoporin 58(NUP58) | -1.060271205 | 0.233393016 | 5.55E-06 | 4.25E-05 |
| ENSG00000249087 | ZNF436 antisense RNA 1(ZNF436-AS1) | -1.060119186 | 0.415600326 | 0.010747162 | 0.030066496 |
| ENSG00000125430 | heparan sulfate-glucosamine 3-sulfotransferase 3B1(HS3ST3B1) | -1.058111302 | 0.201351431 | 1.48E-07 | 1.63E-06 |
| ENSG00000197568 | HERV-H LTR-associating 3(HHLA3) | -1.057163533 | 0.184678454 | 1.04E-08 | 1.46E-07 |
| ENSG00000183186 | C2 calcium dependent domain containing 4C(C2CD4C) | -1.056958142 | 0.270881695 | 9.54E-05 | 0.000523882 |
| ENSG00000000971 | complement factor H(CFH) | -1.056755622 | 0.217798372 | 1.22E-06 | 1.10E-05 |
| ENSG00000176399 | DMRT like family A1(DMRTA1) | -1.055530881 | 0.395097686 | 0.007549746 | 0.022332214 |
| ENSG00000100242 | Sad1 and UNC84 domain containing 2(SUN2) | -1.054841074 | 0.211793323 | 6.34E-07 | 6.11E-06 |
| ENSG00000087842 | pirin(PIR) | -1.054669161 | 0.298921199 | 0.000418315 | 0.001890531 |
| ENSG00000100299 | arylsulfatase A(ARSA) | -1.054056146 | 0.209934112 | 5.14E-07 | 5.04E-06 |
| ENSG00000068137 | pleckstrin homology, MyTH4 and FERM domain containing H3(PLEKHH3) | -1.053612135 | 0.233084063 | 6.17E-06 | 4.64E-05 |
| ENSG00000260342 | NA | -1.053167284 | 0.258649608 | 4.67E-05 | 0.000278733 |
| | v-myc avian myelocytomatosis viral oncogene lung carcinoma derived | | | | |
| ENSG00000116990 | homolog(MYCL) | -1.052690165 | 0.308269197 | 0.000638194 | 0.002722157 |
| ENSG00000131747 | topoisomerase (DNA) II alpha(TOP2A) | -1.051505282 | 0.229060209 | 4.42E-06 | 3.48E-05 |
| ENSG00000111087 | GLI family zinc finger 1(GLI1) | -1.047796702 | 0.196922232 | 1.03E-07 | 1.18E-06 |
| ENSG00000232859 | LYR motif containing 9(LYRM9) | -1.046357888 | 0.447874034 | 0.0194768 | 0.04930602 |
| ENSG00000242612 | 2,4-dienoyl-CoA reductase 2, peroxisomal(DECR2) | -1.046357726 | 0.209595219 | 5.97E-07 | 5.79E-06 |
| ENSG00000150756 | family with sequence similarity 173 member B(FAM173B) | -1.045920098 | 0.406052726 | 0.010000172 | 0.028231827 |
| ENSG00000178773 | copine 7(CPNE7) | -1.045872709 | 0.182142169 | 9.35E-09 | 1.33E-07 |
| ENSG00000114541 | FERM domain containing 4B(FRMD4B) | -1.044519023 | 0.43139187 | 0.015466106 | 0.040684988 |
| ENSG00000118292 | chromosome 1 open reading frame 54(C1orf54) | -1.044397219 | 0.313587252 | 0.000866953 | 0.003543721 |
| ENSG00000110492 | midkine (neurite growth-promoting factor 2)(MDK) | -1.043617133 | 0.169594146 | 7.57E-10 | 1.30E-08 |
| ENSG00000050555 | laminin subunit gamma 3(LAMC3) | -1.042807325 | 0.327511571 | 0.00145239 | 0.005515142 |
| ENSG00000128271 | adenosine A2a receptor(ADORA2A) | -1.042707503 | 0.172132477 | 1.38E-09 | 2.25E-08 |
| ENSG00000173193 | poly(ADP-ribose) polymerase family member 14(PARP14) | -1.042462667 | 0.226225583 | 4.06E-06 | 3.23E-05 |
| ENSG00000107159 | carbonic anhydrase 9(CA9) | -1.04222345 | 0.261044583 | 6.54E-05 | 0.000375903 |
| ENSG00000159388 | BTG anti-proliferation factor 2(BTG2) | -1.041884164 | 0.171729704 | 1.30E-09 | 2.13E-08 |
| ENSG00000110011 | DnaJ heat shock protein family (Hsp40) member C4(DNAJC4) | -1.041227453 | 0.165305228 | 3.00E-10 | 5.57E-09 |
| ENSG00000137474 | myosin VIIA(MYO7A) | -1.040470472 | 0.373167742 | 0.005299998 | 0.016569008 |
| ENSG00000065833 | malic enzyme 1(ME1) | -1.039502368 | 0.356148251 | 0.003514549 | 0.011772537 |
| ENSG00000175745 | nuclear receptor subfamily 2 group F member 1(NR2F1) | -1.039183727 | 0.258432497 | 5.79E-05 | 0.000338186 |
| ENSG00000116005 | prenylcysteine oxidase 1(PCYOX1) | -1.038566144 | 0.194584718 | 9.43E-08 | 1.08E-06 |
| ENSG00000144369 | family with sequence similarity 171 member B(FAM171B) | -1.037736007 | 0.408093527 | 0.010994056 | 0.030626113 |
| ENSG00000170190 | solute carrier family 16 member 5(SLC16A5) | -1.0368572 | 0.37237955 | 0.005362526 | 0.016720053 |
| ENSG00000142892 | phosphatidylinositol glycan anchor biosynthesis class K(PIGK) | -1.036343678 | 0.220081965 | 2.49E-06 | 2.09E-05 |
| ENSG00000166352 | chromosome 11 open reading frame 74(C11orf74) | -1.035698031 | 0.316967232 | 0.001084927 | 0.004300229 |
| ENSG00000111678 | chromosome 12 open reading frame 57(C12orf57) | -1.035413952 | 0.201458806 | 2.75E-07 | 2.90E-06 |
| ENSG00000129636 | integrin alpha FG-GAP repeat containing 1(ITFG1) | -1.035303266 | 0.228256128 | 5.74E-06 | 4.36E-05 |
| ENSG00000137804 | nucleolar and spindle associated protein 1(NUSAP1) | -1.035286986 | 0.214181545 | 1.34E-06 | 1.20E-05 |
| ENSG00000106333 | procollagen C-endopeptidase enhancer(PCOLCE) | -1.035218177 | 0.186180405 | 2.69E-08 | 3.47E-07 |
| ENSG00000165475 | crystallin lambda 1(CRYL1) | -1.034612657 | 0.17527815 | 3.58E-09 | 5.42E-08 |
| ENSG00000178202 | KDEL motif containing 2(KDELC2) | -1.034594599 | 0.237561314 | 1.33E-05 | 9.19E-05 |
| ENSG00000111341 | matrix Gla protein(MGP) | -1.034332111 | 0.358996171 | 0.00396191 | 0.012999255 |
| ENSG00000089682 | RNA binding motif protein 41(RBM41) | -1.034170428 | 0.425613437 | 0.015105698 | 0.039924383 |
| ENSG00000196352 | CD55 molecule (Cromer blood group)(CD55) | -1.034116257 | 0.211219553 | 9.78E-07 | 9.05E-06 |
| ENSG00000227471 | aldo-keto reductase family 1 member B15(AKR1B15) | -1.033922365 | 0.273874755 | 0.000159902 | 0.000821821 |
| ENSG00000169020 | ATP synthase, H+ transporting, mitochondrial Fo complex subunit E(ATP5I) | -1.033013418 | 0.155337764 | 2.93E-11 | 6.35E-10 |
| ENSG00000067141 | neogenin 1(NEO1) | -1.032968365 | 0.171574261 | 1.74E-09 | 2.79E-08 |
| ENSG00000107281 | neural proliferation, differentiation and control 1(NPDC1) | -1.029667977 | 0.262077153 | 8.53E-05 | 0.000476731 |
| ENSG00000090674 | mucolipin 1(MCOLN1) | -1.029182945 | 0.156996942 | 5.55E-11 | 1.17E-09 |
| ENSG00000119986 | arginine vasopressin induced 1(AVPI1) | -1.029073004 | 0.165154785 | 4.64E-10 | 8.25E-09 |
| ENSG00000168056 | latent transforming growth factor beta binding protein 3(LTBP3) | -1.029049941 | 0.184068547 | 2.26E-08 | 2.97E-07 |
| ENSG00000100360 | intraflagellar transport 27(IFT27) | -1.026649963 | 0.184655261 | 2.70E-08 | 3.47E-07 |
| ENSG00000174010 | kelch like family member 15(KLHL15) | -1.026339028 | 0.258353153 | 7.11E-05 | 0.000403016 |
| ENSG00000221821 | chromosome 6 open reading frame 226(C6orf226) | -1.026086785 | 0.267381392 | 0.000124273 | 0.000661216 |
| ENSG00000213996 | transmembrane 6 superfamily member 2(TM6SF2) | -1.026038057 | 0.312497304 | 0.001025812 | 0.004092116 |
| ENSG00000096717 | sirtuin 1(SIRT1) | -1.025394218 | 0.321088965 | 0.001405645 | 0.005372322 |
| ENSG00000130303 | bone marrow stromal cell antigen 2(BST2) | -1.025389534 | 0.272953092 | 0.000172202 | 0.000879489 |
| ENSG00000075826 | SEC31 homolog B, COPII coat complex component(SEC31B) | -1.024314404 | 0.288783846 | 0.000389654 | 0.001773953 |
| ENSG00000189143 | claudin 4(CLDN4) | -1.024221558 | 0.232889656 | 1.09E-05 | 7.71E-05 |
| ENSG00000128274 | alpha 1,4-galactosyltransferase(A4GALT) | -1.022710535 | 0.214222621 | 1.81E-06 | 1.57E-05 |
| ENSG00000105472 | C-type lectin domain family 11 member A(CLEC11A) | -1.022079752 | 0.398118875 | 0.010250194 | 0.028854934 |
| ENSG00000256525 | DNA polymerase gamma 2, accessory subunit(POLG2) | -1.022073852 | 0.405873908 | 0.011795453 | 0.032390687 |
| ENSG00000141497 | zinc finger MYND-type containing 15(ZMYND15) | -1.021957913 | 0.241053277 | 2.24E-05 | 0.000145807 |
| ENSG00000127774 | ER membrane protein complex subunit 6(EMC6) | -1.019643467 | 0.360900841 | 0.004724032 | 0.01505591 |
| ENSG00000118418 | high mobility group nucleosomal binding domain 3(HMGN3) | -1.018678975 | 0.188041082 | 6.05E-08 | 7.17E-07 |
| ENSG00000164603 | base methyltransferase of 25S rRNA 2 homolog(BMT2) | -1.017747697 | 0.350923483 | 0.003729266 | 0.012361535 |
| ENSG00000110719 | T-cell immune regulator 1, ATPase H+ transporting V0 subunit a3(TCIRG1) | -1.017103345 | 0.199011199 | 3.21E-07 | 3.32E-06 |
| ENSG00000182492 | biglycan(BGN) | -1.016925075 | 0.14876387 | 8.15E-12 | 1.89E-10 |
| ENSG00000118898 | periplakin(PPL) | -1.01680963 | 0.201548621 | 4.54E-07 | 4.53E-06 |
| ENSG00000248593 | destrin, actin depolymerizing factor pseudogene 2(DSTNP2) | -1.016365823 | 0.317785453 | 0.001382515 | 0.005299417 |
| ENSG00000054282 | serologically defined colon cancer antigen 8(SDCCAG8) | -1.016219537 | 0.396799435 | 0.01043591 | 0.029279287 |
| ENSG00000183628 | DiGeorge syndrome critical region gene 6(DGCR6) | -1.015658323 | 0.236275396 | 1.72E-05 | 0.000115466 |
| ENSG00000262576 | protocadherin gamma subfamily A, 4(PCDHGA4) | -1.01543462 | 0.422392881 | 0.016216549 | 0.042390897 |
| ENSG00000162066 | amidohydrolase domain containing 2(AMDHD2) | -1.014335005 | 0.288825468 | 0.000444864 | 0.00199291 |
| | potassium voltage-gated channel subfamily A regulatory beta subunit | | | | |
| ENSG00000069424 | 2(KCNAB2) | -1.014249222 | 0.172376918 | 4.01E-09 | 6.04E-08 |
| ENSG00000163251 | frizzled class receptor 5(FZD5) | -1.013815793 | 0.212596083 | 1.85E-06 | 1.61E-05 |
| ENSG00000107485 | GATA binding protein 3(GATA3) | -1.013791405 | 0.275256466 | 0.000230433 | 0.001131393 |
| ENSG00000103056 | sphingomyelin phosphodiesterase 3(SMPD3) | -1.012967862 | 0.339883839 | 0.002879329 | 0.009955563 |
| ENSG00000137731 | FXYD domain containing ion transport regulator 2(FXYD2) | -1.012374726 | 0.372731272 | 0.006605633 | 0.019961126 |
| ENSG00000137145 | DENN domain containing 4C(DENND4C) | -1.011560546 | 0.327271744 | 0.001995584 | 0.00725017 |
| ENSG00000104808 | dihydrodiol dehydrogenase(DHDH) | -1.011488994 | 0.213744285 | 2.22E-06 | 1.89E-05 |
| ENSG00000176678 | forkhead box L1(FOXL1) | -1.010718055 | 0.177569813 | 1.26E-08 | 1.74E-07 |
| ENSG00000246922 | ubiquitin associated protein 1 like(UBAP1L) | -1.009391543 | 0.350375149 | 0.003965566 | 0.013005553 |
| ENSG00000184916 | jagged 2(JAG2) | -1.009336905 | 0.265725622 | 0.000145623 | 0.000757676 |
| ENSG00000003096 | kelch like family member 13(KLHL13) | -1.008326899 | 0.378009162 | 0.007642543 | 0.022550008 |
| ENSG00000148400 | notch 1(NOTCH1) | -1.006576702 | 0.248176279 | 4.99E-05 | 0.000295835 |
| ENSG00000166482 | microfibrillar associated protein 4(MFAP4) | -1.006440108 | 0.215487414 | 3.00E-06 | 2.47E-05 |
| ENSG00000095951 | human immunodeficiency virus type I enhancer binding protein 1(HIVEP1) | -1.006245125 | 0.235242722 | 1.89E-05 | 0.000125845 |
| ENSG00000160602 | NIMA related kinase 8(NEK8) | -1.00595539 | 0.204239607 | 8.42E-07 | 7.89E-06 |
| | SWI/SNF related, matrix associated, actin dependent regulator of chromatin, | | | | |
| ENSG00000102038 | subfamily a, member 1(SMARCA1) | -1.005525139 | 0.25360542 | 7.34E-05 | 0.000414453 |
| ENSG00000233929 | NA | -1.002507344 | 0.196372304 | 3.31E-07 | 3.41E-06 |
| ENSG00000007516 | BAI1 associated protein 3(BAIAP3) | -1.002330421 | 0.162548355 | 6.99E-10 | 1.21E-08 |
| ENSG00000196470 | siah E3 ubiquitin protein ligase 1(SIAH1) | -1.00082593 | 0.264693645 | 0.000156154 | 0.000805244 |
| ENSG00000118640 | vesicle associated membrane protein 8(VAMP8) | -1.000059034 | 0.399123933 | 0.012223161 | 0.033337556 |
| ENSG00000117335 | CD46 molecule(CD46) | -0.999024668 | 0.237055893 | 2.51E-05 | 0.000161155 |
| ENSG00000205413 | sterile alpha motif domain containing 9(SAMD9) | -0.998921838 | 0.27503546 | 0.000281261 | 0.001341748 |
| ENSG00000134363 | follistatin(FST) | -0.99881953 | 0.262449485 | 0.00014137 | 0.000738488 |
| ENSG00000197496 | solute carrier family 2 member 10(SLC2A10) | -0.99865719 | 0.198193609 | 4.68E-07 | 4.65E-06 |
| ENSG00000120616 | enhancer of polycomb homolog 1(EPC1) | -0.9983731 | 0.261973282 | 0.000138421 | 0.000724692 |
| ENSG00000142694 | eva-1 homolog B(EVA1B) | -0.998166984 | 0.287341854 | 0.000513151 | 0.002251668 |
| ENSG00000163132 | msh homeobox 1(MSX1) | -0.997550647 | 0.246652987 | 5.25E-05 | 0.000310328 |
| ENSG00000171951 | secretogranin II(SCG2) | -0.997405624 | 0.307058199 | 0.001161121 | 0.004560744 |
| ENSG00000105722 | ETS2 repressor factor(ERF) | -0.997152783 | 0.212701186 | 2.76E-06 | 2.29E-05 |
| ENSG00000186666 | BCDIN3 domain containing RNA methyltransferase(BCDIN3D) | -0.996526728 | 0.246659791 | 5.34E-05 | 0.000315547 |
| ENSG00000162437 | ribonucleoprotein, PTB binding 2(RAVER2) | -0.99569584 | 0.294576328 | 0.000724609 | 0.003028171 |
| ENSG00000253251 | trafficking protein particle complex 13(TRAPPC13) | -0.995128628 | 0.36588961 | 0.006533109 | 0.019762278 |
| | Cbp/p300 interacting transactivator with Glu/Asp rich carboxy-terminal domain | | | | |
| ENSG00000179862 | 4(CITED4) | -0.994633679 | 0.187355718 | 1.10E-07 | 1.25E-06 |
| ENSG00000173530 | TNF receptor superfamily member 10d(TNFRSF10D) | -0.993928565 | 0.158566363 | 3.65E-10 | 6.67E-09 |
| ENSG00000140199 | solute carrier family 12 member 6(SLC12A6) | -0.993833739 | 0.264281369 | 0.000169565 | 0.000868419 |
| ENSG00000137818 | ribosomal protein lateral stalk subunit P1(RPLP1) | -0.993062016 | 0.173502784 | 1.04E-08 | 1.47E-07 |
| ENSG00000132563 | receptor accessory protein 2(REEP2) | -0.992561986 | 0.16806734 | 3.51E-09 | 5.33E-08 |
| ENSG00000105996 | homeobox A2(HOXA2) | -0.992083848 | 0.293411876 | 0.000721703 | 0.003018938 |
| ENSG00000140386 | S-phase cyclin A associated protein in the ER(SCAPER) | -0.991748022 | 0.318047389 | 0.001819347 | 0.006698842 |
| ENSG00000161682 | family with sequence similarity 171 member A2(FAM171A2) | -0.990930387 | 0.261690064 | 0.000152688 | 0.000789548 |
| ENSG00000080822 | claudin domain containing 1(CLDND1) | -0.990102124 | 0.218182907 | 5.68E-06 | 4.33E-05 |
| ENSG00000132510 | lysine demethylase 6B(KDM6B) | -0.989950748 | 0.181144987 | 4.63E-08 | 5.64E-07 |
| ENSG00000123106 | coiled-coil domain containing 91(CCDC91) | -0.989273975 | 0.330884993 | 0.002791767 | 0.009682295 |
| ENSG00000162552 | Wnt family member 4(WNT4) | -0.989132131 | 0.239804174 | 3.71E-05 | 0.000227993 |
| ENSG00000110446 | solute carrier family 15 member 3(SLC15A3) | -0.988787139 | 0.308548423 | 0.001352307 | 0.005200569 |
| ENSG00000072818 | ArfGAP with coiled-coil, ankyrin repeat and PH domains 1(ACAP1) | -0.988673723 | 0.252520154 | 9.03E-05 | 0.000499302 |
| ENSG00000165804 | zinc finger protein 219(ZNF219) | -0.988637924 | 0.258542953 | 0.000131366 | 0.000692691 |
| ENSG00000114439 | BBX, HMG-box containing(BBX) | -0.987932783 | 0.251533077 | 8.58E-05 | 0.000478036 |
| ENSG00000126603 | GLIS family zinc finger 2(GLIS2) | -0.987204874 | 0.195820849 | 4.62E-07 | 4.60E-06 |
| ENSG00000166750 | schlafen family member 5(SLFN5) | -0.985939903 | 0.315684141 | 0.001789054 | 0.00661006 |
| ENSG00000126804 | zinc finger and BTB domain containing 1(ZBTB1) | -0.985099746 | 0.303434398 | 0.001168335 | 0.004584487 |
| ENSG00000176597 | UDP-GIcNAc:betaGal beta-1,3-N-acetylglucosaminyltransferase 5(B3GNT5) | -0.984991067 | 0.245246943 | 5.91E-05 | 0.000344477 |
| ENSG00000170325 | PR/SET domain 10(PRDM10) | -0.984984348 | 0.314917031 | 0.00176145 | 0.006524338 |
| ENSG00000114626 | ankyrin repeat and BTB domain containing 1(ABTB1) | -0.984315105 | 0.211678416 | 3.32E-06 | 2.70E-05 |
| ENSG00000176978 | dipeptidyl peptidase 7(DPP7) | -0.984193624 | 0.264407119 | 0.000197443 | 0.000988811 |
| ENSG00000103260 | meteorin, glial cell differentiation regulator(METRN) | -0.983738862 | 0.255336546 | 0.000116815 | 0.000625782 |
| ENSG00000166455 | chromosome 16 open reading frame 46(C16orf46) | -0.983110632 | 0.307572837 | 0.001391779 | 0.005327981 |
| ENSG00000259295 | chondroitin sulfate proteoglycan 4 pseudogene(LOC440300) | -0.982369755 | 0.269215051 | 0.000263248 | 0.001267122 |
| ENSG00000137563 | gamma-glutamyl hydrolase(GGH) | -0.982255217 | 0.244567673 | 5.91E-05 | 0.000344477 |
| ENSG00000167543 | tumor protein p53 inducible protein 13(TP53I13) | -0.981536193 | 0.219401399 | 7.69E-06 | 5.65E-05 |
| ENSG00000217801 | uncharacterized LOC100288175(LOC100288175) | -0.981017039 | 0.180769124 | 5.73E-08 | 6.83E-07 |
| ENSG00000105696 | transmembrane protein 59 like(TMEM59L) | -0.980654393 | 0.216507634 | 5.91E-06 | 4.48E-05 |
| ENSG00000187688 | transient receptor potential cation channel subfamily V member 2(TRPV2) | -0.980500904 | 0.336345003 | 0.003555015 | 0.011887775 |
| ENSG00000132026 | retbindin(RTBDN) | -0.979329438 | 0.323317983 | 0.002453665 | 0.008652553 |
| ENSG00000099219 | endoplasmic reticulum metallopeptidase 1(ERMP1) | -0.979253156 | 0.21559107 | 5.57E-06 | 4.26E-05 |
| ENSG00000133433 | glutathione S-transferase theta 2B (gene/pseudogene)(GSTT2B) | -0.979244105 | 0.282685104 | 0.000532046 | 0.002323308 |
| ENSG00000178878 | apolipoprotein L domain containing 1(APOLD1) | -0.978901227 | 0.261537003 | 0.000181924 | 0.000921687 |
| ENSG00000180998 | G protein-coupled receptor 137C(GPR137C) | -0.978793905 | 0.349443157 | 0.005094288 | 0.016023852 |
| ENSG00000105223 | phospholipase D family member 3(PLD3) | -0.978534697 | 0.173914417 | 1.84E-08 | 2.45E-07 |
| ENSG00000109738 | glycine receptor beta(GLRB) | -0.977860639 | 0.327286532 | 0.002810104 | 0.009731546 |
| ENSG00000136295 | tweety family member 3(TTYH3) | -0.976782781 | 0.215878108 | 6.05E-06 | 4.56E-05 |
| ENSG00000143382 | ADAMTS like 4(ADAMTSL4) | -0.975323003 | 0.172275684 | 1.50E-08 | 2.04E-07 |
| ENSG00000126709 | interferon alpha inducible protein 6(IFI6) | -0.974472908 | 0.187475606 | 2.02E-07 | 2.17E-06 |
| ENSG00000116209 | transmembrane protein 59(TMEM59) | -0.974378614 | 0.170369148 | 1.07E-08 | 1.50E-07 |
| ENSG00000127415 | iduronidase, alpha-L-(IDUA) | -0.973211632 | 0.246590248 | 7.92E-05 | 0.000445402 |
| ENSG00000101298 | syntaphilin(SNPH) | -0.973023698 | 0.370472276 | 0.008628287 | 0.025006938 |
| ENSG00000167157 | paired related homeobox 2(PRRX2) | -0.972838337 | 0.362654299 | 0.00730632 | 0.021704937 |
| ENSG00000111816 | fyn related Src family tyrosine kinase(FRK) | -0.972761149 | 0.334937558 | 0.003680683 | 0.012232142 |
| ENSG00000120915 | epoxide hydrolase 2(EPHX2) | -0.971393145 | 0.398411191 | 0.01476194 | 0.039156585 |
| ENSG00000139793 | muscleblind like splicing regulator 2(MBNL2) | -0.97130544 | 0.232245657 | 2.89E-05 | 0.00018257 |
| ENSG00000095209 | transmembrane protein 38B(TMEM38B) | -0.971197853 | 0.250620905 | 0.000106552 | 0.00057684 |
| ENSG00000062822 | DNA polymerase delta 1, catalytic subunit(POLD1) | -0.971127236 | 0.221798151 | 1.20E-05 | 8.37E-05 |
| ENSG00000109107 | aldolase, fructose-bisphosphate C(ALDOC) | -0.968393086 | 0.17046845 | 1.34E-08 | 1.84E-07 |
| ENSG00000100258 | lipase maturation factor 2(LMF2) | -0.967369442 | 0.227436927 | 2.11E-05 | 0.000138172 |
| ENSG00000119950 | MAX interactor 1, dimerization protein(MXI1) | -0.96667005 | 0.286320121 | 0.000734982 | 0.003066072 |
| ENSG00000143816 | Wnt family member 9A(WNT9A) | -0.96622986 | 0.229009028 | 2.45E-05 | 0.000158053 |
| ENSG00000107372 | zinc finger AN1-type containing 5(ZFAND5) | -0.965912714 | 0.200894803 | 1.52E-06 | 1.34E-05 |
| ENSG00000155111 | cyclin dependent kinase 19(CDK19) | -0.96473776 | 0.24744292 | 9.67E-05 | 0.000529373 |
| ENSG00000064989 | calcitonin receptor like receptor(CALCRL) | -0.962954411 | 0.30685662 | 0.001700324 | 0.006321937 |
| ENSG00000172428 | COP9 signalosome subunit 9(COPS9) | -0.962515348 | 0.202684586 | 2.05E-06 | 1.75E-05 |
| ENSG00000184497 | transmembrane protein 255B(TMEM255B) | -0.961228212 | 0.18923294 | 3.78E-07 | 3.85E-06 |
| ENSG00000189339 | solute carrier family 35 member E2B(SLC35E2B) | -0.961118433 | 0.340509592 | 0.004763768 | 0.015142847 |
| ENSG00000232748 | NA | -0.960934566 | 0.379908058 | 0.011426191 | 0.031546047 |
| ENSG00000105662 | CREB regulated transcription coactivator 1(CRTC1) | -0.959765229 | 0.194218862 | 7.75E-07 | 7.33E-06 |
| ENSG00000164414 | solute carrier family 35 member A1(SLC35A1) | -0.959550796 | 0.285590377 | 0.000779749 | 0.003225377 |
| ENSG00000107317 | prostaglandin D2 synthase(PTGDS) | -0.957556969 | 0.402250892 | 0.01728931 | 0.044760441 |
| ENSG00000107902 | phospholysine phosphohistidine inorganic pyrophosphate phosphatase(LHPP) | -0.957510389 | 0.170893995 | 2.11E-08 | 2.78E-07 |
| ENSG00000112659 | cullin 9(CUL9) | -0.956621717 | 0.17295337 | 3.18E-08 | 4.03E-07 |
| ENSG00000108582 | carboxypeptidase D(CPD) | -0.956107946 | 0.215231667 | 8.90E-06 | 6.42E-05 |
| ENSG00000178567 | EPM2A interacting protein 1(EPM2AIP1) | -0.955692901 | 0.263821038 | 0.000291764 | 0.001386234 |
| ENSG00000154146 | neurogranin(NRGN) | -0.95555342 | 0.186078481 | 2.82E-07 | 2.94E-06 |
| ENSG00000107821 | Kazal type serine peptidase inhibitor domain 1(KAZALD1) | -0.955001913 | 0.247292637 | 0.000112541 | 0.000606753 |
| ENSG00000066422 | zinc finger and BTB domain containing 11(ZBTB11) | -0.954891785 | 0.234363431 | 4.61E-05 | 0.000275998 |
| ENSG00000161671 | ER membrane protein complex subunit 10(EMC10) | -0.954724339 | 0.189904538 | 4.97E-07 | 4.90E-06 |
| ENSG00000204176 | synaptotagmin 15(SYT15) | -0.954460262 | 0.267570785 | 0.00036091 | 0.001664998 |
| ENSG00000133103 | component of oligomeric golgi complex 6(COG6) | -0.954371213 | 0.295127834 | 0.001221741 | 0.004759125 |
| ENSG00000141577 | centrosomal protein 131(CEP131) | -0.953421894 | 0.231550904 | 3.83E-05 | 0.000234499 |
| ENSG00000140398 | nei like DNA glycosylase 1(NEIL1) | -0.95260038 | 0.284392528 | 0.000809292 | 0.00332885 |
| ENSG00000115365 | LanC like 1(LANCL1) | -0.952031195 | 0.235805694 | 5.41E-05 | 0.000318755 |
| ENSG00000011028 | mannose receptor C type 2(MRC2) | -0.951541267 | 0.194533551 | 1.00E-06 | 9.24E-06 |
| ENSG00000169992 | neuroligin 2(NLGN2) | -0.951440271 | 0.162213575 | 4.48E-09 | 6.68E-08 |
| ENSG00000219545 | UBAP1-MVB12-associated (UMA) domain containing 1(UMAD1) | -0.95124537 | 0.301503849 | 0.001604968 | 0.006028366 |
| ENSG00000198026 | zinc finger protein 335(ZNF335) | -0.950294927 | 0.194557066 | 1.04E-06 | 9.54E-06 |
| ENSG00000116285 | ERBB receptor feedback inhibitor 1(ERRFI1) | -0.950143127 | 0.190377578 | 6.01E-07 | 5.82E-06 |
| ENSG00000271447 | matrix metallopeptidase 28(MMP28) | -0.950022009 | 0.30165975 | 0.001636529 | 0.006125398 |
| ENSG00000118513 | MYB proto-oncogene, transcription factor(MYB) | -0.949829942 | 0.332255831 | 0.004253397 | 0.013830006 |
| ENSG00000105270 | CAP-Gly domain containing linker protein 3(CLIP3) | -0.949492916 | 0.155642447 | 1.06E-09 | 1.76E-08 |
| ENSG00000213903 | leukotriene B4 receptor(LTB4R) | -0.949470755 | 0.212396057 | 7.81E-06 | 5.72E-05 |
| ENSG00000131171 | SH3 domain binding glutamate rich protein like(SH3BGRL) | -0.949197812 | 0.189142143 | 5.21E-07 | 5.10E-06 |
| ENSG00000027697 | interferon gamma receptor 1(IFNGR1) | -0.948321742 | 0.258855678 | 0.000248777 | 0.001203379 |
| ENSG00000124762 | cyclin dependent kinase inhibitor 1A(CDKN1A) | -0.948183656 | 0.15062976 | 3.08E-10 | 5.70E-09 |
| ENSG00000107537 | phytanoyl-CoA 2-hydroxylase(PHYH) | -0.947936686 | 0.26943153 | 0.000434347 | 0.001950996 |
| ENSG00000242960 | NA | -0.9468074 | 0.330342988 | 0.004155178 | 0.013544338 |
| ENSG00000170264 | family with sequence similarity 161 member A(FAM161A) | -0.946730223 | 0.337765767 | 0.005064232 | 0.015946367 |
| ENSG00000175482 | DNA polymerase delta 4, accessory subunit(POLD4) | -0.945968538 | 0.157719622 | 2.00E-09 | 3.18E-08 |
| ENSG00000174007 | centrosomal protein 19(CEP19) | -0.945615346 | 0.334737113 | 0.004728811 | 0.015063561 |
| ENSG00000143409 | family with sequence similarity 63 member A(FAM63A) | -0.945069889 | 0.214203237 | 1.02E-05 | 7.29E-05 |
| ENSG00000168564 | CDKN2A interacting protein(CDKN2AIP) | -0.944944629 | 0.330844204 | 0.004287963 | 0.013923158 |
| ENSG00000153721 | CNKSR family member 3(CNKSR3) | -0.944595555 | 0.185647271 | 3.62E-07 | 3.69E-06 |
| ENSG00000254531 | uncharacterized LOC90024(FU20021) | -0.94459094 | 0.261064183 | 0.000296622 | 0.001404209 |
| ENSG00000234745 | major histocompatibility complex, class I, B(HLA-B) | -0.94405569 | 0.257764295 | 0.000249789 | 0.001206782 |
| ENSG00000147853 | adenylate kinase 3(AK3) | -0.943200025 | 0.2372533 | 7.02E-05 | 0.000398558 |
| ENSG00000133250 | zinc finger protein 414(ZNF414) | -0.943114914 | 0.28009945 | 0.00075971 | 0.003153572 |
| ENSG00000137868 | stimulated by retinoic acid 6(STRA6) | -0.9422994 | 0.389260794 | 0.015488926 | 0.040735911 |
| ENSG00000197121 | post-GPI attachment to proteins 1(PGAP1) | -0.94204851 | 0.280847321 | 0.000795637 | 0.003282703 |
| ENSG00000162004 | coiled-coil domain containing 78(CCDC78) | -0.941884972 | 0.264569899 | 0.000370769 | 0.001699152 |
| ENSG00000085871 | microsomal glutathione S-transferase 2(MGST2) | -0.941510159 | 0.204629791 | 4.20E-06 | 3.33E-05 |
| ENSG00000149131 | serpin family G member 1(SERPING1) | -0.941110636 | 0.356077879 | 0.008217722 | 0.023988406 |
| ENSG00000059145 | unkempt family like zinc-finger(UNKL) | -0.940832974 | 0.303113214 | 0.001909882 | 0.006984099 |
| ENSG00000102471 | Nedd4 family interacting protein 2(NDFIP2) | -0.939152889 | 0.227501262 | 3.66E-05 | 0.000225264 |
| ENSG00000137404 | nurim (nuclear envelope membrane protein)(NRM) | -0.937831263 | 0.200207542 | 2.81E-06 | 2.33E-05 |
| ENSG00000180776 | zinc finger DHHC-type containing 20(ZDHHC20) | -0.937737894 | 0.330057619 | 0.004495343 | 0.014468732 |
| ENSG00000107854 | tankyrase 2(TNKS2) | -0.937718319 | 0.276597863 | 0.000698451 | 0.00293659 |
| ENSG00000176809 | leucine rich repeat containing 37 member A3(LRRC37A3) | -0.937208392 | 0.281776544 | 0.000880799 | 0.003592831 |
| ENSG00000097046 | cell division cycle 7(CDC7) | -0.936888166 | 0.268807374 | 0.000491491 | 0.00217283 |
| ENSG00000006118 | transmembrane protein 132A(TMEM132A) | -0.935732842 | 0.201578878 | 3.45E-06 | 2.79E-05 |
| ENSG00000129003 | vacuolar protein sorting 13 homolog C(VPS13C) | -0.93549123 | 0.268710655 | 0.000498786 | 0.002197649 |
| ENSG00000179094 | microRNA 6883(MIR6883) | -0.93541657 | 0.173864364 | 7.44E-08 | 8.70E-07 |
| ENSG00000139597 | NEDD4 binding protein 2 like 1(N4BP2L1) | -0.935271412 | 0.251451569 | 0.000199626 | 0.000996774 |
| ENSG00000132182 | nucleoporin 210(NUP210) | -0.935135782 | 0.162767355 | 9.18E-09 | 1.31E-07 |
| ENSG00000164830 | oxidation resistance 1(OXR1) | -0.934760078 | 0.247880442 | 0.000162587 | 0.000835228 |
| ENSG00000239887 | chromosome 1 open reading frame 226(C1orf226) | -0.934578827 | 0.185197852 | 4.50E-07 | 4.50E-06 |
| ENSG00000008311 | aminoadipate-semialdehyde synthase(AASS) | -0.933636531 | 0.20628993 | 6.02E-06 | 4.54E-05 |
| ENSG00000154589 | lymphocyte antigen 96(LY96) | -0.932610293 | 0.366593132 | 0.010959334 | 0.030548038 |
| ENSG00000111897 | serine incorporator 1(SERINC1) | -0.931906927 | 0.216592637 | 1.69E-05 | 0.000113877 |
| ENSG00000165406 | membrane associated ring-CH-type finger 8(MARCH8) | -0.93102948 | 0.398539385 | 0.019485818 | 0.049318237 |
| ENSG00000071967 | cytochrome b reductase 1(CYBRD1) | -0.930805769 | 0.207759166 | 7.46E-06 | 5.50E-05 |
| ENSG00000177666 | patatin like phospholipase domain containing 2(PNPLA2) | -0.929446422 | 0.180395849 | 2.57E-07 | 2.72E-06 |
| ENSG00000123636 | bromodomain adjacent to zinc finger domain 2B(BAZ2B) | -0.928990075 | 0.28714626 | 0.001215361 | 0.00473741 |
| ENSG00000220785 | NA | -0.928802877 | 0.335366238 | 0.005613928 | 0.017397853 |
| ENSG00000161513 | ferredoxin reductase(FDXR) | -0.928461806 | 0.191180678 | 1.20E-06 | 1.08E-05 |
| ENSG00000156787 | TBC1 domain family member 31(TBC1D31) | -0.928450115 | 0.248675108 | 0.000188772 | 0.000953096 |
| ENSG00000105701 | FK506 binding protein 8(FKBP8) | -0.927435242 | 0.216828515 | 1.89E-05 | 0.000125887 |
| ENSG00000239697 | tumor necrosis factor superfamily member 12(TNFSF12) | -0.92721889 | 0.329697518 | 0.00491837 | 0.015537823 |
| ENSG00000163346 | PBX homeobox interacting protein 1(PBXIP1) | -0.927024589 | 0.181812258 | 3.42E-07 | 3.51E-06 |
| ENSG00000105519 | calcyphosine(CAPS) | -0.926669337 | 0.307274058 | 0.002563236 | 0.008985075 |
| ENSG00000099864 | paralemmin(PALM) | -0.926619093 | 0.201914974 | 4.45E-06 | 3.50E-05 |
| ENSG00000179588 | zinc finger protein, FOG family member 1(ZFPM1) | -0.925885032 | 0.289107746 | 0.001362117 | 0.005234873 |
| ENSG00000151006 | protease, serine 53(PRSS53) | -0.923944969 | 0.305207404 | 0.002467741 | 0.008699582 |
| ENSG00000125652 | alkB homolog 7(ALKBH7) | -0.923709234 | 0.211303208 | 1.23E-05 | 8.59E-05 |
| ENSG00000244242 | interferon induced transmembrane protein 10(IFITM10) | -0.923640344 | 0.377026643 | 0.014293508 | 0.038059944 |
| ENSG00000163040 | coiled-coil domain containing 74A(CCDC74A) | -0.923249548 | 0.332597871 | 0.005505366 | 0.017120043 |
| ENSG00000104671 | dynactin subunit 6(DCTN6) | -0.921867514 | 0.304915543 | 0.002499899 | 0.008793815 |
| ENSG00000206560 | ankyrin repeat domain 28(ANKRD28) | -0.921156708 | 0.274624785 | 0.000795841 | 0.003282703 |
| ENSG00000172037 | laminin subunit beta 2(LAMB2) | -0.921033412 | 0.184888727 | 6.31E-07 | 6.09E-06 |
| ENSG00000131196 | nuclear factor of activated T-cells 1(NFATC1) | -0.92067862 | 0.184880356 | 6.36E-07 | 6.13E-06 |
| ENSG00000173846 | polo like kinase 3(PLK3) | -0.920601806 | 0.15812409 | 5.81E-09 | 8.54E-08 |
| ENSG00000105717 | PBX homeobox 4(PBX4) | -0.920354563 | 0.225055528 | 4.32E-05 | 0.000261348 |
| ENSG00000177700 | RNA polymerase II subunit L(POLR2L) | -0.919933809 | 0.222949617 | 3.69E-05 | 0.00022692 |
| ENSG00000187325 | TATA-box binding protein associated factor 9b(TAF9B) | -0.919819356 | 0.277338524 | 0.000911218 | 0.003705151 |
| ENSG00000168264 | interferon regulatory factor 2 binding protein 2(IRF2BP2) | -0.919502576 | 0.14727035 | 4.28E-10 | 7.68E-09 |
| ENSG00000120539 | microtubule associated serine/threonine kinase like(MASTL) | -0.919370509 | 0.264058621 | 0.000498258 | 0.002196968 |
| ENSG00000053524 | MCF.2 cell line derived transforming sequence-like 2(MCF2L2) | -0.918138136 | 0.275343511 | 0.000854471 | 0.003503659 |
| ENSG00000095752 | interleukin 11(IL11) | -0.917892215 | 0.161038388 | 1.20E-08 | 1.67E-07 |
| ENSG00000095539 | semaphorin 4G(SEMA4G) | -0.917869571 | 0.236530221 | 0.000104216 | 0.00056576 |
| ENSG00000005075 | RNA polymerase II subunit J(POLR2J) | -0.917010612 | 0.159933472 | 9.83E-09 | 1.39E-07 |
| ENSG00000134070 | interleukin 1 receptor associated kinase 2(IRAK2) | -0.91640372 | 0.149926724 | 9.82E-10 | 1.64E-08 |
| ENSG00000169100 | solute carrier family 25 member 6(SLC25A6) | -0.915479655 | 0.200184963 | 4.80E-06 | 3.73E-05 |
| ENSG00000123219 | centromere protein K(CENPK) | -0.914844026 | 0.218534859 | 2.84E-05 | 0.000179748 |
| ENSG00000105404 | Rab acceptor 1(RABAC1) | -0.914285457 | 0.195483357 | 2.91E-06 | 2.40E-05 |
| ENSG00000101665 | SMAD family member 7(SMAD7) | -0.913408847 | 0.201301167 | 5.69E-06 | 4.33E-05 |
| ENSG00000065361 | erb-b2 receptor tyrosine kinase 3(ERBB3) | -0.912689724 | 0.222793757 | 4.19E-05 | 0.000254415 |
| ENSG00000169193 | coiled-coil domain containing 126(CCDC126) | -0.91162663 | 0.265840159 | 0.0006053 | 0.002596833 |
| ENSG00000138764 | cyclin G2(CCNG2) | -0.910213603 | 0.21168941 | 1.71E-05 | 0.00011494 |
| ENSG00000251196 | NA | -0.910190543 | 0.332819162 | 0.006242007 | 0.019013716 |
| ENSG00000268218 | NA | -0.909978199 | 0.389005727 | 0.019322957 | 0.049023092 |
| ENSG00000181467 | RAP2B, member of RAS oncogene family(RAP2B) | -0.908776252 | 0.169739408 | 8.61E-08 | 9.96E-07 |
| ENSG00000163703 | cysteine rich with EGF like domains 1(CRELD1) | -0.90821978 | 0.161595282 | 1.91E-08 | 2.53E-07 |
| ENSG00000130844 | zinc finger protein 331(ZNF331) | -0.908019924 | 0.275840304 | 0.000995371 | 0.003981483 |
| ENSG00000155066 | prominin 2(PROM2) | -0.907882806 | 0.318935838 | 0.004418883 | 0.014265535 |
| ENSG00000112297 | absent in melanoma 1(AIM1) | -0.906677225 | 0.347358445 | 0.009048762 | 0.025989767 |
| ENSG00000188613 | nanos C2HC-type zinc finger 1(NANOS1) | -0.906295643 | 0.262752804 | 0.000562181 | 0.002432329 |
| ENSG00000161618 | aldehyde dehydrogenase 16 family member A1(ALDH16A1) | -0.906196983 | 0.241932547 | 0.00017992 | 0.000912714 |
| ENSG00000253953 | protocadherin gamma subfamily B, 4(PCDHGB4) | -0.906134096 | 0.187826569 | 1.40E-06 | 1.25E-05 |
| ENSG00000185633 | NDUFA4, mitochondrial complex associated like 2(NDUFA4L2) | -0.906025922 | 0.327551928 | 0.005673935 | 0.017560687 |
| ENSG00000178053 | myeloid leukemia factor 1(MLF1) | -0.905185286 | 0.279388903 | 0.001195819 | 0.004675178 |
| ENSG00000134020 | phosphatidylethanolamine binding protein 4(PEBP4) | -0.904621125 | 0.345930929 | 0.008921859 | 0.025712089 |
| ENSG00000154721 | junctional adhesion molecule 2(JAM2) | -0.901904772 | 0.326614011 | 0.0057558 | 0.017747275 |
| ENSG00000101017 | CD40 molecule(CD40) | -0.900848522 | 0.174242877 | 2.34E-07 | 2.50E-06 |
| ENSG00000117650 | NIMA related kinase 2(NEK2) | -0.900537004 | 0.268738586 | 0.000805268 | 0.003316937 |
| ENSG00000213999 | myocyte enhancer factor 2B(MEF2B) | -0.900247983 | 0.251824746 | 0.000350363 | 0.001623331 |
| ENSG00000133687 | transmembrane and tetratricopeptide repeat containing 1(TMTC1) | -0.89989881 | 0.290948505 | 0.001981554 | 0.007210332 |
| ENSG00000232671 | uncharacterized LOC100507670(LOC100507670) | -0.898965766 | 0.313066437 | 0.004085474 | 0.013357842 |
| ENSG00000260456 | chromosome 16 open reading frame 95(C16orf95) | -0.897871141 | 0.249560053 | 0.000320901 | 0.001501023 |
| ENSG00000135999 | enhancer of polycomb homolog 2(EPC2) | -0.89718763 | 0.258537951 | 0.000520002 | 0.002276629 |
| ENSG00000137699 | tripartite motif containing 29(TRIM29) | -0.897086316 | 0.160988712 | 2.51E-08 | 3.25E-07 |
| ENSG00000161677 | Josephin domain containing 2(JOSD2) | -0.896686954 | 0.242093416 | 0.00021232 | 0.00105434 |
| ENSG00000137103 | transmembrane protein 8B(TMEM8B) | -0.896022452 | 0.169794836 | 1.31E-07 | 1.46E-06 |
| ENSG00000121905 | hippocalcin(HPCA) | -0.894990912 | 0.224978468 | 6.95E-05 | 0.000395146 |
| ENSG00000105711 | sodium voltage-gated channel beta subunit 1(SCN1B) | -0.893207721 | 0.235524546 | 0.000149187 | 0.000773575 |
| ENSG00000162745 | olfactomedin like 2B(OLFML2B) | -0.892798994 | 0.250094801 | 0.000357196 | 0.001652401 |
| ENSG00000154734 | ADAM metallopeptidase with thrombospondin type 1 motif 1(ADAMTS1) | -0.892236627 | 0.211967438 | 2.56E-05 | 0.000164665 |
| ENSG00000213066 | FGFR1 oncogene partner(FGFR1OP) | -0.889438919 | 0.257375079 | 0.000548645 | 0.002386932 |
| ENSG00000105808 | RAS p21 protein activator 4(RASA4) | -0.889361429 | 0.183293657 | 1.22E-06 | 1.10E-05 |
| ENSG00000177106 | EPS8 like 2(EPS8L2) | -0.888748176 | 0.190422691 | 3.05E-06 | 2.50E-05 |
| ENSG00000162976 | PQ loop repeat containing 3(PQLC3) | -0.888608054 | 0.254020099 | 0.000468445 | 0.002083472 |
| ENSG00000124593 | prickle planar cell polarity protein 4(PRICKLE4) | -0.88826341 | 0.251591694 | 0.000414657 | 0.001875442 |
| ENSG00000248712 | coiled-coil domain containing 153(CCDC153) | -0.888161132 | 0.253881037 | 0.000468171 | 0.002083043 |
| ENSG00000160685 | zinc finger and BTB domain containing 7B(ZBTB7B) | -0.888141652 | 0.189582276 | 2.80E-06 | 2.32E-05 |
| ENSG00000008710 | microRNA 6511b-1(MIR6511B1) | -0.885436225 | 0.26026624 | 0.000668848 | 0.002834075 |
| ENSG00000129007 | calmodulin like 4(CALML4) | -0.88516308 | 0.199152589 | 8.80E-06 | 6.37E-05 |
| ENSG00000196628 | transcription factor 4(TCF4) | -0.885106734 | 0.370533674 | 0.016906495 | 0.043933732 |
| ENSG00000099282 | tetraspanin 15(TSPAN15) | -0.885011277 | 0.159647172 | 2.96E-08 | 3.77E-07 |
| ENSG00000198270 | transmembrane protein 116(TMEM116) | -0.884676399 | 0.231005418 | 0.000128311 | 0.000679933 |
| ENSG00000169733 | RFNG O-fucosylpeptide 3-beta-N-acetylglucosaminyltransferase(RFNG) | -0.884253806 | 0.201947456 | 1.19E-05 | 8.36E-05 |
| ENSG00000153936 | heparan sulfate 2-O-sulfotransferase 1(HS2ST1) | -0.884103344 | 0.245101411 | 0.000309648 | 0.001456455 |
| ENSG00000104765 | BCL2 interacting protein 3 like(BNIP3L) | -0.883112141 | 0.206617897 | 1.92E-05 | 0.000127388 |
| ENSG00000261052 | sulfotransferase family 1A member 3(SULT1A3) | -0.882484497 | 0.259051336 | 0.000657772 | 0.002796543 |
| ENSG00000099385 | BCL tumor suppressor 7C(BCL7C) | -0.882437532 | 0.198605216 | 8.86E-06 | 6.40E-05 |
| ENSG00000105612 | deoxyribonuclease 2, lysosomal(DNASE2) | -0.882139837 | 0.168603805 | 1.68E-07 | 1.83E-06 |
| ENSG00000239332 | long intergenic non-protein coding RNA 1119(LINC01119) | -0.882071184 | 0.290032747 | 0.002355736 | 0.008363091 |
| ENSG00000198353 | homeobox C4(HOXC4) | -0.881884159 | 0.301753627 | 0.003471997 | 0.01165323 |
| ENSG00000115524 | splicing factor 3b subunit 1(SF3B1) | -0.881403335 | 0.234989296 | 0.000176255 | 0.000897603 |
| ENSG00000157637 | solute carrier family 38 member 10(SLC38A10) | -0.881185712 | 0.199384201 | 9.89E-06 | 7.07E-05 |
| ENSG00000263513 | family with sequence similarity 72 member C(FAM72C) | -0.880783761 | 0.31429292 | 0.00507193 | 0.015966331 |
| ENSG00000143303 | ribosomal RNA adenine dimethylase domain containing 1(RRNAD1) | -0.880463002 | 0.166293588 | 1.19E-07 | 1.34E-06 |
| ENSG00000122484 | RNA polymerase II associated protein 2(RPAP2) | -0.880322368 | 0.254786779 | 0.00055002 | 0.002391462 |
| ENSG00000268193 | NA | -0.8795612 | 0.329651725 | 0.007626954 | 0.022509657 |
| ENSG00000197774 | essential meiotic structure-specific endonuclease subunit 2(EME2) | -0.879416869 | 0.207189724 | 2.19E-05 | 0.000143023 |
| ENSG00000070269 | transmembrane protein 260(TMEM260) | -0.878891359 | 0.290437651 | 0.00247736 | 0.008728262 |
| ENSG00000010818 | human immunodeficiency virus type I enhancer binding protein 2(HIVEP2) | -0.878886413 | 0.2144666 | 4.17E-05 | 0.000253215 |
| ENSG00000103479 | RB transcriptional corepressor like 2(RBL2) | -0.878123612 | 0.239475063 | 0.000245539 | 0.001191633 |
| ENSG00000103145 | host cell factor C1 regulator 1(HCFC1R1) | -0.877567133 | 0.163400657 | 7.85E-08 | 9.15E-07 |
| ENSG00000130304 | solute carrier family 27 member 1(SLC27A1) | -0.877504239 | 0.212514871 | 3.64E-05 | 0.000224384 |
| ENSG00000163539 | cytoplasmic linker associated protein 2(CLASP2) | -0.876594108 | 0.249007041 | 0.000430964 | 0.001938018 |
| ENSG00000104164 | biogenesis of lysosomal organelles complex 1 subunit 6(BLOC1S6) | -0.876292577 | 0.234462849 | 0.000185898 | 0.000940199 |
| ENSG00000000003 | tetraspanin 6(TSPAN6) | -0.876286987 | 0.173009382 | 4.08E-07 | 4.12E-06 |
| ENSG00000172270 | basigin (Ok blood group)(BSG) | -0.875432071 | 0.191309321 | 4.74E-06 | 3.69E-05 |
| ENSG00000204764 | RAN binding protein 17(RANBP17) | -0.874778285 | 0.328095299 | 0.007670667 | 0.022627313 |
| ENSG00000183354 | KIAA2026(KIAA2026) | -0.87472373 | 0.298698689 | 0.003406582 | 0.011482818 |
| ENSG00000167074 | TEF, PAR bZIP transcription factor(TEF) | -0.874036424 | 0.153562097 | 1.26E-08 | 1.74E-07 |
| ENSG00000090013 | biliverdin reductase B(BLVRB) | -0.872536897 | 0.187797081 | 3.38E-06 | 2.74E-05 |
| ENSG00000145348 | TBC1 domain containing kinase(TBCK) | -0.871345077 | 0.306964247 | 0.00453136 | 0.014548831 |
| ENSG00000188015 | S100 calcium binding protein A3(S100A3) | -0.871301696 | 0.183536402 | 2.06E-06 | 1.76E-05 |
| ENSG00000176623 | regulator of microtubule dynamics 1(RMDN1) | -0.871140469 | 0.266656876 | 0.001087333 | 0.004308316 |
| ENSG00000186832 | keratin 16(KRT16) | -0.869620654 | 0.237258687 | 0.000247064 | 0.001197057 |
| ENSG00000165795 | microRNA 6717(MIR6717) | -0.869520446 | 0.257106074 | 0.000719727 | 0.003013119 |
| ENSG00000258311 | NA | -0.869397188 | 0.217998377 | 6.66E-05 | 0.00038167 |
| ENSG00000204822 | mitochondrial ribosomal protein L53(MRPL53) | -0.869316805 | 0.18280152 | 1.98E-06 | 1.70E-05 |
| ENSG00000131015 | UL16 binding protein 2(ULBP2) | -0.869094967 | 0.190573932 | 5.11E-06 | 3.94E-05 |
| ENSG00000196296 | ATPase sarcoplasmic/endoplasmic reticulum Ca2+ transporting 1(ATP2A1) | -0.869060723 | 0.23948857 | 0.000284721 | 0.001355505 |
| ENSG00000181045 | solute carrier family 26 member 11(SLC26A11) | -0.868556326 | 0.191632868 | 5.83E-06 | 4.42E-05 |
| ENSG00000268798 | NA | -0.868513469 | 0.261364983 | 0.000890578 | 0.003627687 |
| ENSG00000154040 | calcium binding tyrosine phosphorylation regulated(CABYR) | -0.868356741 | 0.290273148 | 0.002775961 | 0.009638844 |
| ENSG00000065243 | protein kinase N2(PKN2) | -0.868175462 | 0.233158421 | 0.000196446 | 0.000985498 |
| ENSG00000123364 | homeobox C13(HOXC13) | -0.86770255 | 0.169680928 | 3.16E-07 | 3.28E-06 |
| ENSG00000125995 | reactive oxygen species modulator 1(ROMO1) | -0.867643724 | 0.228785492 | 0.000149205 | 0.000773575 |
| ENSG00000176531 | pleckstrin homology like domain family B member 3(PHLDB3) | -0.867486804 | 0.236739974 | 0.000248014 | 0.001200179 |
| ENSG00000170442 | keratin 86(KRT86) | -0.867283489 | 0.341325153 | 0.01105579 | 0.03076894 |
| ENSG00000250571 | GLI family zinc finger 4(GLI4) | -0.867190717 | 0.225892816 | 0.000123563 | 0.000657734 |
| ENSG00000144476 | atypical chemokine receptor 3(ACKR3) | -0.86706359 | 0.168825462 | 2.81E-07 | 2.94E-06 |
| ENSG00000178607 | endoplasmic reticulum to nucleus signaling 1(ERN1) | -0.864750808 | 0.179373645 | 1.43E-06 | 1.27E-05 |
| ENSG00000170634 | acylphosphatase 2(ACYP2) | -0.864683685 | 0.224088535 | 0.000114006 | 0.00061301 |
| ENSG00000233927 | ribosomal protein S28(RPS28) | -0.864432834 | 0.17317681 | 5.99E-07 | 5.80E-06 |
| ENSG00000113391 | microRNA 2277(MIR2277) | -0.863661444 | 0.315366396 | 0.006170197 | 0.018814463 |
| ENSG00000162194 | LBH domain containing 1(LBHD1) | -0.863263083 | 0.242318612 | 0.000367322 | 0.001687559 |
| ENSG00000166575 | transmembrane protein 135(TMEM135) | -0.862227428 | 0.312162485 | 0.005742895 | 0.017727478 |
| ENSG00000130164 | microRNA 6886(MIR6886) | -0.860665656 | 0.155818707 | 3.32E-08 | 4.17E-07 |
| ENSG00000203666 | EF-hand calcium binding domain 2(EFCAB2) | -0.860132149 | 0.188700667 | 5.16E-06 | 3.97E-05 |
| ENSG00000039319 | zinc finger FYVE-type containing 16(ZFYVE16) | -0.860110643 | 0.240273297 | 0.000343965 | 0.001596846 |
| ENSG00000127914 | A-kinase anchoring protein 9(AKAP9) | -0.860064152 | 0.235004158 | 0.000252429 | 0.001218538 |
| ENSG00000156535 | CD109 molecule(CD109) | -0.859530022 | 0.212997678 | 5.45E-05 | 0.00032079 |
| ENSG00000102003 | synaptophysin(SYP) | -0.859383876 | 0.348786425 | 0.013742455 | 0.036859609 |
| ENSG00000106330 | motile sperm domain containing 3(MOSPD3) | -0.857674789 | 0.178404517 | 1.53E-06 | 1.35E-05 |
| ENSG00000167733 | hydroxysteroid 11-beta dehydrogenase 1 like(HSD11B1L) | -0.857565713 | 0.209791048 | 4.36E-05 | 0.000262892 |
| ENSG00000143013 | LIM domain only 4(LMO4) | -0.856187493 | 0.176796107 | 1.28E-06 | 1.15E-05 |
| ENSG00000127540 | ubiquinol-cytochrome c reductase, complex III subunit XI(UQCR11) | -0.855927565 | 0.179815079 | 1.94E-06 | 1.67E-05 |
| ENSG00000183044 | 4-aminobutyrate aminotransferase(ABAT) | -0.855108983 | 0.188452835 | 5.69E-06 | 4.33E-05 |
| ENSG00000110031 | leupaxin(LPXN) | -0.854916541 | 0.293860159 | 0.003622817 | 0.012076742 |
| ENSG00000144746 | ADP ribosylation factor like GTPase 6 interacting protein 5(ARL6IP5) | -0.854530219 | 0.199789635 | 1.89E-05 | 0.000125887 |
| ENSG00000105520 | phospholipid phosphatase related 2(PLPPR2) | -0.854356306 | 0.155008135 | 3.55E-08 | 4.42E-07 |
| ENSG00000129946 | SHC adaptor protein 2(SHC2) | -0.853544215 | 0.253841324 | 0.000772371 | 0.003197551 |
| ENSG00000188419 | CHM, Rab escort protein 1(CHM) | -0.853300672 | 0.330804956 | 0.009895274 | 0.027982692 |
| ENSG00000133935 | chromosome 14 open reading frame 1(C14orf1) | -0.85311354 | 0.166010996 | 2.76E-07 | 2.91E-06 |
| ENSG00000162174 | asparaginase like 1(ASRGL1) | -0.852535983 | 0.211401017 | 5.51E-05 | 0.00032356 |
| ENSG00000064655 | EYA transcriptional coactivator and phosphatase 2(EYA2) | -0.852482611 | 0.258510577 | 0.000974906 | 0.0039156 |
| ENSG00000189057 | family with sequence similarity 111 member B(FAM111B) | -0.852072685 | 0.240780254 | 0.000401953 | 0.001821486 |
| ENSG00000113580 | nuclear receptor subfamily 3 group C member 1(NR3C1) | -0.851931343 | 0.236860296 | 0.000322197 | 0.001506487 |
| ENSG00000153029 | major histocompatibility complex, class I-related(MR1) | -0.851721966 | 0.209127887 | 4.65E-05 | 0.00027775 |
| ENSG00000148339 | solute carrier family 25 member 25(SLC25A25) | -0.85171855 | 0.205627398 | 3.44E-05 | 0.000213293 |
| ENSG00000183779 | zinc finger protein 703(ZNF703) | -0.851284012 | 0.20815601 | 4.32E-05 | 0.000261348 |
| ENSG00000116001 | TIA1 cytotoxic granule associated RNA binding protein(TIA1) | -0.850609902 | 0.246225569 | 0.000551118 | 0.002395031 |
| ENSG00000110628 | solute carrier family 22 member 18(SLC22A18) | -0.850549868 | 0.228143195 | 0.000192898 | 0.000970595 |
| ENSG00000059915 | pleckstrin and Sec7 domain containing(PSD) | -0.850164397 | 0.264305386 | 0.001297198 | 0.005009928 |
| ENSG00000226564 | NA | -0.849653642 | 0.304324479 | 0.005239416 | 0.016410951 |
| ENSG00000188042 | ADP ribosylation factor like GTPase 4C(ARL4C) | -0.849637587 | 0.225839951 | 0.000168477 | 0.000863603 |
| ENSG00000117906 | reticulocalbin 2(RCN2) | -0.849457561 | 0.208922394 | 4.78E-05 | 0.000285095 |
| ENSG00000154889 | metallophosphoesterase 1(MPPE1) | -0.848606854 | 0.190250497 | 8.18E-06 | 5.96E-05 |
| ENSG00000212123 | proline rich 22(PRR22) | -0.848248516 | 0.309444979 | 0.006121647 | 0.018695499 |
| ENSG00000172915 | neurobeachin(NBEA) | -0.848244773 | 0.360877959 | 0.01874805 | 0.047894041 |
| ENSG00000135365 | PHD finger protein 21A(PHF21A) | -0.847918998 | 0.237552333 | 0.000357801 | 0.001654551 |
| ENSG00000245958 | septin 7 pseudogene(LOC645513) | -0.847769794 | 0.252010617 | 0.000768148 | 0.003182986 |
| ENSG00000030582 | granulin precursor(GRN) | -0.84728715 | 0.180311027 | 2.61E-06 | 2.19E-05 |
| ENSG00000114933 | INO80 complex subunit D(INO80D) | -0.847044471 | 0.289655997 | 0.003452187 | 0.011606609 |
| ENSG00000160255 | integrin subunit beta 2(ITGB2) | -0.846385865 | 0.235859433 | 0.000332563 | 0.0015488 |
| ENSG00000158201 | abhydrolase domain containing 3(ABHD3) | -0.845866113 | 0.250172686 | 0.000721887 | 0.003018938 |
| ENSG00000164070 | heat shock protein family A (Hsp70) member 4 like(HSPA4L) | -0.845337527 | 0.242996995 | 0.000503668 | 0.002218325 |
| ENSG00000196776 | CD47 molecule(CD47) | -0.844578592 | 0.198467393 | 2.09E-05 | 0.000136927 |
| ENSG00000101104 | poly(A) binding protein cytoplasmic 1 like(PABPCLL) | -0.844445827 | 0.183565328 | 4.22E-06 | 3.33E-05 |
| ENSG00000168918 | inositol polyphosphate-5-phosphatase D(INPP5D) | -0.84337308 | 0.328954849 | 0.010353442 | 0.029096486 |
| ENSG00000095739 | BMP and activin membrane bound inhibitor(BAMBI) | -0.84259946 | 0.166783173 | 4.37E-07 | 4.38E-06 |
| ENSG00000102158 | magnesium transporter 1(MAGT1) | -0.8423176 | 0.210039864 | 6.06E-05 | 0.00035146 |
| ENSG00000071889 | family with sequence similarity 3 member A(FAM3A) | -0.842299189 | 0.186010712 | 5.95E-06 | 4.50E-05 |
| ENSG00000176124 | deleted in lymphocytic leukemia 1(DLEU1) | -0.841575688 | 0.261198678 | 0.001273101 | 0.004931433 |
| ENSG00000109390 | NADH:ubiquinone oxidoreductase subunit C1(NDUFC1) | -0.841331809 | 0.253171432 | 0.000890005 | 0.00362661 |
| ENSG00000215301 | DEAD-box helicase 3, X-linked(DDX3X) | -0.840864832 | 0.232980536 | 0.000307186 | 0.001447231 |
| ENSG00000149311 | ATM serine/threonine kinase(ATM) | -0.840845406 | 0.222324098 | 0.000155529 | 0.000802766 |
| ENSG00000125676 | THO complex 2(THOC2) | -0.840364845 | 0.24856041 | 0.000722415 | 0.003020074 |
| ENSG00000135678 | carboxypeptidase M(CPM) | -0.840240824 | 0.276499098 | 0.002374787 | 0.008417292 |
| ENSG00000121988 | zinc finger RANBP2-type containing 3(ZRANB3) | -0.839275796 | 0.321289313 | 0.008995845 | 0.025874603 |
| ENSG00000130005 | guanidinoacetate N-methyltransferase(GAMT) | -0.839222936 | 0.16959708 | 7.49E-07 | 7.11E-06 |
| ENSG00000105613 | microtubule associated serine/threonine kinase 1(MAST1) | -0.839200423 | 0.228756871 | 0.000243953 | 0.001184637 |
| ENSG00000228474 | oligosaccharyltransferase complex subunit 4, non-catalytic(OST4) | -0.838302488 | 0.159209194 | 1.40E-07 | 1.56E-06 |
| ENSG00000107789 | multiple inositol-polyphosphate phosphatase 1(MINPP1) | -0.838124794 | 0.267373877 | 0.001720565 | 0.006385087 |
| ENSG00000160683 | C-X-C motif chemokine receptor 5(CXCR5) | -0.837645274 | 0.172507309 | 1.20E-06 | 1.08E-05 |
| ENSG00000132549 | vacuolar protein sorting 13 homolog B(VPS13B) | -0.837296011 | 0.250480413 | 0.000829495 | 0.003407185 |
| ENSG00000171604 | CXXC finger protein 5(CXXC5) | -0.837009167 | 0.192062261 | 1.31E-05 | 9.08E-05 |
| ENSG00000110696 | chromosome 11 open reading frame 58(C11orf58) | -0.836839901 | 0.271345915 | 0.002042146 | 0.007394214 |
| ENSG00000164920 | odd-skipped related transciption factor 2(OSR2) | -0.83582823 | 0.317093488 | 0.008391328 | 0.024422461 |
| ENSG00000146729 | glioblastoma amplified sequence(GBAS) | -0.835652522 | 0.264284262 | 0.001567187 | 0.005901533 |
| ENSG00000124145 | syndecan 4(SDC4) | -0.835330075 | 0.175832743 | 2.03E-06 | 1.74E-05 |
| ENSG00000135916 | integral membrane protein 2C(ITM2C) | -0.835121285 | 0.15768399 | 1.18E-07 | 1.33E-06 |
| ENSG00000185215 | TNF alpha induced protein 2(TNFAIP2) | -0.835043905 | 0.153870623 | 5.73E-08 | 6.83E-07 |
| ENSG00000177917 | ADP ribosylation factor like GTPase 6 interacting protein 6(ARL6IP6) | -0.833727239 | 0.171732092 | 1.21E-06 | 1.09E-05 |
| ENSG00000174720 | La ribonucleoprotein domain family member 7(LARP7) | -0.833549525 | 0.24442506 | 0.000649044 | 0.002761428 |
| ENSG00000177728 | microRNA 6785(MIR6785) | -0.833372266 | 0.153960305 | 6.20E-08 | 7.32E-07 |
| ENSG00000188783 | proline and arginine rich end leucine rich repeat protein(PRELP) | -0.833099452 | 0.348657501 | 0.016873649 | 0.043867758 |
| ENSG00000073169 | selenoprotein O(SELENOO) | -0.833079814 | 0.241474302 | 0.000560643 | 0.002427457 |
| ENSG00000139921 | thioredoxin related transmembrane protein 1(TMX1) | -0.833044655 | 0.216037965 | 0.000115253 | 0.000618818 |
| ENSG00000119969 | helicase, lymphoid-specific(HELLS) | -0.83290566 | 0.25497124 | 0.001088223 | 0.00431039 |
| ENSG00000121741 | zinc finger MYM-type containing 2(ZMYM2) | -0.832872984 | 0.256603654 | 0.00117139 | 0.004594941 |
| ENSG00000095970 | triggering receptor expressed on myeloid cells 2(TREM2) | -0.832753118 | 0.320159046 | 0.0092936 | 0.026594482 |
| ENSG00000174165 | zinc finger DHHC-type containing 24(ZDHHC24) | -0.832657695 | 0.19903079 | 2.87E-05 | 0.000181603 |
| ENSG00000179532 | dynein heavy chain domain 1(DNHD1) | -0.832182756 | 0.255964794 | 0.001149348 | 0.004523561 |
| ENSG00000178467 | prolyl 4-hydroxylase, transmembrane(P4HTM) | -0.832133754 | 0.156457341 | 1.05E-07 | 1.19E-06 |
| ENSG00000176340 | cytochrome c oxidase subunit 8A(COX8A) | -0.832007722 | 0.165709108 | 5.14E-07 | 5.04E-06 |
| ENSG00000105669 | coatomer protein complex subunit epsilon(COPE) | -0.831578796 | 0.195157738 | 2.03E-05 | 0.000134015 |
| ENSG00000196465 | myosin light chain 6B(MYL6B) | -0.831442763 | 0.158535979 | 1.57E-07 | 1.72E-06 |
| ENSG00000157796 | WD repeat domain 19(WDR19) | -0.831416772 | 0.284448713 | 0.003467815 | 0.011645839 |
| ENSG00000004838 | zinc finger MYND-type containing 10(ZMYND10) | -0.831302461 | 0.180599531 | 4.16E-06 | 3.30E-05 |
| ENSG00000071537 | SEL1L ERAD E3 ligase adaptor subunit(SEL1L) | -0.829333147 | 0.229885377 | 0.000309049 | 0.001454845 |
| ENSG00000205105 | NA | -0.828798747 | 0.320058108 | 0.00961082 | 0.02734261 |
| ENSG00000109906 | zinc finger and BTB domain containing 16(ZBTB16) | -0.828533638 | 0.225067696 | 0.00023208 | 0.001137581 |
| ENSG00000108387 | septin 4(SEPT4) | -0.828451143 | 0.222687855 | 0.000199038 | 0.000994258 |
| ENSG00000139324 | transmembrane and tetratricopeptide repeat containing 3(TMTC3) | -0.828354359 | 0.242719362 | 0.000642974 | 0.002738577 |
| ENSG00000065717 | transducin like enhancer of split 2(TLE2) | -0.828158585 | 0.185647788 | 8.16E-06 | 5.96E-05 |
| ENSG00000070214 | solute carrier family 44 member 1(SLC44A1) | -0.828072194 | 0.243851818 | 0.000684281 | 0.002886299 |
| ENSG00000180229 | hect domain and RLD 2 pseudogene 3(HERC2P3) | -0.827896974 | 0.328395685 | 0.011701026 | 0.032161417 |
| ENSG00000204536 | coiled-coil alpha-helical rod protein 1(CCHCR1) | -0.827623587 | 0.191225945 | 1.50E-05 | 0.000102741 |
| ENSG00000239779 | WW domain binding protein 1(WBP1) | -0.827579207 | 0.151973061 | 5.16E-08 | 6.22E-07 |
| ENSG00000079931 | monooxygenase DBH like 1(MOXD1) | -0.827122467 | 0.245603621 | 0.000757945 | 0.003148469 |
| ENSG00000197594 | ectonucleotide pyrophosphatase/phosphodiesterase 1(ENPP1) | -0.827107499 | 0.265094436 | 0.001808213 | 0.006668648 |
| ENSG00000171310 | carbohydrate sulfotransferase 11(CHST11) | -0.82677569 | 0.231803088 | 0.000361483 | 0.00166699 |
| ENSG00000157150 | TIMP metallopeptidase inhibitor 4(TIMP4) | -0.826324541 | 0.190394557 | 1.42E-05 | 9.78E-05 |
| ENSG00000176533 | G protein subunit gamma 7(GNG7) | -0.826254094 | 0.197197171 | 2.79E-05 | 0.000177281 |
| ENSG00000165533 | tetratricopeptide repeat domain 8(TTC8) | -0.826146836 | 0.283731464 | 0.003594436 | 0.011992328 |
| ENSG00000137947 | general transcription factor IIB(GTF2B) | -0.825518348 | 0.266941851 | 0.001984765 | 0.007217552 |
| ENSG00000177169 | unc-51 like autophagy activating kinase 1(ULK1) | -0.825243899 | 0.227988193 | 0.00029497 | 0.001398075 |
| ENSG00000154640 | BTG anti-proliferation factor 3(BTG3) | -0.825169011 | 0.211164008 | 9.32E-05 | 0.000513388 |
| ENSG00000198929 | nitric oxide synthase 1 adaptor protein(NOS1AP) | -0.825106321 | 0.302267331 | 0.006338741 | 0.019253538 |
| ENSG00000198892 | shisa family member 4(SHISA4) | -0.825040569 | 0.154881387 | 9.99E-08 | 1.14E-06 |
| ENSG00000182628 | spindle and kinetochore associated complex subunit 2(SKA2) | -0.824959895 | 0.205374179 | 5.90E-05 | 0.000343985 |
| ENSG00000166037 | centrosomal protein 57(CEP57) | -0.82452716 | 0.257622969 | 0.001371804 | 0.005265221 |
| ENSG00000133678 | transmembrane protein 254(TMEM254) | -0.824494296 | 0.242426374 | 0.000671376 | 0.00284001 |
| ENSG00000145214 | diacylglycerol kinase theta(DGKQ) | -0.824476185 | 0.23131098 | 0.000364742 | 0.001679382 |
| ENSG00000143162 | cellular repressor of E1A stimulated genes 1(CREG1) | -0.824214494 | 0.227769287 | 0.000296158 | 0.001402578 |
| ENSG00000128185 | DiGeorge syndrome critical region gene 6-like(DGCR6L) | -0.823878161 | 0.176590082 | 3.08E-06 | 2.52E-05 |
| ENSG00000136943 | cathepsin V(CTSV) | -0.823617879 | 0.260210846 | 0.001549794 | 0.005847268 |
| ENSG00000106460 | transmembrane protein 106B(TMEM106B) | -0.823186396 | 0.283998732 | 0.003748847 | 0.012412449 |
| ENSG00000100300 | translocator protein(TSPO) | -0.822267356 | 0.236094502 | 0.000496219 | 0.002189618 |
| ENSG00000105650 | phosphodiesterase 4C(PDE4C) | -0.822219399 | 0.28877608 | 0.00440979 | 0.014244033 |
| ENSG00000243646 | interleukin 10 receptor subunit beta(IL10RB) | -0.821499664 | 0.177788175 | 3.83E-06 | 3.05E-05 |
| ENSG00000160285 | lanosterol synthase (2,3-oxidosqualene-lanosterol cyclase)(LSS) | -0.820987875 | 0.16223495 | 4.18E-07 | 4.21E-06 |
| ENSG00000143164 | DDB1 and CUL4 associated factor 6(DCAF6) | -0.820782739 | 0.183096784 | 7.37E-06 | 5.45E-05 |
| ENSG00000186132 | chromosome 2 open reading frame 76(C2orf76) | -0.820528506 | 0.29041638 | 0.00472287 | 0.01505591 |
| ENSG00000160808 | myosin light chain 3(MYL3) | -0.820026651 | 0.286773118 | 0.004243145 | 0.013800485 |
| ENSG00000125505 | membrane bound O-acyltransferase domain containing 7(MBOAT7) | -0.819351155 | 0.176409308 | 3.41E-06 | 2.76E-05 |
| ENSG00000021300 | pleckstrin homology domain containing B1(PLEKHB1) | -0.819191598 | 0.274091122 | 0.002801154 | 0.009703407 |
| ENSG00000106546 | aryl hydrocarbon receptor(AHR) | -0.817713176 | 0.220365103 | 0.000206671 | 0.001028023 |
| ENSG00000130748 | transmembrane protein 160(TMEM160) | -0.817466183 | 0.236781718 | 0.000555615 | 0.002411012 |
| ENSG00000132024 | coiled-coil and C2 domain containing 1A(CC2D1A) | -0.816803848 | 0.185737403 | 1.09E-05 | 7.72E-05 |
| ENSG00000163975 | melanotransferrin(MELTF) | -0.81651158 | 0.199666764 | 4.33E-05 | 0.000261348 |
| ENSG00000167685 | zinc finger protein 444(ZNF444) | -0.816193278 | 0.189412068 | 1.64E-05 | 0.000110895 |
| ENSG00000104375 | serine/threonine kinase 3(STK3) | -0.815418249 | 0.298227494 | 0.00625291 | 0.019037068 |
| ENSG00000139182 | calsyntenin 3(CLSTN3) | -0.815362253 | 0.164858661 | 7.58E-07 | 7.19E-06 |
| ENSG00000138495 | COX17, cytochrome c oxidase copper chaperone(COX17) | -0.815321623 | 0.18833457 | 1.50E-05 | 0.000102277 |
| ENSG00000142627 | EPH receptor A2(EPHA2) | -0.81507255 | 0.162184421 | 5.02E-07 | 4.94E-06 |
| ENSG00000205593 | DENN domain containing 6B(DENND6B) | -0.81311092 | 0.216670805 | 0.000174907 | 0.000891895 |
| ENSG00000032444 | patatin like phospholipase domain containing 6(PNPLA6) | -0.812746537 | 0.211684023 | 0.000123319 | 0.000656734 |
| ENSG00000035499 | DEP domain containing 1B(DEPDC1B) | -0.812433447 | 0.291654717 | 0.005342872 | 0.016680877 |
| ENSG00000178026 | leucine rich repeat containing 75B(LRRC75B) | -0.812397956 | 0.237951713 | 0.000639862 | 0.002728283 |
| ENSG00000102805 | ceroid-lipofuscinosis, neuronal 5(CLN5) | -0.812210701 | 0.217466866 | 0.000187811 | 0.000949061 |
| ENSG00000122884 | prolyl 4-hydroxylase subunit alpha 1(P4HA1) | -0.812048931 | 0.238506599 | 0.000662312 | 0.002810763 |
| ENSG00000186193 | suppressor APC domain containing 2(SAPCD2) | -0.812028474 | 0.213468164 | 0.00014239 | 0.000743157 |
| ENSG00000204397 | caspase recruitment domain family member 16(CARD16) | -0.811958692 | 0.283463573 | 0.004177738 | 0.013609377 |
| ENSG00000099326 | myeloid zinc finger 1(MZF1) | -0.811934088 | 0.228616544 | 0.000383027 | 0.001747159 |
| ENSG00000189060 | H1 histone family member 0(H1F0) | -0.811719314 | 0.167142451 | 1.20E-06 | 1.08E-05 |
| ENSG00000111554 | Mdm1 nuclear protein(MDM1) | -0.811289543 | 0.324373266 | 0.012380868 | 0.033714938 |
| ENSG00000140025 | EF-hand calcium binding domain 11(EFCAB11) | -0.810974995 | 0.295346743 | 0.006035613 | 0.018480727 |
| ENSG00000164761 | TNF receptor superfamily member 11b(TNFRSF11B) | -0.809943305 | 0.289034123 | 0.005074893 | 0.015967114 |
| ENSG00000115194 | solute carrier family 30 member 3(SLC30A3) | -0.809777972 | 0.184874541 | 1.19E-05 | 8.31E-05 |
| ENSG00000140545 | milk fat globule-EGF factor 8 protein(MFGE8) | -0.809396253 | 0.154171513 | 1.52E-07 | 1.67E-06 |
| ENSG00000078699 | CBFA2/RUNX1 translocation partner 2(CBFA2T2) | -0.80884206 | 0.181985484 | 8.81E-06 | 6.37E-05 |
| ENSG00000157764 | B-Raf proto-oncogene, serine/threonine kinase(BRAF) | -0.808804273 | 0.231940072 | 0.000488241 | 0.002160899 |
| ENSG00000130449 | zinc finger SWIM-type containing 6(ZSWIM6) | -0.808407088 | 0.202370428 | 6.48E-05 | 0.000372828 |
| ENSG00000165487 | mitochondrial calcium uptake 2(MICU2) | -0.808301938 | 0.247873014 | 0.001110389 | 0.004390797 |
| ENSG00000137809 | integrin subunit alpha 11(ITGA11) | -0.808065486 | 0.177272896 | 5.16E-06 | 3.97E-05 |
| ENSG00000164808 | scaffolding protein involved in DNA repair(SPIDR) | -0.807514607 | 0.17250721 | 2.85E-06 | 2.36E-05 |
| ENSG00000224420 | adrenomedullin 5 (putative)(ADM5) | -0.806802241 | 0.261411455 | 0.002026432 | 0.007346362 |
| ENSG00000007541 | phosphatidylinositol glycan anchor biosynthesis class Q(PIGQ) | -0.806620406 | 0.222679915 | 0.00029196 | 0.001386604 |
| ENSG00000174233 | adenylate cyclase 6(ADCY6) | -0.806371153 | 0.161956476 | 6.39E-07 | 6.14E-06 |
| ENSG00000007392 | LUC7 like(LUC7L) | -0.804955129 | 0.158661946 | 3.91E-07 | 3.96E-06 |
| ENSG00000013288 | mannosidase alpha class 2B member 2(MAN2B2) | -0.804303359 | 0.188460548 | 1.97E-05 | 0.000130642 |
| ENSG00000125967 | N-terminal EF-hand calcium binding protein 3(NECAB3) | -0.804285035 | 0.193473896 | 3.22E-05 | 0.000201495 |
| ENSG00000198919 | DAZ interacting zinc finger protein 3(DZIP3) | -0.803261117 | 0.288081007 | 0.00529827 | 0.016568009 |
| ENSG00000259781 | NA | -0.803223501 | 0.276572932 | 0.003681912 | 0.012232142 |
| ENSG00000214530 | StAR related lipid transfer domain containing 10(STARD10) | -0.803157491 | 0.154479101 | 2.00E-07 | 2.16E-06 |
| ENSG00000106479 | zinc finger protein 862(ZNF862) | -0.803010738 | 0.236091472 | 0.000670737 | 0.002839347 |
| ENSG00000175854 | SWIS homologous recombination repair protein(SWI5) | -0.80193006 | 0.172543906 | 3.36E-06 | 2.73E-05 |
| ENSG00000072310 | sterol regulatory element binding transcription factor 1(SREBF1) | -0.801395592 | 0.170940883 | 2.76E-06 | 2.29E-05 |
| ENSG00000196535 | myosin XVIIIA(MYO18A) | -0.801332255 | 0.232665259 | 0.000572874 | 0.002474042 |
| ENSG00000102879 | coronin 1A(CORO1A) | -0.80089726 | 0.228115658 | 0.000446548 | 0.001998172 |
| ENSG00000104870 | Fc fragment of IgG receptor and transporter(FCGRT) | -0.800726832 | 0.169096534 | 2.19E-06 | 1.86E-05 |
| ENSG00000083807 | solute carrier family 27 member 5(SLC27A5) | -0.800603906 | 0.208112544 | 0.000119585 | 0.000638872 |
| ENSG00000142178 | salt inducible kinase 1(SIK1) | -0.800206308 | 0.162943081 | 9.06E-07 | 8.43E-06 |
| ENSG00000178096 | bolA family member 1(BOLA1) | -0.800085953 | 0.210601219 | 0.000145248 | 0.00075606 |
| ENSG00000123453 | sarcosine dehydrogenase(SARDH) | -0.800015144 | 0.178287837 | 7.22E-06 | 5.35E-05 |
| ENSG00000163069 | sarcoglycan beta(SGCB) | -0.799980554 | 0.225792134 | 0.000395609 | 0.001794812 |
| ENSG00000130175 | protein kinase C substrate 80K-H(PRKCSH) | -0.799392585 | 0.166225256 | 1.52E-06 | 1.34E-05 |
| ENSG00000052802 | methylsterol monooxygenase 1(MSMO1) | -0.79869947 | 0.226738894 | 0.000427414 | 0.001925735 |
| ENSG00000135679 | MDM2 proto-oncogene(MDM2) | -0.798190821 | 0.21752395 | 0.000243084 | 0.001181667 |
| ENSG00000105767 | cell adhesion molecule 4(CADM4) | -0.79792139 | 0.175559184 | 5.49E-06 | 4.21E-05 |
| ENSG00000186908 | zinc finger DHHC-type containing 17(ZDHHC17) | -0.797916992 | 0.299495713 | 0.007717127 | 0.022741554 |
| ENSG00000140262 | transcription factor 12(TCF12) | -0.79777943 | 0.250066382 | 0.001421396 | 0.005423713 |
| ENSG00000103811 | cathepsin H(CTSH) | -0.797576898 | 0.308401269 | 0.009705016 | 0.027537338 |
| ENSG00000226742 | heat shock factor binding protein 1 like 1(HSBP1L1) | -0.797291464 | 0.224804614 | 0.00039024 | 0.001775743 |
| ENSG00000150347 | AT-rich interaction domain 5B(ARID5B) | -0.795756401 | 0.224712207 | 0.000398274 | 0.001806206 |
| ENSG00000117151 | chitobiase(CTBS) | -0.795234409 | 0.268659315 | 0.003076285 | 0.010495771 |
| ENSG00000261819 | NA | -0.795037022 | 0.337372649 | 0.018445362 | 0.0473057 |
| ENSG00000136710 | coiled-coil domain containing 115(CCDC115) | -0.794014604 | 0.178029391 | 8.20E-06 | 5.97E-05 |
| ENSG00000253537 | protocadherin gamma subfamily A, 7(PCDHGA7) | -0.793706748 | 0.271876362 | 0.003507436 | 0.011752058 |
| ENSG00000184702 | SEPT5-GP1BB readthrough(SEPT5-GP1BB) | -0.792975346 | 0.17645524 | 6.99E-06 | 5.20E-05 |
| ENSG00000120875 | dual specificity phosphatase 4(DUSP4) | -0.792973581 | 0.165475459 | 1.65E-06 | 1.44E-05 |
| ENSG00000174684 | beta-1,4-glucuronyltransferase 1(B4GAT1) | -0.792864852 | 0.205034196 | 0.000110188 | 0.000594461 |
| ENSG00000129214 | sex hormone binding globulin(SHBG) | -0.792697053 | 0.230541615 | 0.000585137 | 0.0025196 |
| ENSG00000255468 | uncharacterized LOC102724064(LOC102724064) | -0.791458426 | 0.293819344 | 0.007066571 | 0.021093922 |
| ENSG00000152078 | transmembrane protein 56(TMEM56) | -0.79136844 | 0.274324359 | 0.003916736 | 0.01288132 |
| ENSG00000109323 | mannosidase beta(MANBA) | -0.789991386 | 0.232770551 | 0.000689141 | 0.002904715 |
| ENSG00000253873 | protocadherin gamma subfamily A, 11(PCDHGA11) | -0.789582476 | 0.195574694 | 5.41E-05 | 0.000318755 |
| ENSG00000116663 | F-box protein 6(FBXO6) | -0.788628855 | 0.306061862 | 0.009974918 | 0.028174055 |
| ENSG00000174405 | DNA ligase 4(LIG4) | -0.788410663 | 0.309227711 | 0.010784288 | 0.030156018 |
| ENSG00000102575 | acid phosphatase 5, tartrate resistant(ACP5) | -0.788360414 | 0.241143837 | 0.001078315 | 0.004275463 |
| ENSG00000213676 | activating transcription factor 6 beta(ATF6B) | -0.788014606 | 0.165844079 | 2.02E-06 | 1.73E-05 |
| ENSG00000173852 | dpy-19 like 1(DPY19L1) | -0.787750012 | 0.217123899 | 0.0002855 | 0.001358668 |
| ENSG00000088881 | early B-cell factor 4(EBF4) | -0.787165389 | 0.183548011 | 1.80E-05 | 0.000120041 |
| ENSG00000234648 | NA | -0.786855669 | 0.249605959 | 0.001619391 | 0.006072844 |
| ENSG00000105290 | amyloid beta precursor like protein 1(APLP1) | -0.786574828 | 0.180499359 | 1.31E-05 | 9.09E-05 |
| ENSG00000095321 | carnitine O-acetyltransferase(CRAT) | -0.786262182 | 0.156160512 | 4.78E-07 | 4.74E-06 |
| ENSG00000105655 | inositol-3-phosphate synthase 1(ISYNA1) | -0.785784243 | 0.183170394 | 1.79E-05 | 0.000119542 |
| ENSG00000274049 | INO80B-WBP1 readthrough (NMD candidate)(INO80B-WBP1) | -0.784921198 | 0.255941736 | 0.002163662 | 0.007772005 |
| ENSG00000006576 | putative homeodomain transcription factor 2(PHTF2) | -0.784637796 | 0.263987291 | 0.002956201 | 0.010177568 |
| ENSG00000124920 | myelin regulatory factor(MYRF) | -0.784087854 | 0.17141373 | 4.78E-06 | 3.72E-05 |
| ENSG00000140691 | armadillo repeat containing 5(ARMC5) | -0.784029515 | 0.212267387 | 0.000221107 | 0.001091066 |
| ENSG00000181885 | claudin 7(CLDN7) | -0.784014772 | 0.30093043 | 0.00917932 | 0.026318676 |
| ENSG00000148362 | chromosome 9 open reading frame 142(C9orf142) | -0.784011109 | 0.194589777 | 5.60E-05 | 0.000327938 |
| ENSG00000177683 | THAP domain containing 5(THAP5) | -0.783553584 | 0.306594247 | 0.010598371 | 0.029699676 |
| ENSG00000108107 | microRNA 6805(MIR6805) | -0.783265239 | 0.201546012 | 0.00010179 | 0.000554379 |
| ENSG00000111247 | RAD51 associated protein 1(RAD51AP1) | -0.7831469 | 0.249034809 | 0.001662409 | 0.006206469 |
| ENSG00000143971 | Ewing tumor associated antigen 1(ETAA1) | -0.782734025 | 0.274084049 | 0.004292618 | 0.013934425 |
| ENSG00000137070 | interleukin 11 receptor subunit alpha(IL11RA) | -0.782498915 | 0.161955664 | 1.35E-06 | 1.21E-05 |
| ENSG00000052841 | tetratricopeptide repeat domain 17(TTC17) | -0.782124696 | 0.198684962 | 8.27E-05 | 0.000463807 |
| ENSG00000175455 | coiled-coil domain containing 14(CCDC14) | -0.782091732 | 0.26355473 | 0.00300258 | 0.010288431 |
| ENSG00000165355 | F-box protein 33(FBXO33) | -0.781938201 | 0.267044923 | 0.003410241 | 0.011491856 |
| ENSG00000106992 | adenylate kinase 1(AK1) | -0.781095014 | 0.154675108 | 4.42E-07 | 4.43E-06 |
| ENSG00000116106 | EPH receptor A4(EPHA4) | -0.780783216 | 0.241765873 | 0.001240063 | 0.004819326 |
| ENSG00000174886 | NADH:ubiquinone oxidoreductase subunit A11(NDUFA11) | -0.78049908 | 0.238684353 | 0.001075455 | 0.00426556 |
| ENSG00000104518 | gasdermin D(GSDMD) | -0.780469403 | 0.204288327 | 0.000133219 | 0.000701202 |
| ENSG00000269113 | TraB domain containing 2B(TRABD2B) | -0.780235204 | 0.332257396 | 0.018860062 | 0.048138375 |
| ENSG00000073331 | alpha kinase 1(ALPK1) | -0.780063377 | 0.257074044 | 0.002410212 | 0.008519751 |
| ENSG00000172869 | Dmx like 1(DMXL1) | -0.779696063 | 0.283863947 | 0.006019367 | 0.018442712 |
| ENSG00000158246 | family with sequence similarity 46 member B(FAM46B) | -0.779100716 | 0.154045247 | 4.25E-07 | 4.27E-06 |
| ENSG00000164638 | solute carrier family 29 member 4(SLC29A4) | -0.778938193 | 0.205851666 | 0.000154341 | 0.000797047 |
| ENSG00000001617 | semaphorin 3F(SEMA3F) | -0.778503447 | 0.171636489 | 5.74E-06 | 4.36E-05 |
| ENSG00000156162 | dpy-19 like 4 (C. elegans)(DPY19L4) | -0.777729558 | 0.27229694 | 0.004287696 | 0.013923158 |
| ENSG00000100626 | polypeptide N-acetylgalactosaminyltransferase 16(GALNT16) | -0.777059936 | 0.171387459 | 5.79E-06 | 4.39E-05 |
| ENSG00000005486 | rhomboid domain containing 2(RHBDD2) | -0.776104395 | 0.15466832 | 5.22E-07 | 5.11E-06 |
| ENSG00000084636 | collagen type XVI alpha 1 chain(COL16A1) | -0.775850301 | 0.220370255 | 0.000430463 | 0.001937247 |
| ENSG00000111911 | histidine triad nucleotide binding protein 3(HINT3) | -0.775843954 | 0.319860406 | 0.015284348 | 0.040315039 |
| ENSG00000184924 | peptidyl-tRNA hydrolase domain containing 1(PTRHD1) | -0.772968788 | 0.176255088 | 1.16E-05 | 8.13E-05 |
| ENSG00000087301 | thioredoxin domain containing 16(TXNDC16) | -0.772843682 | 0.271550144 | 0.004426607 | 0.01427875 |
| ENSG00000206527 | 3-hydroxyacyl-CoA dehydratase 2(HACD2) | -0.772563826 | 0.235251338 | 0.001023473 | 0.004085435 |
| ENSG00000240344 | peptidylprolyl isomerase like 3(PPIL3) | -0.772033151 | 0.29498809 | 0.00886629 | 0.025583281 |
| ENSG00000086065 | charged multivesicular body protein 5(CHMP5) | -0.772017919 | 0.226145399 | 0.000640607 | 0.002730472 |
| ENSG00000025156 | heat shock transcription factor 2(HSF2) | -0.771730502 | 0.283230927 | 0.006435223 | 0.019521394 |
| ENSG00000167393 | protein phosphatase 2 regulatory subunit B"beta(PPP2R3B) | -0.77154039 | 0.208811815 | 0.000219967 | 0.001086351 |
| ENSG00000060339 | cell division cycle and apoptosis regulator 1(CCAR1) | -0.771459853 | 0.239429785 | 0.001272674 | 0.004931403 |
| ENSG00000115159 | glycerol-3-phosphate dehydrogenase 2(GPD2) | -0.771094916 | 0.284855988 | 0.006790161 | 0.020420063 |
| ENSG00000108654 | microRNA 3064(MIR3064) | -0.770475627 | 0.215961128 | 0.000360185 | 0.001662958 |
| ENSG00000134294 | solute carrier family 38 member 2(SLC38A2) | -0.770034942 | 0.19583485 | 8.42E-05 | 0.000471568 |
| ENSG00000103363 | transcription elongation factor B subunit 2(TCEB2) | -0.769991061 | 0.183072936 | 2.60E-05 | 0.000166785 |
| ENSG00000121104 | family with sequence similarity 117 member A(FAM117A) | -0.76995685 | 0.182980794 | 2.58E-05 | 0.000165547 |
| ENSG00000187164 | shootin 1(SHTN1) | -0.769306456 | 0.327203084 | 0.018715038 | 0.047851273 |
| ENSG00000059728 | MAX dimerization protein 1(MXD1) | -0.769128452 | 0.270699562 | 0.004493534 | 0.014466868 |
| ENSG00000100429 | histone deacetylase 10(HDAC10) | -0.769089917 | 0.212067397 | 0.000287145 | 0.001365388 |
| ENSG00000100979 | phospholipid transfer protein(PLTP) | -0.768991391 | 0.212190173 | 0.000290006 | 0.001378439 |
| ENSG00000122966 | microRNA 1178(MIR1178) | -0.768769349 | 0.165249852 | 3.28E-06 | 2.67E-05 |
| ENSG00000168002 | RNA polymerase II subunit G(POLR2G) | -0.768718276 | 0.171807223 | 7.67E-06 | 5.64E-05 |
| ENSG00000125730 | complement C3(C3) | -0.768608731 | 0.204417372 | 0.000169915 | 0.000869836 |
| ENSG00000103254 | family with sequence similarity 173 member A(FAM173A) | -0.768402292 | 0.300796779 | 0.010632325 | 0.029773547 |
| ENSG00000196850 | PTC7 protein phosphatase homolog(PPTC7) | -0.768121363 | 0.241116053 | 0.001444085 | 0.005494248 |
| ENSG00000159314 | Rho GTPase activating protein 27(ARHGAP27) | -0.767596463 | 0.213572028 | 0.000325531 | 0.001521473 |
| ENSG00000122417 | outer dense fiber of sperm tails 2 like(ODF2L) | -0.767015855 | 0.26144908 | 0.003349367 | 0.011312647 |
| ENSG00000119681 | latent transforming growth factor beta binding protein 2(LTBP2) | -0.766894784 | 0.185685791 | 3.63E-05 | 0.000223601 |
| ENSG00000156171 | DNA damage regulated autophagy modulator 2(DRAM2) | -0.766432693 | 0.21208758 | 0.000301793 | 0.00142525 |
| ENSG00000135423 | glutaminase 2(GLS2) | -0.766212913 | 0.284212014 | 0.007019448 | 0.020974545 |
| ENSG00000260280 | SLX1B-SULT1A4 readthrough (NMD candidate)(SLX1B-SULT1A4) | -0.765809694 | 0.210887659 | 0.000281928 | 0.001344386 |
| ENSG00000136425 | calcium and integrin binding family member 2(CIB2) | -0.765712629 | 0.178231287 | 1.74E-05 | 0.000116548 |
| ENSG00000139687 | RB transcriptional corepressor 1(RB1) | -0.764999782 | 0.24074714 | 0.001484959 | 0.005624295 |
| ENSG00000099625 | CACN beta subunit associated regulatory protein(CBARP) | -0.764827035 | 0.277022908 | 0.005764583 | 0.017761802 |
| ENSG00000158286 | ring finger protein 207(RNF207) | -0.764678948 | 0.22700343 | 0.000755568 | 0.00314049 |
| ENSG00000196683 | translocase of outer mitochondrial membrane 7(TOMM7) | -0.76428064 | 0.165030824 | 3.64E-06 | 2.92E-05 |
| ENSG00000187239 | formin binding protein 1(FNBP1) | -0.763637726 | 0.157242817 | 1.20E-06 | 1.08E-05 |
| ENSG00000120860 | coiled-coil domain containing 53(CCDC53) | -0.763361953 | 0.257080756 | 0.002984335 | 0.010248673 |
| ENSG00000188985 | NA | -0.762741627 | 0.302232389 | 0.011612944 | 0.031943223 |
| ENSG00000180530 | nuclear receptor interacting protein 1(NRIP1) | -0.762737697 | 0.304599504 | 0.012277463 | 0.033472092 |
| ENSG00000184185 | potassium voltage-gated channel subfamily J member 12(KCNJ12) | -0.761742156 | 0.201743184 | 0.000159494 | 0.000820415 |
| ENSG00000134352 | interleukin 6 signal transducer(IL6ST) | -0.761663044 | 0.210419811 | 0.00029491 | 0.001398075 |
| ENSG00000141552 | anaphase promoting complex subunit 11(ANAPC11) | -0.761622558 | 0.191045992 | 6.70E-05 | 0.000383526 |
| ENSG00000197563 | phosphatidylinositol glycan anchor biosynthesis class N(PIGN) | -0.761020839 | 0.267663952 | 0.004466372 | 0.014387295 |
| ENSG00000072210 | aldehyde dehydrogenase 3 family member A2(ALDH3A2) | -0.76025099 | 0.197746599 | 0.000120763 | 0.000644799 |
| ENSG00000143228 | NUF2, NDC80 kinetochore complex component(NUF2) | -0.759953563 | 0.228007433 | 0.000859085 | 0.003518896 |
| ENSG00000180730 | shisa family member 2(SHISA2) | -0.759738739 | 0.260143827 | 0.003495189 | 0.0117177 |
| ENSG00000135272 | MyoD family inhibitor domain containing(MDFIC) | -0.758716 | 0.245049042 | 0.001960312 | 0.007144085 |
| ENSG00000183137 | centrosomal protein 57 like 1(CEP57L1) | -0.757798837 | 0.261159511 | 0.003711788 | 0.012314008 |
| ENSG00000166479 | thioredoxin related transmembrane protein 3(TMX3) | -0.757782557 | 0.244418705 | 0.001932947 | 0.007055286 |
| ENSG00000128708 | histone acetyltransferase 1(HAT1) | -0.757775426 | 0.221131514 | 0.000610719 | 0.002617303 |
| ENSG00000213190 | myeloid/lymphoid or mixed-lineage leukemia; translocated to, 11(MLLT11) | -0.757465648 | 0.194279464 | 9.67E-05 | 0.000529373 |
| ENSG00000178498 | deltex E3 ubiquitin ligase 3(DTX3) | -0.756825428 | 0.152520332 | 6.97E-07 | 6.65E-06 |
| ENSG00000137507 | leucine rich repeat containing 32(LRRC32) | -0.755709311 | 0.242212018 | 0.001808312 | 0.006668648 |
| ENSG00000167107 | acyl-CoA synthetase family member 2(ACSF2) | -0.755228895 | 0.163126195 | 3.66E-06 | 2.94E-05 |
| ENSG00000165494 | PCF11 cleavage and polyadenylation factor subunit(PCF11) | -0.755205395 | 0.217737755 | 0.000523534 | 0.002289535 |
| ENSG00000266074 | BAH domain and coiled-coil containing 1(BAHCC1) | -0.754833192 | 0.251644574 | 0.002703339 | 0.009420048 |
| ENSG00000133243 | BTB domain containing 2(BTBD2) | -0.754650855 | 0.220070116 | 0.000605501 | 0.002596833 |
| ENSG00000184445 | kinetochore associated 1(KNTC1) | -0.754411503 | 0.235710558 | 0.001371493 | 0.005265221 |
| ENSG00000166803 | KIAA0101(KIAA0101) | -0.75436483 | 0.201544818 | 0.000181899 | 0.000921687 |
| ENSG00000165698 | sperm acrosome associated 9(SPACA9) | -0.754363265 | 0.245429788 | 0.002114631 | 0.007616817 |
| ENSG00000139880 | cadherin 24(CDH24) | -0.753266182 | 0.164262745 | 4.52E-06 | 3.55E-05 |
| ENSG00000071794 | helicase like transcription factor(HLTF) | -0.75294788 | 0.267332714 | 0.004854705 | 0.01538544 |
| ENSG00000167100 | sterile alpha motif domain containing 14(SAMD14) | -0.752920902 | 0.169437375 | 8.84E-06 | 6.39E-05 |
| ENSG00000196576 | plexin B2(PLXNB2) | -0.752444479 | 0.19207972 | 8.95E-05 | 0.000496316 |
| ENSG00000172828 | carboxylesterase 3(CES3) | -0.752257549 | 0.319847973 | 0.018676681 | 0.047763581 |
| ENSG00000167476 | uncharacterized LOC105372240(LOC105372240) | -0.751411828 | 0.217818085 | 0.000561164 | 0.002428821 |
| ENSG00000131408 | nuclear receptor subfamily 1 group H member 2(NR1H2) | -0.751361767 | 0.206956356 | 0.000282837 | 0.001347629 |
| ENSG00000214455 | NA | -0.751246692 | 0.28070654 | 0.007444649 | 0.022065721 |
| ENSG00000126351 | thyroid hormone receptor, alpha(THRA) | -0.751138122 | 0.157894682 | 1.96E-06 | 1.69E-05 |
| ENSG00000114854 | troponin C1, slow skeletal and cardiac type(TNNC1) | -0.750957278 | 0.152080543 | 7.90E-07 | 7.45E-06 |
| ENSG00000214756 | methyltransferase like 12(METTL12) | -0.750114281 | 0.192566827 | 9.81E-05 | 0.000536043 |
| ENSG00000240053 | lymphocyte antigen 6 complex, locus G5B(LY6G5B) | -0.750048835 | 0.310273858 | 0.015632824 | 0.041031803 |
| ENSG00000142166 | interferon alpha and beta receptor subunit 1(IFNAR1) | -0.749486218 | 0.224895683 | 0.000860401 | 0.003523062 |
| ENSG00000131584 | ArfGAP with coiled-coil, ankyrin repeat and PH domains 3(ACAP3) | -0.747648854 | 0.208579777 | 0.000337758 | 0.001569886 |
| ENSG00000105373 | glioma tumor suppressor candidate region gene 2(GLTSCR2) | -0.74727192 | 0.190634767 | 8.86E-05 | 0.000492002 |
| ENSG00000138386 | NGFI-A binding protein 1(NAB1) | -0.746225686 | 0.237952693 | 0.001712544 | 0.00636335 |
| ENSG00000070081 | uncharacterized LOC105376575(LOC105376575) | -0.745029466 | 0.276609982 | 0.007072112 | 0.021105104 |
| ENSG00000116171 | sterol carrier protein 2(SCP2) | -0.744510712 | 0.23180693 | 0.001319196 | 0.005086537 |
| ENSG00000006715 | VPS41, HOPS complex subunit(VPS41) | -0.743096114 | 0.249982484 | 0.00295296 | 0.010169385 |
| ENSG00000103502 | CDP-diacylglycerol--inositol 3-phosphatidyltransferase(CDIPT) | -0.742952265 | 0.152742238 | 1.15E-06 | 1.04E-05 |
| ENSG00000149541 | beta-1,3-glucuronyltransferase 3(B3GAT3) | -0.742666415 | 0.201192988 | 0.000223099 | 0.001099052 |
| ENSG00000136156 | integral membrane protein 2B(ITM2B) | -0.742361791 | 0.169323536 | 1.16E-05 | 8.16E-05 |
| ENSG00000115947 | origin recognition complex subunit 4(ORC4) | -0.742339818 | 0.224591438 | 0.000948782 | 0.003830292 |
| ENSG00000178078 | signal transducing adaptor family member 2(STAP2) | -0.741431537 | 0.186732506 | 7.17E-05 | 0.000406136 |
| ENSG00000204580 | microRNA 4640(MIR4640) | -0.741386202 | 0.22884468 | 0.00119659 | 0.00467664 |
| ENSG00000131149 | Gse1 coiled-coil protein(GSE1) | -0.741384602 | 0.186962928 | 7.33E-05 | 0.000413801 |
| ENSG00000180488 | mitoguardin 1(MIGA1) | -0.741310433 | 0.297025541 | 0.012568049 | 0.034161397 |
| ENSG00000110237 | Rho guanine nucleotide exchange factor 17(ARHGEF17) | -0.741300618 | 0.17691113 | 2.79E-05 | 0.000177281 |
| ENSG00000147650 | LDL receptor related protein 12(LRP12) | -0.741176073 | 0.237488398 | 0.00180303 | 0.006655428 |
| ENSG00000114573 | ATPase H+ transporting V1 subunit A(ATP6V1A) | -0.741168388 | 0.258615957 | 0.004158209 | 0.013550463 |
| ENSG00000179046 | tripartite motif family like 2(TRIML2) | -0.7411519 | 0.189804559 | 9.43E-05 | 0.000518612 |
| ENSG00000154736 | ADAM metallopeptidase with thrombospondin type 1 motif 5(ADAMTS5) | -0.741145175 | 0.278696193 | 0.007829621 | 0.023015407 |
| ENSG00000087884 | adipogenesis associated Mth938 domain containing(AAMDC) | -0.74098767 | 0.197884241 | 0.000180717 | 0.000916357 |
| ENSG00000106052 | Tax1 binding protein 1(TAX1BP1) | -0.74097236 | 0.219970373 | 0.000755757 | 0.00314049 |
| ENSG00000160753 | RUN and SH3 domain containing 1(RUSC1) | -0.740856891 | 0.151987961 | 1.09E-06 | 9.98E-06 |
| ENSG00000104897 | splicing factor 3a subunit 2(SF3A2) | -0.740684295 | 0.219987117 | 0.000760061 | 0.003153919 |
| ENSG00000109255 | neuromedin U(NMU) | -0.740518287 | 0.314403874 | 0.018507307 | 0.047433542 |
| ENSG00000173531 | macrophage stimulating 1(MST1) | -0.740361152 | 0.261929927 | 0.004705065 | 0.015006064 |
| ENSG00000167995 | bestrophin 1(BEST1) | -0.738293351 | 0.174191858 | 2.25E-05 | 0.000146401 |
| ENSG00000170667 | RAS p21 protein activator 4B(RASA4B) | -0.736572082 | 0.185699648 | 7.29E-05 | 0.00041256 |
| ENSG00000166681 | brain expressed X-linked 3(BEX3) | -0.736567926 | 0.153005649 | 1.48E-06 | 1.31E-05 |
| ENSG00000188186 | late endosomal/lysosomal adaptor, MAPK and MTOR activator 4(LAMTOR4) | -0.735891739 | 0.162046293 | 5.59E-06 | 4.27E-05 |
| ENSG00000109861 | cathepsin C(CTSC) | -0.735432948 | 0.154049761 | 1.81E-06 | 1.57E-05 |
| ENSG00000188559 | Ral GTPase activating protein catalytic alpha subunit 2(RALGAPA2) | -0.734884399 | 0.262423982 | 0.005104397 | 0.01604278 |
| ENSG00000115652 | UDP-glucuronate decarboxylase 1(UXS1) | -0.734525404 | 0.18558139 | 7.56E-05 | 0.00042588 |
| ENSG00000166734 | cancer susceptibility candidate 4(CASC4) | -0.734503949 | 0.215000793 | 0.000634818 | 0.002709724 |
| ENSG00000132842 | adaptor related protein complex 3 beta 1 subunit(AP3B1) | -0.733977182 | 0.262357561 | 0.005148038 | 0.01616267 |
| ENSG00000069702 | transforming growth factor beta receptor 3(TGFBR3) | -0.732884677 | 0.254137676 | 0.003928962 | 0.012910701 |
| ENSG00000080802 | CCR4-NOT transcription complex subunit 4(CNOT4) | -0.732880153 | 0.184295504 | 6.99E-05 | 0.000397003 |
| ENSG00000173064 | microRNA 6861(MIR6861) | -0.732581603 | 0.186474967 | 8.54E-05 | 0.000477089 |
| ENSG00000139168 | zinc finger CCHC-type and RNA binding motif containing 1(ZCRB1) | -0.732085817 | 0.210987463 | 0.000520832 | 0.002279416 |
| ENSG00000155368 | diazepam binding inhibitor, acyl-CoA binding protein(DBI) | -0.73179492 | 0.160315326 | 5.00E-06 | 3.87E-05 |
| ENSG00000151725 | centromere protein U(CENPU) | -0.731728537 | 0.278261267 | 0.00854739 | 0.024818665 |
| | ATP synthase, H+ transporting, mitochondrial F1 complex, epsilon | | | | |
| ENSG00000124172 | subunit(ATP5E) | -0.729364172 | 0.157144262 | 3.46E-06 | 2.80E-05 |
| ENSG00000075568 | transmembrane protein 131(TMEM131) | -0.729114045 | 0.195019649 | 0.000184997 | 0.000936446 |
| ENSG00000100321 | synaptogyrin 1(SYNGR1) | -0.728662541 | 0.186530784 | 9.37E-05 | 0.000515723 |
| ENSG00000074695 | lectin, mannose binding 1(LMAN1) | -0.728057728 | 0.22403125 | 0.001154844 | 0.004542156 |
| ENSG00000163964 | phosphatidylinositol glycan anchor biosynthesis class X(PIGX) | -0.727123328 | 0.234330205 | 0.001915787 | 0.007003519 |
| ENSG00000185236 | RAB11B, member RAS oncogene family(RAB11B) | -0.726546697 | 0.206451233 | 0.000432822 | 0.001944891 |
| ENSG00000170075 | G protein-coupled receptor 37 like 1(GPR37L1) | -0.726479761 | 0.228516702 | 0.00147728 | 0.00559701 |
| ENSG00000130669 | p21 (RAC1) activated kinase 4(PAK4) | -0.726140964 | 0.199698675 | 0.000276708 | 0.001323785 |
| ENSG00000116266 | syntaxin binding protein 3(STXBP3) | -0.724548901 | 0.269078238 | 0.007087449 | 0.021140149 |
| ENSG00000135541 | Abelson helper integration site 1(AHI1) | -0.724307442 | 0.289034146 | 0.012212011 | 0.033324541 |
| ENSG00000101843 | proteasome 26S subunit, non-ATPase 10(PSMD10) | -0.724305242 | 0.215114132 | 0.000759702 | 0.003153572 |
| ENSG00000004700 | RecQ like helicase(RECQL) | -0.724283394 | 0.237785158 | 0.002319409 | 0.008258332 |
| ENSG00000115109 | erythrocyte membrane protein band 4.1 like 5(EPB41L5) | -0.723731603 | 0.276254604 | 0.008798154 | 0.025442844 |
| ENSG00000140463 | Bardet-Biedl syndrome 4(BBS4) | -0.722825294 | 0.212277761 | 0.000661418 | 0.002807982 |
| ENSG00000043143 | jade family PHD finger 2(JADE2) | -0.72239028 | 0.18470293 | 9.19E-05 | 0.000506737 |
| ENSG00000174943 | potassium channel tetramerization domain containing 13(KCTD13) | -0.722114884 | 0.18503276 | 9.52E-05 | 0.000522581 |
| ENSG00000167969 | enoyl-CoA delta isomerase 1(ECI1) | -0.721775312 | 0.216646242 | 0.00086353 | 0.003532186 |
| ENSG00000106009 | BRCA1 associated ATM activator 1(BRAT1) | -0.720900613 | 0.23236425 | 0.0019192 | 0.007011635 |
| ENSG00000125734 | microRNA 6791(MIR6791) | -0.720791116 | 0.148453425 | 1.20E-06 | 1.09E-05 |
| ENSG00000125726 | CD70 molecule(CD70) | -0.7206539 | 0.187183613 | 0.000118126 | 0.000632512 |
| ENSG00000196586 | myosin VI(MYO6) | -0.720278101 | 0.27222971 | 0.008148672 | 0.023834156 |
| ENSG00000167925 | GH3 domain containing(GHDC) | -0.719893551 | 0.176819817 | 4.67E-05 | 0.000279132 |
| ENSG00000119632 | interferon alpha inducible protein 27 like 2(IFI27L2) | -0.719662162 | 0.153607927 | 2.80E-06 | 2.32E-05 |
| ENSG00000077942 | fibulin 1(FBLN1) | -0.719444822 | 0.165665609 | 1.41E-05 | 9.67E-05 |
| ENSG00000162729 | immunoglobulin superfamily member 8(IGSF8) | -0.719146825 | 0.18686549 | 0.000118856 | 0.000635557 |
| ENSG00000163605 | protein phosphatase 4 regulatory subunit 2(PPP4R2) | -0.719033275 | 0.23856571 | 0.002578412 | 0.00903289 |
| ENSG00000177464 | G protein-coupled receptor 4(GPR4) | -0.718951351 | 0.176640139 | 4.70E-05 | 0.000280423 |
| ENSG00000170835 | carboxyl ester lipase(CEL) | -0.718928104 | 0.2955976 | 0.015010836 | 0.03970656 |
| ENSG00000157227 | matrix metallopeptidase 14(MMP14) | -0.717507149 | 0.183153321 | 8.95E-05 | 0.000496206 |
| ENSG00000146556 | WAS protein family homolog 2 pseudogene(WASH2P) | -0.717413318 | 0.216934774 | 0.000942853 | 0.00381148 |
| ENSG00000188486 | H2A histone family member X(H2AFX) | -0.717186826 | 0.245800337 | 0.003525536 | 0.011802619 |
| ENSG00000187231 | SEC14 and spectrin domain containing 1(SESTD1) | -0.716693246 | 0.280665752 | 0.010663182 | 0.02985285 |
| ENSG00000096092 | transmembrane protein 14A(TMEM14A) | -0.716514488 | 0.196464695 | 0.000265279 | 0.001275856 |
| ENSG00000143434 | semaphorin 6C(SEMA6C) | -0.716192308 | 0.245042273 | 0.003469777 | 0.011649102 |
| ENSG00000185585 | olfactomedin like 2A(OLFML2A) | -0.715520457 | 0.170382072 | 2.67E-05 | 0.000171211 |
| ENSG00000203668 | CHM like, Rab escort protein 2(CHML) | -0.71464443 | 0.275468947 | 0.009478841 | 0.027052178 |
| ENSG00000115457 | insulin like growth factor binding protein 2(IGFBP2) | -0.714326452 | 0.181746431 | 8.48E-05 | 0.000474045 |
| ENSG00000149260 | calpain 5(CAPN5) | -0.714253769 | 0.161783888 | 1.01E-05 | 7.21E-05 |
| ENSG00000235912 | NA | -0.713961837 | 0.194725049 | 0.000245881 | 0.001192801 |
| ENSG00000100243 | cytochrome b5 reductase 3(CYB5R3) | -0.713358145 | 0.160492562 | 8.80E-06 | 6.37E-05 |
| ENSG00000117616 | arginine and serine rich protein 1(RSRP1) | -0.713346079 | 0.178517412 | 6.44E-05 | 0.000371036 |
| ENSG00000090581 | N-acetylglucosamine-1-phosphate transferase gamma subunit(GNPTG) | -0.71152969 | 0.171414786 | 3.31E-05 | 0.000206329 |
| ENSG00000205155 | presenilin enhancer gamma-secretase subunit(PSENEN) | -0.710458049 | 0.149708011 | 2.08E-06 | 1.78E-05 |
| ENSG00000137101 | CD72 molecule(CD72) | -0.710068463 | 0.227326906 | 0.001786795 | 0.006605863 |
| ENSG00000081320 | serine/threonine kinase 17b(STK17B) | -0.710018652 | 0.233119745 | 0.0023212 | 0.008259708 |
| ENSG00000259132 | NA | -0.709643966 | 0.201481751 | 0.000428102 | 0.001928097 |
| ENSG00000155760 | frizzled class receptor 7(FZD7) | -0.709146275 | 0.185114613 | 0.000127701 | 0.000677008 |
| ENSG00000073670 | ADAM metallopeptidase domain 11(ADAM11) | -0.708866223 | 0.183708284 | 0.000114014 | 0.00061301 |
| ENSG00000166225 | fibroblast growth factor receptor substrate 2(FRS2) | -0.708298807 | 0.279187337 | 0.011180634 | 0.031035626 |
| ENSG00000164543 | serine/threonine kinase 17a(STK17A) | -0.708175793 | 0.279741337 | 0.011356355 | 0.031397452 |
| ENSG00000126705 | AT-hook DNA binding motif containing 1(AHDC1) | -0.708036459 | 0.230095679 | 0.002089976 | 0.007541708 |
| ENSG00000132329 | receptor activity modifying protein 1(RAMP1) | -0.708034199 | 0.245748914 | 0.003962541 | 0.012999255 |
| ENSG00000112406 | hdc homolog, cell cycle regulator(HECA) | -0.707981149 | 0.239763665 | 0.003148761 | 0.010705789 |
| ENSG00000131495 | NADH:ubiquinone oxidoreductase subunit A2(NDUFA2) | -0.70754844 | 0.168692288 | 2.74E-05 | 0.000174704 |
| ENSG00000183828 | nudix hydrolase 14(NUDT14) | -0.707044012 | 0.191838843 | 0.000228152 | 0.001121126 |
| ENSG00000185885 | interferon induced transmembrane protein 1(IFITM1) | -0.706252271 | 0.243733187 | 0.003759758 | 0.012434578 |
| ENSG00000138316 | ADAM metallopeptidase with thrombospondin type 1 motif 14(ADAMTS14) | -0.706191737 | 0.213223382 | 0.000926379 | 0.003752729 |
| ENSG00000012822 | calcium binding and coiled-coil domain 1(CALCOCO1) | -0.705768533 | 0.158797624 | 8.81E-06 | 6.37E-05 |
| ENSG00000163170 | bolA family member 3(BOLA3) | -0.705411524 | 0.179286196 | 8.34E-05 | 0.000467368 |
| ENSG00000167644 | chromosome 19 open reading frame 33(C19orf33) | -0.705334727 | 0.193520928 | 0.000267655 | 0.001285478 |
| ENSG00000169554 | zinc finger E-box binding homeobox 2(ZEB2) | -0.705291837 | 0.220605925 | 0.001388329 | 0.005319967 |
| ENSG00000144401 | methyltransferase like 21A(METTL21A) | -0.704770381 | 0.202624118 | 0.000504764 | 0.00222232 |
| ENSG00000078674 | pericentriolar material 1(PCM1) | -0.70476342 | 0.262964682 | 0.007360705 | 0.021844432 |
| ENSG00000173705 | sushi domain containing 5(SUSD5) | -0.704246302 | 0.226740921 | 0.001896678 | 0.006953108 |
| ENSG00000162585 | Fanconi anemia core complex associated protein 20(FAAP20) | -0.704155031 | 0.207536031 | 0.000691495 | 0.00291255 |
| ENSG00000196743 | GM2 ganglioside activator(GM2A) | -0.703423436 | 0.20201736 | 0.000497693 | 0.002195298 |
| ENSG00000104774 | mannosidase alpha class 2B member 1(MAN2B1) | -0.702929759 | 0.1753327 | 6.09E-05 | 0.000352836 |
| ENSG00000087086 | ferritin light chain(FTL) | -0.702605466 | 0.151760362 | 3.66E-06 | 2.94E-05 |
| ENSG00000116691 | migration and invasion inhibitory protein(MIIP) | -0.701651095 | 0.216207613 | 0.001173412 | 0.004599806 |
| ENSG00000005243 | coatomer protein complex subunit zeta 2(COPZ2) | -0.700751018 | 0.276890139 | 0.01138057 | 0.031457008 |
| ENSG00000135473 | PAN2 poly(A) specific ribonuclease subunit(PAN2) | -0.700099999 | 0.179794136 | 9.86E-05 | 0.000538753 |
| ENSG00000020426 | MNAT1, CDK activating kinase assembly factor(MNAT1) | -0.700051275 | 0.262993838 | 0.007771151 | 0.02287211 |
| ENSG00000184205 | TSPY like 2(TSPYL2) | -0.69982909 | 0.161293104 | 1.43E-05 | 9.81E-05 |
| ENSG00000109625 | carboxypeptidase Z(CPZ) | -0.699474958 | 0.17486784 | 6.33E-05 | 0.000365422 |
| ENSG00000075945 | kinesin associated protein 3(KIFAP3) | -0.699332449 | 0.298351205 | 0.019078647 | 0.048538323 |
| ENSG00000140264 | small EDRK-rich factor 2(SERF2) | -0.69901516 | 0.157492311 | 9.06E-06 | 6.52E-05 |
| ENSG00000180720 | cholinergic receptor muscarinic 4(CHRM4) | -0.698643326 | 0.259046449 | 0.006997128 | 0.020915 |
| ENSG00000138658 | zinc finger GRF-type containing 1(ZGRF1) | -0.698639938 | 0.275795416 | 0.01130323 | 0.031292137 |
| ENSG00000157326 | dehydrogenase/reductase 4(DHRS4) | -0.698189222 | 0.240356836 | 0.003674845 | 0.012219015 |
| ENSG00000149582 | transmembrane protein 25(TMEM25) | -0.698135328 | 0.153953127 | 5.77E-06 | 4.38E-05 |
| ENSG00000189362 | nuclear envelope integral membrane protein 2(NEMP2) | -0.6980164 | 0.2680004 | 0.009199905 | 0.026371266 |
| ENSG00000196549 | membrane metalloendopeptidase(MME) | -0.697594204 | 0.246040515 | 0.004578527 | 0.014672241 |
| ENSG00000198142 | sosondowah ankyrin repeat domain family member C(SOWAHC) | -0.697442095 | 0.213608401 | 0.001094448 | 0.004332128 |
| ENSG00000156931 | VPS8, CORVET complex subunit(VPS8) | -0.696070141 | 0.266197289 | 0.008926256 | 0.025713396 |
| ENSG00000169682 | sphingolipid transporter 1 (putative)(SPNS1) | -0.695619335 | 0.172980805 | 5.79E-05 | 0.000337975 |
| ENSG00000186350 | retinoid X receptor alpha(RXRA) | -0.695445005 | 0.198432587 | 0.000457137 | 0.002039346 |
| ENSG00000077254 | ubiquitin specific peptidase 33(USP33) | -0.694794452 | 0.252043327 | 0.005839738 | 0.017951052 |
| ENSG00000130770 | ATPase inhibitory factor 1(ATPIF1) | -0.694545217 | 0.14898508 | 3.13E-06 | 2.56E-05 |
| ENSG00000147400 | centrin 2(CETN2) | -0.69334498 | 0.16140843 | 1.74E-05 | 0.00011671 |
| ENSG00000131067 | gamma-glutamyltransferase 7(GGT7) | -0.69312167 | 0.243498461 | 0.004420085 | 0.014265535 |
| ENSG00000100060 | MFNG O-fucosylpeptide 3-beta-N-acetylglucosaminyltransferase(MFNG) | -0.692927241 | 0.257107335 | 0.007036976 | 0.021016244 |
| ENSG00000173276 | zinc finger and BTB domain containing 21(ZBTB21) | -0.692684048 | 0.264572694 | 0.008841474 | 0.02553673 |
| ENSG00000161955 | tumor necrosis factor superfamily member 13(TNFSF13) | -0.692419127 | 0.184623885 | 0.000176531 | 0.000897945 |
| ENSG00000112972 | 3-hydroxy-3-methylglutaryl-CoA synthase 1(HMGCS1) | -0.692084134 | 0.21521425 | 0.001300854 | 0.005022396 |
| ENSG00000122691 | twist family bHLH transcription factor 1(TWIST1) | -0.691781024 | 0.244745886 | 0.004705568 | 0.015006064 |
| ENSG00000264230 | long intergenic non-protein coding RNA 842(LINC00842) | -0.690983983 | 0.279322501 | 0.01336899 | 0.035980964 |
| ENSG00000050426 | LETM1 domain containing 1(LETMD1) | -0.690628833 | 0.172070585 | 5.98E-05 | 0.000347688 |
| ENSG00000120837 | nuclear transcription factor Y subunit beta(NFYB) | -0.690178548 | 0.254778407 | 0.006749924 | 0.020329264 |
| ENSG00000132471 | WW domain binding protein 2(WBP2) | -0.689903757 | 0.15949069 | 1.52E-05 | 0.000103768 |
| ENSG00000126368 | nuclear receptor subfamily 1 group D member 1(NR1D1) | -0.689272378 | 0.187342602 | 0.00023396 | 0.001144883 |
| ENSG00000187609 | exonuclease 3'-5' domain containing 3(EXD3) | -0.689088743 | 0.219761949 | 0.001714945 | 0.00637026 |
| ENSG00000146066 | HIG1 hypoxia inducible domain family member 2A(HIGD2A) | -0.688875375 | 0.157864696 | 1.28E-05 | 8.86E-05 |
| ENSG00000068885 | intraflagellar transport 80(IFT80) | -0.688599403 | 0.254564084 | 0.006830152 | 0.020528846 |
| ENSG00000213462 | endogenous retrovirus group 3 member 1(ERV3-1) | -0.688337622 | 0.27794047 | 0.013265349 | 0.035742916 |
| ENSG00000112697 | transmembrane protein 30A(TMEM30A) | -0.687971463 | 0.218530042 | 0.001642916 | 0.006144614 |
| ENSG00000137812 | kinetochore scaffold 1(KNL1) | -0.685965978 | 0.234133688 | 0.003391751 | 0.011439381 |
| ENSG00000167671 | UBX domain protein 6(UBXN6) | -0.685605868 | 0.185206657 | 0.00021404 | 0.001061088 |
| ENSG00000079432 | capicua transcriptional repressor(CIC) | -0.685547451 | 0.216452496 | 0.001539166 | 0.005810896 |
| ENSG00000126778 | SIX homeobox 1(SIX1) | -0.684867702 | 0.22592604 | 0.002434386 | 0.008587141 |
| ENSG00000078304 | protein phosphatase 2 regulatory subunit B'gamma(PPP2R5C) | -0.684480202 | 0.220718112 | 0.001927699 | 0.007038316 |
| ENSG00000143401 | acidic nuclear phosphoprotein 32 family member E(ANP32E) | -0.684430269 | 0.253904153 | 0.00702568 | 0.020987838 |
| ENSG00000112655 | protein tyrosine kinase 7 (inactive)(PTK7) | -0.684116316 | 0.159337683 | 1.76E-05 | 0.000117692 |
| ENSG00000126777 | kinectin 1(KTN1) | -0.682875648 | 0.2151021 | 0.001500131 | 0.005678101 |
| ENSG00000258289 | churchill domain containing 1(CHURC1) | -0.682538945 | 0.27124342 | 0.011858286 | 0.032532855 |
| ENSG00000123268 | activating transcription factor 1(ATF1) | -0.682479965 | 0.251815597 | 0.006723513 | 0.020260095 |
| ENSG00000131051 | RNA binding motif protein 39(RBM39) | -0.682060578 | 0.218301563 | 0.001781704 | 0.006591183 |
| ENSG00000161914 | zinc finger protein 653(ZNF653) | -0.68169955 | 0.207654924 | 0.001027641 | 0.004096633 |
| ENSG00000166275 | BLOC-1 related complex subunit 7(BORCS7) | -0.681486202 | 0.26472376 | 0.010043456 | 0.028333624 |
| ENSG00000144061 | nephrocystin 1(NPHP1) | -0.681246582 | 0.223955955 | 0.002351075 | 0.008353383 |
| ENSG00000102580 | DnaJ heat shock protein family (Hsp40) member C3(DNAJC3) | -0.680642001 | 0.238997186 | 0.004400764 | 0.014218783 |
| ENSG00000138175 | ADP ribosylation factor like GTPase 3(ARL3) | -0.680480089 | 0.179330554 | 0.000147907 | 0.000767863 |
| ENSG00000157216 | single stranded DNA binding protein 3(SSBP3) | -0.680007478 | 0.200391094 | 0.000690303 | 0.00290857 |
| ENSG00000075223 | semaphorin 3C(SEMA3C) | -0.679862756 | 0.22273789 | 0.002270954 | 0.008117745 |
| ENSG00000188910 | gap junction protein beta 3(GJB3) | -0.679758133 | 0.280373691 | 0.01533067 | 0.040410039 |
| ENSG00000166165 | creatine kinase B(CKB) | -0.679540594 | 0.20698074 | 0.001026685 | 0.004094211 |
| ENSG00000114923 | solute carrier family 4 member 3(SLC4A3) | -0.679333805 | 0.191216382 | 0.000381304 | 0.001741327 |
| ENSG00000133315 | MACRO domain containing 1(MACROD1) | -0.679321547 | 0.186573092 | 0.000271531 | 0.001301135 |
| ENSG00000176624 | mex-3 RNA binding family member C(MEX3C) | -0.678120396 | 0.221842837 | 0.002237431 | 0.008009517 |
| | extracellular leucine rich repeat and fibronectin type III domain containing | | | | |
| ENSG00000166897 | 2(ELFN2) | -0.677866053 | 0.206680084 | 0.001038866 | 0.004132977 |
| ENSG00000060656 | protein tyrosine phosphatase, receptor type U(PTPRU) | -0.676901537 | 0.180190777 | 0.000172249 | 0.000879489 |
| ENSG00000100154 | tetratricopeptide repeat domain 28(TTC28) | -0.676581603 | 0.170182437 | 7.02E-05 | 0.000398537 |
| ENSG00000155304 | heat shock protein family A (Hsp70) member 13(HSPA13) | -0.676442214 | 0.221043181 | 0.002211696 | 0.007932437 |
| ENSG00000164758 | mediator complex subunit 30(MED30) | -0.675497044 | 0.2024067 | 0.000845888 | 0.003469675 |
| ENSG00000130714 | protein O-mannosyltransferase 1(POMT1) | -0.675345775 | 0.176419664 | 0.000129155 | 0.000682561 |
| ENSG00000215375 | myosin light chain 5(MYL5) | -0.674727819 | 0.176742552 | 0.00013477 | 0.0007081 |
| ENSG00000197150 | ATP binding cassette subfamily B member 8(ABCB8) | -0.674688715 | 0.181284288 | 0.000197873 | 0.000990545 |
| ENSG00000171130 | ATPase H+ transporting V0 subunit e2(ATP6V0E2) | -0.674410357 | 0.164323824 | 4.06E-05 | 0.00024709 |
| ENSG00000133131 | MORC family CW-type zinc finger 4(MORC4) | -0.674356016 | 0.267877067 | 0.011822166 | 0.032441325 |
| ENSG00000157557 | ETS proto-oncogene 2, transcription factor(ETS2) | -0.672733366 | 0.146748189 | 4.56E-06 | 3.57E-05 |
| ENSG00000172292 | ceramide synthase 6(CERS6) | -0.672548046 | 0.237555323 | 0.004638506 | 0.014828093 |
| ENSG00000181035 | solute carrier family 25 member 42(SLC25A42) | -0.672519709 | 0.182662349 | 0.000231626 | 0.001135824 |
| ENSG00000156467 | ubiquinol-cytochrome c reductase binding protein(UQCRB) | -0.671966297 | 0.178490908 | 0.000166743 | 0.000855459 |
| ENSG00000168078 | PDZ binding kinase(PBK) | -0.671035512 | 0.273926813 | 0.014297963 | 0.038063191 |
| ENSG00000112249 | activating signal cointegrator 1 complex subunit 3(ASCC3) | -0.670722153 | 0.246064777 | 0.006414681 | 0.019464099 |
| ENSG00000161958 | fibroblast growth factor 11(FGF11) | -0.670144735 | 0.275268842 | 0.01491197 | 0.039492213 |
| ENSG00000159173 | troponin I1, slow skeletal type(TNNI1) | -0.669990446 | 0.231167622 | 0.003752054 | 0.012419572 |
| ENSG00000014641 | malate dehydrogenase 1(MDH1) | -0.669340366 | 0.221494519 | 0.002511707 | 0.008828123 |
| ENSG00000160284 | spermatogenesis and centriole associated 1-like(SPATC1L) | -0.669317179 | 0.242876138 | 0.005854944 | 0.017983696 |
| ENSG00000075275 | cadherin EGF LAG seven-pass G-type receptor 1(CELSR1) | -0.669276872 | 0.202000478 | 0.000922205 | 0.003739682 |
| ENSG00000118454 | ankyrin repeat domain 13C(ANKRD13C) | -0.669229804 | 0.234203342 | 0.004270285 | 0.013881082 |
| ENSG00000140319 | signal recognition particle 14(SRP14) | -0.669122752 | 0.155021204 | 1.59E-05 | 0.000107505 |
| ENSG00000168734 | protein kinase (cAMP-dependent, catalytic) inhibitor gamma(PKIG) | -0.669009263 | 0.173545411 | 0.000115752 | 0.000620928 |
| ENSG00000139428 | methylmalonic aciduria (cobalamin deficiency) cblB type(MMAB) | -0.668559399 | 0.164273294 | 4.71E-05 | 0.000280699 |
| ENSG00000108821 | collagen type I alpha 1 chain(COL1A1) | -0.668186497 | 0.205754869 | 0.001164285 | 0.00457012 |
| ENSG00000088930 | 5'-3' exoribonuclease 2(XRN2) | -0.667860394 | 0.253820977 | 0.00850774 | 0.024736791 |
| | SWI/SNF related, matrix associated, actin dependent regulator of chromatin, | | | | |
| ENSG00000080503 | subfamily a, member 2(SMARCA2) | -0.667695335 | 0.246633998 | 0.006784692 | 0.020418296 |
| ENSG00000206503 | major histocompatibility complex, class I, A(HLA-A) | -0.667693133 | 0.214291886 | 0.001834395 | 0.006745801 |
| ENSG00000114859 | chloride voltage-gated channel 2(CLCN2) | -0.666940912 | 0.171563244 | 0.000101309 | 0.000552527 |
| ENSG00000146555 | sidekick cell adhesion molecule 1(SDK1) | -0.66627197 | 0.188897751 | 0.000420048 | 0.001897637 |
| ENSG00000133069 | transmembrane and coiled-coil domain family 2(TMCC2) | -0.665424441 | 0.202871539 | 0.001037966 | 0.004130795 |
| ENSG00000112667 | 2'-deoxynucleoside 5'-phosphate N-hydrolase 1(DNPH1) | -0.664621592 | 0.200168339 | 0.000899165 | 0.003657599 |
| ENSG00000171703 | transcription elongation factor A2(TCEA2) | -0.664274903 | 0.162367981 | 4.29E-05 | 0.000259998 |
| ENSG00000141524 | transmembrane channel like 6(TMC6) | -0.6642277 | 0.227214784 | 0.003462902 | 0.011635982 |
| ENSG00000142784 | WD and tetratricopeptide repeats 1(WDTC1) | -0.664117388 | 0.148569077 | 7.82E-06 | 5.72E-05 |
| ENSG00000155816 | formin 2(FMN2) | -0.663574953 | 0.223541855 | 0.002992965 | 0.010268078 |
| ENSG00000185100 | adenylosuccinate synthase like 1(ADSSL1) | -0.662811786 | 0.239141772 | 0.005577691 | 0.017303788 |
| ENSG00000177674 | angiotensin II receptor associated protein(AGTRAP) | -0.662238988 | 0.178112219 | 0.000200727 | 0.001001422 |
| ENSG00000214021 | tubulin tyrosine ligase like 3(TTLL3) | -0.661935457 | 0.197590781 | 0.000808022 | 0.003327116 |
| ENSG00000213390 | Rho GTPase activating protein 19(ARHGAP19) | -0.661914499 | 0.279774025 | 0.0179868 | 0.046351963 |
| ENSG00000161277 | THAP domain containing 8(THAP8) | -0.661766135 | 0.233505074 | 0.004596148 | 0.014712675 |
| ENSG00000170989 | sphingosine-1-phosphate receptor 1(S1PR1) | -0.660978937 | 0.22891356 | 0.00388365 | 0.012790385 |
| ENSG00000171522 | prostaglandin E receptor 4(PTGER4) | -0.660501717 | 0.254593753 | 0.009477384 | 0.027052178 |
| ENSG00000131669 | ninjurin 1(NINJ1) | -0.660126553 | 0.156196856 | 2.38E-05 | 0.000153507 |
| ENSG00000188603 | CLN3, battenin(CLN3) | -0.65962672 | 0.154756572 | 2.02E-05 | 0.000133392 |
| ENSG00000141295 | secernin 2(SCRN2) | -0.658945501 | 0.173504541 | 0.000145952 | 0.000759055 |
| ENSG00000113108 | microRNA 6831(MIR6831) | -0.658015875 | 0.227054452 | 0.003754869 | 0.012421902 |
| ENSG00000187186 | uncharacterized LOC730098(LOC730098) | -0.657734301 | 0.199533905 | 0.000979487 | 0.003929973 |
| ENSG00000020181 | adhesion G protein-coupled receptor A2(ADGRA2) | -0.65771697 | 0.187532875 | 0.000452834 | 0.002024756 |
| ENSG00000166033 | HtrA serine peptidase 1(HTRA1) | -0.657342399 | 0.217547028 | 0.002514338 | 0.008834729 |
| ENSG00000121310 | enoyl-CoA hydratase domain containing 2(ECHDC2) | -0.657247609 | 0.260806401 | 0.011733505 | 0.032243156 |
| ENSG00000116962 | nidogen 1(NID1) | -0.65706415 | 0.148132652 | 9.18E-06 | 6.60E-05 |
| ENSG00000162604 | TM2 domain containing 1(TM2D1) | -0.657000203 | 0.193862439 | 0.000701475 | 0.00294825 |
| ENSG00000164050 | plexin B1(PLXNB1) | -0.656954754 | 0.180657561 | 0.000276406 | 0.001322881 |
| ENSG00000166920 | microRNA 147b(MIR147B) | -0.656849369 | 0.227568896 | 0.003897064 | 0.012823795 |
| ENSG00000118518 | ring finger protein 146(RNF146) | -0.656510853 | 0.279897779 | 0.018999646 | 0.048389659 |
| ENSG00000063660 | glypican 1(GPC1) | -0.656010625 | 0.221570525 | 0.003069103 | 0.010477134 |
| ENSG00000156381 | ankyrin repeat domain 9(ANKRD9) | -0.655924911 | 0.254743435 | 0.010028496 | 0.028298207 |
| ENSG00000167779 | insulin like growth factor binding protein 6(IGFBP6) | -0.65493881 | 0.16727885 | 9.03E-05 | 0.000499302 |
| ENSG00000075292 | zinc finger protein 638(ZNF638) | -0.654708785 | 0.206943873 | 0.001557761 | 0.005871676 |
| ENSG00000105856 | HMG-box transcription factor 1(HBP1) | -0.654605052 | 0.215180674 | 0.002349196 | 0.008349227 |
| ENSG00000079691 | capping protein regulator and myosin 1 linker 1(CARMIL1) | -0.654477411 | 0.229803157 | 0.004399612 | 0.014218783 |
| ENSG00000198517 | MAF bZIP transcription factor K(MAFK) | -0.654031465 | 0.245962828 | 0.007835755 | 0.023027683 |
| ENSG00000183336 | bolA family member 2(BOLA2) | -0.653950731 | 0.249146922 | 0.008671021 | 0.025112233 |
| ENSG00000111674 | enolase 2(ENO2) | -0.65379631 | 0.171179552 | 0.000133798 | 0.000703938 |
| ENSG00000211450 | selenoprotein H(SELENOH) | -0.653292429 | 0.183188686 | 0.000362158 | 0.001669445 |
| ENSG00000064545 | transmembrane protein 161A(TMEM161A) | -0.652528463 | 0.1961167 | 0.00087709 | 0.00357894 |
| ENSG00000214413 | BBSome interacting protein 1(BBIP1) | -0.652353913 | 0.2665799 | 0.014400152 | 0.038291906 |
| ENSG00000143622 | Ras like without CAAX 1(RIT1) | -0.652304391 | 0.236360451 | 0.005783914 | 0.017816697 |
| ENSG00000167182 | Sp2 transcription factor(SP2) | -0.652095855 | 0.153389528 | 2.13E-05 | 0.000139248 |
| ENSG00000115687 | PAS domain containing serine/threonine kinase(PASK) | -0.652044317 | 0.221336273 | 0.003219704 | 0.01091885 |
| ENSG00000101210 | eukaryotic translation elongation factor 1 alpha 2(EEF1A2) | -0.65169627 | 0.248387065 | 0.008697715 | 0.025170951 |
| ENSG00000197818 | solute carrier family 9 member A8(SLC9A8) | -0.651491873 | 0.160934508 | 5.16E-05 | 0.000305592 |
| ENSG00000177426 | TGFB induced factor homeobox 1(TGIF1) | -0.651020253 | 0.150514336 | 1.52E-05 | 0.000103819 |
| ENSG00000068305 | myocyte enhancer factor 2A(MEF2A) | -0.650976452 | 0.250137909 | 0.009255486 | 0.026498306 |
| ENSG00000255112 | charged multivesicular body protein 1B(CHMP1B) | -0.650574642 | 0.169729119 | 0.000126581 | 0.000671976 |
| ENSG00000172183 | interferon stimulated exonuclease gene 20(ISG20) | -0.650547899 | 0.219821827 | 0.00308206 | 0.010509379 |
| ENSG00000120458 | Myb/SANT DNA binding domain containing 2(MSANTD2) | -0.650516043 | 0.203393416 | 0.001382336 | 0.005299417 |
| ENSG00000150281 | cardiotrophin 1(CTF1) | -0.6503044 | 0.235264999 | 0.005707379 | 0.017645625 |
| ENSG00000121900 | transmembrane protein 54(TMEM54) | -0.650042885 | 0.167447658 | 0.000103573 | 0.000562785 |
| ENSG00000163281 | glucosamine-6-phosphate deaminase 2(GNPDA2) | -0.649636098 | 0.274494933 | 0.017949428 | 0.046265792 |
| ENSG00000160404 | torsin family 2 member A(TOR2A) | -0.649473244 | 0.196664021 | 0.000958439 | 0.003863975 |
| ENSG00000173230 | golgin B1(GOLGB1) | -0.649291365 | 0.185990183 | 0.00048122 | 0.002133033 |
| ENSG00000072954 | transmembrane protein 38A(TMEM38A) | -0.64907682 | 0.18830236 | 0.000566864 | 0.002451688 |
| ENSG00000178691 | SUZ12 polycomb repressive complex 2 subunit(SUZ12) | -0.648959705 | 0.241828581 | 0.007284453 | 0.021645441 |
| ENSG00000138032 | protein phosphatase, Mg2+/Mn2+ dependent 1B(PPM1B) | -0.648780067 | 0.223909787 | 0.003761419 | 0.012436576 |
| ENSG00000074657 | zinc finger protein 532(ZNF532) | -0.6487441 | 0.22528386 | 0.003980864 | 0.013052087 |
| ENSG00000162430 | selenoprotein N(SELENON) | -0.648329803 | 0.152776787 | 2.20E-05 | 0.000143413 |
| ENSG00000149489 | retinal outer segment membrane protein 1(ROM1) | -0.647485211 | 0.234446421 | 0.005749037 | 0.017732479 |
| ENSG00000106066 | carboxypeptidase, vitellogenic like(CPVL) | -0.647259367 | 0.217221713 | 0.002885142 | 0.009967933 |
| ENSG00000114784 | eukaryotic translation initiation factor 1B(EIF1B) | -0.646425418 | 0.165797484 | 9.66E-05 | 0.000529373 |
| ENSG00000025434 | nuclear receptor subfamily 1 group H member 3(NR1H3) | -0.645760954 | 0.168732725 | 0.00012965 | 0.000684559 |
| ENSG00000178695 | potassium channel tetramerization domain containing 12(KCTD12) | -0.645722287 | 0.235490281 | 0.006106008 | 0.018657422 |
| ENSG00000101773 | microRNA 4741(MIR4741) | -0.645630429 | 0.22789064 | 0.00461031 | 0.014749984 |
| ENSG00000115935 | WAS/WASL interacting protein family member 1(WIPF1) | -0.645575098 | 0.184707807 | 0.000473857 | 0.002104361 |
| ENSG00000135441 | biogenesis of lysosomal organelles complex 1 subunit 1(BLOC1S1) | -0.645255854 | 0.154892993 | 3.10E-05 | 0.000194441 |
| ENSG00000232112 | translation machinery associated 7 homolog(TMA7) | -0.645138637 | 0.160050652 | 5.56E-05 | 0.000326093 |
| ENSG00000258315 | chromosome 17 open reading frame 49(C17orf49) | -0.644868842 | 0.148655864 | 1.44E-05 | 9.84E-05 |
| ENSG00000152193 | ring finger protein 219(RNF219) | -0.64483726 | 0.259862372 | 0.013084655 | 0.035304561 |
| ENSG00000099797 | microRNA 639(MIR639) | -0.644423497 | 0.148988354 | 1.52E-05 | 0.000103819 |
| ENSG00000138326 | ribosomal protein S24(RPS24) | -0.644397107 | 0.194647444 | 0.000931008 | 0.003767589 |
| ENSG00000171940 | zinc finger protein 217(ZNF217) | -0.644300386 | 0.211178647 | 0.00228101 | 0.008148739 |
| ENSG00000105835 | nicotinamide phosphoribosyltransferase(NAMPT) | -0.643683468 | 0.212191984 | 0.002417382 | 0.008539963 |
| ENSG00000041357 | proteasome subunit alpha 4(PSMA4) | -0.642858859 | 0.203939034 | 0.001620392 | 0.006074661 |
| ENSG00000163945 | UV stimulated scaffold protein A(UVSSA) | -0.642660627 | 0.250611125 | 0.010336213 | 0.029061953 |
| ENSG00000160211 | glucose-6-phosphate dehydrogenase(G6PD) | -0.642548656 | 0.164099146 | 9.02E-05 | 0.000498974 |
| ENSG00000138686 | Bardet-Biedl syndrome 7(BBS7) | -0.641960936 | 0.226498902 | 0.004592931 | 0.014708262 |
| ENSG00000151694 | ADAM metallopeptidase domain 17(ADAM17) | -0.64179274 | 0.239085544 | 0.007266784 | 0.021609314 |
| ENSG00000170312 | cyclin dependent kinase 1(CDK1) | -0.641399113 | 0.247716193 | 0.009618528 | 0.027357921 |
| ENSG00000164663 | ubiquitin specific peptidase 49(USP49) | -0.641226015 | 0.252840375 | 0.011209783 | 0.031094528 |
| ENSG00000150457 | large tumor suppressor kinase 2(LATS2) | -0.641183233 | 0.163902806 | 9.15E-05 | 0.000505843 |
| ENSG00000087903 | regulatory factor X2(RFX2) | -0.640986855 | 0.188984974 | 0.000694517 | 0.002922138 |
| ENSG00000111077 | tensin 2(TNS2) | -0.640652004 | 0.163269227 | 8.71E-05 | 0.000484656 |
| ENSG00000149084 | hydroxysteroid 17-beta dehydrogenase 12(HSD17B12) | -0.640614812 | 0.272349079 | 0.018663602 | 0.047740511 |
| ENSG00000179348 | GATA binding protein 2(GATA2) | -0.639992971 | 0.194052294 | 0.000973609 | 0.003911728 |
| ENSG00000186522 | septin 10(SEPT10) | -0.6396749 | 0.224501605 | 0.004381402 | 0.014167899 |
| ENSG00000117054 | acyl-CoA dehydrogenase, C-4 to C-12 straight chain(ACADM) | -0.639512302 | 0.239928016 | 0.007688901 | 0.022666327 |
| ENSG00000141012 | galactosamine (N-acetyl)-6-sulfatase(GALNS) | -0.639171518 | 0.179427706 | 0.00036765 | 0.001687808 |
| ENSG00000177600 | ribosomal protein lateral stalk subunit P2(RPLP2) | -0.638863099 | 0.180297847 | 0.000395039 | 0.001792915 |
| ENSG00000233276 | glutathione peroxidase 1(GPX1) | -0.638646911 | 0.222257892 | 0.004060155 | 0.013282443 |
| ENSG00000101400 | syntrophin alpha 1(SNTA1) | -0.638564204 | 0.162913765 | 8.87E-05 | 0.000492319 |
| ENSG00000185222 | transcription elongation factor A like 9(TCEAL9) | -0.638539943 | 0.199754544 | 0.001390466 | 0.005326422 |
| ENSG00000173156 | ras homolog family member D(RHOD) | -0.638469721 | 0.18141567 | 0.000432565 | 0.001944478 |
| ENSG00000158427 | thymosin beta 15B(TMSB15B) | -0.638182652 | 0.238092296 | 0.007353417 | 0.021828312 |
| ENSG00000105968 | H2A histone family member V(H2AFV) | -0.637490501 | 0.142007041 | 7.15E-06 | 5.31E-05 |
| ENSG00000158615 | protein phosphatase 1 regulatory subunit 15B(PPP1R15B) | -0.636957671 | 0.202646529 | 0.001671141 | 0.00623709 |
| ENSG00000239521 | GATS, stromal antigen 3 opposite strand(GATS) | -0.636691937 | 0.192985865 | 0.000969734 | 0.003900153 |
| ENSG00000169951 | zinc finger protein 764(ZNF764) | -0.636545178 | 0.216655127 | 0.00330276 | 0.011168056 |
| ENSG00000023318 | endoplasmic reticulum protein 44(ERP44) | -0.63654269 | 0.204808327 | 0.00188363 | 0.006910759 |
| ENSG00000164919 | cytochrome c oxidase subunit 6C(COX6C) | -0.63604132 | 0.167927758 | 0.000152116 | 0.000786937 |
| ENSG00000063245 | epsin 1(EPN1) | -0.636027151 | 0.20438143 | 0.001858487 | 0.006827995 |
| ENSG00000166881 | nuclear envelope integral membrane protein 1(NEMP1) | -0.635885622 | 0.205309321 | 0.001953529 | 0.007123781 |
| ENSG00000011566 | mitogen-activated protein kinase kinase kinase kinase 3(MAP4K3) | -0.635409986 | 0.241636424 | 0.008548243 | 0.024818665 |
| ENSG00000185883 | ATPase H+ transporting V0 subunit c(ATP6V0C) | -0.634369896 | 0.180192794 | 0.000430723 | 0.001937679 |
| ENSG00000101752 | mindbomb E3 ubiquitin protein ligase 1(MIB1) | -0.632965096 | 0.217235895 | 0.003571473 | 0.011930514 |
| ENSG00000130592 | microRNA 7847(MIR7847) | -0.63217559 | 0.235220627 | 0.007197063 | 0.02142907 |
| ENSG00000116586 | late endosomal/lysosomal adaptor, MAPK and MTOR activator 2(LAMTOR2) | -0.63175396 | 0.189722092 | 0.000868799 | 0.003548801 |
| ENSG00000179750 | apolipoprotein B mRNA editing enzyme catalytic subunit 3B(APOBEC3B) | -0.631122317 | 0.183118924 | 0.000567865 | 0.002455112 |
| ENSG00000117298 | endothelin converting enzyme 1(ECE1) | -0.630270055 | 0.175774374 | 0.000336205 | 0.001563907 |
| ENSG00000160867 | fibroblast growth factor receptor 4(FGFR4) | -0.629238805 | 0.197601116 | 0.0014506 | 0.005511904 |
| ENSG00000182903 | zinc finger protein 721(ZNF721) | -0.628027519 | 0.21612376 | 0.003662337 | 0.012187762 |
| ENSG00000172893 | 7-dehydrocholesterol reductase(DHCR7) | -0.627666305 | 0.155374403 | 5.35E-05 | 0.000315905 |
| ENSG00000058063 | ATPase phospholipid transporting 11B (putative)(ATP11B) | -0.627129686 | 0.248541882 | 0.011628052 | 0.031968311 |
| ENSG00000169062 | UPF3 regulator of nonsense transcripts homolog A (yeast)(UPF3A) | -0.627129429 | 0.190388669 | 0.000987925 | 0.003958431 |
| ENSG00000160932 | lymphocyte antigen 6 complex, locus E(LY6E) | -0.626728818 | 0.165333449 | 0.000150226 | 0.000778184 |
| ENSG00000077454 | leucine rich repeats and calponin homology domain containing 4(LRCH4) | -0.626084802 | 0.193722551 | 0.001229859 | 0.004784411 |
| ENSG00000141959 | phosphofructokinase, liver type(PFKL) | -0.625529716 | 0.191962817 | 0.001119641 | 0.004418468 |
| ENSG00000168938 | peptidylprolyl isomerase C(PPIC) | -0.625173793 | 0.174270961 | 0.000334035 | 0.00155504 |
| ENSG00000099203 | transmembrane p24 trafficking protein 1(TMED1) | -0.624674268 | 0.184477816 | 0.00070874 | 0.002972414 |
| ENSG00000163001 | cilia and flagella associated protein 36(CFAP36) | -0.624016811 | 0.219723908 | 0.004511295 | 0.014504201 |
| ENSG00000138101 | dystrobrevin beta(DTNB) | -0.623463885 | 0.166371614 | 0.000178665 | 0.000907518 |
| ENSG00000171155 | C1GALT1 specific chaperone 1(C1GALT1C1) | -0.622955613 | 0.222594778 | 0.005132334 | 0.016117666 |
| ENSG00000108379 | uncharacterized LOC101929777(LOC101929777) | -0.62294385 | 0.235527798 | 0.008171878 | 0.023890152 |
| ENSG00000163815 | C-type lectin domain family 3 member B(CLEC3B) | -0.62172309 | 0.235087348 | 0.008177614 | 0.023900981 |
| ENSG00000163956 | LDL receptor related protein associated protein 1(LRPAP1) | -0.62126941 | 0.177434009 | 0.000462801 | 0.00206149 |
| ENSG00000183337 | BCL6 corepressor(BCOR) | -0.621147367 | 0.169303509 | 0.000243654 | 0.001183952 |
| ENSG00000169379 | ADP ribosylation factor like GTPase 13B(ARL13B) | -0.620854091 | 0.245879105 | 0.011568577 | 0.031856915 |
| ENSG00000104805 | nucleobindin 1(NUCB1) | -0.620752031 | 0.207317432 | 0.00275156 | 0.0095739 |
| ENSG00000146904 | EPH receptor A1(EPHA1) | -0.620580358 | 0.253016837 | 0.014177925 | 0.037837804 |
| ENSG00000167566 | NCK associated protein 5 like(NCKAP5L) | -0.620415487 | 0.203285452 | 0.002273657 | 0.008124941 |
| ENSG00000075790 | B-cell receptor associated protein 29(BCAP29) | -0.620390472 | 0.204138202 | 0.00237309 | 0.008416349 |
| ENSG00000221890 | neuronal pentraxin receptor(NPTXR) | -0.619284671 | 0.167640816 | 0.000220653 | 0.001089282 |
| ENSG00000166341 | dachsous cadherin-related 1(DCHS1) | -0.618835193 | 0.219948959 | 0.004899929 | 0.015495367 |
| ENSG00000183648 | NADH:ubiquinone oxidoreductase subunit B1(NDUFB1) | -0.618397985 | 0.208929169 | 0.003077937 | 0.010498363 |
| ENSG00000125398 | SRY-box 9(SOX9) | -0.61834364 | 0.203201921 | 0.002342316 | 0.008332327 |
| ENSG00000153914 | splicing regulatory glutamic acid and lysine rich protein 1(SREK1) | -0.618122717 | 0.246432881 | 0.012132042 | 0.033147673 |
| ENSG00000106268 | nudix hydrolase 1(NUDT1) | -0.617969975 | 0.175483566 | 0.00042907 | 0.001931719 |
| ENSG00000132207 | SLX1 homolog A, structure-specific endonuclease subunit(SLX1A) | -0.617898973 | 0.214955796 | 0.004046169 | 0.01324037 |
| ENSG00000086666 | zinc finger AN1-type containing 6(ZFAND6) | -0.616945533 | 0.218460753 | 0.004742001 | 0.015085154 |
| ENSG00000104325 | 2,4-dienoyl-CoA reductase 1, mitochondrial(DECR1) | -0.616861455 | 0.258011323 | 0.016810291 | 0.043751386 |
| ENSG00000130827 | plexin A3(PLXNA3) | -0.615795889 | 0.201203786 | 0.002209249 | 0.007926075 |
| ENSG00000099999 | ring finger protein 215(RNF215) | -0.615421317 | 0.172208633 | 0.000351977 | 0.001629541 |
| ENSG00000115268 | ribosomal protein S15(RPS15) | -0.614872529 | 0.167276056 | 0.00023711 | 0.00115789 |
| ENSG00000119912 | insulin degrading enzyme(IDE) | -0.61472466 | 0.262494816 | 0.019188195 | 0.048785374 |
| ENSG00000169967 | mitogen-activated protein kinase kinase kinase 2(MAP3K2) | -0.614590956 | 0.227042015 | 0.006790487 | 0.020420063 |
| ENSG00000121716 | microRNA 6840(MIR6840) | -0.613970735 | 0.240830449 | 0.010791155 | 0.030168049 |
| ENSG00000167964 | RAB26, member RAS oncogene family(RAB26) | -0.613882717 | 0.211322635 | 0.003673061 | 0.012216535 |
| ENSG00000163795 | zinc finger protein 513(ZNF513) | -0.613663372 | 0.204414737 | 0.002681676 | 0.009353441 |
| ENSG00000137413 | TATA-box binding protein associated factor 8(TAF8) | -0.612661404 | 0.166860577 | 0.000240946 | 0.001172729 |
| ENSG00000033800 | protein inhibitor of activated STAT 1(PIAS1) | -0.612530054 | 0.23476891 | 0.009078693 | 0.026049207 |
| ENSG00000063854 | hydroxyacylglutathione hydrolase(HAGH) | -0.611482882 | 0.175479693 | 0.000492807 | 0.002176193 |
| ENSG00000197249 | serpin family A member 1(SERPINA1) | -0.6111485 | 0.174911338 | 0.000475757 | 0.002111173 |
| ENSG00000141504 | spermidine/spermine N1-acetyltransferase family member 2(SAT2) | -0.610500538 | 0.178010073 | 0.000604507 | 0.002596402 |
| ENSG00000166710 | beta-2-microglobulin(B2M) | -0.610452318 | 0.184882741 | 0.000960545 | 0.003871139 |
| ENSG00000137133 | histidine triad nucleotide binding protein 2(HINT2) | -0.610295972 | 0.170837048 | 0.000353743 | 0.001637069 |
| ENSG00000166340 | tripeptidyl peptidase 1(TPP1) | -0.610220987 | 0.158223036 | 0.000114922 | 0.000617323 |
| ENSG00000134569 | LDL receptor related protein 4(LRP4) | -0.610150417 | 0.193535281 | 0.001617916 | 0.006070635 |
| ENSG00000006016 | cytokine receptor like factor 1(CRLF1) | -0.610042234 | 0.168726159 | 0.000299681 | 0.001416979 |
| ENSG00000105379 | electron transfer flavoprotein beta subunit(ETFB) | -0.609956351 | 0.180291229 | 0.000716536 | 0.003000828 |
| ENSG00000130830 | membrane palmitoylated protein 1(MPP1) | -0.609671446 | 0.209152785 | 0.003557372 | 0.011892276 |
| ENSG00000072274 | transferrin receptor(TFRC) | -0.609558233 | 0.253582831 | 0.016226379 | 0.042400515 |
| ENSG00000129559 | neural precursor cell expressed, developmentally down-regulated 8(NEDD8) | -0.609280841 | 0.165905427 | 0.000240228 | 0.001170828 |
| ENSG00000183011 | N(alpha)-acetyltransferase 38, NatC auxiliary subunit(NAA38) | -0.60906524 | 0.174782795 | 0.000492693 | 0.002176193 |
| ENSG00000183762 | kringle containing transmembrane protein 1(KREMEN1) | -0.608886905 | 0.209332716 | 0.003629303 | 0.012094936 |
| ENSG00000131374 | TBC1 domain family member 5(TBC1D5) | -0.608867299 | 0.206344002 | 0.003170147 | 0.010766904 |
| ENSG00000026297 | ribonuclease T2(RNASET2) | -0.608793781 | 0.20932191 | 0.003632726 | 0.012102913 |
| ENSG00000067836 | rogdi homolog(ROGDI) | -0.608579495 | 0.163168093 | 0.000191654 | 0.000965577 |
| ENSG00000111231 | GPN-loop GTPase 3(GPN3) | -0.607852474 | 0.202213433 | 0.002647138 | 0.009250859 |
| ENSG00000125356 | NADH:ubiquinone oxidoreductase subunit A1(NDUFA1) | -0.607697642 | 0.187297767 | 0.001176349 | 0.004609782 |
| ENSG00000104823 | enoyl-CoA hydratase 1(ECH1) | -0.606938799 | 0.162982014 | 0.000196121 | 0.000984284 |
| ENSG00000171962 | dynein regulatory complex subunit 3(DRC3) | -0.606441098 | 0.214538329 | 0.004702653 | 0.015006064 |
| ENSG00000174483 | Bardet-Biedl syndrome 1(BBS1) | -0.605903129 | 0.165057844 | 0.000241742 | 0.001176118 |
| ENSG00000071242 | ribosomal protein S6 kinase A2(RPS6KA2) | -0.605734002 | 0.168236099 | 0.000317606 | 0.001487981 |
| ENSG00000136636 | potassium channel tetramerization domain containing 3(KCTD3) | -0.605649527 | 0.234770637 | 0.009887189 | 0.027966553 |
| ENSG00000172638 | EGF containing fibulin like extracellular matrix protein 2(EFEMP2) | -0.604998567 | 0.1722125 | 0.000442922 | 0.001985722 |
| ENSG00000164405 | ubiquinol-cytochrome c reductase complex III subunit VII(UQCRQ) | -0.604917716 | 0.18408756 | 0.001016093 | 0.004057473 |
| ENSG00000140948 | zinc finger CCHC-type containing 14(ZCCHC14) | -0.604768712 | 0.178330974 | 0.000695692 | 0.002926035 |
| ENSG00000125898 | family with sequence similarity 110 member A(FAM110A) | -0.604440248 | 0.218294442 | 0.005624213 | 0.017420547 |
| ENSG00000167619 | transmembrane protein 145(TMEM145) | -0.604321395 | 0.221480999 | 0.006361396 | 0.019317361 |
| ENSG00000161896 | inositol hexakisphosphate kinase 3(IP6K3) | -0.604216072 | 0.17388075 | 0.000511067 | 0.00224671 |
| ENSG00000128609 | NADH:ubiquinone oxidoreductase subunit A5(NDUFA5) | -0.603703244 | 0.199578537 | 0.002487231 | 0.008755171 |
| ENSG00000177311 | zinc finger and BTB domain containing 38(ZBTB38) | -0.603643564 | 0.218791786 | 0.005798095 | 0.01785103 |
| ENSG00000134186 | pre-mRNA processing factor 38B(PRPF38B) | -0.603397925 | 0.230619887 | 0.008885782 | 0.025626952 |
| ENSG00000166801 | family with sequence similarity 111 member A(FAM111A) | -0.603182532 | 0.221153914 | 0.006382918 | 0.019377715 |
| ENSG00000132196 | hydroxysteroid 17-beta dehydrogenase 7(HSD17B7) | -0.602839373 | 0.215923076 | 0.005239675 | 0.016410951 |
| ENSG00000214022 | replication initiator 1(REPIN1) | -0.602544808 | 0.172286684 | 0.000469926 | 0.002088479 |
| ENSG00000135919 | serpin family E member 2(SERPINE2) | -0.602002929 | 0.142817311 | 2.50E-05 | 0.000160604 |
| ENSG00000216490 | IFI30, lysosomal thiol reductase(IFI30) | -0.601200888 | 0.192219966 | 0.001761971 | 0.006524338 |
| ENSG00000168000 | BSCL2, seipin lipid droplet biogenesis associated(BSCL2) | -0.600612228 | 0.178795884 | 0.000781667 | 0.003230172 |
| ENSG00000113161 | 3-hydroxy-3-methylglutaryl-CoA reductase(HMGCR) | -0.600497215 | 0.203509083 | 0.003170398 | 0.010766904 |
| ENSG00000064651 | solute carrier family 12 member 2(SLC12A2) | -0.600180391 | 0.244337578 | 0.014035354 | 0.037533963 |
| ENSG00000115041 | potassium voltage-gated channel interacting protein 3(KCNIP3) | -0.599957652 | 0.218428534 | 0.006019817 | 0.018442712 |
| ENSG00000171984 | chromosome 20 open reading frame 196(C20orf196) | -0.59965831 | 0.230971305 | 0.009424858 | 0.026943871 |
| ENSG00000135677 | glucosamine (N-acetyl)-6-sulfatase(GNS) | -0.599512702 | 0.150490384 | 6.78E-05 | 0.000387239 |
| ENSG00000033178 | ubiquitin like modifier activating enzyme 6(UBA6) | -0.599000066 | 0.229485054 | 0.00904914 | 0.025989767 |
| ENSG00000130332 | LSM7 homolog, U6 small nuclear RNA and mRNA degradation associated(LSM7) | -0.59876138 | 0.158605706 | 0.000159907 | 0.000821821 |
| ENSG00000130707 | argininosuccinate synthase 1(ASS1) | -0.598436877 | 0.166199175 | 0.000317336 | 0.001487306 |
| ENSG00000013563 | deoxyribonuclease 1 like 1(DNASE1L1) | -0.59772675 | 0.190291459 | 0.001683078 | 0.006269704 |
| ENSG00000104964 | amino-terminal enhancer of split(AES) | -0.597046188 | 0.156782973 | 0.000140035 | 0.000732166 |
| ENSG00000170248 | programmed cell death 6 interacting protein(PDCD6IP) | -0.596660254 | 0.209414604 | 0.004383184 | 0.014169766 |
| ENSG00000021826 | carbamoyl-phosphate synthase 1(CPS1) | -0.596344103 | 0.239606034 | 0.012815608 | 0.034729973 |
| ENSG00000091136 | laminin subunit beta 1(LAMB1) | -0.595290851 | 0.169279532 | 0.000437088 | 0.00196181 |
| ENSG00000112984 | kinesin family member 20A(KIF20A) | -0.595076599 | 0.180352175 | 0.000968484 | 0.00389715 |
| ENSG00000104904 | ornithine decarboxylase antizyme 1(OAZ1) | -0.592825412 | 0.175663127 | 0.000738732 | 0.003080625 |
| ENSG00000103202 | NME/NM23 nucleoside diphosphate kinase 4(NME4) | -0.592047753 | 0.164049287 | 0.000307424 | 0.001447772 |
| ENSG00000127184 | microRNA 3607(MIR3607) | -0.591996318 | 0.201487965 | 0.003302066 | 0.011168056 |
| ENSG00000139438 | family with sequence similarity 222 member A(FAM222A) | -0.591972222 | 0.190666469 | 0.001904382 | 0.006977001 |
| ENSG00000136021 | SCY1 like pseudokinase 2(SCYL2) | -0.591886578 | 0.236789088 | 0.01243209 | 0.033837267 |
| ENSG00000162694 | exostosin like glycosyltransferase 2(EXTL2) | -0.591637056 | 0.238132339 | 0.01297377 | 0.035067992 |
| ENSG00000141401 | inositol monophosphatase 2(IMPA2) | -0.591611396 | 0.191265496 | 0.001980491 | 0.007208695 |
| ENSG00000132424 | PNN interacting serine and arginine rich protein(PNISR) | -0.591309843 | 0.196586587 | 0.002630727 | 0.009199723 |
| ENSG00000119711 | aldehyde dehydrogenase 6 family member A1(ALDH6A1) | -0.591017657 | 0.187842103 | 0.001653201 | 0.006174048 |
| ENSG00000196544 | BLOC-1 related complex subunit 6(BORCS6) | -0.590951491 | 0.235677136 | 0.012160161 | 0.033206161 |
| ENSG00000109103 | unc-119 lipid binding chaperone(UNC119) | -0.589755335 | 0.15653512 | 0.000164851 | 0.000846122 |
| ENSG00000160471 | cytochrome c oxidase subunit 6B2(COX6B2) | -0.589585113 | 0.186581738 | 0.001578074 | 0.005936828 |
| ENSG00000146067 | family with sequence similarity 193 member B(FAM193B) | -0.589461496 | 0.178982869 | 0.000989849 | 0.003964788 |
| ENSG00000128791 | twisted gastrulation BMP signaling modulator 1(TWSG1) | -0.588699731 | 0.22377925 | 0.008520512 | 0.024761685 |
| ENSG00000164611 | pituitary tumor-transforming 1(PTTG1) | -0.588613921 | 0.158299829 | 0.00020053 | 0.001000861 |
| ENSG00000226763 | serine/arginine repetitive matrix 5(SRRM5) | -0.588605725 | 0.206577438 | 0.004381246 | 0.014167899 |
| ENSG00000197119 | solute carrier family 25 member 29(SLC25A29) | -0.588223553 | 0.165804844 | 0.000388631 | 0.001769979 |
| ENSG00000135317 | sorting nexin 14(SNX14) | -0.5879329 | 0.24615212 | 0.016917577 | 0.043952822 |
| ENSG00000115355 | coiled-coil domain containing 88A(CCDC88A) | -0.587739639 | 0.242612341 | 0.01541216 | 0.040571497 |
| ENSG00000136114 | thrombospondin type 1 domain containing 1(THSD1) | -0.587345524 | 0.186620935 | 0.001648056 | 0.006158744 |
| ENSG00000155329 | zinc finger CCHC-type containing 10(ZCCHC10) | -0.587320722 | 0.23628891 | 0.01293317 | 0.034983306 |
| ENSG00000178882 | filamin-interacting protein FAM101A(ZNF664-FAM101A) | -0.586668469 | 0.170327794 | 0.000572435 | 0.002473141 |
| ENSG00000102931 | ADP ribosylation factor like GTPase 2 binding protein(ARL2BP) | -0.586610682 | 0.160487487 | 0.000257001 | 0.001238576 |
| ENSG00000181513 | acyl-CoA binding domain containing 4(ACBD4) | -0.58658103 | 0.179901322 | 0.001111881 | 0.004395219 |
| ENSG00000229833 | PET100 homolog(PET100) | -0.58619679 | 0.195266665 | 0.002681839 | 0.009353441 |
| ENSG00000161921 | C-X-C motif chemokine ligand 16(CXCL16) | -0.585945763 | 0.165028909 | 0.000384406 | 0.001752773 |
| ENSG00000196689 | transient receptor potential cation channel subfamily V member 1(TRPV1) | -0.585236559 | 0.247455263 | 0.018029114 | 0.046430495 |
| ENSG00000149547 | EI24, autophagy associated transmembrane protein(EI24) | -0.585005407 | 0.230046442 | 0.010990892 | 0.030624549 |
| ENSG00000088826 | spermine oxidase(SMOX) | -0.583882606 | 0.172105691 | 0.000692373 | 0.00291416 |
| ENSG00000049249 | TNF receptor superfamily member 9(TNFRSF9) | -0.583760226 | 0.20796219 | 0.004999751 | 0.015768651 |
| ENSG00000163527 | STT3B, catalytic subunit of the oligosaccharyltransferase complex(STT3B) | -0.5834321 | 0.221408894 | 0.008411527 | 0.024475193 |
| ENSG00000140400 | mannosidase alpha class 2C member 1(MAN2C1) | -0.583308157 | 0.16498833 | 0.00040708 | 0.001843297 |
| ENSG00000254999 | BRICK1, SCAR/WAVE actin nucleating complex subunit(BRK1) | -0.582893328 | 0.174008999 | 0.000808733 | 0.003327993 |
| ENSG00000132780 | nuclear autoantigenic sperm protein(NASP) | -0.582713068 | 0.18627429 | 0.001758489 | 0.006517599 |
| ENSG00000137509 | prolylcarboxypeptidase(PRCP) | -0.582018843 | 0.173109555 | 0.000773404 | 0.003200257 |
| ENSG00000155957 | transmembrane BAX inhibitor motif containing 4(TMBIM4) | -0.581873196 | 0.180830827 | 0.001291884 | 0.004991043 |
| ENSG00000167740 | cytochrome b5 domain containing 2(CYB5D2) | -0.581804991 | 0.213625749 | 0.006459855 | 0.019579146 |
| ENSG00000184481 | forkhead box O4(FOXO4) | -0.581494578 | 0.213615127 | 0.006485671 | 0.019637326 |
| ENSG00000184584 | transmembrane protein 173(TMEM173) | -0.581365743 | 0.160195175 | 0.000284401 | 0.001354533 |
| ENSG00000167081 | PBX homeobox 3(PBX3) | -0.581116825 | 0.186423144 | 0.001825823 | 0.00671638 |
| ENSG00000197223 | C1D nuclear receptor corepressor(C1D) | -0.580047338 | 0.201078575 | 0.003918117 | 0.012882262 |
| ENSG00000100097 | galectin 1(LGALS1) | -0.580012399 | 0.162705503 | 0.000364135 | 0.001677246 |
| ENSG00000172725 | coronin 1B(CORO1B) | -0.579230434 | 0.206944877 | 0.005126749 | 0.016107529 |
| ENSG00000107984 | dickkopf WNT signaling pathway inhibitor 1(DKK1) | -0.578961495 | 0.179876212 | 0.001287883 | 0.004977219 |
| ENSG00000137770 | CTD small phosphatase like 2(CTDSPL2) | -0.578719323 | 0.228423642 | 0.011291842 | 0.031270534 |
| ENSG00000132561 | matrilin 2(MATN2) | -0.57781878 | 0.195399219 | 0.003105281 | 0.010573233 |
| ENSG00000102312 | porcupine homolog (Drosophila)(PORCN) | -0.577765946 | 0.173809138 | 0.000886916 | 0.003616526 |
| ENSG00000063176 | sphingosine kinase 2(SPHK2) | -0.577611121 | 0.225336441 | 0.010367417 | 0.029128802 |
| ENSG00000136367 | zinc finger homeobox 2(ZFHX2) | -0.5773895 | 0.236324085 | 0.014557242 | 0.038648474 |
| ENSG00000163126 | ankyrin repeat domain 23(ANKRD23) | -0.577321343 | 0.222795974 | 0.00956264 | 0.027251672 |
| ENSG00000213442 | NA | -0.577152991 | 0.212534846 | 0.006616203 | 0.019983031 |
| ENSG00000187535 | intraflagellar transport 140(IFT140) | -0.576610411 | 0.177554496 | 0.001164186 | 0.00457012 |
| ENSG00000063180 | carbonic anhydrase 11(CA11) | -0.576516293 | 0.173513601 | 0.000891827 | 0.003631517 |
| ENSG00000166228 | pterin-4 alpha-carbinolamine dehydratase 1(PCBD1) | -0.576454777 | 0.198789439 | 0.003733699 | 0.012369258 |
| ENSG00000048471 | sorting nexin 29(SNX29) | -0.57572652 | 0.20077769 | 0.004137516 | 0.013490505 |
| ENSG00000141741 | migration and invasion enhancer 1(MIEN1) | -0.575661687 | 0.159650055 | 0.000311226 | 0.001462167 |
| ENSG00000006740 | Rho GTPase activating protein 44(ARHGAP44) | -0.575273228 | 0.207320236 | 0.005523481 | 0.017167194 |
| ENSG00000087111 | phosphatidylinositol glycan anchor biosynthesis class S(PIGS) | -0.574926387 | 0.180388187 | 0.001436762 | 0.005469927 |
| ENSG00000125378 | bone morphogenetic protein 4(BMP4) | -0.574812623 | 0.200677897 | 0.004178602 | 0.013609377 |
| ENSG00000146247 | pleckstrin homology domain interacting protein(PHIP) | -0.574029245 | 0.214919136 | 0.007564637 | 0.022365014 |
| ENSG00000099204 | actin binding LIM protein 1(ABLIM1) | -0.573849091 | 0.195881477 | 0.00339428 | 0.01144463 |
| ENSG00000021645 | neurexin 3(NRXN3) | -0.573684673 | 0.217633547 | 0.008388673 | 0.024420774 |
| ENSG00000101439 | cystatin C(CST3) | -0.573580207 | 0.154453954 | 0.000204332 | 0.001017679 |
| ENSG00000167645 | Yip1 interacting factor homolog B, membrane trafficking protein(YIF1B) | -0.572551836 | 0.170922971 | 0.000808801 | 0.003327993 |
| ENSG00000166313 | amyloid beta precursor protein binding family B member 1(APBB1) | -0.572054851 | 0.164143225 | 0.000491961 | 0.002174091 |
| ENSG00000180304 | ornithine decarboxylase antizyme 2(OAZ2) | -0.570823955 | 0.174523359 | 0.00107259 | 0.004257069 |
| ENSG00000111801 | butyrophilin subfamily 3 member A3(BTN3A3) | -0.57057384 | 0.201757293 | 0.004683675 | 0.014953878 |
| ENSG00000213625 | leptin receptor overlapping transcript(LEPROT) | -0.570360577 | 0.189083418 | 0.002557538 | 0.008967774 |
| ENSG00000248487 | abhydrolase domain containing 14A(ABHD14A) | -0.570357896 | 0.146022492 | 9.39E-05 | 0.000516407 |
| ENSG00000103978 | transmembrane protein 87A(TMEM87A) | -0.56978288 | 0.225438347 | 0.011489603 | 0.031682537 |
| ENSG00000145882 | prenylcysteine oxidase 1 like(PCYOX1L) | -0.569671822 | 0.177717964 | 0.001348362 | 0.00519049 |
| ENSG00000117868 | extended synaptotagmin 2(ESYT2) | -0.568848117 | 0.163586539 | 0.000506391 | 0.002227817 |
| ENSG00000121851 | RNA polymerase III subunit G like(POLR3GL) | -0.568716809 | 0.204712926 | 0.005467464 | 0.017015674 |
| ENSG00000171858 | ribosomal protein S21(RPS21) | -0.568453756 | 0.188025085 | 0.002500452 | 0.008793815 |
| ENSG00000105173 | cyclin E1(CCNE1) | -0.567492788 | 0.206769541 | 0.006059085 | 0.018541287 |
| ENSG00000113583 | chromosome 5 open reading frame 15(C5orf15) | -0.567296996 | 0.15125147 | 0.00017635 | 0.0008977 |
| ENSG00000159214 | coiled-coil domain containing 24(CCDC24) | -0.567010575 | 0.180438551 | 0.001675674 | 0.006250043 |
| ENSG00000113558 | S-phase kinase associated protein 1(SKP1) | -0.566899555 | 0.159846539 | 0.000390349 | 0.001775743 |
| ENSG00000163702 | interleukin 17 receptor C(IL17RC) | -0.566840161 | 0.153270531 | 0.00021705 | 0.001074651 |
| ENSG00000116857 | transmembrane protein 9(TMEM9) | -0.566269705 | 0.143595432 | 8.03E-05 | 0.000450682 |
| ENSG00000178038 | ALS2 C-terminal like(ALS2CL) | -0.566240587 | 0.201490967 | 0.004950257 | 0.015633511 |
| ENSG00000132824 | serine incorporator 3(SERINC3) | -0.566179976 | 0.177171488 | 0.00139511 | 0.005337261 |
| ENSG00000173786 | 2',3'-cyclic nucleotide 3' phosphodiesterase(CNP) | -0.565788295 | 0.175304738 | 0.001248963 | 0.004852313 |
| ENSG00000117262 | G protein-coupled receptor 89A(GPR89A) | -0.565746592 | 0.209244158 | 0.006855919 | 0.02059051 |
| ENSG00000183291 | selenoprotein F(SELENOF) | -0.56540916 | 0.200886754 | 0.004884305 | 0.015454266 |
| ENSG00000060237 | WNK lysine deficient protein kinase 1(WNK1) | -0.565335314 | 0.181817533 | 0.001874961 | 0.006882072 |
| ENSG00000138600 | signal peptide peptidase like 2A(SPPL2A) | -0.565309023 | 0.223694309 | 0.011499245 | 0.031682537 |
| ENSG00000173486 | FK506 binding protein 2(FKBP2) | -0.564989206 | 0.198738844 | 0.004470894 | 0.01439792 |
| ENSG00000091317 | CKLF like MARVEL transmembrane domain containing 6(CMTM6) | -0.564610567 | 0.240154107 | 0.018721303 | 0.04785689 |
| ENSG00000111775 | cytochrome c oxidase subunit 6A1(COX6A1) | -0.56437709 | 0.160501496 | 0.000437548 | 0.001963125 |
| ENSG00000105887 | myotrophin(MTPN) | -0.564282083 | 0.211282795 | 0.007568353 | 0.022370378 |
| ENSG00000177954 | ribosomal protein S27(RPS27) | -0.563836598 | 0.191095546 | 0.003172107 | 0.010769599 |
| ENSG00000173264 | G protein-coupled receptor 137(GPR137) | -0.56331564 | 0.169511164 | 0.000889984 | 0.00362661 |
| ENSG00000055950 | mitochondrial ribosomal protein L43(MRPL43) | -0.563268122 | 0.190437727 | 0.003098884 | 0.010556321 |
| ENSG00000197006 | methyltransferase like 9(METTL9) | -0.563174937 | 0.159780021 | 0.00042398 | 0.001913194 |
| ENSG00000179912 | R3H domain containing 2(R3HDM2) | -0.562829108 | 0.166119753 | 0.000703805 | 0.002954876 |
| ENSG00000177042 | transmembrane protein 80(TMEM80) | -0.562342604 | 0.21041284 | 0.007527314 | 0.022280987 |
| ENSG00000172340 | succinate-CoA ligase GDP-forming beta subunit(SUCLG2) | -0.561954093 | 0.222626997 | 0.011596338 | 0.031925887 |
| ENSG00000136997 | v-myc avian myelocytomatosis viral oncogene homolog(MYC) | -0.561499944 | 0.154439582 | 0.000277199 | 0.001325598 |
| ENSG00000215712 | transmembrane protein 242(TMEM242) | -0.561417417 | 0.221158929 | 0.011132093 | 0.030930075 |
| ENSG00000168763 | cyclin and CBS domain divalent metal cation transport mediator 3(CNNM3) | -0.560582068 | 0.169844752 | 0.000964935 | 0.00388484 |
| ENSG00000164855 | transmembrane protein 184A(TMEM184A) | -0.560354628 | 0.229014966 | 0.014412938 | 0.038317244 |
| ENSG00000162444 | retinol binding protein 7(RBP7) | -0.559590388 | 0.19778724 | 0.004665662 | 0.014902754 |
| ENSG00000168101 | nudix hydrolase 16 like 1(NUDT16L1) | -0.559339117 | 0.15950985 | 0.000453851 | 0.002026807 |
| ENSG00000145284 | stearoyl-CoA desaturase 5(SCD5) | -0.559245426 | 0.160415999 | 0.000489898 | 0.002167418 |
| ENSG00000158104 | 4-hydroxyphenylpyruvate dioxygenase(HPD) | -0.559236125 | 0.227727087 | 0.014059895 | 0.037591045 |
| ENSG00000100526 | cyclin dependent kinase inhibitor 3(CDKN3) | -0.558952905 | 0.212699495 | 0.008591388 | 0.024912273 |
| ENSG00000117461 | phosphoinositide-3-kinase regulatory subunit 3(PIK3R3) | -0.558690813 | 0.209955165 | 0.007790883 | 0.022918715 |
| ENSG00000133321 | retinoic acid receptor responder 3(RARRES3) | -0.558178657 | 0.224560679 | 0.012931604 | 0.034983306 |
| ENSG00000114416 | FMR1 autosomal homolog 1(FXR1) | -0.558139174 | 0.237734398 | 0.018887569 | 0.048177224 |
| ENSG00000133027 | phosphatidylethanolamine N-methyltransferase(PEMT) | -0.55734524 | 0.19775173 | 0.00482623 | 0.015307568 |
| ENSG00000102804 | TSC22 domain family member 1(TSC22D1) | -0.556854223 | 0.157888766 | 0.000420492 | 0.001898915 |
| ENSG00000159055 | MIS18 kinetochore protein A(MIS18A) | -0.556313645 | 0.226239539 | 0.013934071 | 0.037322498 |
| ENSG00000130520 | LSM4 homolog, U6 small nuclear RNA and mRNA degradation associated(LSM4) | -0.55620941 | 0.163663635 | 0.000677587 | 0.002862165 |
| ENSG00000125304 | transmembrane 9 superfamily member 2(TM9SF2) | -0.556056373 | 0.187368382 | 0.003000202 | 0.01028391 |
| ENSG00000130159 | ECSIT signalling integrator(ECSIT) | -0.55529867 | 0.168706984 | 0.000996554 | 0.003984861 |
| ENSG00000005882 | pyruvate dehydrogenase kinase 2(PDK2) | -0.555149552 | 0.158341047 | 0.000454832 | 0.002029835 |
| ENSG00000160469 | BR serine/threonine kinase 1(BRSK1) | -0.554310105 | 0.200075784 | 0.005597017 | 0.017351095 |
| ENSG00000146416 | androgen induced 1(AlG1) | -0.55430761 | 0.174294587 | 0.001471269 | 0.005577832 |
| ENSG00000136003 | iron-sulfur cluster assembly enzyme(ISCU) | -0.552331044 | 0.155307835 | 0.000376024 | 0.001720556 |
| ENSG00000141446 | establishment of sister chromatid cohesion N-acetyltransferase 1(ESCO1) | -0.552277054 | 0.234579529 | 0.018556251 | 0.047527919 |
| ENSG00000093010 | microRNA 4761(MIR4761) | -0.552025878 | 0.186447841 | 0.003068886 | 0.010477134 |
| ENSG00000139990 | DDB1 and CUL4 associated factor 5(DCAF5) | -0.551771991 | 0.159411908 | 0.000537579 | 0.002344312 |
| ENSG00000189171 | S100 calcium binding protein A13(S100A13) | -0.551459965 | 0.169674115 | 0.001153594 | 0.004538754 |
| ENSG00000134744 | zinc finger CCHC-type containing 11(ZCCHC11) | -0.551328793 | 0.223988651 | 0.013839058 | 0.037110254 |
| ENSG00000170906 | NADH:ubiquinone oxidoreductase subunit A3(NDUFA3) | -0.551181529 | 0.163515807 | 0.000749462 | 0.00311984 |
| ENSG00000132819 | RNA binding motif protein 38(RBM38) | -0.551165131 | 0.208530395 | 0.008215258 | 0.023987167 |
| ENSG00000183426 | nuclear pore complex interacting protein family member A1(NPIPA1) | -0.551071672 | 0.217944091 | 0.011455103 | 0.03161578 |
| | hydroxy-delta-5-steroid dehydrogenase, 3 beta- and steroid delta-isomerase | | | | |
| ENSG00000099377 | 7(HSD3B7) | -0.550728222 | 0.231657594 | 0.017438138 | 0.045115956 |
| ENSG00000143369 | extracellular matrix protein 1(ECM1) | -0.550715388 | 0.162738311 | 0.000714237 | 0.002993333 |
| ENSG00000229320 | NA | -0.550239311 | 0.235118879 | 0.019270319 | 0.048939455 |
| ENSG00000139531 | sulfite oxidase(SUOX) | -0.549769719 | 0.175138305 | 0.001694905 | 0.006305776 |
| ENSG00000100246 | dynein axonemal light chain 4(DNAL4) | -0.549597454 | 0.160810986 | 0.000631617 | 0.002698816 |
| ENSG00000099817 | RNA polymerase II subunit E(POLR2E) | -0.549442674 | 0.19862838 | 0.00567165 | 0.017558234 |
| ENSG00000128581 | intraflagellar transport 22(IFT22) | -0.549130139 | 0.196586825 | 0.005216987 | 0.016352953 |
| ENSG00000135940 | cytochrome c oxidase subunit 5B(COX5B) | -0.548249805 | 0.153988728 | 0.000370397 | 0.001698111 |
| ENSG00000235655 | NA | -0.548243164 | 0.143760302 | 0.000136961 | 0.00071833 |
| ENSG00000135740 | solute carrier family 9 member A5(SLC9A5) | -0.547983332 | 0.180490654 | 0.00239679 | 0.008479951 |
| ENSG00000167815 | peroxiredoxin 2(PRDX2) | -0.546331667 | 0.148587364 | 0.000236143 | 0.001153648 |
| ENSG00000163923 | ribosomal protein L39 like(RPL39L) | -0.546078048 | 0.185929547 | 0.00331387 | 0.011199187 |
| ENSG00000029993 | high mobility group box 3(HMGB3) | -0.54595546 | 0.173881069 | 0.00169051 | 0.006293405 |
| ENSG00000024862 | coiled-coil domain containing 28A(CCDC28A) | -0.545824772 | 0.233164818 | 0.019235275 | 0.048873386 |
| ENSG00000162923 | WD repeat domain 26(WDR26) | -0.545100985 | 0.193141055 | 0.004768133 | 0.015151896 |
| ENSG00000255302 | EP300 interacting inhibitor of differentiation 1(EID1) | -0.545004608 | 0.203617409 | 0.007437092 | 0.022051758 |
| ENSG00000100949 | Rab geranylgeranyltransferase alpha subunit(RABGGTA) | -0.544512947 | 0.150410197 | 0.000294391 | 0.001396457 |
| ENSG00000116251 | ribosomal protein L22(RPL22) | -0.543514906 | 0.151675769 | 0.000339151 | 0.00157574 |
| ENSG00000076382 | sperm associated antigen 5(SPAG5) | -0.54280628 | 0.156162307 | 0.000509121 | 0.002238992 |
| ENSG00000131724 | interleukin 13 receptor subunit alpha 1(IL13RA1) | -0.542498988 | 0.20163078 | 0.007133297 | 0.021249965 |
| ENSG00000142798 | heparan sulfate proteoglycan 2(HSPG2) | -0.542196139 | 0.230460465 | 0.018639446 | 0.04769408 |
| ENSG00000173812 | eukaryotic translation initiation factor 1(EIF1) | -0.541791874 | 0.159982227 | 0.000707708 | 0.002970203 |
| ENSG00000103121 | C-X9-C motif containing 2(CMC2) | -0.541249544 | 0.169552983 | 0.001411863 | 0.005390832 |
| ENSG00000118193 | kinesin family member 14(KIF14) | -0.54121756 | 0.231129105 | 0.019200024 | 0.048804902 |
| ENSG00000102452 | sodium leak channel, non-selective(NALCN) | -0.540276401 | 0.22898713 | 0.018303616 | 0.047003654 |
| ENSG00000116670 | MAD2 mitotic arrest deficient-like 2 (yeast)(MAD2L2) | -0.540102505 | 0.150310273 | 0.000326578 | 0.001524549 |
| ENSG00000090263 | mitochondrial ribosomal protein S33(MRPS33) | -0.54002264 | 0.209560321 | 0.009968164 | 0.028161738 |
| ENSG00000168936 | transmembrane protein 129(TMEM129) | -0.539830077 | 0.181065603 | 0.002869281 | 0.009924793 |
| ENSG00000198843 | selenoprotein T(SELENOT) | -0.5395653 | 0.204188498 | 0.008229981 | 0.024018232 |
| ENSG00000092036 | microRNA 4707(MIR4707) | -0.539132386 | 0.190740594 | 0.004705659 | 0.015006064 |
| ENSG00000179954 | scavenger receptor cysteine rich family member with 5 domains(SSC5D) | -0.53893462 | 0.172294925 | 0.001760138 | 0.006521656 |
| ENSG00000167123 | cerebral endothelial cell adhesion molecule(CERCAM) | -0.538891425 | 0.166047052 | 0.0011728 | 0.004598939 |
| ENSG00000167653 | prostate stem cell antigen(PSCA) | -0.537269291 | 0.221418205 | 0.015245627 | 0.040230945 |
| ENSG00000158555 | glycerophosphodiester phosphodiesterase domain containing 5(GDPD5) | -0.536916689 | 0.1698524 | 0.001571864 | 0.00591725 |
| ENSG00000055211 | glycoprotein integral membrane 1(GINM1) | -0.53683103 | 0.197216658 | 0.006488089 | 0.019637326 |
| ENSG00000137218 | fibroblast growth factor receptor substrate 3(FRS3) | -0.536541587 | 0.220422604 | 0.014926825 | 0.039522659 |
| ENSG00000158290 | cullin 4B(CUL4B) | -0.536126352 | 0.221568533 | 0.0155339 | 0.040818658 |
| ENSG00000124299 | peptidase D(PEPD) | -0.535805863 | 0.15596108 | 0.000591439 | 0.002543947 |
| ENSG00000112096 | uncharacterized LOC100129518(LOC100129518) | -0.535766208 | 0.196150354 | 0.006306477 | 0.019170387 |
| ENSG00000166825 | alanyl aminopeptidase, membrane(ANPEP) | -0.535298168 | 0.20172417 | 0.007963476 | 0.023350532 |
| ENSG00000137100 | dynactin subunit 3(DCTN3) | -0.535276472 | 0.165504645 | 0.001219805 | 0.00475316 |
| ENSG00000184076 | ubiquinol-cytochrome c reductase, complex III subunit X(UQCR10) | -0.535235954 | 0.167865319 | 0.001430207 | 0.005453795 |
| ENSG00000111665 | cell division cycle associated 3(CDCA3) | -0.535202903 | 0.15572523 | 0.00058854 | 0.002533329 |
| ENSG00000100027 | yippee like 1(YPEL1) | -0.535106598 | 0.193801969 | 0.005760705 | 0.017754502 |
| ENSG00000166311 | sphingomyelin phosphodiesterase 1(SMPD1) | -0.534615592 | 0.182642153 | 0.003421163 | 0.011518764 |
| ENSG00000104859 | CLK4 associating serine/arginine rich protein(CLASRP) | -0.534568234 | 0.165365706 | 0.001226509 | 0.004774537 |
| ENSG00000197070 | arrestin domain containing 1(ARRDC1) | -0.533837741 | 0.19366173 | 0.005841514 | 0.017951822 |
| ENSG00000010017 | RAN binding protein 9(RANBP9) | -0.53284936 | 0.226287619 | 0.018535482 | 0.047495405 |
| ENSG00000106771 | transmembrane protein 245(TMEM245) | -0.532679708 | 0.189986724 | 0.00505084 | 0.015908456 |
| ENSG00000172830 | slingshot protein phosphatase 3(SSH3) | -0.532027436 | 0.162625331 | 0.001069817 | 0.004247495 |
| ENSG00000087460 | GNAS complex locus(GNAS) | -0.531834589 | 0.145131976 | 0.000247832 | 0.001199795 |
| ENSG00000108679 | galectin 3 binding protein(LGALS3BP) | -0.531289817 | 0.174020051 | 0.002265373 | 0.008100258 |
| ENSG00000226278 | NA | -0.531121838 | 0.195263619 | 0.006527707 | 0.019751014 |
| ENSG00000002586 | CD99 molecule(CD99) | -0.53102705 | 0.161311887 | 0.000995032 | 0.003981483 |
| ENSG00000230989 | heat shock factor binding protein 1(HSBP1) | -0.530918779 | 0.162141454 | 0.001058801 | 0.00420802 |
| ENSG00000160766 | glucosylceramidase beta pseudogene 1(GBAP1) | -0.530451612 | 0.223322126 | 0.017535848 | 0.045358777 |
| ENSG00000177868 | small vasohibin binding protein(SVBP) | -0.530416379 | 0.213291225 | 0.012889145 | 0.034897101 |
| ENSG00000198715 | glycosylated lysosomal membrane protein(GLMP) | -0.530161485 | 0.171816596 | 0.002031252 | 0.007359301 |
| ENSG00000130255 | ribosomal protein L36(RPL36) | -0.529927822 | 0.183889 | 0.003954345 | 0.01298323 |
| ENSG00000064961 | high mobility group 20B(HMG20B) | -0.528996104 | 0.173458216 | 0.002290675 | 0.008170861 |
| ENSG00000233369 | general transcription factor Ili pseudogene 4(GTF2IP4) | -0.528756016 | 0.16554215 | 0.001402739 | 0.005362957 |
| ENSG00000126432 | peroxiredoxin 5(PRDX5) | -0.528668812 | 0.146649914 | 0.000312187 | 0.001466094 |
| ENSG00000188243 | COMM domain containing 6(COMMD6) | -0.528654759 | 0.200759283 | 0.00845654 | 0.024600083 |
| ENSG00000171603 | calsyntenin 1(CLSTN1) | -0.528586533 | 0.169679218 | 0.001838135 | 0.006757444 |
| ENSG00000180581 | NA | -0.528225584 | 0.215829104 | 0.014388099 | 0.038268505 |
| ENSG00000112335 | sorting nexin 3(SNX3) | -0.527689533 | 0.163103487 | 0.001215128 | 0.00473741 |
| ENSG00000256618 | MT-RNR2-like 1(MTRNR2L1) | -0.527201197 | 0.166104949 | 0.001504033 | 0.005687382 |
| ENSG00000161714 | phospholipase C delta 3(PLCD3) | -0.526806091 | 0.166136216 | 0.001519524 | 0.005740426 |
| ENSG00000144455 | sulfatase modifying factor 1(SUMF1) | -0.526584512 | 0.173324951 | 0.002380477 | 0.008434099 |
| ENSG00000170604 | interferon regulatory factor 2 binding protein 1(IRF2BP1) | -0.52637753 | 0.211709499 | 0.01290719 | 0.034937916 |
| ENSG00000187051 | ribosomal protein S19 binding protein 1(RPS19BP1) | -0.526309531 | 0.183221221 | 0.004071918 | 0.013317222 |
| ENSG00000111206 | forkhead box M1(FOXM1) | -0.525752652 | 0.154607466 | 0.000672469 | 0.00284361 |
| ENSG00000096060 | FK506 binding protein 5(FKBP5) | -0.525550185 | 0.17736807 | 0.003046086 | 0.010413879 |
| ENSG00000196323 | zinc finger and BTB domain containing 44(ZBTB44) | -0.525048622 | 0.182927351 | 0.004101375 | 0.013406106 |
| ENSG00000049860 | hexosaminidase subunit beta(HEXB) | -0.524911177 | 0.198347969 | 0.008134978 | 0.023800021 |
| ENSG00000130749 | zinc finger CCCH-type containing 4(ZC3H4) | -0.524834619 | 0.188613322 | 0.005392596 | 0.01680045 |
| ENSG00000235174 | NA | -0.524502863 | 0.216540253 | 0.015427042 | 0.040600384 |
| ENSG00000185133 | inositol polyphosphate-5-phosphatase J(INPP5J) | -0.523704546 | 0.193635377 | 0.006838848 | 0.020544487 |
| ENSG00000146426 | T-cell lymphoma invasion and metastasis 2(TIAM2) | -0.522776502 | 0.219254048 | 0.017109721 | 0.044367846 |
| ENSG00000102901 | centromere protein T(CENPT) | -0.5220964 | 0.169427642 | 0.002059396 | 0.007449794 |
| ENSG00000141971 | multivesicular body subunit 12A(MVB12A) | -0.52201401 | 0.171384676 | 0.002320146 | 0.008258455 |
| ENSG00000150433 | transmembrane protein 218(TMEM218) | -0.521036172 | 0.209231776 | 0.012765886 | 0.034611174 |
| ENSG00000108797 | contactin associated protein 1(CNTNAP1) | -0.520840801 | 0.174154501 | 0.002783548 | 0.009659487 |
| ENSG00000160014 | calmodulin 3(CALM3) | -0.520184052 | 0.159474171 | 0.001106798 | 0.004378067 |
| ENSG00000153774 | craniofacial development protein 1(CFDP1) | -0.519988458 | 0.182424753 | 0.004366056 | 0.014133815 |
| ENSG00000110047 | EH domain containing 1(EHD1) | -0.519821444 | 0.16343939 | 0.001470135 | 0.005575328 |
| ENSG00000142168 | superoxide dismutase 1, soluble(SOD1) | -0.519624008 | 0.151043885 | 0.000581244 | 0.002504673 |
| ENSG00000147604 | ribosomal protein L7(RPL7) | -0.519618125 | 0.191194257 | 0.006572755 | 0.019871984 |
| ENSG00000204852 | tectonic family member 1(TCTN1) | -0.519487374 | 0.173307637 | 0.002722155 | 0.009479999 |
| ENSG00000143198 | microsomal glutathione S-transferase 3(MGST3) | -0.519365103 | 0.192299679 | 0.006917062 | 0.020719794 |
| ENSG00000164292 | Rho related BTB domain containing 3(RHOBTB3) | -0.519105054 | 0.221100008 | 0.018883178 | 0.048176472 |
| ENSG00000181104 | coagulation factor II thrombin receptor(F2R) | -0.518983963 | 0.157716329 | 0.000999681 | 0.003996006 |
| ENSG00000158402 | cell division cycle 25C(CDC25C) | -0.518659292 | 0.220402087 | 0.018610553 | 0.047635889 |
| ENSG00000166598 | microRNA 3652(MIR3652) | -0.518628648 | 0.213134265 | 0.014960146 | 0.03960197 |
| ENSG00000070182 | microRNA 7855(MIR7855) | -0.517739166 | 0.216663127 | 0.016866528 | 0.043858939 |
| ENSG00000149806 | FAU, ubiquitin like and ribosomal protein S30 fusion(FAU) | -0.517655509 | 0.174054362 | 0.002938469 | 0.010122446 |
| ENSG00000136828 | Ral GEF with PH domain and SH3 binding motif 1(RALGPS1) | -0.517520004 | 0.218425269 | 0.017820685 | 0.04597424 |
| ENSG00000179526 | SHANK associated RH domain interactor(SHARPIN) | -0.517513816 | 0.186001167 | 0.005397264 | 0.016810543 |
| ENSG00000079277 | MAP kinase interacting serine/threonine kinase 1(MKNK1) | -0.517295502 | 0.184697457 | 0.005098049 | 0.016027429 |
| ENSG00000187942 | low density lipoprotein receptor class A domain containing 2(LDLRAD2) | -0.516870218 | 0.204116781 | 0.011334029 | 0.031349884 |
| ENSG00000188153 | collagen type IV alpha 5 chain(COL4A5) | -0.516306964 | 0.148664064 | 0.000514718 | 0.002255175 |
| ENSG00000182054 | isocitrate dehydrogenase (NADP(+)) 2, mitochondrial(IDH2) | -0.515398828 | 0.154416023 | 0.000844661 | 0.00346585 |
| ENSG00000125611 | coiled-coil-helix-coiled-coil-helix domain containing 5(CHCHD5) | -0.515379351 | 0.190719984 | 0.006886514 | 0.020661299 |
| ENSG00000130429 | actin related protein 2/3 complex subunit 1B(ARPC1B) | -0.515204132 | 0.207169886 | 0.012887325 | 0.034897101 |
| ENSG00000225151 | golgin A2 pseudogene 7(GOLGA2P7) | -0.514901161 | 0.180862815 | 0.004414507 | 0.014255356 |
| ENSG00000196476 | chromosome 20 open reading frame 96(C20orf96) | -0.514759959 | 0.206548227 | 0.012695372 | 0.034462523 |
| ENSG00000163521 | galactosidase beta 1 like(GLB1L) | -0.514702616 | 0.220362415 | 0.01950638 | 0.049359659 |
| ENSG00000157353 | fucokinase(FUK) | -0.514082801 | 0.218892509 | 0.018846021 | 0.048112977 |
| ENSG00000167680 | semaphorin 6B(SEMA6B) | -0.514076558 | 0.212144229 | 0.015382725 | 0.040529088 |
| ENSG00000167792 | NADH:ubiquinone oxidoreductase core subunit V1(NDUFV1) | -0.514013303 | 0.171012054 | 0.002649582 | 0.009254656 |
| ENSG00000185339 | transcobalamin 2(TCN2) | -0.513748503 | 0.15339554 | 0.000810526 | 0.00333276 |
| ENSG00000073803 | mitogen-activated protein kinase kinase kinase 13(MAP3K13) | -0.513310382 | 0.209882093 | 0.01445679 | 0.038423921 |
| ENSG00000176155 | coiled-coil domain containing 57(CCDC57) | -0.513068493 | 0.212010533 | 0.015519903 | 0.040808256 |
| ENSG00000131748 | StAR related lipid transfer domain containing 3(STARD3) | -0.51292412 | 0.148228557 | 0.000539454 | 0.002349552 |
| ENSG00000253276 | coiled-coil domain containing 71-like(CCDC71L) | -0.512268795 | 0.2011678 | 0.010881697 | 0.030370649 |
| ENSG00000196976 | L antigen family member 3(LAGE3) | -0.512139131 | 0.164530696 | 0.001853674 | 0.006812441 |
| ENSG00000110046 | autophagy related 2A(ATG2A) | -0.511854195 | 0.198462608 | 0.009905914 | 0.02800605 |
| ENSG00000186312 | carbonic anhydrase 5B pseudogene 1(CA5BP1) | -0.511780947 | 0.189454818 | 0.006906172 | 0.020699161 |
| ENSG00000164880 | integrator complex subunit 1(INTS1) | -0.511645369 | 0.214588603 | 0.017111264 | 0.044367846 |
| ENSG00000136861 | CDK5 regulatory subunit associated protein 2(CDK5RAP2) | -0.51153232 | 0.194587835 | 0.008568697 | 0.024852602 |
| ENSG00000100288 | choline kinase beta(CHKB) | -0.511289866 | 0.181177807 | 0.004772017 | 0.015158715 |
| ENSG00000064489 | BORCS8-MEF2B readthrough(BORCS8-MEF2B) | -0.511229525 | 0.202263963 | 0.011486592 | 0.031682537 |
| ENSG00000090266 | NADH:ubiquinone oxidoreductase subunit B2(NDUFB2) | -0.510974581 | 0.165991168 | 0.002081685 | 0.007518865 |
| ENSG00000182179 | microRNA 5193(MIR5193) | -0.510933676 | 0.162323775 | 0.001646051 | 0.006153208 |
| ENSG00000127445 | peptidylprolyl cis/trans isomerase, NIMA-interacting 1(PIN1) | -0.510766927 | 0.171214291 | 0.00285249 | 0.009869617 |
| ENSG00000143819 | epoxide hydrolase 1(EPHX1) | -0.510401474 | 0.170109896 | 0.002696058 | 0.00939746 |
| ENSG00000160445 | zyg-11 related cell cycle regulator(ZER1) | -0.509861915 | 0.157512996 | 0.00120814 | 0.004713949 |
| ENSG00000127922 | uncharacterized LOC100506136(LOC100506136) | -0.509800601 | 0.175828001 | 0.003738444 | 0.01238149 |
| ENSG00000167468 | glutathione peroxidase 4(GPX4) | -0.509751631 | 0.159109882 | 0.001356403 | 0.005214616 |
| ENSG00000153094 | BCL2 like 11(BCL2L11) | -0.509720489 | 0.183701521 | 0.005524906 | 0.017167194 |
| ENSG00000158856 | dematin actin binding protein(DMTN) | -0.509284227 | 0.162232184 | 0.001693921 | 0.006304109 |
| ENSG00000198937 | coiled-coil domain containing 167(CCDC167) | -0.509132618 | 0.16845526 | 0.002508112 | 0.00881812 |
| ENSG00000103197 | tuberous sclerosis 2(TSC2) | -0.509113911 | 0.160020275 | 0.001464848 | 0.005558862 |
| ENSG00000079805 | dynamin 2(DNM2) | -0.507981641 | 0.153279845 | 0.000919453 | 0.003732381 |
| ENSG00000127920 | G protein subunit gamma 11(GNG11) | -0.507054281 | 0.203218629 | 0.012591556 | 0.034217386 |
| ENSG00000205309 | 5',3'-nucleotidase, mitochondrial(NT5M) | -0.506414983 | 0.160705562 | 0.001626024 | 0.006091895 |
| ENSG00000115350 | DNA polymerase epsilon 4, accessory subunit(POLE4) | -0.506312899 | 0.152664248 | 0.000911485 | 0.003705151 |
| ENSG00000104529 | eukaryotic translation elongation factor 1 delta(EEF1D) | -0.506002371 | 0.185304892 | 0.006321001 | 0.019209572 |
| ENSG00000169223 | lectin, mannose binding 2(LMAN2) | -0.505485408 | 0.188325035 | 0.007272283 | 0.021620202 |
| ENSG00000102908 | nuclear factor of activated T-cells 5(NFAT5) | -0.50529318 | 0.214721426 | 0.018610121 | 0.047635889 |
| ENSG00000105048 | troponin T1, slow skeletal type(TNNT1) | -0.505119655 | 0.156260936 | 0.001226951 | 0.00477468 |
| ENSG00000168904 | leucine rich repeat containing 28(LRRC28) | -0.504647513 | 0.189087996 | 0.007611142 | 0.022468625 |
| ENSG00000141376 | BCAS3, microtubule associated cell migration factor(BCAS3) | -0.504553751 | 0.179549352 | 0.004952429 | 0.015636173 |
| ENSG00000152291 | trans-golgi network protein 2(TGOLN2) | -0.504485491 | 0.156404994 | 0.001257495 | 0.004880449 |
| ENSG00000142192 | amyloid beta precursor protein(APP) | -0.504311097 | 0.153740624 | 0.00103707 | 0.004128625 |
| ENSG00000083838 | zinc finger protein 446(ZNF446) | -0.502926009 | 0.18966272 | 0.008009095 | 0.023466748 |
| ENSG00000157456 | cyclin B2(CCNB2) | -0.502080717 | 0.157518171 | 0.001435351 | 0.005468092 |
| ENSG00000129562 | defender against cell death 1(DAD1) | -0.501948049 | 0.161700831 | 0.00190809 | 0.006981887 |
| ENSG00000064309 | cell adhesion associated, oncogene regulated(CDON) | -0.501328392 | 0.206872466 | 0.015377284 | 0.040523827 |
| ENSG00000187742 | SECIS binding protein 2(SECISBP2) | -0.500937111 | 0.213546627 | 0.018986428 | 0.048366465 |
| ENSG00000072062 | protein kinase cAMP-activated catalytic subunit alpha(PRKACA) | -0.5009151 | 0.158191604 | 0.001542807 | 0.005822772 |
| ENSG00000094631 | histone deacetylase 6(HDAC6) | -0.499972645 | 0.164885003 | 0.002427376 | 0.008572697 |
| | suppression of tumorigenicity 13 (colon carcinoma) (Hsp70 interacting | | | | |
| ENSG00000100380 | protein)(ST13) | -0.499147564 | 0.162417127 | 0.002117448 | 0.007619964 |
| ENSG00000136732 | glycophorin C (Gerbich blood group)(GYPC) | -0.498701881 | 0.156214829 | 0.001410906 | 0.005388928 |
| ENSG00000140391 | tetraspanin 3(TSPAN3) | -0.497801452 | 0.159523039 | 0.001805066 | 0.006660852 |
| ENSG00000120437 | acetyl-CoA acetyltransferase 2(ACAT2) | -0.497591031 | 0.202914294 | 0.014197678 | 0.037864765 |
| ENSG00000178951 | zinc finger and BTB domain containing 7A(ZBTB7A) | -0.497421966 | 0.192258811 | 0.009674485 | 0.027470586 |
| ENSG00000101187 | solute carrier organic anion transporter family member 4A1(SLCO4A1) | -0.4969403 | 0.195927139 | 0.01120139 | 0.031085906 |
| ENSG00000105698 | upstream transcription factor 2, c-fos interacting(USF2) | -0.496205384 | 0.154092308 | 0.001281089 | 0.004955848 |
| ENSG00000177239 | mannosidase alpha class 1B member 1(MAN1B1) | -0.496034048 | 0.159643217 | 0.00188906 | 0.006927339 |
| ENSG00000167930 | family with sequence similarity 234 member A(FAM234A) | -0.495473521 | 0.16848471 | 0.003274054 | 0.011083734 |
| ENSG00000130726 | tripartite motif containing 28(TRIM28) | -0.49540084 | 0.155784928 | 0.001472595 | 0.005581059 |
| ENSG00000272047 | general transcription factor IIH subunit 5(GTF2H5) | -0.495172528 | 0.182346042 | 0.006616279 | 0.019983031 |
| ENSG00000107798 | lipase A, lysosomal acid type(LIPA) | -0.49297345 | 0.170355584 | 0.003806215 | 0.012563498 |
| ENSG00000078618 | microRNA 761(MIR761) | -0.49292136 | 0.205942278 | 0.016688809 | 0.043462498 |
| ENSG00000165389 | serine palmitoyltransferase small subunit A(SPTSSA) | -0.492829205 | 0.190922889 | 0.009842908 | 0.02786672 |
| ENSG00000130584 | zinc finger and BTB domain containing 46(ZBTB46) | -0.492381482 | 0.205722566 | 0.016691905 | 0.043462498 |
| ENSG00000166147 | fibrillin 1(FBN1) | -0.492347797 | 0.171159407 | 0.004020544 | 0.013167506 |
| ENSG00000138796 | hydroxyacyl-CoA dehydrogenase(HADH) | -0.49230461 | 0.182134112 | 0.006872117 | 0.020623363 |
| ENSG00000072042 | retinol dehydrogenase 11 (all-trans/9-cis/11-cis)(RDH11) | -0.492195833 | 0.185240966 | 0.007882608 | 0.02314225 |
| ENSG00000118515 | serum/glucocorticoid regulated kinase 1(SGK1) | -0.4921416 | 0.143927307 | 0.000627649 | 0.002684968 |
| ENSG00000255823 | MT-RNR2-like 8(MTRNR2L8) | -0.492033919 | 0.162278306 | 0.00242909 | 0.008576174 |
| ENSG00000158292 | G protein-coupled receptor 153(GPR153) | -0.492001207 | 0.176661976 | 0.005352996 | 0.01669919 |
| ENSG00000214941 | zinc finger SWIM-type containing 7(ZSWIM7) | -0.491697606 | 0.193115967 | 0.010892583 | 0.030389099 |
| ENSG00000213123 | Tctex1 domain containing 2(TCTEX1D2) | -0.49133345 | 0.193985888 | 0.011314553 | 0.031311314 |
| ENSG00000111328 | cyclin dependent kinase 2 associated protein 1(CDK2AP1) | -0.491306158 | 0.15636652 | 0.001677887 | 0.006256311 |
| ENSG00000092929 | unc-13 homolog D(UNC13D) | -0.491118797 | 0.189336842 | 0.009489705 | 0.0270688 |
| ENSG00000189043 | NDUFA4, mitochondrial complex associated(NDUFA4) | -0.490761418 | 0.202152155 | 0.015195604 | 0.040116941 |
| ENSG00000219200 | ribonuclease K(RNASEK) | -0.490564533 | 0.161516079 | 0.002387482 | 0.008454648 |
| ENSG00000084693 | ATP/GTP binding protein like 5(AGBL5) | -0.4903482 | 0.163805471 | 0.002758167 | 0.009585547 |
| ENSG00000042493 | capping actin protein, gelsolin like(CAPG) | -0.490013224 | 0.148088208 | 0.000936539 | 0.003788668 |
| ENSG00000079459 | farnesyl-diphosphate farnesyltransferase 1(FDFT1) | -0.489818998 | 0.165702109 | 0.003116354 | 0.010604797 |
| ENSG00000131174 | cytochrome c oxidase subunit 7B(COX7B) | -0.489179412 | 0.187466454 | 0.009069486 | 0.02603549 |
| ENSG00000145494 | NADH:ubiquinone oxidoreductase subunit S6(NDUFS6) | -0.488872221 | 0.191946985 | 0.010868061 | 0.030339788 |
| ENSG00000054148 | phosphohistidine phosphatase 1(PHPT1) | -0.488715547 | 0.158227641 | 0.002010436 | 0.007297368 |
| ENSG00000159685 | coiled-coil-helix-coiled-coil-helix domain containing 6(CHCHD6) | -0.488374951 | 0.160573501 | 0.002354481 | 0.008362958 |
| ENSG00000135451 | trophinin associated protein(TROAP) | -0.486791763 | 0.154974043 | 0.001683042 | 0.006269704 |
| ENSG00000197956 | S100 calcium binding protein A6(S100A6) | -0.486615337 | 0.152973435 | 0.00146745 | 0.00556694 |
| ENSG00000091592 | NLR family pyrin domain containing 1(NLRP1) | -0.486111813 | 0.179016788 | 0.006618502 | 0.019984613 |
| ENSG00000123815 | coenzyme Q8B(COQ8B) | -0.486094804 | 0.19819231 | 0.014181135 | 0.037837804 |
| ENSG00000034510 | thymosin beta 10(TMSB10) | -0.48459866 | 0.149122789 | 0.001155423 | 0.004542911 |
| ENSG00000141086 | chymotrypsin like(CTRL) | -0.484473903 | 0.207105236 | 0.019321755 | 0.049023092 |
| ENSG00000187676 | beta 3-glucosyltransferase(B3GLCT) | -0.484229067 | 0.191080724 | 0.011271735 | 0.031236913 |
| ENSG00000120533 | ENY2, transcription and export complex 2 subunit(ENY2) | -0.484092725 | 0.173813346 | 0.00535063 | 0.016696238 |
| ENSG00000283430 | NA | -0.48171141 | 0.167523617 | 0.004034091 | 0.013204521 |
| ENSG00000126456 | interferon regulatory factor 3(IRF3) | -0.481547352 | 0.174390372 | 0.005756851 | 0.017747275 |
| ENSG00000137693 | Yes associated protein 1(YAP1) | -0.481400203 | 0.193950512 | 0.013061886 | 0.035251211 |
| ENSG00000135046 | annexin A1(ANXA1) | -0.481170971 | 0.200580973 | 0.016445015 | 0.0428956 |
| ENSG00000139192 | TAP binding protein like(TAPBPL) | -0.480967149 | 0.186434473 | 0.009885219 | 0.027966553 |
| ENSG00000117009 | kynurenine 3-monooxygenase(KMO) | -0.48095108 | 0.205315312 | 0.019155043 | 0.048711615 |
| ENSG00000268916 | CSAG family member 3(CSAG3) | -0.480878877 | 0.191579632 | 0.012070618 | 0.032992274 |
| ENSG00000164904 | aldehyde dehydrogenase 7 family member A1(ALDH7A1) | -0.480874194 | 0.159993738 | 0.002650737 | 0.009255942 |
| ENSG00000140451 | PIF1 5'-to-3' DNA helicase(PIF1) | -0.480598353 | 0.199874141 | 0.016194389 | 0.042345142 |
| ENSG00000172354 | G protein subunit beta 2(GNB2) | -0.480473135 | 0.178113614 | 0.006984855 | 0.020892406 |
| ENSG00000134955 | solute carrier family 37 member 2(SLC37A2) | -0.480464215 | 0.173797666 | 0.005700954 | 0.01763503 |
| ENSG00000165672 | peroxiredoxin 3(PRDX3) | -0.479905358 | 0.197917046 | 0.015317653 | 0.040384775 |
| ENSG00000107745 | mitochondrial calcium uptake 1(MICU1) | -0.478854553 | 0.201067598 | 0.017239481 | 0.044646981 |
| ENSG00000173818 | endonuclease V(ENDOV) | -0.478455041 | 0.195896136 | 0.014590318 | 0.03872755 |
| ENSG00000122545 | septin 7(SEPT7) | -0.477746869 | 0.200606463 | 0.017241689 | 0.044646981 |
| ENSG00000272647 | NA | -0.477376592 | 0.202162917 | 0.018208734 | 0.046821321 |
| ENSG00000149743 | tRNA phosphotransferase 1(TRPT1) | -0.47717954 | 0.157154615 | 0.002394454 | 0.008474236 |
| ENSG00000106976 | dynamin 1(DNM1) | -0.476652537 | 0.153933931 | 0.001958365 | 0.007139203 |
| ENSG00000108846 | ATP binding cassette subfamily C member 3(ABCC3) | -0.476308932 | 0.164084923 | 0.003698196 | 0.012279304 |
| ENSG00000124313 | IQ motif and Sec7 domain 2(IQSEC2) | -0.475758073 | 0.184164352 | 0.009785048 | 0.027730981 |
| ENSG00000146242 | trophoblast glycoprotein(TPBG) | -0.47505697 | 0.171087123 | 0.005491447 | 0.017081277 |
| ENSG00000106351 | ArfGAP with FG repeats 2(AGFG2) | -0.474023127 | 0.175515391 | 0.00691834 | 0.020719794 |
| ENSG00000160633 | scaffold attachment factor B(SAFB) | -0.473731174 | 0.167291627 | 0.004629134 | 0.01480618 |
| ENSG00000232187 | NA | -0.47348383 | 0.186278743 | 0.011028238 | 0.03070679 |
| ENSG00000213983 | adaptor related protein complex 1 gamma 2 subunit(AP1G2) | -0.473345627 | 0.151758703 | 0.001814244 | 0.006686332 |
| ENSG00000066583 | isochorismatase domain containing 1(ISOC1) | -0.473100141 | 0.202821528 | 0.0196695 | 0.049729635 |
| ENSG00000171953 | ATP synthase mitochondrial F1 complex assembly factor 2(ATPAF2) | -0.471624882 | 0.154824628 | 0.002317631 | 0.008254498 |
| ENSG00000205323 | SAP domain containing ribonucleoprotein(SARNP) | -0.471516509 | 0.191949944 | 0.014031515 | 0.037532229 |
| ENSG00000131116 | zinc finger protein 428(ZNF428) | -0.471372329 | 0.191149889 | 0.013663791 | 0.036679753 |
| ENSG00000134184 | glutathione S-transferase mu 1(GSTM1) | -0.47123085 | 0.169227107 | 0.005359277 | 0.016714352 |
| ENSG00000130731 | methyltransferase like 26(METTL26) | -0.470200883 | 0.153734239 | 0.002224253 | 0.007971143 |
| ENSG00000112306 | ribosomal protein S12(RPS12) | -0.47013057 | 0.16115421 | 0.003531084 | 0.011814468 |
| ENSG00000203778 | family with sequence similarity 229 member B(FAM229B) | -0.469546904 | 0.194955833 | 0.016019176 | 0.041914943 |
| ENSG00000168273 | small integral membrane protein 4(SMIM4) | -0.468965349 | 0.184925777 | 0.011213785 | 0.031098296 |
| ENSG00000075702 | WD repeat domain 62(WDR62) | -0.468892931 | 0.155215034 | 0.00252004 | 0.008852121 |
| ENSG00000149932 | transmembrane protein 219(TMEM219) | -0.468831564 | 0.16115057 | 0.003622663 | 0.012076742 |
| ENSG00000149476 | triokinase and FMN cyclase(TKFC) | -0.468830197 | 0.156318594 | 0.002706926 | 0.009429752 |
| ENSG00000160973 | forkhead box H1(FOXH1) | -0.468563549 | 0.179008129 | 0.008856232 | 0.025573077 |
| ENSG00000119655 | microRNA 4709(MIR4709) | -0.46827572 | 0.168582247 | 0.005474033 | 0.017031611 |
| ENSG00000182196 | ADP ribosylation factor like GTPase 6 interacting protein 4(ARL6IP4) | -0.468274003 | 0.157243845 | 0.00290125 | 0.010012746 |
| ENSG00000188010 | MORN repeat containing 2(MORN2) | -0.467627482 | 0.182641611 | 0.010456509 | 0.029330084 |
| ENSG00000151366 | NADH:ubiquinone oxidoreductase subunit C2(NDUFC2) | -0.467531681 | 0.178985734 | 0.008998345 | 0.025875466 |
| ENSG00000167526 | ribosomal protein L13(RPL13) | -0.467023036 | 0.168536664 | 0.005587634 | 0.017330063 |
| ENSG00000268400 | NA | -0.46698389 | 0.200073724 | 0.019592629 | 0.049556587 |
| ENSG00000113845 | translocase of inner mitochondrial membrane domain containing 1(TIMMDC1) | -0.466959576 | 0.18635371 | 0.0122185 | 0.033334515 |
| ENSG00000161011 | sequestosome 1(SQSTM1) | -0.46686121 | 0.189810706 | 0.013908623 | 0.037262819 |
| ENSG00000111859 | neural precursor cell expressed, developmentally down-regulated 9(NEDD9) | -0.466810527 | 0.171357012 | 0.006445774 | 0.019548359 |
| ENSG00000144810 | collagen type VIII alpha 1 chain(COL8A1) | -0.466620618 | 0.157072386 | 0.002970869 | 0.010214317 |
| ENSG00000169609 | chromosome 15 open reading frame 40(C15orf40) | -0.466458207 | 0.184572561 | 0.01149646 | 0.031682537 |
| ENSG00000144589 | serine/threonine kinase 11 interacting protein(STK11IP) | -0.465399212 | 0.171428564 | 0.006631003 | 0.020012088 |
| ENSG00000186792 | hyaluronoglucosaminidase 3(HYAL3) | -0.465377637 | 0.159564863 | 0.00353935 | 0.011838757 |
| ENSG00000034713 | GABA type A receptor associated protein like 2(GABARAPL2) | -0.465314032 | 0.160168643 | 0.003670764 | 0.01221235 |
| ENSG00000181019 | NAD(P)H quinone dehydrogenase 1(NQO1) | -0.465177586 | 0.160035738 | 0.003652509 | 0.012161934 |
| ENSG00000244398 | NA | -0.465057857 | 0.195914713 | 0.017607176 | 0.045513255 |
| ENSG00000111144 | leukotriene A4 hydrolase(LTA4H) | -0.464795042 | 0.180083019 | 0.009851336 | 0.027878547 |
| ENSG00000163344 | phosphomevalonate kinase(PMVK) | -0.464735513 | 0.173798113 | 0.007495328 | 0.022193565 |
| ENSG00000267368 | uroplakin 3B-like(UPK3BL) | -0.464670045 | 0.173875844 | 0.007530534 | 0.022280987 |
| ENSG00000167772 | angiopoietin like 4(ANGPTL4) | -0.464141993 | 0.167988348 | 0.005728302 | 0.017694379 |
| ENSG00000243749 | transmembrane protein 35B(TMEM35B) | -0.464071098 | 0.186211415 | 0.012696411 | 0.034462523 |
| ENSG00000250982 | NA | -0.463961471 | 0.175164839 | 0.00807988 | 0.023644704 |
| ENSG00000197746 | prosaposin(PSAP) | -0.463931537 | 0.174056983 | 0.007689673 | 0.022666327 |
| ENSG00000243317 | chromosome 7 open reading frame 73(C7orf73) | -0.463171906 | 0.164095638 | 0.004763999 | 0.015142847 |
| ENSG00000232196 | MT-RNR2-like 4(MTRNR2L4) | -0.462993373 | 0.165897304 | 0.005257092 | 0.016447988 |
| ENSG00000166997 | canopy FGF signaling regulator 4(CNPY4) | -0.462108009 | 0.174789691 | 0.008198243 | 0.023943429 |
| ENSG00000213977 | Tax1 binding protein 3(TAX1BP3) | -0.462090867 | 0.173631482 | 0.007783237 | 0.022901952 |
| ENSG00000249115 | HAUS augmin like complex subunit 5(HAUS5) | -0.461828384 | 0.16653987 | 0.005552847 | 0.017235801 |
| ENSG00000147155 | emopamil binding protein (sterol isomerase)(EBP) | -0.461631404 | 0.17489141 | 0.008302068 | 0.024210593 |
| ENSG00000140830 | thioredoxin like 4B(TXNL4B) | -0.460913172 | 0.185011286 | 0.012728714 | 0.034526308 |
| ENSG00000184983 | NADH:ubiquinone oxidoreductase subunit A6(NDUFA6) | -0.460722296 | 0.170698173 | 0.006953847 | 0.020815536 |
| ENSG00000149196 | Hikeshi, heat shock protein nuclear import factor(HIKESHI) | -0.45975794 | 0.190284532 | 0.015685153 | 0.041150789 |
| ENSG00000167900 | thymidine kinase 1(TK1) | -0.459522708 | 0.184175656 | 0.012594856 | 0.034218449 |
| ENSG00000136854 | syntaxin binding protein 1(STXBP1) | -0.459001403 | 0.161667584 | 0.004523136 | 0.014530359 |
| ENSG00000136731 | UDP-glucose glycoprotein glucosyltransferase 1(UGGT1) | -0.458888361 | 0.192521572 | 0.017145691 | 0.044428342 |
| ENSG00000163041 | H3 histone family member 3A(H3F3A) | -0.458630016 | 0.180620954 | 0.011111105 | 0.030893653 |
| ENSG00000197324 | LDL receptor related protein 10(LRP10) | -0.458167922 | 0.162215419 | 0.004736313 | 0.015071134 |
| ENSG00000170275 | cartilage associated protein(CRTAP) | -0.457995523 | 0.171375197 | 0.007529452 | 0.022280987 |
| ENSG00000182087 | transmembrane protein 259(TMEM259) | -0.457907272 | 0.192891762 | 0.017600941 | 0.045507137 |
| ENSG00000127124 | human immunodeficiency virus type I enhancer binding protein 3(HIVEP3) | -0.457880434 | 0.162385391 | 0.00480664 | 0.01525089 |
| ENSG00000005893 | lysosomal associated membrane protein 2(LAMP2) | -0.457678274 | 0.184931131 | 0.013328919 | 0.035897757 |
| ENSG00000170348 | transmembrane p24 trafficking protein 10(TMED10) | -0.456951451 | 0.149966553 | 0.002311205 | 0.008237296 |
| ENSG00000267855 | NADH:ubiquinone oxidoreductase subunit A7(NDUFA7) | -0.456817329 | 0.154231697 | 0.003057569 | 0.010444032 |
| ENSG00000010404 | iduronate 2-sulfatase(IDS) | -0.456617879 | 0.1525321 | 0.002757203 | 0.009585027 |
| ENSG00000129219 | phospholipase D2(PLD2) | -0.456421318 | 0.156676692 | 0.003578128 | 0.011941306 |
| ENSG00000125354 | septin 6(SEPT6) | -0.456376529 | 0.194538882 | 0.018979167 | 0.04835844 |
| ENSG00000164885 | cyclin dependent kinase 5(CDK5) | -0.456302383 | 0.164793488 | 0.005623993 | 0.017420547 |
| ENSG00000156504 | family with sequence similarity 122B(FAM122B) | -0.456293632 | 0.188967766 | 0.015749737 | 0.041298229 |
| ENSG00000169689 | centromere protein X(CENPX) | -0.454144812 | 0.165888264 | 0.006187898 | 0.018858661 |
| ENSG00000157045 | N-terminal asparagine amidase(NTAN1) | -0.452988001 | 0.188745208 | 0.01639519 | 0.042784608 |
| ENSG00000205581 | high mobility group nucleosome binding domain 1(HMGN1) | -0.452784261 | 0.158779145 | 0.004349149 | 0.014082957 |
| ENSG00000114544 | solute carrier family 41 member 3(SLC41A3) | -0.451924184 | 0.159982997 | 0.004730625 | 0.015064035 |
| ENSG00000167004 | protein disulfide isomerase family A member 3(PDIA3) | -0.451807342 | 0.174166234 | 0.00948346 | 0.027058798 |
| ENSG00000161395 | post-GPI attachment to proteins 3(PGAP3) | -0.449998423 | 0.166286001 | 0.00680639 | 0.020462657 |
| ENSG00000126756 | ubiquitously expressed prefoldin like chaperone(UXT) | -0.449483162 | 0.16697117 | 0.007102906 | 0.021176805 |
| ENSG00000064607 | SURP and G-patch domain containing 2(SUGP2) | -0.449451793 | 0.157704261 | 0.004372389 | 0.014150423 |
| ENSG00000117632 | microRNA 3917(MIR3917) | -0.448661416 | 0.149516713 | 0.002693207 | 0.009390303 |
| ENSG00000198276 | microRNA 1914(MIR1914) | -0.448048006 | 0.181293415 | 0.01345863 | 0.036205655 |
| ENSG00000144560 | vestigial like family member 4(VGLL4) | -0.447743968 | 0.183175069 | 0.014511344 | 0.038537138 |
| ENSG00000140988 | ribosomal protein S2(RPS2) | -0.44753469 | 0.143550081 | 0.001823173 | 0.00671083 |
| ENSG00000119638 | NIMA related kinase 9(NEK9) | -0.445314761 | 0.166419348 | 0.007453793 | 0.022081697 |
| ENSG00000066279 | abnormal spindle microtubule assembly(ASPM) | -0.444647646 | 0.187487656 | 0.017710762 | 0.045740437 |
| ENSG00000182379 | neurexophilin 4(NXPH4) | -0.444446229 | 0.179994118 | 0.013540552 | 0.036384434 |
| ENSG00000111676 | atrophin 1(ATN1) | -0.443299525 | 0.155304722 | 0.004312019 | 0.013985827 |
| ENSG00000130158 | dedicator of cytokinesis 6(DOCK6) | -0.442482299 | 0.180598455 | 0.014282088 | 0.038038145 |
| ENSG00000116478 | histone deacetylase 1(HDAC1) | -0.442384859 | 0.180452648 | 0.014225081 | 0.037912066 |
| ENSG00000166797 | family with sequence similarity 96 member A(FAM96A) | -0.442275516 | 0.179425809 | 0.013703265 | 0.036762876 |
| ENSG00000104979 | chromosome 19 open reading frame 53(C19orf53) | -0.441930172 | 0.152526867 | 0.003762789 | 0.012437608 |
| ENSG00000120656 | TATA-box binding protein associated factor 12(TAF12) | -0.441318482 | 0.175277011 | 0.011807883 | 0.032417255 |
| ENSG00000114019 | angiomotin like 2(AMOTL2) | -0.441175555 | 0.168719306 | 0.008926684 | 0.025713396 |
| ENSG00000125733 | thyroid hormone receptor interactor 10(TRIP10) | -0.440619041 | 0.170373624 | 0.009704263 | 0.027537338 |
| ENSG00000081870 | heat shock protein family B (small) member 11(HSPB11) | -0.440360214 | 0.177169513 | 0.012935853 | 0.034983306 |
| ENSG00000137764 | mitogen-activated protein kinase kinase 5(MAP2K5) | -0.440336159 | 0.18649481 | 0.018219957 | 0.046839942 |
| ENSG00000249072 | NA | -0.440045065 | 0.152596656 | 0.003930159 | 0.012911028 |
| ENSG00000167283 | ATP synthase, H+ transporting, mitochondrial Fo complex subunit G(ATP5L) | -0.439685235 | 0.186574431 | 0.018442055 | 0.0473057 |
| ENSG00000085117 | CD82 molecule(CD82) | -0.439674268 | 0.175124863 | 0.01205149 | 0.032947648 |
| ENSG00000081791 | KIAA0141(KIAA0141) | -0.439498695 | 0.153126799 | 0.004102618 | 0.013406443 |
| ENSG00000139200 | PILR alpha associated neural protein(PIANP) | -0.439478085 | 0.179539349 | 0.014372771 | 0.038243191 |
| ENSG00000164010 | erythroblast membrane associated protein (Scianna blood group)(ERMAP) | -0.438439057 | 0.178205712 | 0.013882191 | 0.037208954 |
| ENSG00000196405 | Enah/Vasp-like(EVL) | -0.437911533 | 0.174323888 | 0.012002972 | 0.032868473 |
| ENSG00000182551 | acireductone dioxygenase 1(ADI1) | -0.437435575 | 0.173233897 | 0.011566032 | 0.031856915 |
| ENSG00000184164 | cysteine rich with EGF like domains 2(CRELD2) | -0.436617598 | 0.176163336 | 0.013194289 | 0.03556774 |
| ENSG00000111275 | aldehyde dehydrogenase 2 family (mitochondrial)(ALDH2) | -0.435944499 | 0.177535816 | 0.014067706 | 0.037603385 |
| ENSG00000112290 | WAS protein family member 1(WASF1) | -0.433945866 | 0.181741632 | 0.016953827 | 0.044017837 |
| ENSG00000130313 | 6-phosphogluconolactonase(PGLS) | -0.433815969 | 0.168728386 | 0.01013788 | 0.028565753 |
| ENSG00000160783 | polyamine modulated factor 1(PMF1) | -0.433792099 | 0.152520202 | 0.004452846 | 0.014351584 |
| ENSG00000182796 | transmembrane protein 198B (pseudogene)(TMEM198B) | -0.433776172 | 0.177120137 | 0.014323334 | 0.038122105 |
| ENSG00000105640 | ribosomal protein L18a(RPL18A) | -0.433704385 | 0.148148984 | 0.003417122 | 0.011508451 |
| ENSG00000160741 | CREB regulated transcription coactivator 2(CRTC2) | -0.432157552 | 0.15199401 | 0.004465549 | 0.014387295 |
| ENSG00000108528 | solute carrier family 25 member 11(SLC25A11) | -0.431872027 | 0.143446656 | 0.002606631 | 0.009123597 |
| ENSG00000129354 | adaptor related protein complex 1 mu 2 subunit(AP1M2) | -0.431520398 | 0.164696243 | 0.008790434 | 0.025426769 |
| ENSG00000163374 | YY1 associated protein 1(YY1AP1) | -0.431074701 | 0.152271212 | 0.004640758 | 0.014831263 |
| ENSG00000167565 | SERTA domain containing 3(SERTAD3) | -0.430549909 | 0.173000232 | 0.012820228 | 0.034734492 |
| ENSG00000099974 | D-dopachrome tautomerase-like(DDTL) | -0.429957033 | 0.173166872 | 0.013031554 | 0.035177421 |
| ENSG00000135506 | OS9, endoplasmic reticulum lectin(OS9) | -0.427693169 | 0.173094936 | 0.013478898 | 0.0362436 |
| ENSG00000114956 | deoxyguanosine kinase(DGUOK) | -0.426222361 | 0.176895199 | 0.015976035 | 0.041811362 |
| ENSG00000187764 | semaphorin 4D(SEMA4D) | -0.425489126 | 0.16816533 | 0.011400292 | 0.031496304 |
| ENSG00000131899 | LLGL1, scribble cell polarity complex component(LLGL1) | -0.425163076 | 0.180640914 | 0.018590773 | 0.047605977 |
| ENSG00000090863 | golgi glycoprotein 1(GLG1) | -0.424799297 | 0.15402701 | 0.005816451 | 0.017898177 |
| ENSG00000198258 | ubiquitin like 5(UBL5) | -0.424777269 | 0.17787583 | 0.016937806 | 0.043985948 |
| ENSG00000171530 | tubulin folding cofactor A(TBCA) | -0.422282122 | 0.150154655 | 0.004918638 | 0.015537823 |
| ENSG00000129353 | solute carrier family 44 member 2(SLC44A2) | -0.421169291 | 0.167717673 | 0.012032827 | 0.032919577 |
| ENSG00000187800 | platelet endothelial aggregation receptor 1(PEAR1) | -0.419601204 | 0.161266334 | 0.009270498 | 0.026534826 |
| ENSG00000114353 | G protein subunit alpha i2(GNAI2) | -0.419121993 | 0.152679022 | 0.006048907 | 0.018516613 |
| ENSG00000198171 | DDRGK domain containing 1(DDRGK1) | -0.418934607 | 0.173577874 | 0.015799027 | 0.041412601 |
| ENSG00000143222 | ubiquitin-fold modifier conjugating enzyme 1(UFC1) | -0.417828488 | 0.155793707 | 0.007319783 | 0.021733955 |
| ENSG00000154723 | ATP synthase, H+ transporting, mitochondrial Fo complex subunit F6(ATP5J) | -0.41456491 | 0.151681871 | 0.006273727 | 0.019090567 |
| ENSG00000144040 | sideroflexin 5(SFXN5) | -0.414489658 | 0.170109514 | 0.014825873 | 0.039299588 |
| ENSG00000102125 | tafazzin(TAZ) | -0.41412231 | 0.175150041 | 0.018059984 | 0.046499813 |
| ENSG00000106400 | zinc finger HIT-type containing 1(ZNHIT1) | -0.413910665 | 0.174033002 | 0.017390508 | 0.045002625 |
| ENSG00000105193 | ribosomal protein S16(RPS16) | -0.413899956 | 0.154638353 | 0.007438063 | 0.022051758 |
| ENSG00000124155 | phosphatidylinositol glycan anchor biosynthesis class T(PIGT) | -0.413260057 | 0.158694883 | 0.009211128 | 0.026397006 |
| ENSG00000105755 | ETHE1, persulfide dioxygenase(ETHE1) | -0.412814178 | 0.151218817 | 0.006335109 | 0.019247474 |
| ENSG00000167632 | trafficking protein particle complex 9(TRAPPC9) | -0.412524908 | 0.162083489 | 0.010923444 | 0.03045826 |
| ENSG00000120725 | SIL1 nucleotide exchange factor(SIL1) | -0.412361285 | 0.16973484 | 0.015122002 | 0.039958496 |
| ENSG00000005339 | CREB binding protein(CREBBP) | -0.408738197 | 0.16085962 | 0.011054791 | 0.03076894 |
| ENSG00000241837 | ATP synthase, H+ transporting, mitochondrial F1 complex, O subunit(ATP5O) | -0.408636583 | 0.161919233 | 0.011612491 | 0.031943223 |
| ENSG00000175592 | FOS like 1, AP-1 transcription factor subunit(FOSL1) | -0.408465223 | 0.146752833 | 0.005379991 | 0.016770063 |
| ENSG00000135334 | akirin 2(AKIRIN2) | -0.407803102 | 0.161831909 | 0.011738225 | 0.032248595 |
| ENSG00000134779 | tubulin polyglutamylase complex subunit 2(TPGS2) | -0.407566254 | 0.167332952 | 0.014864631 | 0.039375707 |
| ENSG00000213694 | sphingosine-1-phosphate receptor 3(S1PR3) | -0.405994591 | 0.168410465 | 0.01592 | 0.041673983 |
| ENSG00000129195 | family with sequence similarity 64 member A(FAM64A) | -0.404671269 | 0.149237578 | 0.006696117 | 0.020182709 |
| ENSG00000089063 | transmembrane protein 230(TMEM230) | -0.404448609 | 0.153739621 | 0.008519978 | 0.024761685 |
| ENSG00000106635 | BCL tumor suppressor 7B(BCL7B) | -0.404134373 | 0.146909384 | 0.005943012 | 0.018235157 |
| ENSG00000063177 | ribosomal protein L18(RPL18) | -0.402551844 | 0.164145314 | 0.014190231 | 0.037853487 |
| ENSG00000196547 | mannosidase alpha class 2A member 2(MAN2A2) | -0.400852484 | 0.163567297 | 0.014258331 | 0.037992074 |
| ENSG00000140612 | SEC11 homolog A, signal peptidase complex subunit(SEC11A) | -0.398622284 | 0.152318664 | 0.008869894 | 0.025587403 |
| ENSG00000106853 | prostaglandin reductase 1(PTGR1) | -0.396806355 | 0.169359332 | 0.019130158 | 0.048658849 |
| ENSG00000104853 | CLPTM1, transmembrane protein(CLPTM1) | -0.396315144 | 0.163092171 | 0.015098524 | 0.039914391 |
| ENSG00000054967 | RELT tumor necrosis factor receptor(RELT) | -0.395433627 | 0.167641252 | 0.018333709 | 0.047060386 |
| ENSG00000099785 | membrane associated ring-CH-type finger 2(MARCH2) | -0.394070577 | 0.160740197 | 0.014222288 | 0.037912066 |
| ENSG00000127483 | heterochromatin protein 1 binding protein 3(HP1BP3) | -0.393201475 | 0.145908383 | 0.007041993 | 0.021025891 |
| ENSG00000235043 | NA | -0.392769947 | 0.150347458 | 0.008990506 | 0.02586557 |
| ENSG00000141736 | erb-b2 receptor tyrosine kinase 2(ERBB2) | -0.392393659 | 0.158793545 | 0.013470068 | 0.036228139 |
| ENSG00000269028 | MT-RNR2-like 2(MTRNR2L2) | -0.392263687 | 0.151564583 | 0.009650809 | 0.02743647 |
| ENSG00000105583 | WD repeat domain 83 opposite strand(WDR83OS) | -0.392041076 | 0.148275124 | 0.008192997 | 0.023939993 |
| ENSG00000105372 | ribosomal protein S19(RPS19) | -0.391179418 | 0.161259141 | 0.01527547 | 0.040300657 |
| ENSG00000117691 | neudesin neurotrophic factor(NENF) | -0.390877205 | 0.162044625 | 0.015858417 | 0.041540506 |
| ENSG00000087088 | BCL2 associated X, apoptosis regulator(BAX) | -0.39006776 | 0.16624668 | 0.018959867 | 0.048340676 |
| ENSG00000164096 | chromosome 4 open reading frame 3(C4orf3) | -0.389993521 | 0.163162292 | 0.016838354 | 0.04381473 |
| ENSG00000162231 | nuclear RNA export factor 1(NXF1) | -0.388917139 | 0.150055555 | 0.009546943 | 0.027220126 |
| ENSG00000149485 | microRNA 1908(MIR1908) | -0.386946233 | 0.1524068 | 0.011120033 | 0.03091117 |
| ENSG00000143429 | lymphocyte-specific protein 1 pseudogene(LOC645166) | -0.386055969 | 0.15498874 | 0.012743266 | 0.034557811 |
| | SWI/SNF related, matrix associated, actin dependent regulator of chromatin, | | | | |
| ENSG00000082014 | subfamily d, member 3(SMARCD3) | -0.384016987 | 0.149331603 | 0.010123793 | 0.028532894 |
| ENSG00000042753 | adaptor related protein complex 2 sigma 1 subunit(AP2S1) | -0.383039523 | 0.156855634 | 0.014606646 | 0.038753402 |
| ENSG00000172819 | retinoic acid receptor gamma(RARG) | -0.383026163 | 0.145948652 | 0.008680436 | 0.025133313 |
| ENSG00000008838 | microRNA 6884(MIR6884) | -0.382789887 | 0.152943771 | 0.012321011 | 0.033575254 |
| ENSG00000185201 | interferon induced transmembrane protein 2(IFITM2) | -0.380618792 | 0.144700803 | 0.008528827 | 0.024779729 |
| ENSG00000121064 | serine carboxypeptidase 1(SCPEP1) | -0.379532507 | 0.156852504 | 0.015534268 | 0.040818658 |
| ENSG00000140365 | COMM domain containing 4(COMMD4) | -0.377578391 | 0.160626698 | 0.018740262 | 0.047884546 |
| ENSG00000112576 | cyclin D3(CCND3) | -0.376842165 | 0.152819882 | 0.013666045 | 0.036679753 |
| ENSG00000161021 | mastermind like transcriptional coactivator 1(MAML1) | -0.376609519 | 0.159592471 | 0.0182838 | 0.046973274 |
| ENSG00000022267 | four and a half LIM domains 1(FHL1) | -0.374871932 | 0.14975455 | 0.012306124 | 0.033542457 |
| ENSG00000125648 | solute carrier family 25 member 23(SLC25A23) | -0.374435294 | 0.153957986 | 0.015013157 | 0.03970656 |
| ENSG00000142089 | interferon induced transmembrane protein 3(IFITM3) | -0.371700916 | 0.155521755 | 0.016847216 | 0.043818407 |
| ENSG00000076944 | syntaxin binding protein 2(STXBP2) | -0.370866164 | 0.156801313 | 0.01802043 | 0.046418293 |
| ENSG00000134825 | microRNA 611(MIR611) | -0.367787012 | 0.157321485 | 0.019397347 | 0.049147188 |
| ENSG00000100439 | abhydrolase domain containing 4(ABHD4) | -0.367498857 | 0.156755494 | 0.019057304 | 0.048494511 |
| ENSG00000133318 | reticulon 3(RTN3) | -0.36574911 | 0.14667804 | 0.012647257 | 0.034344951 |
| ENSG00000134824 | fatty acid desaturase 2(FADS2) | -0.363583894 | 0.153316569 | 0.017718032 | 0.045740437 |
| ENSG00000078061 | A-Raf proto-oncogene, serine/threonine kinase(ARAF) | -0.355636423 | 0.146168717 | 0.014972056 | 0.039624581 |
| ENSG00000185043 | calcium and integrin binding 1(CIB1) | -0.351890771 | 0.144919435 | 0.015174724 | 0.040070809 |
| ENSG00000100348 | thioredoxin 2(TXN2) | -0.347490731 | 0.146511263 | 0.017703201 | 0.045731325 |
| ENSG00000159335 | parathymosin(PTMS) | -0.347127344 | 0.14800167 | 0.01900522 | 0.048393378 |

**Table 3B: Genes induced by IL1β and inhibited by BT2 logFC >=2**

| **Gene** | **Description** | **logFC:Induced by IL-1β** | **logFC:Inhibited by BT2** |
|---|---|---|---|
| ENSG00000163739 | C-X-C motif chemokine ligand 1(CXCL1) | 13.96900561 | -3.421722765 |
| ENSG00000115009 | C-C motif chemokine ligand 20(CCL20) | 13.26369408 | -3.088261993 |
| ENSG00000108342 | colony stimulating factor 3(CSF3) | 12.37526618 | -2.486011924 |
| ENSG00000169429 | C-X-C motif chemokine ligand 8(CXCL8) | 12.35654278 | -3.607945957 |
| ENSG00000081041 | C-X-C motif chemokine ligand 2(CXCL2) | 12.11926805 | -4.84494193 |
| ENSG00000163734 | C-X-C motif chemokine ligand 3(CXCL3) | 12.0923909 | -4.487286536 |
| ENSG00000164400 | colony stimulating factor 2(CSF2) | 11.98710322 | -3.818708412 |
| ENSG00000105371 | intercellular adhesion molecule 4 (Landsteiner-Wiener blood group)(ICAM4) | 9.532972736 | -5.291180651 |
| ENSG00000124875 | C-X-C motif chemokine ligand 6(CXCL6) | 9.447801428 | -2.165343451 |
| ENSG00000118503 | TNF alpha induced protein 3(TNFAIP3) | 8.517668436 | -2.937976363 |
| ENSG00000136244 | interleukin 6(IL6) | 8.285711102 | -3.773082183 |
| ENSG00000267059 | ubiquinol-cytochrome c reductase, complex III subunit XI(UQCR11) | 7.168192546 | -3.610917086 |
| ENSG00000163874 | microRNA 6732(MIR6732) | 6.99848928 | -2.240141482 |
| ENSG00000128016 | ZFP36 ring finger protein(ZFP36) | 6.923460299 | -5.233766514 |
| ENSG00000108691 | C-C motif chemokine ligand 2(CCL2) | 6.836075704 | -2.525591194 |
| ENSG00000171223 | JunB proto-oncogene, AP-1 transcription factor subunit(JUNB) | 6.22594493 | -4.439704537 |
| ENSG00000144802 | NFKB inhibitor zeta(NFKBIZ) | 6.072479332 | -2.054645605 |
| ENSG00000221869 | CCAAT/enhancer binding protein delta(CEBPD) | 5.801387656 | -3.11155979 |
| ENSG00000019549 | snail family transcriptional repressor 2(SNAI2) | 5.713577126 | -2.336223423 |
| ENSG00000163435 | E74 like ETS transcription factor 3(ELF3) | 5.633331659 | -2.915501624 |
| ENSG00000188293 | IGF like family member 1(IGFL1) | 5.604522465 | -3.038575344 |
| ENSG00000218426 | - | 5.493003088 | -3.206669489 |
| ENSG00000116741 | regulator of G-protein signaling 2(RGS2) | 5.446653743 | -4.113629168 |
| ENSG00000162692 | vascular cell adhesion molecule 1(VCAM1) | 5.430533097 | -3.357244281 |
| ENSG00000125740 | FosB proto-oncogene, AP-1 transcription factor subunit(FOSB) | 5.20213564 | -4.39246161 |
| ENSG00000120129 | dual specificity phosphatase 1(DUSP1) | 5.135712289 | -2.659219592 |
| ENSG00000177606 | Jun proto-oncogene, AP-1 transcription factor subunit(JUN) | 5.076087449 | -3.713380171 |
| ENSG00000173391 | oxidized low density lipoprotein receptor 1(OLR1) | 5.006555758 | -2.426590495 |
| ENSG00000198576 | activity regulated cytoskeleton associated protein(ARC) | 4.902741252 | -4.770568608 |
| ENSG00000130518 | KIAA1683(KIAA1683) | 4.768469531 | -6.046181314 |
| ENSG00000127129 | endothelin 2(EDN2) | 4.593994666 | -2.848547622 |
| ENSG00000184371 | colony stimulating factor 1(CSF1) | 4.559262331 | -2.085693014 |
| ENSG00000170345 | Fos proto-oncogene, AP-1 transcription factor subunit(FOS) | 4.487202447 | -5.81280372 |
| ENSG00000123358 | nuclear receptor subfamily 4 group A member 1(NR4A1) | 4.476568832 | -4.035692161 |
| ENSG00000179388 | early growth response 3(EGR3) | 4.372265836 | -4.748606448 |
| ENSG00000109321 | amphiregulin(AREG) | 3.931790234 | -2.491152934 |
| ENSG00000136826 | Kruppel like factor 4(KLF4) | 3.793211171 | -2.684611128 |
| ENSG00000160888 | immediate early response 2(IER2) | 3.698751836 | -3.10770709 |
| ENSG00000188483 | immediate early response 5 like(IER5L) | 3.698131042 | -4.233292009 |
| ENSG00000099860 | growth arrest and DNA damage inducible beta(GADD45B) | 3.669582901 | -2.155058852 |
| ENSG00000272196 | histone cluster 2 H2A family member a4(HIST2H2AA4) | 3.654733117 | -3.149273568 |
| ENSG00000185650 | ZFP36 ring finger protein like 1(ZFP36L1) | 3.642709132 | -2.089088105 |
| ENSG00000162772 | activating transcription factor 3(ATF3) | 3.635121332 | -2.20839475 |
| ENSG00000108551 | ras related dexamethasone induced 1(RASD1) | 3.62445948 | -2.512046377 |
| ENSG00000152518 | ZFP36 ring finger protein like 2(ZFP36L2) | 3.488339502 | -2.598134923 |
| ENSG00000203811 | histone cluster 2 H3 family member c(HIST2H3C) | 3.428484423 | -4.763925928 |
| ENSG00000169242 | ephrin A1(EFNA1) | 3.42346034 | -2.409787071 |
| ENSG00000105376 | intercellular adhesion molecule 5(ICAM5) | 3.318325272 | -2.593632739 |
| ENSG00000137449 | cytoplasmic polyadenylation element binding protein 2(CPEB2) | 3.294341864 | -2.416506755 |
| ENSG00000183496 | mex-3 RNA binding family member B(MEX3B) | 3.292666719 | -2.273063239 |
| ENSG00000115844 | distal-less homeobox 2(DLX2) | 3.261131993 | -3.200287357 |
| ENSG00000120738 | early growth response 1(EGR1) | 3.235227809 | -4.070710146 |
| ENSG00000168477 | tenascin XB(TNXB) | 3.157931975 | -3.263931496 |
| ENSG00000139329 | lumican(LUM) | 3.145317007 | -2.7112042 |
| ENSG00000130522 | JunD proto-oncogene, AP-1 transcription factor subunit(JUND) | 3.072038382 | -2.075823946 |
| ENSG00000243449 | chromosome 4 open reading frame 48(C4orf48) | 2.96463919 | -2.112571645 |
| ENSG00000105327 | microRNA 3191(MIR3191) | 2.902423182 | -2.152382139 |
| ENSG00000164949 | GTP binding protein overexpressed in skeletal muscle(GEM) | 2.88683507 | -2.820631629 |
| ENSG00000104899 | microRNA 4321(MIR4321) | 2.858218738 | -2.479501612 |
| ENSG00000181218 | histone cluster 3 H2A(HIST3H2A) | 2.851693019 | -3.620778057 |
| ENSG00000162924 | REL proto-oncogene, NF-kB subunit(REL) | 2.790557104 | -2.517805862 |
| ENSG00000143878 | ras homolog family member B(RHOB) | 2.788482624 | -2.194356004 |
| ENSG00000102878 | heat shock transcription factor 4(HSF4) | 2.768266578 | -2.650455425 |
| ENSG00000089692 | lymphocyte activating 3(LAG3) | 2.765530963 | -3.415913177 |
| ENSG00000139289 | pleckstrin homology like domain family A member 1(PHLDA1) | 2.729157328 | -2.567199114 |
| ENSG00000164442 | Cbp/p300 interacting transactivator with Glu/Asp rich carboxy-terminal domain 2(CITED2) | 2.690190316 | -2.290371541 |
| ENSG00000158373 | histone cluster 1 H2B family member d(HIST1H2BD) | 2.645317177 | -3.501034382 |
| ENSG00000087074 | protein phosphatase 1 regulatory subunit 15A(PPP1R15A) | 2.638898606 | -2.101900503 |
| ENSG00000197483 | zinc finger protein 628(ZNF628) | 2.629470566 | -2.020027279 |
| ENSG00000222047 | chromosome 10 open reading frame 55(C10orf55) | 2.610278529 | -2.534916862 |
| ENSG00000102554 | Kruppel like factor 5(KLF5) | 2.527027281 | -2.05370008 |
| ENSG00000114315 | hes family bHLH transcription factor 1(HES1) | 2.520479519 | -2.238445441 |
| ENSG00000147883 | cyclin dependent kinase inhibitor 2B(CDKN2B) | 2.492912588 | -2.024327858 |
| ENSG00000156427 | fibroblast growth factor 18(FGF18) | 2.459828118 | -3.320172319 |
| ENSG00000162733 | discoidin domain receptor tyrosine kinase 2(DDR2) | 2.455979518 | -2.538720005 |
| ENSG00000125968 | inhibitor of DNA binding 1, HLH protein(ID1) | 2.453492819 | -3.021043337 |
| ENSG00000185338 | suppressor of cytokine signaling 1(SOCS1) | 2.437248307 | -2.130055169 |
| ENSG00000115738 | inhibitor of DNA binding 2, HLH protein(ID2) | 2.389577065 | -2.582567927 |
| ENSG00000100625 | SIX homeobox 4(SIX4) | 2.324820181 | -2.081865037 |
| ENSG00000130513 | growth differentiation factor 15(GDF15) | 2.316722037 | -3.349977694 |
| ENSG00000182759 | MAF bZIP transcription factor A(MAFA) | 2.30059938 | -2.741653406 |
| ENSG00000128917 | delta like canonical Notch ligand 4(DLL4) | 2.272772187 | -2.473176483 |
| ENSG00000004777 | Rho GTPase activating protein 33(ARHGAP33) | 2.269803341 | -2.365826313 |
| ENSG00000204839 | maestro heat like repeat family member 6(MROH6) | 2.255049118 | -2.110544407 |
| ENSG00000165312 | OTU deubiquitinase 1(OTUD1) | 2.219300098 | -2.029552583 |
| ENSG00000162572 | sodium channel epithelial 1 delta subunit(SCNN1D) | 2.185901322 | -2.026636946 |
| ENSG00000146278 | proline rich nuclear receptor coactivator 1(PNRC1) | 2.090639266 | -2.392607114 |
| ENSG00000274272 | - | 2.080023968 | -2.27836831 |
| ENSG00000113742 | cytoplasmic polyadenylation element binding protein 4(CPEB4) | 2.057832248 | -2.183085711 |

**Table 3C: Difference in gene expression induced by IL-1β compared untreated (UT)**

| | | FC= Fold Change | | | |
|---|---|---|---|---|---|
| **Gene** | **Description** | **logFC** | **IfcSE** | **PValue** | **FDR** |
| ENSG00000163739 | C-X-C motif chemokine ligand 1(CXCL1) | 13.9690056 | 0.76568123 | 2.31E-74 | 1.13E-71 |
| ENSG00000115009 | C-C motif chemokine ligand 20(CCL20) | 13.2636941 | 1.39963197 | 2.63E-21 | 2.24E-19 |
| ENSG00000108342 | colony stimulating factor 3(CSF3) | 12.3752662 | 0.69903117 | 3.94E-70 | 1.66E-67 |
| ENSG00000169429 | C-X-C motif chemokine ligand 8(CXCL8) | 12.3565428 | 0.39002216 | 2.78E-220 | 1.64E-216 |
| ENSG00000081041 | C-X-C motif chemokine ligand 2(CXCL2) | 12.1192681 | 0.52130801 | 1.50E-119 | 2.52E-116 |
| ENSG00000163734 | C-X-C motif chemokine ligand 3(CXCL3) | 12.0923909 | 0.57106864 | 1.62E-99 | 1.36E-96 |
| ENSG00000164400 | colony stimulating factor 2(CSF2) | 11.9871032 | 0.69226859 | 3.58E-67 | 1.41E-64 |
| ENSG00000269590 | NA | 10.3567974 | 1.23664032 | 5.52E-17 | 3.74E-15 |
| ENSG00000196136 | serpin family A member 3(SERPINA3) | 10.2091097 | 0.52561354 | 4.91E-84 | 3.04E-81 |
| ENSG00000105371 | intercellular adhesion molecule 4 (Landsteiner-Wiener blood group)(ICAM4) | 9.53297274 | 0.41264316 | 4.40E-118 | 6.47E-115 |
| ENSG00000124875 | C-X-C motif chemokine ligand 6(CXCL6) | 9.44780143 | 1.05692403 | 3.93E-19 | 3.08E-17 |
| ENSG00000124102 | peptidase inhibitor 3(PI3) | 9.37685556 | 1.22716226 | 2.15E-14 | 1.16E-12 |
| ENSG00000235333 | NA | 8.75397213 | 1.24321385 | 1.90E-12 | 8.65E-11 |
| ENSG00000118503 | TNF alpha induced protein 3(TNFAIP3) | 8.51766844 | 0.25325038 | 5.45E-248 | 6.42E-244 |
| ENSG00000136244 | interleukin 6(IL6) | 8.2857111 | 0.29282102 | 3.85E-176 | 1.51E-172 |
| ENSG00000113504 | solute carrier family 12 member 7(SLC12A7) | 8.06662084 | 0.33719771 | 1.79E-126 | 4.20E-123 |
| ENSG00000140379 | BCL2 related protein A1(BCL2A1) | 7.46338772 | 1.08015704 | 4.86E-12 | 2.16E-10 |
| ENSG00000267059 | ubiquinol-cytochrome c reductase, complex III subunit XI(UQCR11) | 7.16819255 | 2.08549669 | 0.00058784 | 0.00432284 |
| ENSG00000163874 | microRNA 6732(MIR6732) | 6.99848928 | 0.29813937 | 7.54E-122 | 1.48E-118 |
| ENSG00000128016 | ZFP36 ring finger protein(ZFP36) | 6.9234603 | 0.35100518 | 1.32E-86 | 9.74E-84 |
| ENSG00000023445 | baculoviral IAP repeat containing 3(BIRC3) | 6.8686717 | 0.38352705 | 9.99E-72 | 4.35E-69 |
| ENSG00000108691 | C-C motif chemokine ligand 2(CCL2) | 6.8360757 | 0.26178504 | 2.57E-150 | 7.57E-147 |
| ENSG00000164283 | endothelial cell specific molecule 1(ESM1) | 6.45765596 | 0.93096666 | 4.02E-12 | 1.79E-10 |
| ENSG00000122641 | inhibin beta A subunit(INHBA) | 6.43800623 | 0.36549824 | 1.91E-69 | 7.76E-67 |
| ENSG00000171223 | JunB proto-oncogene, AP-1 transcription factor subunit(JUNB) | 6.22594493 | 0.37095808 | 3.23E-63 | 1.22E-60 |
| ENSG00000144802 | NFKB inhibitor zeta(NFKBIZ) | 6.07247933 | 0.26984559 | 3.83E-112 | 4.51E-109 |
| ENSG00000268465 | NA | 6.03501295 | 0.76941664 | 4.38E-15 | 2.52E-13 |
| ENSG00000090339 | intercellular adhesion molecule 1(ICAM1) | 5.89868503 | 0.26386957 | 1.09E-110 | 1.17E-107 |
| ENSG00000221869 | CCAAT/enhancer binding protein delta(CEBPD) | 5.80138766 | 0.29830006 | 3.02E-84 | 1.97E-81 |
| ENSG00000019549 | snail family transcriptional repressor 2(SNAI2) | 5.71357713 | 0.39672693 | 5.04E-47 | 1.03E-44 |
| ENSG00000163661 | pentraxin 3(PTX3) | 5.67717561 | 0.299096 | 2.44E-80 | 1.31E-77 |
| ENSG00000163435 | E74 like ETS transcription factor 3(ELF3) | 5.63333166 | 0.29919743 | 4.44E-79 | 2.27E-76 |
| ENSG00000255439 | NA | 5.62000598 | 0.59407463 | 3.08E-21 | 2.60E-19 |
| ENSG00000188293 | IGF like family member 1(IGFL1) | 5.60452247 | 0.31166184 | 2.66E-72 | 1.20E-69 |
| ENSG00000218426 | NA | 5.49300309 | 0.4131418 | 2.45E-40 | 4.06E-38 |
| ENSG00000116741 | regulator of G-protein signaling 2(RGS2) | 5.44665374 | 0.37275176 | 2.35E-48 | 5.43E-46 |
| ENSG00000162692 | vascular cell adhesion molecule 1(VCAM1) | 5.4305331 | 0.37765035 | 6.93E-47 | 1.38E-44 |
| ENSG00000166016 | ankyrin repeat and BTB domain containing 2(ABTB2) | 5.3908287 | 0.36455779 | 1.77E-49 | 4.25E-47 |
| ENSG00000078401 | endothelin 1(EDN1) | 5.36673129 | 0.35871664 | 1.32E-50 | 3.24E-48 |
| ENSG00000109906 | zinc finger and BTB domain containing 16(ZBTB16) | 5.2969402 | 0.32164204 | 6.19E-61 | 2.08E-58 |
| ENSG00000125740 | FosB proto-oncogene, AP-1 transcription factor subunit(FOSB) | 5.20213564 | 0.26023449 | 6.70E-89 | 5.26E-86 |
| ENSG00000128271 | adenosine A2a receptor(ADORA2A) | 5.19867877 | 1.10418235 | 2.50E-06 | 4.08E-05 |
| ENSG00000185215 | TNF alpha induced protein 2(TNFAIP2) | 5.1755334 | 0.27174939 | 7.19E-81 | 4.03E-78 |
| ENSG00000120129 | dual specificity phosphatase 1(DUSP1) | 5.13571229 | 0.22780309 | 1.52E-112 | 1.99E-109 |
| ENSG00000177606 | Jun proto-oncogene, AP-1 transcription factor subunit(JUN) | 5.07608745 | 0.23416988 | 3.39E-104 | 3.32E-101 |
| ENSG00000104856 | RELB proto-oncogene, NF-kB subunit(RELB) | 5.04278616 | 0.30057985 | 3.60E-63 | 1.33E-60 |
| ENSG00000173391 | oxidized low density lipoprotein receptor 1(OLR1) | 5.00655576 | 0.33233126 | 2.75E-51 | 7.04E-49 |
| ENSG00000167772 | angiopoietin like 4(ANGPTL4) | 4.93524945 | 0.35830517 | 3.66E-43 | 6.73E-41 |
| ENSG00000100906 | NFKB inhibitor alpha(NFKBIA) | 4.91280613 | 0.23072331 | 1.32E-100 | 1.19E-97 |
| ENSG00000198576 | activity regulated cytoskeleton associated protein(ARC) | 4.90274125 | 0.32489314 | 1.88E-51 | 4.90E-49 |
| ENSG00000106366 | serpin family E member 1(SERPINE1) | 4.88578101 | 0.26936956 | 1.60E-73 | 7.54E-71 |
| ENSG00000176692 | forkhead box C2(FOXC2) | 4.85693426 | 0.31125949 | 6.82E-55 | 1.96E-52 |
| ENSG00000130518 | KIAA1683(KIAA1683) | 4.76846953 | 0.24720748 | 6.60E-83 | 3.88E-80 |
| ENSG00000134339 | serum amyloid A2(SAA2) | 4.69466557 | 0.4645727 | 5.23E-24 | 5.00E-22 |
| ENSG00000232133 | NA | 4.68727617 | 0.33703144 | 5.70E-44 | 1.08E-41 |
| ENSG00000135477 | NA | 4.67179644 | 0.47454367 | 7.22E-23 | 6.48E-21 |
| ENSG00000143507 | dual specificity phosphatase 10(DUSP10) | 4.63718675 | 0.3655276 | 7.05E-37 | 1.14E-34 |
| ENSG00000125538 | interleukin 1 beta(IL1B) | 4.61924702 | 0.31885446 | 1.46E-47 | 3.13E-45 |
| ENSG00000127129 | endothelin 2(EDN2) | 4.59399467 | 0.28204521 | 1.20E-59 | 3.92E-57 |
| ENSG00000184371 | colony stimulating factor 1(CSF1) | 4.55926233 | 0.28070187 | 2.53E-59 | 8.04E-57 |
| ENSG00000070404 | follistatin like 3(FSTL3) | 4.49966822 | 0.2707599 | 5.11E-62 | 1.82E-59 |
| ENSG00000170345 | Fos proto-oncogene, AP-1 transcription factor subunit(FOS) | 4.48720245 | 0.23059659 | 2.44E-84 | 1.69E-81 |
| ENSG00000123358 | nuclear receptor subfamily 4 group A member 1(NR4A1) | 4.47656883 | 0.29619153 | 1.31E-51 | 3.51E-49 |
| ENSG00000137507 | leucine rich repeat containing 32(LRRC32) | 4.41942157 | 0.40918493 | 3.42E-27 | 3.99E-25 |
| ENSG00000173432 | serum amyloid A1(SAA1) | 4.4107103 | 0.32783204 | 2.91E-41 | 5.03E-39 |
| ENSG00000101096 | nuclear factor of activated T-cells 2(NFATC2) | 4.40924342 | 0.30216224 | 3.14E-48 | 7.11E-46 |
| ENSG00000118515 | serum/glucocorticoid regulated kinase 1(SGK1) | 4.39958283 | 0.27347806 | 3.12E-58 | 9.66E-56 |
| ENSG00000179388 | early growth response 3(EGR3) | 4.37226584 | 0.31780722 | 4.58E-43 | 8.30E-41 |
| ENSG00000178882 | filamin-interacting protein FAM101A(ZNF664-FAM101A) | 4.36951189 | 0.31529629 | 1.13E-43 | 2.11E-41 |
| ENSG00000119138 | Kruppel like factor 9(KLF9) | 4.26502875 | 0.3743521 | 4.53E-30 | 5.61E-28 |
| ENSG00000128342 | leukemia inhibitory factor(LIF) | 4.22779088 | 0.25531575 | 1.38E-61 | 4.77E-59 |
| ENSG00000056558 | TNF receptor associated factor 1(TRAF1) | 4.22694679 | 0.26678613 | 1.55E-56 | 4.67E-54 |
| ENSG00000067082 | Kruppel like factor 6(KLF6) | 4.08480949 | 0.27131955 | 3.18E-51 | 7.97E-49 |
| ENSG00000166920 | microRNA 147b(MIR147B) | 3.98718604 | 0.33760144 | 3.45E-32 | 4.67E-30 |
| ENSG00000141682 | phorbol-12-myristate-13-acetate-induced protein 1(PMAIP1) | 3.97114162 | 0.34781983 | 3.43E-30 | 4.29E-28 |
| ENSG00000109321 | amphiregulin(AREG) | 3.93179023 | 0.32387829 | 6.51E-34 | 9.57E-32 |
| ENSG00000179862 | Cbp/p300 interacting transactivator with Glu/Asp rich carboxy-terminal domain 4 | 3.8359609 | 0.324449 | 2.97E-32 | 4.06E-30 |
| ENSG00000134070 | interleukin 1 receptor associated kinase 2(IRAK2) | 3.79477908 | 0.24315569 | 6.59E-55 | 1.94E-52 |
| ENSG00000136826 | Kruppel like factor 4(KLF4) | 3.79321117 | 0.27584518 | 5.01E-43 | 8.93E-41 |
| ENSG00000148344 | prostaglandin E synthase(PTGES) | 3.7583197 | 0.35202477 | 1.31E-26 | 1.39E-24 |
| ENSG00000148677 | ankyrin repeat domain 1(ANKRD1) | 3.74915003 | 0.35084771 | 1.18E-26 | 1.29E-24 |
| ENSG00000160888 | immediate early response 2(IER2) | 3.69875184 | 0.2551971 | 1.33E-47 | 2.89E-45 |
| ENSG00000188483 | immediate early response 5 like(IER5L) | 3.69813104 | 0.26585052 | 5.46E-44 | 1.05E-41 |
| ENSG00000173334 | tribbles pseudokinase 1(TRIB1) | 3.69520343 | 0.25454665 | 9.48E-48 | 2.11E-45 |
| ENSG00000099860 | growth arrest and DNA damage inducible beta(GADD45B) | 3.6695829 | 0.24039058 | 1.31E-52 | 3.58E-50 |
| ENSG00000112096 | uncharacterized LOC100129518(LOC100129518) | 3.6668101 | 0.2743907 | 9.89E-41 | 1.66E-38 |
| ENSG00000272196 | histone cluster 2 H2A family member a4(HIST2H2AA4) | 3.65473312 | 0.35763992 | 1.63E-24 | 1.61E-22 |
| ENSG00000185650 | ZFP36 ring finger protein like 1(ZFP36L1) | 3.64270913 | 0.23470706 | 2.53E-54 | 7.09E-52 |
| ENSG00000003989 | solute carrier family 7 member 2(SLC7A2) | 3.63569485 | 0.30935783 | 6.86E-32 | 9.08E-30 |
| ENSG00000162772 | activating transcription factor 3(ATF3) | 3.63512133 | 0.25242172 | 5.10E-47 | 1.03E-44 |
| ENSG00000108551 | ras related dexamethasone induced 1(RASD1) | 3.62445948 | 0.30497465 | 1.43E-32 | 2.00E-30 |
| ENSG00000184557 | suppressor of cytokine signaling 3(SOCS3) | 3.62081658 | 0.26700817 | 6.85E-42 | 1.20E-39 |
| ENSG00000069399 | microRNA 8085(MIR8085) | 3.60254579 | 0.29975767 | 2.85E-33 | 4.09E-31 |
| ENSG00000115963 | Rho family GTPase 3(RND3) | 3.59838896 | 0.33677579 | 1.20E-26 | 1.29E-24 |
| ENSG00000197149 | NA | 3.58854736 | 1.16517018 | 0.00207106 | 0.01190571 |
| ENSG00000049249 | TNF receptor superfamily member 9(TNFRSF9) | 3.58636611 | 0.34180762 | 9.37E-26 | 9.67E-24 |
| ENSG00000171522 | prostaglandin E receptor 4(PTGER4) | 3.54461994 | 0.37015449 | 1.01E-21 | 8.79E-20 |
| ENSG00000125148 | metallothionein 2A(MT2A) | 3.53612142 | 0.37765837 | 7.73E-21 | 6.36E-19 |
| ENSG00000064270 | ATPase secretory pathway Ca2+ transporting 2(ATP2C2) | 3.48961206 | 0.39865148 | 2.07E-18 | 1.56E-16 |
| ENSG00000152518 | ZFP36 ring finger protein like 2(ZFP36L2) | 3.4883395 | 0.25938352 | 3.14E-41 | 5.35E-39 |
| ENSG00000187193 | metallothionein 1X(MT1X) | 3.44577167 | 0.29450539 | 1.27E-31 | 1.64E-29 |
| ENSG00000172216 | CCAAT/enhancer binding protein beta(CEBPB) | 3.43407386 | 0.27569896 | 1.30E-35 | 2.01E-33 |
| ENSG00000173918 | C1q and tumor necrosis factor related protein 1(C1QTNF1) | 3.43065875 | 0.32962809 | 2.29E-25 | 2.34E-23 |
| ENSG00000203811 | histone cluster 2 H3 family member c(HIST2H3C) | 3.42848442 | 0.27012546 | 6.53E-37 | 1.07E-34 |
| ENSG00000169242 | ephrin A1(EFNA1) | 3.42346034 | 0.23708905 | 2.92E-47 | 6.13E-45 |
| ENSG00000136997 | v-myc avian myelocytomatosis viral oncogene homolog(MYC) | 3.40928807 | 0.2332175 | 2.14E-48 | 5.04E-46 |
| ENSG00000141448 | GATA binding protein 6(GATA6) | 3.3969746 | 0.35825605 | 2.50E-21 | 2.14E-19 |
| ENSG00000164056 | sprouty RTK signaling antagonist 1(SPRY1) | 3.38970255 | 0.29247883 | 4.65E-31 | 5.95E-29 |
| ENSG00000233929 | NA | 3.37924322 | 0.37904989 | 4.88E-19 | 3.80E-17 |
| ENSG00000165507 | chromosome 10 open reading frame 10(C10orf10) | 3.36684379 | 0.31187608 | 3.62E-27 | 4.17E-25 |
| ENSG00000170214 | adrenoceptor alpha 1B(ADRA1B) | 3.34365175 | 0.3775852 | 8.34E-19 | 6.41E-17 |
| ENSG00000124766 | SRY-box 4(SOX4) | 3.34364636 | 0.30112327 | 1.20E-28 | 1.43E-26 |
| ENSG00000105376 | intercellular adhesion molecule 5(ICAM5) | 3.31832527 | 0.4195873 | 2.60E-15 | 1.52E-13 |
| ENSG00000137449 | cytoplasmic polyadenylation element binding protein 2(CPEB2) | 3.29434186 | 0.34004316 | 3.39E-22 | 3.00E-20 |
| ENSG00000183496 | mex-3 RNA binding family member B(MEX3B) | 3.29266672 | 0.26229322 | 3.81E-36 | 5.98E-34 |
| ENSG00000240972 | macrophage migration inhibitory factor (glycosylation-inhibiting factor)(MIF) | 3.27648573 | 1.0330842 | 0.00151624 | 0.00930719 |
| ENSG00000115844 | distal-less homeobox 2(DLX2) | 3.26113199 | 0.32790835 | 2.64E-23 | 2.43E-21 |
| ENSG00000183458 | NA | 3.26044571 | 1.14854382 | 0.00452885 | 0.02205567 |
| ENSG00000120738 | early growth response 1(EGR1) | 3.23522781 | 0.2270113 | 4.39E-46 | 8.62E-44 |
| ENSG00000107984 | dickkopf WNT signaling pathway inhibitor 1(DKK1) | 3.23267632 | 0.29218866 | 1.88E-28 | 2.21E-26 |
| ENSG00000130066 | spermidine/spermine N1-acetyltransferase 1(SAT1) | 3.22076677 | 0.30326881 | 2.40E-26 | 2.50E-24 |
| ENSG00000110876 | selectin P ligand(SELPLG) | 3.22064093 | 0.31038344 | 3.18E-25 | 3.22E-23 |
| ENSG00000197632 | serpin family B member 2(SERPINB2) | 3.21718329 | 0.428056 | 5.66E-14 | 2.94E-12 |
| ENSG00000176597 | UDP-GIcNAc:betaGal beta-1,3-N-acetylglucosaminyltransferase 5(B3GNT5) | 3.19767378 | 0.31974858 | 1.51E-23 | 1.43E-21 |
| ENSG00000015568 | RANBP2-like and GRIP domain containing 5(RGPD5) | 3.18061809 | 0.50879097 | 4.07E-10 | 1.43E-08 |
| ENSG00000179094 | microRNA 6883(MIR6883) | 3.17309371 | 0.31570875 | 9.12E-24 | 8.66E-22 |
| ENSG00000111859 | neural precursor cell expressed, developmentally down-regulated 9(NEDD9) | 3.16916397 | 0.26736199 | 2.06E-32 | 2.86E-30 |
| ENSG00000168477 | tenascin XB(TNXB) | 3.15793198 | 0.42248403 | 7.74E-14 | 3.96E-12 |
| ENSG00000010818 | human immunodeficiency virus type I enhancer binding protein 2(HIVEP2) | 3.14643901 | 0.3080596 | 1.72E-24 | 1.69E-22 |
| ENSG00000139329 | lumican(LUM) | 3.14531701 | 0.42049021 | 7.43E-14 | 3.82E-12 |
| ENSG00000135111 | T-box 3(TBX3) | 3.14116586 | 0.24792045 | 8.67E-37 | 1.38E-34 |
| ENSG00000041982 | tenascin C(TNC) | 3.12356951 | 0.25221873 | 3.18E-35 | 4.85E-33 |
| ENSG00000146592 | cAMP responsive element binding protein 5(CREB5) | 3.11347967 | 0.31422505 | 3.83E-23 | 3.49E-21 |
| ENSG00000130522 | JunD proto-oncogene, AP-1 transcription factor subunit(JUND) | 3.07203838 | 0.24830651 | 3.71E-35 | 5.59E-33 |
| ENSG00000105246 | Epstein-Barr virus induced 3(EBI3) | 3.06624204 | 0.42399407 | 4.77E-13 | 2.32E-11 |
| ENSG00000175602 | coiled-coil domain containing 85B(CCDC85B) | 3.02544649 | 0.39731864 | 2.64E-14 | 1.41E-12 |
| ENSG00000183691 | noggin(NOG) | 2.99037578 | 0.36364537 | 1.98E-16 | 1.29E-14 |
| ENSG00000243449 | chromosome 4 open reading frame 48(C4orf48) | 2.96463919 | 0.96589819 | 0.00214555 | 0.01226981 |
| ENSG00000105088 | olfactomedin 2(OLFM2) | 2.9544716 | 0.45548284 | 8.79E-11 | 3.37E-09 |
| ENSG00000142178 | salt inducible kinase 1(SIK1) | 2.95436138 | 0.25146088 | 7.16E-32 | 9.37E-30 |
| ENSG00000243649 | complement factor B(CFB) | 2.94080237 | 0.34764008 | 2.69E-17 | 1.86E-15 |
| ENSG00000113916 | B-cell CLL/lymphoma 6(BCL6) | 2.93724523 | 0.2493217 | 4.89E-32 | 6.54E-30 |
| ENSG00000111780 | NA | 2.92837169 | 0.48463352 | 1.52E-09 | 4.92E-08 |
| ENSG00000144476 | atypical chemokine receptor 3(ACKR3) | 2.91739003 | 0.24213279 | 1.97E-33 | 2.86E-31 |
| ENSG00000185950 | insulin receptor substrate 2(IRS2) | 2.91230824 | 0.27181598 | 8.72E-27 | 9.68E-25 |
| ENSG00000218175 | NA | 2.91057886 | 1.17236723 | 0.01304087 | 0.04972097 |
| ENSG00000105327 | microRNA 3191(MIR3191) | 2.90242318 | 0.35298371 | 1.99E-16 | 1.30E-14 |
| ENSG00000164949 | GTP binding protein overexpressed in skeletal muscle(GEM) | 2.88683507 | 0.38543023 | 6.89E-14 | 3.56E-12 |
| ENSG00000213694 | sphingosine-1-phosphate receptor 3(S1PR3) | 2.87994427 | 0.26915868 | 1.02E-26 | 1.12E-24 |
| ENSG00000135842 | family with sequence similarity 129 member A(FAM129A) | 2.87145064 | 0.4840647 | 2.99E-09 | 9.22E-08 |
| ENSG00000104899 | microRNA 4321(MIR4321) | 2.85821874 | 0.42009575 | 1.02E-11 | 4.33E-10 |
| ENSG00000181218 | histone cluster 3 H2A(HIST3H2A) | 2.85169302 | 0.47986189 | 2.80E-09 | 8.70E-08 |
| ENSG00000141384 | TATA-box binding protein associated factor 4b(TAF4B) | 2.82860803 | 0.48002045 | 3.80E-09 | 1.14E-07 |
| ENSG00000185022 | MAF bZIP transcription factor F(MAFF) | 2.82736526 | 0.22970434 | 8.13E-35 | 1.21E-32 |
| ENSG00000112149 | CD83 molecule(CD83) | 2.82486043 | 0.26448091 | 1.25E-26 | 1.34E-24 |
| ENSG00000107968 | mitogen-activated protein kinase kinase kinase 8(MAP3K8) | 2.81835239 | 0.36071926 | 5.58E-15 | 3.19E-13 |
| ENSG00000149289 | zinc finger CCCH-type containing 12C(ZC3H12C) | 2.81574699 | 0.30559403 | 3.14E-20 | 2.55E-18 |
| ENSG00000187908 | deleted in malignant brain tumors 1(DMBT1) | 2.79295469 | 0.39449119 | 1.44E-12 | 6.58E-11 |
| ENSG00000162924 | REL proto-oncogene, NF-kB subunit(REL) | 2.7905571 | 0.32994279 | 2.73E-17 | 1.88E-15 |
| ENSG00000049192 | ADAM metallopeptidase with thrombospondin type 1 motif 6(ADAMTS6) | 2.78904643 | 0.41280693 | 1.42E-11 | 5.93E-10 |
| ENSG00000143878 | ras homolog family member B(RHOB) | 2.78848262 | 0.25955703 | 6.38E-27 | 7.21E-25 |
| ENSG00000145779 | TNF alpha induced protein 8(TNFAIP8) | 2.78802173 | 0.32011394 | 3.05E-18 | 2.25E-16 |
| ENSG00000053524 | MCF.2 cell line derived transforming sequence-like 2(MCF2L2) | 2.77248098 | 0.34543965 | 1.01E-15 | 6.08E-14 |
| ENSG00000102878 | heat shock transcription factor 4(HSF4) | 2.76826658 | 0.36726819 | 4.79E-14 | 2.52E-12 |
| ENSG00000089692 | lymphocyte activating 3(LAG3) | 2.76553096 | 0.32187742 | 8.56E-18 | 6.06E-16 |
| ENSG00000256040 | PAPPA antisense RNA 1(PAPPA-AS1) | 2.76054875 | 0.48778683 | 1.52E-08 | 4.15E-07 |
| ENSG00000095951 | human immunodeficiency virus type I enhancer binding protein 1(HIVEP1) | 2.75812424 | 0.31656233 | 2.97E-18 | 2.21E-16 |
| ENSG00000127666 | toll like receptor adaptor molecule 1(TICAM1) | 2.73465289 | 0.33409179 | 2.72E-16 | 1.72E-14 |
| ENSG00000139289 | pleckstrin homology like domain family A member 1(PHLDA1) | 2.72915733 | 0.22810763 | 5.46E-33 | 7.74E-31 |
| ENSG00000165030 | nuclear factor, interleukin 3 regulated(NFIL3) | 2.7132794 | 0.30301167 | 3.42E-19 | 2.70E-17 |
| ENSG00000184545 | dual specificity phosphatase 8(DUSP8) | 2.69889349 | 0.3254541 | 1.11E-16 | 7.28E-15 |
| ENSG00000164442 | Cbp/p300 interacting transactivator with Glu/Asp rich carboxy-terminal domain 2 | 2.69019032 | 0.24009226 | 3.86E-29 | 4.64E-27 |
| ENSG00000197249 | serpin family A member 1(SERPINA1) | 2.68538995 | 0.25883734 | 3.23E-25 | 3.25E-23 |
| ENSG00000137193 | Pim-1 proto-oncogene, serine/threonine kinase(PIM1) | 2.67701451 | 0.23440567 | 3.31E-30 | 4.18E-28 |
| ENSG00000144655 | cysteine and serine rich nuclear protein 1(CSRNP1) | 2.6752014 | 0.28634721 | 9.41E-21 | 7.69E-19 |
| ENSG00000124145 | syndecan 4(SDC4) | 2.67476949 | 0.26116867 | 1.29E-24 | 1.29E-22 |
| ENSG00000132510 | lysine demethylase 6B(KDM6B) | 2.67317521 | 0.31423 | 1.78E-17 | 1.24E-15 |
| ENSG00000117479 | solute carrier family 19 member 2(SLC19A2) | 2.65137682 | 0.36132039 | 2.17E-13 | 1.07E-11 |
| ENSG00000158373 | histone cluster 1 H2B family member d(HIST1H2BD) | 2.64531718 | 0.27406874 | 4.82E-22 | 4.23E-20 |
| ENSG00000087074 | protein phosphatase 1 regulatory subunit 15A(PPP1R15A) | 2.63889861 | 0.23171026 | 4.76E-30 | 5.83E-28 |
| ENSG00000165617 | dishevelled binding antagonist of beta catenin 1(DACT1) | 2.63018744 | 0.32328548 | 4.09E-16 | 2.55E-14 |
| ENSG00000197483 | zinc finger protein 628(ZNF628) | 2.62947057 | 0.73484743 | 0.0003459 | 0.00281066 |
| ENSG00000163545 | NUAK family kinase 2(NUAK2) | 2.62942175 | 0.24504152 | 7.32E-27 | 8.20E-25 |
| ENSG00000181649 | pleckstrin homology like domain family A member 2(PHLDA2) | 2.62727831 | 0.38222897 | 6.26E-12 | 2.73E-10 |
| ENSG00000008517 | interleukin 32(IL32) | 2.62432541 | 0.26313034 | 1.99E-23 | 1.86E-21 |
| ENSG00000169715 | metallothionein 1E(MT1E) | 2.62299828 | 0.31913977 | 2.05E-16 | 1.33E-14 |
| ENSG00000109320 | nuclear factor kappa B subunit 1(NFKB1) | 2.61866006 | 0.26467295 | 4.42E-23 | 4.00E-21 |
| ENSG00000222047 | chromosome 10 open reading frame 55(C10orf55) | 2.61027853 | 0.30365693 | 8.24E-18 | 5.88E-16 |
| ENSG00000171056 | SRY-box 7(SOX7) | 2.57440731 | 0.391496 | 4.84E-11 | 1.89E-09 |
| ENSG00000152402 | guanylate cyclase 1 soluble subunit alpha 2(GUCY1A2) | 2.57375597 | 0.45232303 | 1.27E-08 | 3.52E-07 |
| ENSG00000105835 | nicotinamide phosphoribosyltransferase(NAMPT) | 2.57010019 | 0.30735183 | 6.16E-17 | 4.14E-15 |
| ENSG00000162645 | guanylate binding protein 2(GBP2) | 2.56793432 | 0.47099166 | 4.98E-08 | 1.20E-06 |
| ENSG00000185112 | family with sequence similarity 43 member A(FAM43A) | 2.56263 | 0.27914223 | 4.30E-20 | 3.46E-18 |
| ENSG00000150907 | forkhead box O1(FOXO1) | 2.55336601 | 0.29759947 | 9.50E-18 | 6.65E-16 |
| ENSG00000241978 | A-kinase anchoring protein 2(AKAP2) | 2.55222586 | 0.29639162 | 7.24E-18 | 5.20E-16 |
| ENSG00000215218 | ubiquitin conjugating enzyme E2 Q family like 1(UBE2QL1) | 2.54450991 | 0.44936087 | 1.49E-08 | 4.09E-07 |
| ENSG00000249936 | NA | 2.53771598 | 0.54997146 | 3.94E-06 | 6.16E-05 |
| ENSG00000168389 | major facilitator superfamily domain containing 2A(MFSD2A) | 2.53374307 | 0.26479596 | 1.08E-21 | 9.37E-20 |
| ENSG00000157514 | TSC22 domain family member 3(TSC22D3) | 2.53128378 | 0.23559565 | 6.31E-27 | 7.21E-25 |
| ENSG00000102554 | Kruppel like factor 5(KLF5) | 2.52702728 | 0.28692744 | 1.28E-18 | 9.74E-17 |
| ENSG00000170873 | MTSS1, I-BAR domain containing(MTSS1) | 2.52254264 | 0.47568808 | 1.14E-07 | 2.57E-06 |
| ENSG00000114315 | hes family bHLH transcription factor 1(HES1) | 2.52047952 | 0.24704064 | 1.93E-24 | 1.88E-22 |
| ENSG00000155090 | Kruppel like factor 10(KLF10) | 2.50971495 | 0.28949523 | 4.35E-18 | 3.16E-16 |
| ENSG00000196428 | TSC22 domain family member 2(TSC22D2) | 2.50593186 | 0.2975164 | 3.67E-17 | 2.51E-15 |
| ENSG00000269113 | TraB domain containing 2B(TRABD2B) | 2.50448734 | 0.43703084 | 1.00E-08 | 2.80E-07 |
| ENSG00000120217 | CD274 molecule(CD274) | 2.49629367 | 0.30838175 | 5.74E-16 | 3.53E-14 |
| ENSG00000116285 | ERBB receptor feedback inhibitor 1(ERRFI1) | 2.49419893 | 0.31327358 | 1.70E-15 | 1.00E-13 |
| ENSG00000186832 | keratin 16(KRT16) | 2.49413722 | 0.32020857 | 6.75E-15 | 3.82E-13 |
| ENSG00000147883 | cyclin dependent kinase inhibitor 2B(CDKN2B) | 2.49291259 | 0.28681793 | 3.57E-18 | 2.61E-16 |
| ENSG00000168264 | interferon regulatory factor 2 binding protein 2(IRF2BP2) | 2.48535416 | 0.23335238 | 1.73E-26 | 1.82E-24 |
| ENSG00000124225 | prostate transmembrane protein, androgen induced 1(PMEPA1) | 2.4843773 | 0.27473371 | 1.53E-19 | 1.22E-17 |
| ENSG00000168386 | filamin A interacting protein 1 like(FILIP1L) | 2.46521111 | 0.38292763 | 1.21E-10 | 4.56E-09 |
| ENSG00000180730 | shisa family member 2(SHISA2) | 2.46213074 | 0.33468407 | 1.89E-13 | 9.37E-12 |
| ENSG00000156427 | fibroblast growth factor 18(FGF18) | 2.45982812 | 0.29968479 | 2.25E-16 | 1.45E-14 |
| ENSG00000134954 | ETS proto-oncogene 1, transcription factor(ETS1) | 2.45612389 | 0.30005049 | 2.71E-16 | 1.72E-14 |
| ENSG00000162733 | discoidin domain receptor tyrosine kinase 2(DDR2) | 2.45597952 | 0.38800691 | 2.46E-10 | 8.81E-09 |
| ENSG00000125968 | inhibitor of DNA binding 1, HLH protein(ID1) | 2.45349282 | 0.29190908 | 4.28E-17 | 2.91E-15 |
| ENSG00000131669 | ninjurin 1(NINJ1) | 2.45024666 | 0.33471114 | 2.47E-13 | 1.22E-11 |
| ENSG00000185338 | suppressor of cytokine signaling 1(SOCS1) | 2.43724831 | 0.38197103 | 1.76E-10 | 6.44E-09 |
| ENSG00000142675 | connector enhancer of kinase suppressor of Ras 1(CNKSR1) | 2.43599731 | 0.49561306 | 8.87E-07 | 1.66E-05 |
| ENSG00000179431 | four jointed box 1(FJX1) | 2.43219375 | 0.25764701 | 3.73E-21 | 3.09E-19 |
| ENSG00000110330 | baculoviral IAP repeat containing 2(BIRC2) | 2.42907091 | 0.29664193 | 2.64E-16 | 1.69E-14 |
| ENSG00000131435 | PDZ and LIM domain 4(PDLIM4) | 2.42757614 | 0.36613879 | 3.35E-11 | 1.32E-09 |
| ENSG00000169554 | zinc finger E-box binding homeobox 2(ZEB2) | 2.42248705 | 0.31818302 | 2.67E-14 | 1.41E-12 |
| ENSG00000054598 | forkhead box C1(FOXC1) | 2.41861962 | 0.24782848 | 1.68E-22 | 1.50E-20 |
| ENSG00000225614 | zinc finger protein 469(ZNF469) | 2.41456533 | 0.39476655 | 9.57E-10 | 3.20E-08 |
| ENSG00000198355 | Pim-3 proto-oncogene, serine/threonine kinase(PIM3) | 2.41255047 | 0.30561929 | 2.93E-15 | 1.70E-13 |
| ENSG00000136048 | DNA damage regulated autophagy modulator 1(DRAM1) | 2.40004397 | 0.29883876 | 9.65E-16 | 5.85E-14 |
| ENSG00000115738 | inhibitor of DNA binding 2, HLH protein(ID2) | 2.38957707 | 0.31218682 | 1.94E-14 | 1.06E-12 |
| ENSG00000178726 | thrombomodulin(THBD) | 2.36929452 | 0.38948918 | 1.18E-09 | 3.89E-08 |
| ENSG00000100403 | zinc finger CCCH-type containing 7B(ZC3H7B) | 2.36542415 | 0.92150269 | 0.01026061 | 0.04157897 |
| ENSG00000136603 | SKI like proto-oncogene(SKIL) | 2.36380173 | 0.34656305 | 9.06E-12 | 3.89E-10 |
| ENSG00000244115 | DNAJC25-GNG10 readthrough(DNAJC25-GNG10) | 2.36364339 | 0.50845752 | 3.34E-06 | 5.30E-05 |
| ENSG00000167034 | NK3 homeobox 1(NKX3-1) | 2.36200508 | 0.32928094 | 7.33E-13 | 3.45E-11 |
| ENSG00000128274 | alpha 1,4-galactosyltransferase(A4GALT) | 2.36146786 | 0.35349831 | 2.38E-11 | 9.71E-10 |
| ENSG00000152082 | mitotic spindle organizing protein 2B(MZT2B) | 2.34364384 | 0.68655556 | 0.00064105 | 0.00465302 |
| ENSG00000131196 | nuclear factor of activated T-cells 1(NFATC1) | 2.34314389 | 0.26824647 | 2.44E-18 | 1.83E-16 |
| ENSG00000186567 | carcinoembryonic antigen related cell adhesion molecule 19(CEACAM19) | 2.34198473 | 0.30169641 | 8.31E-15 | 4.66E-13 |
| ENSG00000157680 | diacylglycerol kinase iota(DGKI) | 2.33456836 | 0.43022655 | 5.75E-08 | 1.36E-06 |
| ENSG00000228981 | NA | 2.32892605 | 0.42245474 | 3.53E-08 | 8.90E-07 |
| ENSG00000100625 | SIX homeobox 4(SIX4) | 2.32482018 | 0.35408011 | 5.18E-11 | 2.01E-09 |
| ENSG00000148426 | proline and serine rich 2(PROSER2) | 2.32190197 | 0.28654082 | 5.35E-16 | 3.31E-14 |
| ENSG00000170989 | sphingosine-1-phosphate receptor 1(S1PR1) | 2.31913368 | 0.30921586 | 6.38E-14 | 3.31E-12 |
| ENSG00000130513 | growth differentiation factor 15(GDF15) | 2.31672204 | 0.33798309 | 7.15E-12 | 3.11E-10 |
| ENSG00000113240 | CDC like kinase 4(CLK4) | 2.30874875 | 0.32329257 | 9.24E-13 | 4.28E-11 |
| ENSG00000134313 | kinase D-interacting substrate 220kDa(KIDINS220) | 2.30711755 | 0.41566045 | 2.85E-08 | 7.38E-07 |
| ENSG00000182759 | MAF bZIP transcription factor A(MAFA) | 2.30059938 | 0.33054948 | 3.40E-12 | 1.53E-10 |
| ENSG00000143867 | odd-skipped related transciption factor 1(OSR1) | 2.29368734 | 0.2975853 | 1.28E-14 | 7.08E-13 |
| ENSG00000128917 | delta like canonical Notch ligand 4(DLL4) | 2.27277219 | 0.29362229 | 9.91E-15 | 5.50E-13 |
| ENSG00000004777 | Rho GTPase activating protein 33(ARHGAP33) | 2.26980334 | 0.33520337 | 1.28E-11 | 5.36E-10 |
| ENSG00000204839 | maestro heat like repeat family member 6(MROH6) | 2.25504912 | 0.38913463 | 6.83E-09 | 1.98E-07 |
| ENSG00000134107 | basic helix-loop-helix family member e40(BHLHE40) | 2.25475495 | 0.23867609 | 3.49E-21 | 2.91E-19 |
| ENSG00000205189 | zinc finger and BTB domain containing 10(ZBTB10) | 2.24974945 | 0.40632519 | 3.08E-08 | 7.91E-07 |
| ENSG00000117228 | guanylate binding protein 1(GBP1) | 2.23514547 | 0.346236 | 1.08E-10 | 4.08E-09 |
| ENSG00000131737 | keratin 34(KRT34) | 2.23276361 | 0.25326553 | 1.19E-18 | 9.07E-17 |
| ENSG00000160223 | inducible T-cell costimulator ligand(ICOSLG) | 2.2321475 | 0.32085422 | 3.48E-12 | 1.56E-10 |
| ENSG00000131650 | kringle containing transmembrane protein 2(KREMEN2) | 2.22733646 | 0.40523098 | 3.87E-08 | 9.60E-07 |
| ENSG00000179776 | cadherin 5(CDH5) | 2.2265067 | 0.30030853 | 1.22E-13 | 6.13E-12 |
| ENSG00000165312 | OTU deubiquitinase 1(OTUD1) | 2.2193001 | 0.35919909 | 6.47E-10 | 2.19E-08 |
| ENSG00000030419 | IKAROS family zinc finger 2(IKZF2) | 2.19825261 | 0.45587109 | 1.42E-06 | 2.47E-05 |
| ENSG00000276107 | NA | 2.1975662 | 0.58725531 | 0.00018249 | 0.00164788 |
| ENSG00000008516 | matrix metallopeptidase 25(MMP25) | 2.19552687 | 0.35316486 | 5.08E-10 | 1.76E-08 |
| ENSG00000163602 | RING1 and YY1 binding protein(RYBP) | 2.18701425 | 0.30609321 | 9.00E-13 | 4.19E-11 |
| ENSG00000162572 | sodium channel epithelial 1 delta subunit(SCNN1D) | 2.18590132 | 0.39702172 | 3.68E-08 | 9.20E-07 |
| ENSG00000158714 | SLAM family member 8(SLAMF8) | 2.18453724 | 0.33729878 | 9.38E-11 | 3.57E-09 |
| ENSG00000077150 | nuclear factor kappa B subunit 2(NFKB2) | 2.18279765 | 0.28595322 | 2.29E-14 | 1.23E-12 |
| ENSG00000176678 | forkhead box L1(FOXL1) | 2.17825694 | 0.26727614 | 3.64E-16 | 2.28E-14 |
| ENSG00000244257 | NA | 2.17551791 | 0.72186514 | 0.00258044 | 0.01421417 |
| ENSG00000240342 | NA | 2.16523089 | 0.38655143 | 2.13E-08 | 5.66E-07 |
| ENSG00000168685 | interleukin 7 receptor(IL7R) | 2.15992015 | 0.34744789 | 5.08E-10 | 1.76E-08 |
| ENSG00000250251 | NA | 2.15886669 | 0.30081712 | 7.14E-13 | 3.38E-11 |
| ENSG00000116717 | growth arrest and DNA damage inducible alpha(GADD45A) | 2.15860219 | 0.28965295 | 9.17E-14 | 4.63E-12 |
| ENSG00000148926 | adrenomedullin(ADM) | 2.15781167 | 0.26774634 | 7.68E-16 | 4.71E-14 |
| ENSG00000158246 | family with sequence similarity 46 member B(FAM46B) | 2.15549058 | 0.28521023 | 4.11E-14 | 2.17E-12 |
| ENSG00000095739 | BMP and activin membrane bound inhibitor(BAMBI) | 2.1529692 | 0.25767195 | 6.52E-17 | 4.36E-15 |
| ENSG00000142102 | protein-glucosylgalactosylhydroxylysine glucosidase(PGGHG) | 2.15074267 | 0.3316151 | 8.83E-11 | 3.37E-09 |
| ENSG00000149948 | high mobility group AT-hook 2(HMGA2) | 2.14159552 | 0.35109916 | 1.06E-09 | 3.51E-08 |
| ENSG00000177943 | MAM domain containing 4(MAMDC4) | 2.141313 | 0.40352634 | 1.12E-07 | 2.52E-06 |
| ENSG00000198142 | sosondowah ankyrin repeat domain family member C(SOWAHC) | 2.14119702 | 0.29062634 | 1.74E-13 | 8.67E-12 |
| ENSG00000173272 | microRNA 4784(MIR4784) | 2.13008983 | 0.41990248 | 3.92E-07 | 7.92E-06 |
| ENSG00000101187 | solute carrier organic anion transporter family member 4A1(SLCO4A1) | 2.12880362 | 0.33854243 | 3.21E-10 | 1.14E-08 |
| ENSG00000159200 | regulator of calcineurin 1(RCAN1) | 2.1195123 | 0.30795216 | 5.88E-12 | 2.58E-10 |
| ENSG00000020577 | sterile alpha motif domain containing 4A(SAMD4A) | 2.11525947 | 0.2379326 | 6.10E-19 | 4.72E-17 |
| ENSG00000126561 | signal transducer and activator of transcription 5A(STAT5A) | 2.11088049 | 0.27050167 | 6.02E-15 | 3.42E-13 |
| ENSG00000140961 | oxidative stress induced growth inhibitor 1(OSGIN1) | 2.11078446 | 0.37895549 | 2.55E-08 | 6.68E-07 |
| ENSG00000131188 | proline rich 7, synaptic(PRR7) | 2.11060916 | 0.83496836 | 0.01147891 | 0.04512556 |
| ENSG00000075426 | FOS like 2, AP-1 transcription factor subunit(FOSL2) | 2.10647903 | 0.23301714 | 1.57E-19 | 1.25E-17 |
| ENSG00000027697 | interferon gamma receptor 1(IFNGR1) | 2.10570455 | 0.33944341 | 5.53E-10 | 1.88E-08 |
| ENSG00000170684 | zinc finger protein 296(ZNF296) | 2.10483224 | 0.41662986 | 4.37E-07 | 8.75E-06 |
| ENSG00000170075 | G protein-coupled receptor 37 like 1(GPR37L1) | 2.09909449 | 0.35443228 | 3.17E-09 | 9.75E-08 |
| ENSG00000218537 | NA | 2.09444286 | 0.79255687 | 0.00822623 | 0.03499272 |
| ENSG00000146278 | proline rich nuclear receptor coactivator 1(PNRC1) | 2.09063927 | 0.25044246 | 6.96E-17 | 4.62E-15 |
| ENSG00000162004 | coiled-coil domain containing 78(CCDC78) | 2.08976496 | 0.4237712 | 8.17E-07 | 1.54E-05 |
| ENSG00000153714 | leucine rich adaptor protein 1 like(LURAP1L) | 2.08821159 | 0.42852616 | 1.10E-06 | 2.00E-05 |
| ENSG00000118263 | Kruppel like factor 7(KLF7) | 2.08582589 | 0.31671694 | 4.53E-11 | 1.77E-09 |
| ENSG00000274272 | NA | 2.08002397 | 0.4243973 | 9.53E-07 | 1.77E-05 |
| ENSG00000158014 | solute carrier family 30 member 2(SLC30A2) | 2.07802761 | 0.3874294 | 8.16E-08 | 1.87E-06 |
| ENSG00000146904 | EPH receptor A1(EPHA1) | 2.0757147 | 0.32675482 | 2.12E-10 | 7.69E-09 |
| ENSG00000124216 | snail family transcriptional repressor 1(SNAI1) | 2.07453611 | 0.30461679 | 9.74E-12 | 4.17E-10 |
| ENSG00000182752 | pappalysin 1(PAPPA) | 2.07082181 | 0.29971762 | 4.87E-12 | 2.16E-10 |
| ENSG00000255513 | NA | 2.07047691 | 0.30733353 | 1.62E-11 | 6.70E-10 |
| ENSG00000122862 | serglycin(SRGN) | 2.06566404 | 0.37967119 | 5.31E-08 | 1.26E-06 |
| ENSG00000185507 | interferon regulatory factor 7(IRF7) | 2.06159485 | 0.42575316 | 1.28E-06 | 2.27E-05 |
| ENSG00000113742 | cytoplasmic polyadenylation element binding protein 4(CPEB4) | 2.05783225 | 0.33711772 | 1.03E-09 | 3.42E-08 |
| ENSG00000159216 | uncharacterized LOC100506403(LOC100506403) | 2.05620427 | 0.23602872 | 2.99E-18 | 2.22E-16 |
| ENSG00000268193 | NA | 2.05450038 | 0.4890513 | 2.66E-05 | 0.00032008 |
| ENSG00000125726 | CD70 molecule(CD70) | 2.05222213 | 0.32483064 | 2.65E-10 | 9.43E-09 |
| ENSG00000266074 | BAH domain and coiled-coil containing 1(BAHCC1) | 2.05193922 | 0.39518674 | 2.08E-07 | 4.43E-06 |
| ENSG00000212123 | proline rich 22(PRR22) | 2.05094683 | 0.45254012 | 5.84E-06 | 8.71E-05 |
| ENSG00000232748 | NA | 2.04851693 | 0.77212344 | 0.00797574 | 0.03419918 |
| ENSG00000171150 | suppressor of cytokine signaling 5(SOCS5) | 2.04505402 | 0.31065285 | 4.61E-11 | 1.80E-09 |
| ENSG00000028277 | POU class 2 homeobox 2(POU2F2) | 2.04322218 | 0.45938761 | 8.68E-06 | 0.00012274 |
| ENSG00000141933 | tubulin polyglutamylase complex subunit 1(TPGS1) | 2.038328 | 0.42711002 | 1.82E-06 | 3.11E-05 |
| ENSG00000182871 | collagen type XVIII alpha 1 chain(COL18A1) | 2.03792103 | 0.4062434 | 5.26E-07 | 1.04E-05 |
| ENSG00000251196 | NA | 2.03656863 | 0.3803909 | 8.61E-08 | 1.97E-06 |
| ENSG00000165655 | zinc finger protein 503(ZNF503) | 2.0338611 | 0.31855831 | 1.72E-10 | 6.30E-09 |
| ENSG00000158106 | rhophilin Rho GTPase binding protein 1(RHPN1) | 2.02698696 | 0.42638819 | 2.00E-06 | 3.36E-05 |
| ENSG00000136630 | H2.0 like homeobox(HLX) | 2.02460271 | 0.35498224 | 1.17E-08 | 3.26E-07 |
| ENSG00000197982 | chromosome 1 open reading frame 122(C1orf122) | 2.02358958 | 0.4351828 | 3.32E-06 | 5.27E-05 |
| ENSG00000160712 | interleukin 6 receptor(IL6R) | 2.01599011 | 0.27714065 | 3.48E-13 | 1.70E-11 |
| ENSG00000104312 | receptor interacting serine/threonine kinase 2(RIPK2) | 2.01332332 | 0.41332632 | 1.11E-06 | 2.01E-05 |
| ENSG00000150347 | AT-rich interaction domain 5B(ARID5B) | 2.01297338 | 0.30280742 | 2.98E-11 | 1.19E-09 |
| ENSG00000178773 | copine 7(CPNE7) | 2.01289068 | 0.31631946 | 1.97E-10 | 7.18E-09 |
| ENSG00000196850 | PTC7 protein phosphatase homolog(PPTC7) | 2.00784491 | 0.30787212 | 6.95E-11 | 2.69E-09 |
| ENSG00000055208 | TGF-beta activated kinase 1/MAP3K7 binding protein 2(TAB2) | 2.00464468 | 0.4259593 | 2.52E-06 | 4.10E-05 |

## Claims

1. A compound of formula (II), or a pharmaceutically acceptable salt thereof: 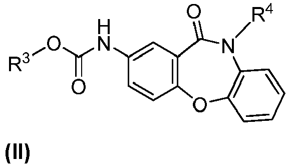 wherein:
**R³** is straight or branched C₁-C₆ alkyl; and
R⁴ is straight or branched C₁-C₆ alkyl,
or R⁴ is 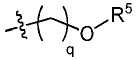 wherein q is 1, 2, 3 or 4; and R⁵ is straight or branched C₁-C₆ alkyl, for use in treating a disease or condition associated with vascular permeability, neovascularisation, angiogenesis, inflammation, cell migration and/or cell proliferation in the subject, wherein the disease or condition is selected from the group consisting of
• arthritis;
• rheumatoid arthritis;
• bone destruction;
• age-related macular degeneration;
• diabetic retinopathy;
• macular edema;
• vascular leakage;
• vascular permeability;
• retinal vascular permeability;
• angiogenesis;
• endothelial cell dysfunction;
• atherosclerosis;
• stroke;
• myocardial infarction;
• peripheral vascular disease;
• stenosis;
• restenosis;
• inflammation;
• cytokine storm;
• **pulmonary** inflammation; and
• pulmonary fibrosis.

2. The compound for use of claim 1, wherein R³ is -CH₂CH₃ or -CH₂CH(CH₃)₂.

3. The compound for use of claim 1, wherein R⁴ is straight C₁-C₆ alkyl or branched C₁-C₆ alkyl, preferably wherein R⁴ is -CH₂CH₃ or -CH₂CH(CH₃)₂, or wherein R⁴ is 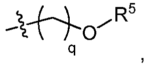 **wherein q is** 1, 2, 3 or 4; and R⁵ is straight C₁-C₆ alkyl or branched C₁-C₆ **alkyl,** preferably wherein q is 2, preferably wherein R⁵ is -CH₃, preferably wherein q is 2 and R⁵ is -CH₃.

4. The compound for use of any one of claims 1 to 3, wherein the compound of formula (II) is a compound of formula (II-1): 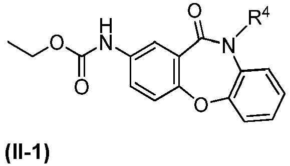 wherein:
R⁴ is straight or branched C₁-C₆ alkyl;
or R⁴ is: 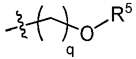 wherein q is 1, 2, 3 or 4; and R⁵ is straight or branched C₁-C₆ alkyl.

5. The compound for use of any one of claims 1 to 4, wherein compound of formula (II-1) is selected from: 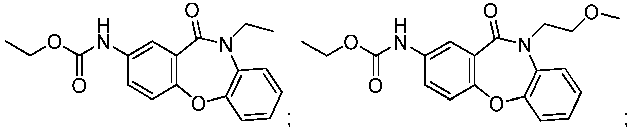 and

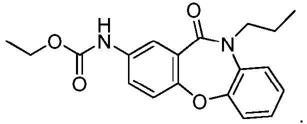

6. The compound for use of any one of claims 1 to 5, wherein the compound of formula (II) is:

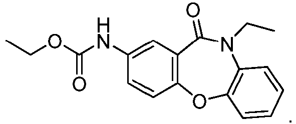

7. A pharmaceutical composition comprising a compound of formula II, or a pharmaceutically acceptable salt thereof 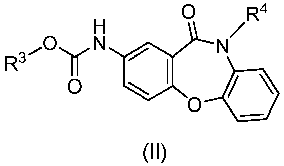 wherein:
R³ is straight or branched C₁-C₆ alkyl; and
R⁴ is straight or branched C₁-C₆ alkyl,
or R⁴ is 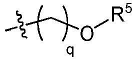 wherein q is 1, 2, 3 or 4; and R⁵ is straight or branched C₁-C₆ alkyl, and a pharmaceutically acceptable carrier.

8. The pharmaceutical composition of claim 7, wherein R³ is straight C₁-C₆ alkyl or branched C₁-C₆ alkyl, preferably wherein R³ is -CH₂CH₃ or -CH₂CH(CH₃)₂, preferably wherein R⁴ is straight C₁-C₆ alkyl or branched C₁-C₆ alkyl.

9. The pharmaceutical composition of claim 7, wherein R⁴ is -CH₂CH₃ or -CH₂CH(CH₃)₂, preferably wherein R⁴ is 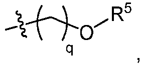 **wherein q is** 1, 2, 3 or 4; and R⁵ is straight C₁-C₆ **alkyl or branched** C₁-C₆ alkyl, preferably wherein q is 2; preferably wherein R⁵ is -CH₃, preferably wherein q is 2 and R⁵ is -CH₃.

10. The pharmaceutical composition of any one of claims 7 to 9, wherein the compound of formula (II) is a compound of formula (II-1): 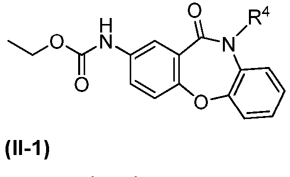 wherein:
R⁴ is straight or branched C₁-C₆ alkyl;
or R⁴ is: 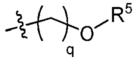 **wherein q is** 1, 2, 3 or 4; and R⁵ is straight or branched C₁-C₆ alkyl.

11. The pharmaceutical composition of any one of claims 7 to 12, wherein the compound of formula (II) is:

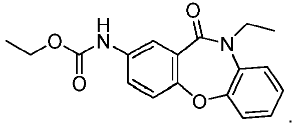

12. A compound of formula (II), or a pharmaceutically acceptable salt thereof: 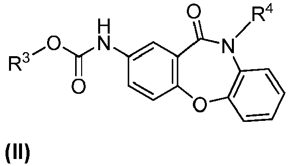 wherein:
R³ is straight or branched C₁-C₆ alkyl; and
R⁴ is straight or branched C₁-C₆ alkyl,
or R⁴ is 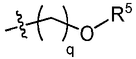 **wherein q is** 1, 2, 3 or 4; and R⁵ is straight or branched C₁-C₆ alkyl, for use as a medicament.

13. The compound for use of claim 12, wherein R³ is -CH₂CH₃ or -CH₂CH(CH₃)₂.

14. The compound for use of claim 12, wherein R⁴ is straight C₁-C₆ alkyl or branched C₁-C₆ alkyl, preferably wherein R⁴ is -CH₂CH₃ or -CH₂CH(CH₃)₂, or wherein R⁴ is 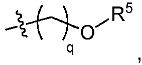 **wherein q is** 1, 2, 3 or 4; and R⁵ is straight C₁-C₆ alkyl or branched C₁-C₆ **alkyl,** preferably wherein q is 2, preferably wherein R⁵ is -CH₃, preferably wherein q is 2 and R⁵ is -CH₃.

15. The compound for use of claim 12, wherein the compound of formula (II) is a compound of formula (II-1): 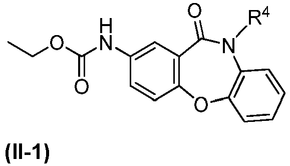 wherein:
R⁴ is straight or branched C₁-C₆ alkyl;
or R⁴ is: 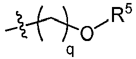 wherein q is 1, 2, 3 or 4; and R⁵ is straight or branched C₁-C₆ alkyl.

16. The compound for use of claim 15, wherein compound of formula (II-1) is a compound of formula (II):

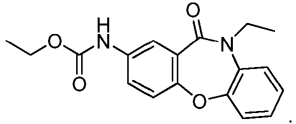

## Patentansprüche

1. Verbindung der Formel (II) oder pharmazeutisch verträgliches Salz davon: 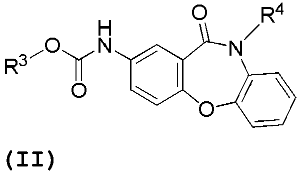 wobei:
R³ für geradkettiges oder verzweigtes C₁-C₆-Alkyl steht; und
R⁴ für geradkettiges oder verzweigtes C₁-C₆-Alkyl steht,
oder R⁴ für 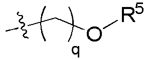
steht, wobei q für 1, 2, 3 oder 4 steht; und R⁵ für geradkettiges oder verzweigtes C₁-C₆-Alkyl steht,
zur Verwendung bei der Behandlung einer Erkrankung oder Krankheit, die mit Gefäßpermeabilität, Neovaskularisation, Angiogenese, Entzündung, Zellmigration und/oder Zellproliferation bei dem Subjekt verknüpft ist, wobei die Erkrankung oder Krankheit ausgewählt ist aus der Gruppe bestehend aus
• Arthritis;
• rheumatoider Arthritis;
• Knochenabbau;
• altersbedingter Makuladegeneration;
• diabetischer Retinopathie;
• Makulaödem;
• Gefäßleckage;
• Gefäßpermeabilität;
• retinaler Gefäßpermeabilität;
• Angiogenese;
• Endothelzelldysfunktion;
• Arteriosklerose;
• Schlaganfall;
• Herzinfarkt;
• peripherer Gefäßerkrankung;
• Stenose;
• Restenose;
• Entzündung;
• Zytokinsturm;
• Lungenentzündung; und
• Lungenfibrose.

2. Verbindung zur Verwendung nach Anspruch 1, wobei R³ für - CH₂CH₃ oder -CH₂CH(CH₃)₂ steht.

3. Verbindung zur Verwendung nach Anspruch 1, wobei R⁴ für geradkettiges C₁-C₆-Alkyl oder verzweigtes C₁-C₆-Alkyl steht, bevorzugt wobei R⁴ für -CH₂CH₃ oder -CH₂CH(CH₃)₂ steht oder wobei R⁴ für 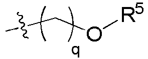 steht, wobei q für 1, 2, 3 oder 4 steht; und R⁵ für geradkettiges C₁-C₆-Alkyl oder verzweigtes C₁-C₆-Alkyl steht, bevorzugt wobei q für 2 steht, bevorzugt wobei R⁵ für - CH₃ steht, bevorzugt wobei q für 2 steht und R⁵ für -CH₃ steht.

4. Verbindung zur Verwendung nach einem der Ansprüche 1 bis 3, wobei es sich bei der Verbindung der Formel (II) um eine Verbindung der Formel (II-1) handelt: 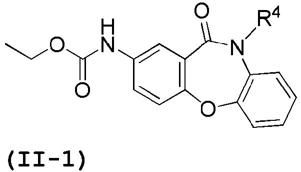 wobei:
R⁴ für geradkettiges oder verzweigtes C₁-C₆-Alkyl steht;
oder R⁴ für: 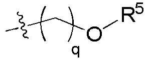
steht, wobei q für 1, 2, 3 oder 4 steht; und R⁵ für geradkettiges oder verzweigtes C₁-C₆-Alkyl steht.

5. Verbindung zur Verwendung nach einem der Ansprüche 1 bis 4, wobei die Verbindung der Formel (II-1) ausgewählt ist aus: 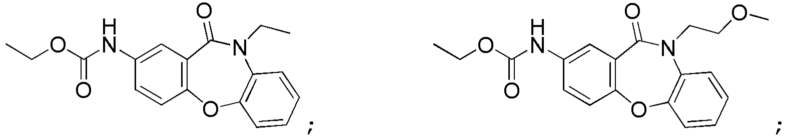 und

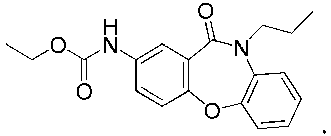

6. Verbindung zur Verwendung nach einem der Ansprüche 1 bis 5, wobei es sich bei der Verbindung der Formel (II) um Folgendes handelt:

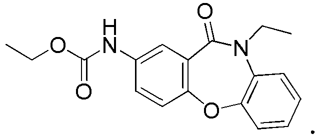

7. Pharmazeutische Zusammensetzung, umfassend eine Verbindung der Formel II oder ein pharmazeutisch verträgliches Salz davon 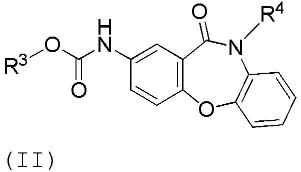 wobei:
R³ für geradkettiges oder verzweigtes C₁-C₆-Alkyl steht; und
R⁴ für geradkettiges oder verzweigtes C₁-C₆-Alkyl steht,
oder R⁴ für 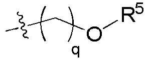
steht, wobei q für 1, 2, 3 oder 4 steht; und R⁵ für geradkettiges oder verzweigtes C₁-C₆-Alkyl steht,
und einen pharmazeutisch verträglichen Träger.

8. Pharmazeutische Zusammensetzung nach Anspruch 7, wobei R³ für geradkettiges C₁-C₆-Alkyl oder verzweigtes C₁-C₆-Alkyl steht, bevorzugt wobei R³ für -CH₂CH₃ oder -CH₂CH(CH₃)₂ steht, bevorzugt wobei R⁴ für geradkettiges C₁-C₆-Alkyl oder verzweigtes C₁-C₆-Alkyl steht.

9. Pharmazeutische Zusammensetzung nach Anspruch 7, wobei R⁴ für -CH₂CH₃ oder -CH₂CH(CH₃)₂ steht, bevorzugt wobei R⁴ für 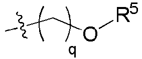 steht, wobei q für 1, 2, 3 oder 4 steht; und R⁵ für geradkettiges C₁-C₆-Alkyl oder verzweigtes C₁-C₆-Alkyl steht, bevorzugt wobei q für 2 steht; bevorzugt wobei R⁵ für -CH₃ steht, bevorzugt wobei q für 2 steht und R⁵ für -CH₃ steht.

10. Pharmazeutische Zusammensetzung nach einem der Ansprüche 7 bis 9, wobei es sich bei der Verbindung der Formel (II) um eine Verbindung der Formel (II-1) handelt: 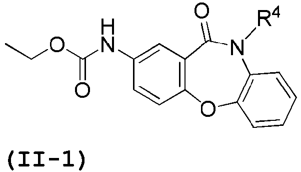 wobei:
R⁴ für geradkettiges oder verzweigtes C₁-C₆-Alkyl steht;
oder R⁴ für: 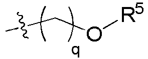
steht, wobei q für 1, 2, 3 oder 4 steht; und R⁵ für geradkettiges oder verzweigtes C₁-C₆-Alkyl steht.

11. Pharmazeutische Zusammensetzung nach einem der Ansprüche 7 bis 12, wobei es sich bei der Verbindung der Formel (II) um Folgendes handelt:

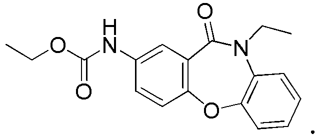

12. Verbindung der Formel (II) oder pharmazeutisch verträgliches Salz davon: 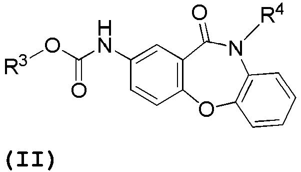 wobei:
R³ für geradkettiges oder verzweigtes C₁-C₆-Alkyl steht; und
R⁴ für geradkettiges oder verzweigtes C₁-C₆-Alkyl steht,
oder R⁴ für 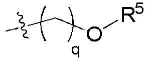
steht, wobei q für 1, 2, 3 oder 4 steht; und R⁵ für geradkettiges oder verzweigtes C₁-C₆-Alkyl steht,
zur Verwendung als Arzneimittel.

13. Verbindung zur Verwendung nach Anspruch 12, wobei R³ für - CH₂CH₃ oder -CH₂CH(CH₃)₂ steht.

14. Verbindung zur Verwendung nach Anspruch 12, wobei R⁴ für geradkettiges C₁-C₆-Alkyl oder verzweigtes C₁-C₆-Alkyl steht, bevorzugt wobei R⁴ für -CH₂CH₃ oder -CH₂CH(CH₃)₂ steht oder wobei R⁴ für 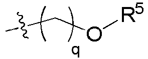 steht, wobei q für 1, 2, 3 oder 4 steht; und R⁵ für geradkettiges C₁-C₆-Alkyl oder verzweigtes C₁-C₆-Alkyl steht, bevorzugt wobei q für 2 steht, bevorzugt wobei R⁵ für - CH₃ steht, bevorzugt wobei q für 2 steht und R⁵ für -CH₃ steht.

15. Verbindung zur Verwendung nach Anspruch 12, wobei es sich bei der Verbindung der Formel (II) um eine Verbindung der Formel (II-1) handelt: 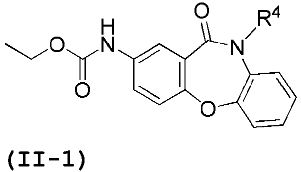 wobei:
R⁴ für geradkettiges oder verzweigtes C₁-C₆-Alkyl steht;
oder R⁴ für: 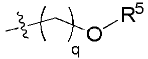
steht, wobei q für 1, 2, 3 oder 4 steht; und R⁵ für geradkettiges oder verzweigtes C₁-C₆-Alkyl steht.

16. Verbindung zur Verwendung nach Anspruch 15, wobei es sich bei der Verbindung der Formel (II-1) um eine Verbindung der Formel (II) handelt:

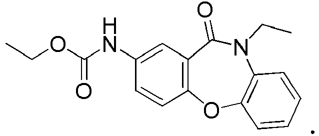

## Revendications

1. Composé de formule (II), ou sel pharmaceutiquement acceptable de celui-ci : 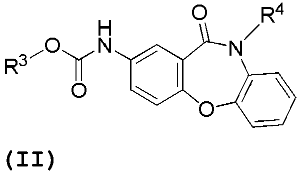 dans lequel :
R³ représente un alkyle en C₁-C₆ linéaire ou ramifié ; et
R⁴ représente un alkyle en C₁-C₆ linéaire ou ramifié,
ou R⁴ représente 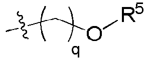
où q vaut 1, 2, 3 ou 4 ; et R⁵ représente un alkyle en C₁-C₆ linéaire ou ramifié,
pour utilisation dans le traitement d'une maladie ou d'un état associé à la perméabilité vasculaire, néovascularisation, angiogenèse, inflammation, migration cellulaire et/ou prolifération cellulaire chez le sujet, dans lequel la maladie ou l'état est choisi dans le groupe constitué de
• arthrite ;
• polyarthrite rhumatoïde ;
• destruction osseuse ;
• dégénérescence maculaire liée à l'âge ;
• rétinopathie diabétique ;
• œdème maculaire ;
• fuite vasculaire ;
• perméabilité vasculaire ;
• perméabilité vasculaire rétinienne ;
• angiogenèse ;
• dysfonctionnement des cellules endothéliales ;
• athérosclérose ;
• accident vasculaire cérébral ;
• infarctus du myocarde ;
• maladie vasculaire périphérique ;
• sténose ;
• resténose ;
• inflammation ;
• orage cytokinique ;
• inflammation pulmonaire ; et
• fibrose pulmonaire.

2. Composé pour utilisation selon la revendication 1, dans lequel R³ représente -CH₂CH₃ ou -CH₂CH(CH₃)₂.

3. Composé pour utilisation selon la revendication 1, dans lequel R⁴ représente un alkyle en C₁-C₆ linéaire ou un alkyle en C₁-C₆ ramifié, de préférence dans lequel R⁴ représente -CH₂CH₃ ou -CH₂CH(CH₃)₂, ou dans lequel R⁴ représente 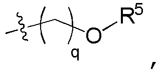 où q vaut 1, 2, 3 ou 4 ; et R⁵ représente un alkyle en C₁-C₆ linéaire ou un alkyle en C₁-C₆ ramifié, de préférence où q vaut 2, de préférence dans lequel R⁵ représente -CH₃, de préférence où q vaut 2 et R⁵ représente -CH₃.

4. Composé pour utilisation selon l'une quelconque des revendications 1 à 3, dans lequel le composé de formule (II) est un composé de formule (II-1) : 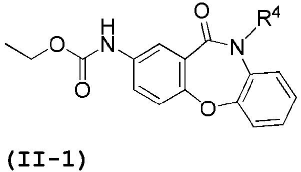 dans lequel :
R⁴ représente un alkyle en C₁-C₆ linéaire ou ramifié ;
ou R⁴ représente : 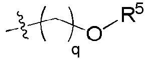
où q vaut 1, 2, 3 ou 4 ; et R⁵ représente un alkyle en C₁-C₆ linéaire ou ramifié.

5. Composé pour utilisation selon l'une quelconque des revendications 1 à 4, dans lequel le composé de formule (II-1) est choisi parmi : 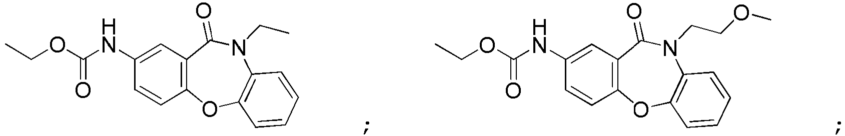 et

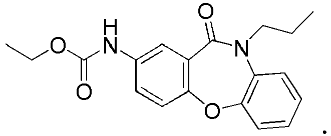

6. Composé pour utilisation selon l'une quelconque des revendications 1 à 5, dans lequel le composé de formule (II) est :

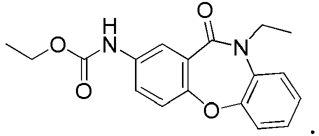

7. Composition pharmaceutique comprenant un composé de formule (II), ou sel pharmaceutiquement acceptable de celui-ci 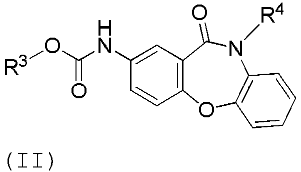 dans laquelle :
R³ représente un alkyle en C₁-C₆ linéaire ou ramifié ; et
R⁴ représente un alkyle en C₁-C₆ linéaire ou ramifié,
ou R⁴ représente 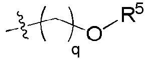
où q vaut 1, 2, 3 ou 4 ; et R⁵ représente un alkyle en C₁-C₆ linéaire ou ramifié,
et un véhicule pharmaceutiquement acceptable.

8. Composition pharmaceutique selon la revendication 7, dans laquelle R³ représente un alkyle en C₁-C₆ linéaire ou un alkyle en C₁-C₆ ramifié, de préférence dans laquelle R³ représente - CH₂CH₃ ou -CH₂CH(CH₃)₂, de préférence dans laquelle R⁴ représente un alkyle en C₁-C₆ linéaire ou un alkyle en C₁-C₆ ramifié.

9. Composition pharmaceutique selon la revendication 7, dans laquelle R⁴ représente -CH₂CH₃ ou -CH₂CH(CH₃)₂, de préférence dans laquelle R⁴ représente 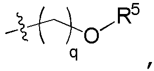 où q vaut 1, 2, 3 ou 4 ; et R⁵ représente un alkyle en C₁-C₆ linéaire ou un alkyle en C₁-C₆ ramifié, de préférence où q vaut 2 ; de préférence dans laquelle R⁵ représente -CH₃, de préférence où q vaut 2 et R⁵ représente - CH₃.

10. Composition pharmaceutique selon l'une quelconque des revendications 7 à 9, dans laquelle le composé de formule (II) est un composé de formule (II-1) : 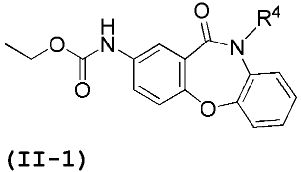 dans laquelle :
R⁴ représente un alkyle en C₁-C₆ linéaire ou ramifié ;
ou R⁴ représente : 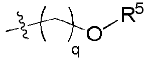
où q vaut 1, 2, 3 ou 4 ; et R⁵ représente un alkyle en C₁-C₆ linéaire ou ramifié.

11. Composition pharmaceutique selon l'une quelconque des revendications 7 à 12, dans laquelle le composé de formule (II) est :

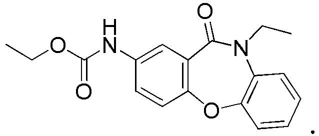

12. Composé de formule (II), ou sel pharmaceutiquement acceptable de celui-ci : 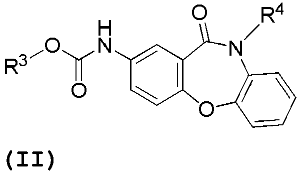 dans lequel :
R³ représente un alkyle en C₁-C₆ linéaire ou ramifié ; et
R⁴ représente un alkyle en C₁-C₆ linéaire ou ramifié,
ou R⁴ représente 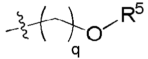
où q vaut 1, 2, 3 ou 4 ; et R⁵ représente un alkyle en C₁-C₆ linéaire ou ramifié,
pour utilisation comme médicament.

13. Composé pour utilisation selon la revendication 12, dans lequel R³ représente -CH₂CH₃ ou -CH₂CH(CH₃)₂.

14. Composé pour utilisation selon la revendication 12, dans lequel R⁴ représente un alkyle en C₁-C₆ linéaire ou un alkyle en C₁-C₆ ramifié, de préférence dans lequel R⁴ représente -CH₂CH₃ ou -CH₂CH(CH₃)₂, ou dans lequel R⁴ représente 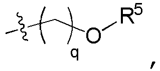 où q vaut 1, 2, 3 ou 4 ; et R⁵ représente un alkyle en C₁-C₆ linéaire ou un alkyle en C₁-C₆ ramifié, de préférence où q vaut 2, de préférence dans lequel R⁵ représente -CH₃, de préférence où q vaut 2 et R⁵ représente -CH₃.

15. Composé pour utilisation selon la revendication 12, dans lequel le composé de formule (II) est un composé de formule (II-1) : 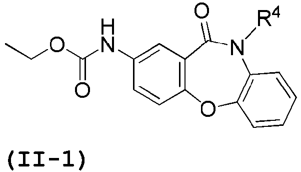 dans lequel :
R⁴ représente un alkyle en C₁-C₆ linéaire ou ramifié ;
ou R⁴ représente : 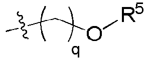
où q vaut 1, 2, 3 ou 4 ; et R⁵ représente un alkyle en C₁-C₆ linéaire ou ramifié.

16. Composé pour utilisation selon la revendication 15, dans lequel le composé de formule (II-1) est un composé de formule (II) :
